# EUROPEAN PATENT APPLICATION

(11) **EP 4 257 657 A1**
(43) Date of publication of application: **11.10.2023**
(21) Application number: 21900408.2
(22) Date of filing: 17.11.2021
(51) Int. Cl.: C09K 11/06, C07D 307/77, H05B 33/12, H01L 51/50

(54) **COMPOUND, ORGANIC ELECTROLUMINESCENT ELEMENT AND ELECTRONIC DEVICE**

(30) Priority: 04.12.2020 JP 2020202018; 04.12.2020 JP 2020202119; 04.12.2020 JP 2020202149; 05.10.2021 JP 2021163838
(71) Applicant: Idemitsu Kosan Co.,Ltd., Tokyo 100-8321 (JP)
(72) Inventor: ITOI, Hiroaki, Sodegaura-shi, Chiba 299-0293 (JP); YAMAKI, Taro, Sodegaura-shi, Chiba 299-0293 (JP); IIDA, Maiko, Sodegaura-shi, Chiba 299-0293 (JP); BAN, Shintaro, Sodegaura-shi, Chiba 299-0293 (JP); MORITA, Takamoto, Sodegaura-shi, Chiba 299-0293 (JP); KUDO, Yu, Sodegaura-shi, Chiba 299-0293 (JP)
(74) Representative: Hoffmann Eitle
(86) International application number: PCT/JP2021/042208
(87) International publication number: WO 2022/118653

(57) **Abstract**

A compound represented by the following formula (1).

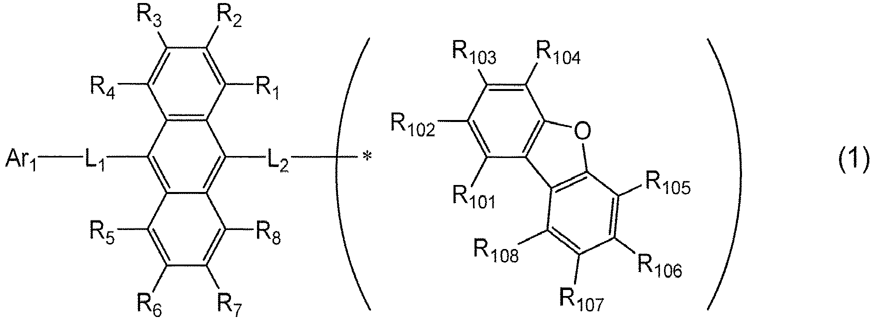

## Description

### Technical Field

The present invention relates to a compound, an organic electroluminescence device and an electronic apparatus.

### Background Art

When voltage is applied to an organic electroluminescence device (hereinafter, also referred to as an organic EL device), holes and electrons are injected into an emitting layer from an anode and a cathode, respectively. Then, thus injected holes and electrons are recombined in the emitting layer, and excitons are formed therein.

Conventional organic EL devices have not yet had sufficient device performance. Although materials used for the organic EL device are gradually improved to enhance the device performance, further performance enhancement is required.

Patent Documents 1 to 3 disclose that a deuterated anthracene host compound having the specific structure is used for an emitting layer in an organic EL device.

### Related Art Documents

### Patent Documents

[Patent Document 1] WO 2020/153650 A1
[Patent Document 2] KR 10-2017-055411
[Patent Document 3] US 2020/0259086 A1

### Summary of the Invention

It is an object of the present invention to provide an organic EL device having long lifetime.

As a result of intensive studies to achieve the above object, the present inventors have found that an organic EL device having long lifetime can be obtained by using a compound having the specific structure, and have completed the present invention.

According to the present invention, the following compound, organic electroluminescence device and electronic apparatus are provided.
1. A compound represented by the following formula (1): 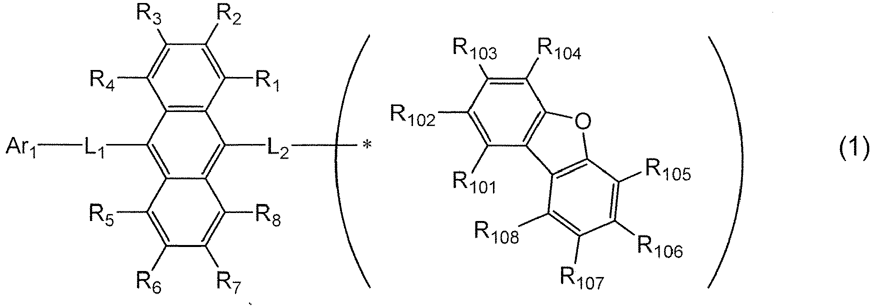 wherein in the formula (1),
   R₁ to R₈ are independently a hydrogen atom, or a substituent R;
   L₁ and L₂ are independently
   a single bond,
   a substituted or unsubstituted phenylene group,
   a substituted or unsubstituted naphthylene group, or
   a divalent group in which these two groups are combined;
   Ar₁ is
   a substituted or unsubstituted phenyl group,
   a substituted or unsubstituted p-biphenyl group,
   a substituted or unsubstituted m-biphenyl group,
   a substituted or unsubstituted o-biphenyl group,
   a substituted or unsubstituted 1-naphthyl group,
   a substituted or unsubstituted 2-naphthyl group, or
   a substituted or unsubstituted phenanthryl group;
   the adjacent two of R₁₀₁ to R₁₀₈ form a substituted or unsubstituted benzene ring by bonding with each other, or do not bond with each other;
   one of, R₁₀₁ to R₁₀₈ which do not form the substituted or unsubstituted benzene ring, and carbon atoms in the substituted or unsubstituted benzene ring in the case where the adjacent two of R₁₀₁ to R₁₀₈ form a substituted or unsubstituted benzene ring by bonding with each other is bonded with L₂ via a single bond;
   R₁₀₁ to R₁₀₈ which do not form the substituted or unsubstituted benzene ring and which are not bonded with L₂ are independently a hydrogen atom, or a substituent R;
   the substituent R is selected from the group consisting of
   a substituted or unsubstituted alkyl group having 1 to 50 carbon atoms,
   a substituted or unsubstituted alkenyl group having 2 to 50 carbon atoms,
   a substituted or unsubstituted alkynyl group having 2 to 50 carbon atoms,
   a substituted or unsubstituted cycloalkyl group having 3 to 50 ring carbon atoms,
   -Si(R₉₀₁)(R₉₀₂)(R₉₀₃),
   -O-(R₉₀₄),
   -S-(R905),
   -N(R₉₀₆)(R₉₀₇)
   (wherein R₉₀₁ to R₉₀₇ are independently
   a hydrogen atom,
   a substituted or unsubstituted alkyl group having 1 to 50 carbon atoms,
   a substituted or unsubstituted cycloalkyl group having 3 to 50 ring carbon atoms,
   a substituted or unsubstituted aryl group having 6 to 50 ring carbon atoms, or
   a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
   a halogen atom, a cyano group, a nitro group,
   a substituted or unsubstituted aryl group having 6 to 50 ring carbon atoms, and
   a substituted or unsubstituted monovalent heterocyclic group having 5 to 50 ring atoms; provided that the compound represented by the formula (1) satisfies all of the following conditions 1 to 3;
   condition 1: R₁ to R₈ which are hydrogen atoms are not a deuterium atom;
   condition 2: one or more of, hydrogen atoms possessed by L₁, L₂ and Ar₁, hydrogen atoms possessed by the substituent in the case where L₁, L₂ and Ar₁ are substituted, hydrogen atoms possessed by R₁ to R₈ which are the substituents R, R₁₀₁ to R₁₀₈ which are hydrogen atoms, hydrogen atoms possessed by R₁₀₁ to R₁₀₈ which are the substituents R, and hydrogen atoms possessed by the substituted or unsubstituted benzene ring in the case where the adjacent two of R₁₀₁ to R₁₀₈ form a substituted or unsubstituted benzene ring by bonding with each other are a deuterium atom; and
   condition 3: when L₁ is a single bond, Ar₁ is not a phenyl group.
2. An organic electroluminescence device comprising
   a cathode;
   an anode; and
   one or more emitting layers arranged between the cathode and the anode,
   wherein at least one layer of the one or more emitting layers comprises
   a compound represented by the following formula (1A), and
   a compound represented by the following formula (41): 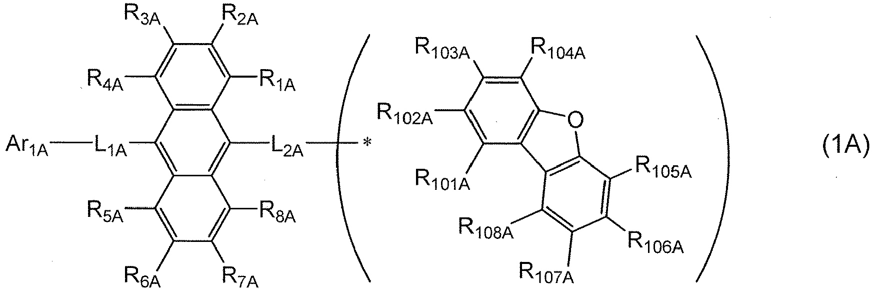 wherein in the formula (1A),
   R_{1A} to R_{8A} are independently a hydrogen atom, or a substituent R;
   L_{1A} and L_{2A} are independently
   a single bond,
   a substituted or unsubstituted arylene group having 6 to 18 ring carbon atoms, or
   a substituted or unsubstituted divalent heterocyclic group having 5 to 18 ring atoms;
   Ar_{1A} is
   a substituted or unsubstituted phenyl group,
   a substituted or unsubstituted p-biphenyl group,
   a substituted or unsubstituted m-biphenyl group,
   a substituted or unsubstituted o-biphenyl group,
   a substituted or unsubstituted 1-naphthyl group,
   a substituted or unsubstituted 2-naphthyl group, or
   a substituted or unsubstituted phenanthryl group;
   one of R_{101A} to R_{104A} is bonded with L_{2A} via a single bond;
   R_{101A} to R_{104A} which are not bonded with L_{2A} are independently
   a hydrogen atom,
   a substituted or unsubstituted aryl group having 6 to 12 ring carbon atoms, or
   a substituted or unsubstituted monovalent heterocyclic group having 5 to 12 ring atoms;
   R_{105A} to R_{108A} are hydrogen atoms;
   the substituent R is selected from the group consisting of
   a substituted or unsubstituted alkyl group having 1 to 50 carbon atoms,
   a substituted or unsubstituted alkenyl group having 2 to 50 carbon atoms,
   a substituted or unsubstituted alkynyl group having 2 to 50 carbon atoms,
   a substituted or unsubstituted cycloalkyl group having 3 to 50 ring carbon atoms,
   -Si(R₉₀₁)(R₉₀₂)(R₉₀₃),
   -O-(R₉₀₄),
   -S-(R905),
   -N(R₉₀₆)(R₉₀₇)
   (wherein R₉₀₁ to R₉₀₇ are independently
   a hydrogen atom,
   a substituted or unsubstituted alkyl group having 1 to 50 carbon atoms,
   a substituted or unsubstituted cycloalkyl group having 3 to 50 ring carbon atoms,
   a substituted or unsubstituted aryl group having 6 to 50 ring carbon atoms, or
   a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
   a halogen atom, a cyano group, a nitro group,
   a substituted or unsubstituted aryl group having 6 to 50 ring carbon atoms, and
   a substituted or unsubstituted monovalent heterocyclic group having 5 to 50 ring atoms; provided that the compound represented by the formula (1A) satisfies the following conditions A1 and A2;
   condition A1: R_{1A} to R_{8A} which are hydrogen atoms are not a deuterium atom;
   condition A2: one or more of, hydrogen atoms possessed by L_{1A}, L_{2A} and Ar_{1A}; hydrogen atoms possessed by the substituent in the case where L_{1A}, L_{2A} and Ar_{1A} are substituted; hydrogen atoms possessed by R_{1A} to R_{8A} which are the substituents R; R_{101A} to R_{108A} which are hydrogen atoms; and hydrogen atoms possessed by R_{101A} to R_{104A} which are a substituted or unsubstituted aryl group having 6 to 12 ring carbon atoms, or a substituted or unsubstituted monovalent heterocyclic group having 5 to 12 ring atoms are a deuterium atom: 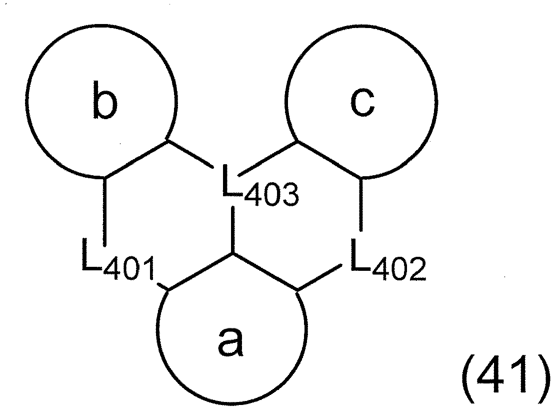 wherein in the formula (41),
   a ring a, a ring b and a ring c are independently
   a substituted or unsubstituted aromatic hydrocarbon ring having 6 to 50 ring carbon atoms, or
   a substituted or unsubstituted heterocycle having 5 to 50 ring atoms;
   L₄₀₁ and L₄₀₂ are independently O, S, Se, NR₄₀₁, C(R₄₀₂)(R₄₀₃), or Si(R₄₀₄)(R₄₀₅);
   (wherein R₄₀₁ to R₄₀₅ form a substituted or unsubstituted, saturated or unsaturated ring by bonding with the ring a, the ring b or the ring c, or do not form the substituted or unsubstituted, saturated or unsaturated ring;
   R₄₀₂ and R₄₀₃ form a substituted or unsubstituted, saturated or unsaturated ring by bonding with each other, or do not form the substituted or unsubstituted, saturated or unsaturated ring;
   R₄₀₄ and R₄₀₅ form a substituted or unsubstituted, saturated or unsaturated ring by bonding with each other, or do not form the substituted or unsubstituted, saturated or unsaturated ring;
   R₄₀₁ to R₄₀₅ which do not form the substituted or unsubstituted, saturated or unsaturated ring are independently
   a substituted or unsubstituted alkyl group having 1 to 50 carbon atoms,
   a substituted or unsubstituted alkenyl group having 2 to 50 carbon atoms,
   a substituted or unsubstituted alkynyl group having 2 to 50 carbon atoms,
   a substituted or unsubstituted cycloalkyl group having 3 to 50 ring carbon atoms,
   a substituted or unsubstituted aryl group having 6 to 50 ring carbon atoms, or a substituted or unsubstituted monovalent heterocyclic group having 5 to 50 ring atoms;
   when two or more of each of R₄₀₁ to R₄₀₅ are present, the two or more of each of R₄₀₁ to R₄₀₅ may be the same as or different from each other; and
   L₄₀₃ is B, P, or P=O.
3. An organic electroluminescence device comprising
   a cathode;
   an anode; and
   one or more emitting layers arranged between the cathode and the anode,
   wherein at least one layer of the one or more emitting layers comprises
   a compound represented by the following formula (1B), and
   a compound represented by the following formula (41): 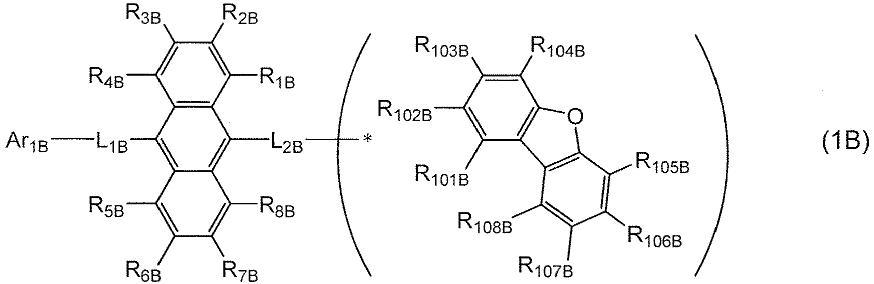 wherein in the formula (1B),
   R_{1B} to R_{8B} are independently a hydrogen atom, or a substituent R;
   L_{1B} and L_{2B} are independently
   a single bond,
   a substituted or unsubstituted arylene group having 6 to 18 ring carbon atoms, or a substituted or unsubstituted divalent heterocyclic group having 5 to 18 ring atoms;
   Ar_{1B} is
   a substituted or unsubstituted phenyl group,
   a substituted or unsubstituted p-biphenyl group,
   a substituted or unsubstituted m-biphenyl group,
   a substituted or unsubstituted o-biphenyl group,
   a substituted or unsubstituted 1-naphthyl group,
   a substituted or unsubstituted 2-naphthyl group, or
   a substituted or unsubstituted phenanthryl group;
   the adjacent two of R_{101B} to R_{108B} form a substituted or unsubstituted benzene ring by bonding with each other;
   one of, R_{101B} to R_{108B} which do not form the substituted or unsubstituted benzene ring, and carbon atoms in the substituted or unsubstituted benzene ring is bonded with L_{2B} via a single bond;
   R_{101B} to R_{108B} which do not form the substituted or unsubstituted benzene ring and which are not bonded with L_{2B} are independently a hydrogen atom, or a substituent R;
   the substituent R is selected from the group consisting of
   a substituted or unsubstituted alkyl group having 1 to 50 carbon atoms,
   a substituted or unsubstituted alkenyl group having 2 to 50 carbon atoms,
   a substituted or unsubstituted alkynyl group having 2 to 50 carbon atoms,
   a substituted or unsubstituted cycloalkyl group having 3 to 50 ring carbon atoms,
   -Si(R₉₀₁)(R₉₀₂)(R₉₀₃),
   -O-(R₉₀₄),
   -S-(R905),
   -N(R₉₀₆)(R₉₀₇)
   (wherein R₉₀₁ to R₉₀₇ are independently
   a hydrogen atom,
   a substituted or unsubstituted alkyl group having 1 to 50 carbon atoms,
   a substituted or unsubstituted cycloalkyl group having 3 to 50 ring carbon atoms,
   a substituted or unsubstituted aryl group having 6 to 50 ring carbon atoms, or
   a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
   a halogen atom, a cyano group, a nitro group,
   a substituted or unsubstituted aryl group having 6 to 50 ring carbon atoms, and
   a substituted or unsubstituted monovalent heterocyclic group having 5 to 50 ring atoms;
   provided that the compound represented by the formula (1B) satisfies the following conditions B1 and B2;
   condition B1: R_{1B} to R_{8B} which are hydrogen atoms are not a deuterium atom;
   condition B2: one or more of, hydrogen atoms possessed by L_{1B}, L_{2B} and Ar_{1B}, hydrogen atoms possessed by the substituent in the case where L_{1B}, L_{2B} and Ar_{1B} are substituted, hydrogen atoms possessed by R_{1B} to R_{8B} which are the substituents R, R_{101B} to R_{108B} which are hydrogen atoms, hydrogen atoms possessed by R_{101B} to R_{108B} which are the substituents R, and hydrogen atoms possessed by the substituted or unsubstituted benzene ring are a deuterium atom: 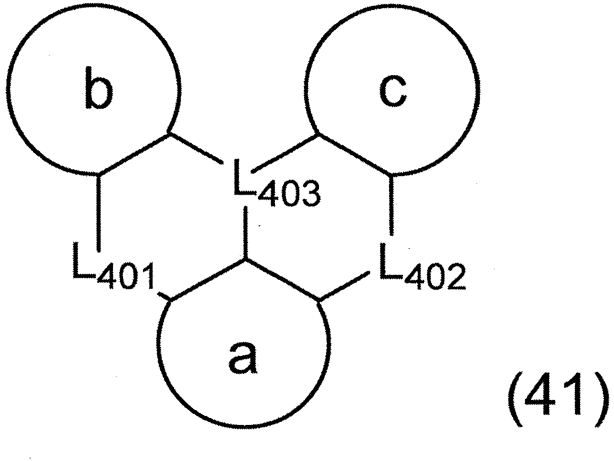 wherein in the formula (41),
   a ring a, a ring b and a ring c are independently
   a substituted or unsubstituted aromatic hydrocarbon ring having 6 to 50 ring carbon atoms, or a substituted or unsubstituted heterocycle having 5 to 50 ring atoms;
   L₄₀₁ and L₄₀₂ are independently O, S, Se, NR₄₀₁, C(R₄₀₂)(R₄₀₃), or Si(R₄₀₄)(R₄₀₅);
   (wherein R₄₀₁ to R₄₀₅ form a substituted or unsubstituted, saturated or unsaturated ring by bonding with the ring a, the ring b or the ring c, or do not form the substituted or unsubstituted, saturated or unsaturated ring;
   R₄₀₂ and R₄₀₃ form a substituted or unsubstituted, saturated or unsaturated ring by bonding with each other, or do not form the substituted or unsubstituted, saturated or unsaturated ring;
   R₄₀₄ and R₄₀₅ form a substituted or unsubstituted, saturated or unsaturated ring by bonding with each other, or do not form the substituted or unsubstituted, saturated or unsaturated ring;
   R₄₀₁ to R₄₀₅ which do not form the substituted or unsubstituted, saturated or unsaturated ring are independently
   a substituted or unsubstituted alkyl group having 1 to 50 carbon atoms,
   a substituted or unsubstituted alkenyl group having 2 to 50 carbon atoms,
   a substituted or unsubstituted alkynyl group having 2 to 50 carbon atoms,
   a substituted or unsubstituted cycloalkyl group having 3 to 50 ring carbon atoms,
   a substituted or unsubstituted aryl group having 6 to 50 ring carbon atoms, or a substituted or unsubstituted monovalent heterocyclic group having 5 to 50 ring atoms;
   when two or more of each of R₄₀₁ to R₄₀₅ are present, the two or more of each of R₄₀₁ to R₄₀₅ may be the same as or different from each other; and
   L₄₀₃ is B, P, or P=O.
4. An electronic apparatus comprising the organic electroluminescence device according to 2 or 3.

According to the present invention, there can be provided an organic EL device having long lifetime.

### Brief Description of the Drawings

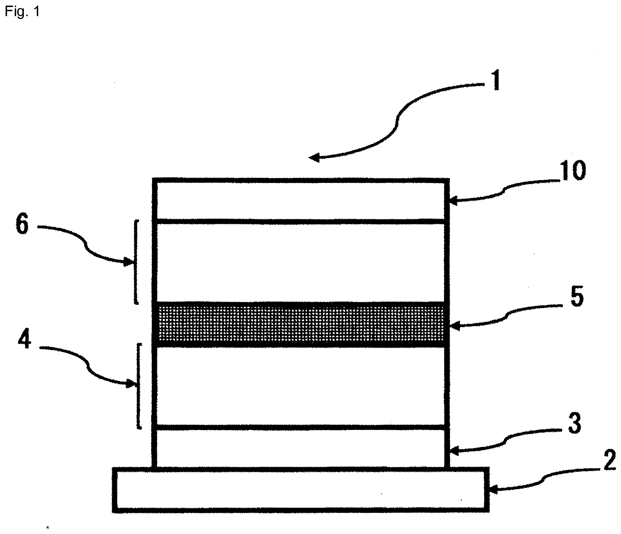

Fig. 1 is a diagram showing a schematic configuration of an organic EL device according to an aspect of the present invention.

### Mode for Carrying out the Invention

### [Definition]

In this specification, a hydrogen atom includes its isotopes different in the number of neutrons, namely, a protium, a deuterium and a tritium.

In this specification, at a bondable position in a chemical formula where a symbol such as "R", or "D" representing a deuterium atom is not indicated, a hydrogen atom, that is, a protium atom, a deuterium atom or a tritium atom is bonded.

In this specification, the number of ring carbon atoms represents the number of carbon atoms forming a subject ring itself among the carbon atoms of a compound having a structure in which atoms are bonded in a ring form (for example, a monocyclic compound, a fused ring compound, a cross-linked compound, a carbocyclic compound, or a heterocyclic compound). When the subject ring is substituted by a substituent, the carbon contained in the substituent is not included in the number of ring carbon atoms. The same shall apply to "the number of ring carbon atoms" described below, unless otherwise specified. For example, a benzene ring has 6 ring carbon atoms, a naphthalene ring includes 10 ring carbon atoms, a pyridine ring includes 5 ring carbon atoms, and a furan ring includes 4 ring carbon atoms. Further, for example, a 9,9-diphenylfluorenyl group includes 13 ring carbon atoms, and a 9,9'-spirobifluorenyl group includes 25 ring carbon atoms.

When a benzene ring is substituted by, for example, an alkyl group as a substituent, the number of carbon atoms of the alkyl group is not included in the number of ring carbon atoms of the benzene ring. Therefore, the number of ring carbon atoms of the benzene ring substituted by the alkyl group is 6. When a naphthalene ring is substituted by, for example, an alkyl group as a substituent, the number of carbon atoms of the alkyl group is not included in the number of ring carbon atoms of the naphthalene ring. Therefore, the number of ring carbon atoms of the naphthalene ring substituted by the alkyl group is 10.

In this specification, the number of ring atoms represents the number of atoms forming a subject ring itself among the atoms of a compound having a structure in which atoms are bonded in a ring form (for example, the structure includes a monocyclic ring, a fused ring and a ring assembly) (for example, a monocyclic compound, a fused ring compound, a cross-linked compound, a carbocyclic compound and a heterocyclic compound). The number of ring atoms does not include atoms which do not form the ring (for example, a hydrogen atom which terminates a bond of the atoms forming the ring), or atoms contained in a substituent when the ring is substituted by the substituent. The same shall apply to "the number of ring atoms" described below, unless otherwise specified. For example, the number of atoms of a pyridine ring is 6, the number of atoms of a quinazoline ring is 10, and the number of a furan ring is 5. For example, hydrogen atoms bonded to a pyridine ring and atoms constituting a substituent substituted on the pyridine ring are not included in the number of ring atoms of the pyridine ring. Therefore, the number of ring atoms of a pyridine ring with which a hydrogen atom or a substituent is bonded is 6. For example, hydrogen atoms and atoms constituting a substituent which are bonded with a quinazoline ring is not included in the number of ring atoms of the quinazoline ring. Therefore, the number of ring atoms of a quinazoline ring with which a hydrogen atom or a substituent is bonded is 10.

In this specification, "XX to YY carbon atoms" in the expression "a substituted or unsubstituted ZZ group including XX to YY carbon atoms" represents the number of carbon atoms in the case where the ZZ group is unsubstituted by a substituent, and does not include the number of carbon atoms of a substituent in the case where the ZZ group is substituted by the substituent. Here, "YY" is larger than "XX", and "XX" means an integer of 1 or more and "YY" means an integer of 2 or more.

In this specification, "XX to YY atoms" in the expression "a substituted or unsubstituted ZZ group including XX to YY atoms" represents the number of atoms in the case where the ZZ group is unsubstituted by a substituent, and does not include the number of atoms of a substituent in the case where the ZZ group is substituted by the substituent. Here, "YY" is larger than "XX", and "XX" means an integer of 1 or more and "YY" means an integer of 2 or more.

In this specification, the unsubstituted ZZ group represents the case where the "substituted or unsubstituted ZZ group" is a "ZZ group unsubstituted by a substituent", and the substituted ZZ group represents the case where the "substituted or unsubstituted ZZ group" is a " ZZ group substituted by a substituent" .

In this specification, a term "unsubstituted" in the case of "a substituted or unsubstituted ZZ group" means that hydrogen atoms in the ZZ group are not substituted by a substituent. Hydrogen atoms in a term "unsubstituted ZZ group" are a protium atom, a deuterium atom, or a tritium atom.

In this specification, a term "substituted" in the case of "a substituted or unsubstituted ZZ group" means that one or more hydrogen atoms in the ZZ group are substituted by a substituent. Similarly, a term "substituted" in the case of "a BB group substituted by an AA group" means that one or more hydrogen atoms in the BB group are substituted by the AA group.

### "Substituent as described in this specification"

Hereinafter, the substituent described in this specification will be explained.

The number of ring carbon atoms of the "unsubstituted aryl group" described in this specification is 6 to 50, preferably 6 to 30, and more preferably 6 to 18, unless otherwise specified.

The number of ring atoms of the "unsubstituted heterocyclic group" described in this specification is 5 to 50, preferably 5 to 30, and more preferably 5 to 18, unless otherwise specified.

The number of carbon atoms of the "unsubstituted alkyl group" described in this specification is 1 to 50, preferably 1 to 20, and more preferably 1 to 6, unless otherwise specified.

The number of carbon atoms of the "unsubstituted alkenyl group" described in this specification is 2 to 50, preferably 2 to 20, and more preferably 2 to 6, unless otherwise specified.

The number of carbon atoms of the "unsubstituted alkynyl group" described in this specification is 2 to 50, preferably 2 to 20, and more preferably 2 to 6, unless otherwise specified.

The number of ring carbon atoms of the "unsubstituted cycloalkyl group" described in this specification is 3 to 50, preferably 3 to 20, and more preferably 3 to 6, unless otherwise specified.

The number of ring carbon atoms of the "unsubstituted arylene group" described in this specification is 6 to 50, preferably 6 to 30, and more preferably 6 to 18, unless otherwise specified.

The number of ring atoms of the "unsubstituted divalent heterocyclic group" described in this specification is 5 to 50, preferably 5 to 30, and more preferably 5 to 18, unless otherwise specified.

The number of carbon atoms of the "unsubstituted alkylene group" described in this specification is 1 to 50, preferably 1 to 20, and more preferably 1 to 6, unless otherwise specified.

### · "Substituted or unsubstituted aryl group"

Specific examples of the "substituted or unsubstituted aryl group" described in this specification (specific example group G1) include the following unsubstituted aryl groups (specific example group G1A), substituted aryl groups (specific example group G1B), and the like. (Here, the unsubstituted aryl group refers to the case where the "substituted or unsubstituted aryl group" is an " aryl group unsubstituted by a substituent" , and the substituted aryl group refers to the case where the "substituted or unsubstituted aryl group" is an " aryl group substituted by a substituent" .). In this specification, in the case where simply referred as an "aryl group", it includes both a "unsubstituted aryl group" and a "substituted aryl group."

The "substituted aryl group" means a group in which one or more hydrogen atoms of the "unsubstituted aryl group" are substituted by a substituent. Specific examples of the "substituted aryl group" include, for example, groups in which one or more hydrogen atoms of the "unsubstituted aryl group" of the following specific example group G1A are substituted by a substituent, the substituted aryl groups of the following specific example group G1B, and the like. It should be noted that the examples of the "unsubstituted aryl group" and the examples of the "substituted aryl group" enumerated in this specification are mere examples, and the "substituted aryl group" described in this specification also includes a group in which a hydrogen atom bonded with a carbon atom of the aryl group itself in the "substituted aryl group" of the following specific group G1B is further substituted by a substituent, and a group in which a hydrogen atom of a substituent in the "substituted aryl group" of the following specific group G1B is further substituted by a substituent.
· Unsubstituted aryl group (specific example group G1A):
   a phenyl group,
   a p-biphenyl group,
   a m-biphenyl group,
   an o-biphenyl group,
   a p-terphenyl-4-yl group,
   a p-terphenyl-3-yl group,
   a p-terphenyl-2-yl group,
   a m-terphenyl-4-yl group,
   a m-terphenyl-3-yl group,
   a m-terphenyl-2-yl group,
   an o-terphenyl-4-yl group,
   an o-terphenyl-3-yl group,
   an o-terphenyl-2-yl group,
   a 1-naphthyl group,
   a 2-naphthyl group,
   an anthryl group,
   a benzanthryl group,
   a phenanthryl group,
   a benzophenanthryl group,
   a phenalenyl group,
   a pyrenyl group,
   a chrysenyl group,
   a benzochrysenyl group,
   a triphenylenyl group,
   a benzotriphenylenyl group,
   a tetracenyl group,
   a pentacenyl group,
   a fluorenyl group,
   a 9,9'-spirobifluorenyl group,
   a benzofluorenyl group,
   a dibenzofluorenyl group,
   a fluoranthenyl group,
   a benzofluoranthenyl group,
   a perylenyl group, and
   a monovalent aryl group derived by removing one hydrogen atom from the ring structures represented by any of the following general formulas (TEMP-1) to (TEMP-15). 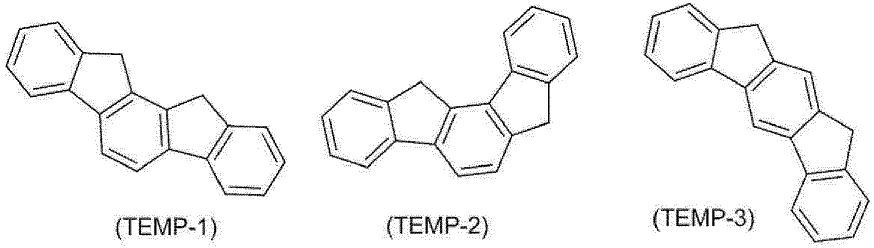 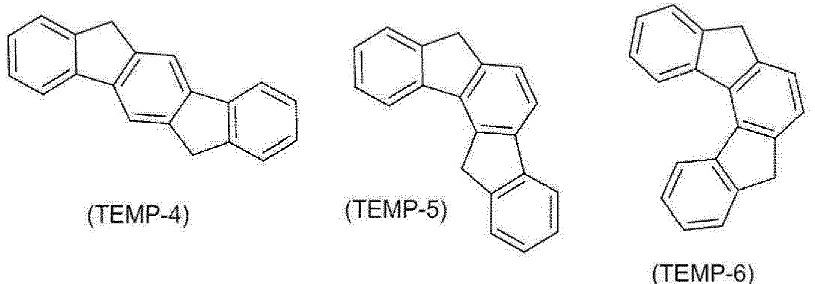 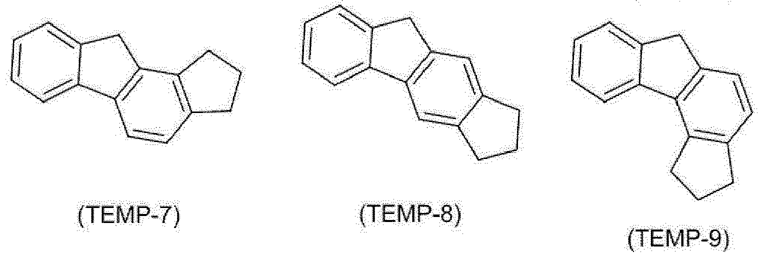 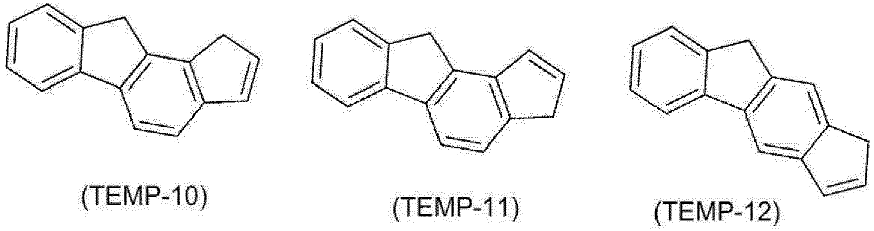 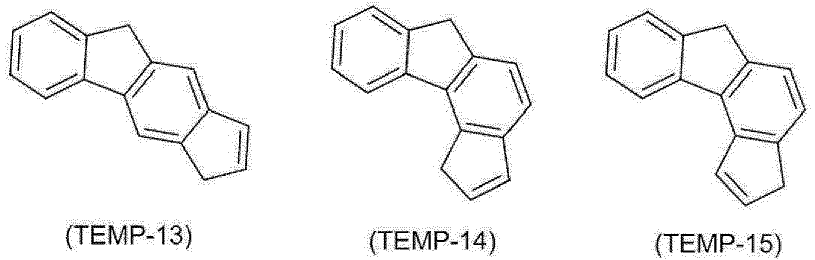
· Substituted aryl group (specific example group G1B):
   an o-tolyl group,
   a m-tolyl group,
   a p-tolyl group,
   a p-xylyl group,
   a m-xylyl group,
   an o-xylyl group,
   a p-isopropylphenyl group,
   a m-isopropylphenyl group,
   an o-isopropylphenyl group,
   a p-t-butylphenyl group,
   a m-t-butylphenyl group,
   an o-t-butylphenyl group,
   a 3,4,5-trimethylphenyl group,
   a 9,9-dimethylfluorenyl group,
   a 9,9-diphenylfluorenyl group,
   a 9,9-bis(4-methylphenyl)fluorenyl group,
   a 9,9-bis(4-isopropylphenyl)fluorenyl group,
   a 9,9-bis(4-t-butylphenyl)fluorenyl group,
   a cyanophenyl group,
   a triphenylsilylphenyl group,
   a trimethylsilylphenyl group,
   a phenylnaphthyl group,
   a naphthylphenyl group, and
   a group in which one or more hydrogen atoms of a monovalent group derived from the ring structures represented by any of the general formulas (TEMP-1) to (TEMP-15) are substituted by a substituent.

### · "Substituted or unsubstituted heterocyclic group"

The "heterocyclic group" described in this specification is a ring group having at least one hetero atom in the ring atom. Specific examples of the hetero atom include a nitrogen atom, an oxygen atom, a sulfur atom, a silicon atom, a phosphorus atom, and a boron atom.

The "heterocyclic group" in this specification is a monocyclic group or a fused ring group.

The "heterocyclic group" in this specification is an aromatic heterocyclic group or a non-aromatic heterocyclic group.

Specific examples of the "substituted or unsubstituted heterocyclic group" (specific example group G2) described in this specification include the following unsubstituted heterocyclic group (specific example group G2A), the following substituted heterocyclic group (specific example group G2B), and the like. (Here, the unsubstituted heterocyclic group refers to the case where the "substituted or unsubstituted heterocyclic group" is a " heterocyclic group unsubstituted by a substituent", and the substituted heterocyclic group refers to the case where the "substituted or unsubstituted heterocyclic group" is a " heterocyclic group substituted by a substituent".). In this specification, in the case where simply referred as a "heterocyclic group", it includes both the "unsubstituted heterocyclic group" and the "substituted heterocyclic group."

The "substituted heterocyclic group" means a group in which one or more hydrogen atom of the "unsubstituted heterocyclic group" are substituted by a substituent. Specific examples of the "substituted heterocyclic group" include a group in which a hydrogen atom of "unsubstituted heterocyclic group" of the following specific example group G2A is substituted by a substituent, the substituted heterocyclic groups of the following specific example group G2B, and the like. It should be noted that the examples of the "unsubstituted heterocyclic group" and the examples of the "substituted heterocyclic group" enumerated in this specification are mere examples, and the "substituted heterocyclic group" described in this specification includes groups in which hydrogen atom bonded with a ring atom of the heterocyclic group itself in the "substituted heterocyclic group" of the specific example group G2B is further substituted by a substituent, and a group in which hydrogen atom of a substituent in the "substituted heterocyclic group" of the specific example group G2B is further substituted by a substituent.

Specific example group G2A includes, for example, the following unsubstituted heterocyclic group containing a nitrogen atom (specific example group G2A1), the following unsubstituted heterocyclic group containing an oxygen atom (specific example group G2A2), the following unsubstituted heterocyclic group containing a sulfur atom (specific example group G2A3), and the monovalent heterocyclic group derived by removing one hydrogen atom from the ring structures represented by any of the following general formulas (TEMP-16) to (TEMP-33) (specific example group G2A4).

Specific example group G2B includes, for example, the following substituted heterocyclic group containing a nitrogen atom (specific example group G2B1), the following substituted heterocyclic group containing an oxygen atom (specific example group G2B2), the following substituted heterocyclic group containing a sulfur atom (specific example group G2B3), and the following group in which one or more hydrogen atoms of the monovalent heterocyclic group derived from the ring structures represented by any of the following general formulas (TEMP-16) to (TEMP-33) are substituted by a substituent (specific example group G2B4).
· Unsubstituted heterocyclic group containing a nitrogen atom (specific example group G2A1):
   a pyrrolyl group,
   an imidazolyl group,
   a pyrazolyl group,
   a triazolyl group,
   a tetrazolyl group,
   an oxazolyl group,
   an isoxazolyl group,
   an oxadiazolyl group,
   a thiazolyl group,
   an isothiazolyl group,
   a thiadiazolyl group,
   a pyridyl group,
   a pyridazinyl group,
   a pyrimidinyl group,
   a pyrazinyl group,
   a triazinyl group,
   an indolyl group,
   an isoindolyl group,
   an indolizinyl group,
   a quinolizinyl group,
   a quinolyl group,
   an isoquinolyl group,
   a cinnolyl group,
   a phthalazinyl group,
   a quinazolinyl group,
   a quinoxalinyl group,
   a benzimidazolyl group,
   an indazolyl group,
   a phenanthrolinyl group,
   a phenanthridinyl group,
   an acridinyl group,
   a phenazinyl group,
   a carbazolyl group,
   a benzocarbazolyl group,
   a morpholino group,
   a phenoxazinyl group,
   a phenothiazinyl group,
   an azacarbazolyl group, and
   a diazacarbazolyl group.
· Unsubstituted heterocyclic group containing an oxygen atom (specific example group G2A2):
   a furyl group,
   an oxazolyl group,
   an isoxazolyl group,
   an oxadiazolyl group,
   a xanthenyl group,
   a benzofuranyl group,
   an isobenzofuranyl group,
   a dibenzofuranyl group,
   a naphthobenzofuranyl group,
   a benzoxazolyl group,
   a benzisoxazolyl group,
   a phenoxazinyl group,
   a morpholino group,
   a dinaphthofuranyl group,
   an azadibenzofuranyl group,
   a diazadibenzofuranyl group,
   an azanaphthobenzofuranyl group, and
   a diazanaphthobenzofuranyl group.
· Unsubstituted heterocyclic group containing a sulfur atom (specific example group G2A3):
   a thienyl group,
   a thiazolyl group,
   an isothiazolyl group,
   a thiadiazolyl group,
   a benzothiophenyl group (benzothienyl group),
   an isobenzothiophenyl group (isobenzothienyl group),
   a dibenzothiophenyl group (dibenzothienyl group),
   a naphthobenzothiophenyl group (naphthobenzothienyl group),
   a benzothiazolyl group,
   a benzisothiazolyl group,
   a phenothiazinyl group,
   a dinaphthothiophenyl group (dinaphthothienyl group),
   an azadibenzothiophenyl group (azadibenzothienyl group),
   a diazadibenzothiophenyl group (diazadibenzothienyl group),
   an azanaphthobenzothiophenyl group (azanaphthobenzothienyl group), and
   a diazanaphthobenzothiophenyl group (diazanaphthobenzothienyl group).
· Monovalent heterocyclic group derived by removing one hydrogen atom from the ring structures represented by any of the following general formulas (TEMP-16) to (TEMP-33) (specific example group G2A4):

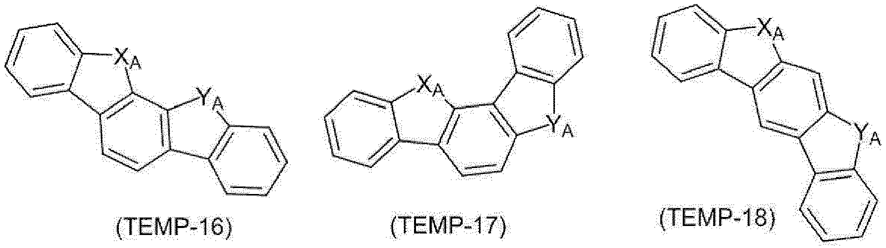

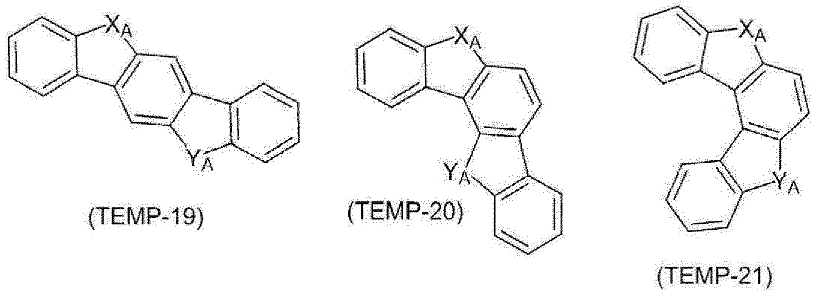

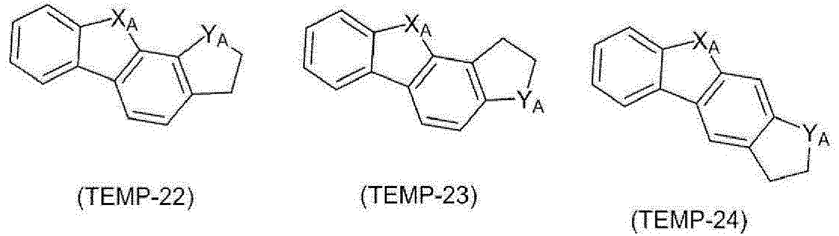

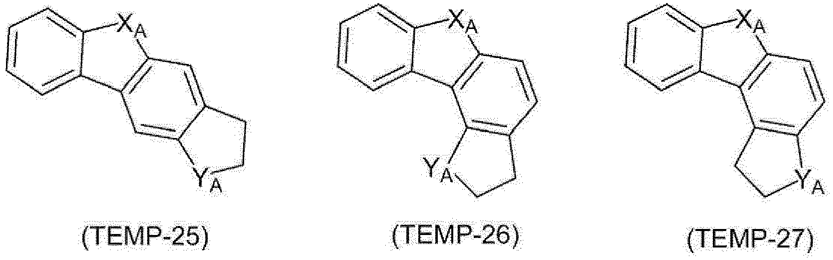

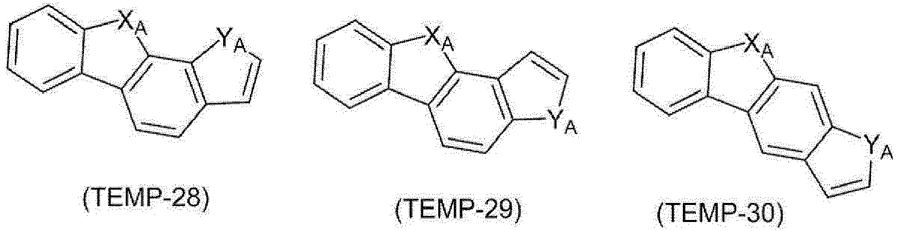

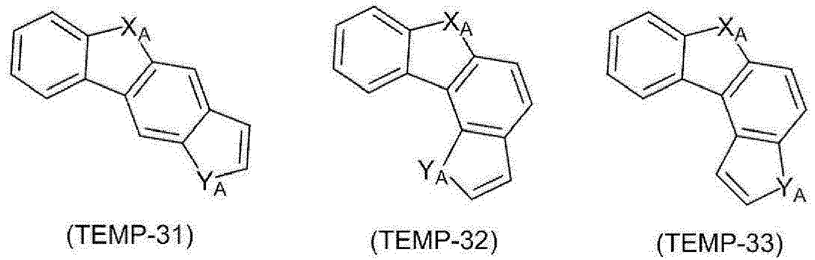

In the general formulas (TEMP-16) to (TEMP-33), X_{A} and Y_{A} are independently an oxygen atom, a sulfur atom, NH, or CH₂. Provided that at least one of X_{A} and Y_{A} is an oxygen atom, a sulfur atom, or NH.

In the general formulas (TEMP-16) to (TEMP-33), when at least one of X_{A} and Y_{A} is NH or CH₂, the monovalent heterocyclic group derived from the ring structures represented by any of the general formulas (TEMP-16) to (TEMP-33) includes a monovalent group derived by removing one hydrogen atom from these NH or CH₂.
· Substituted heterocyclic group containing a nitrogen atom (specific example group G2B1):
   a (9-phenyl)carbazolyl group,
   a (9-biphenylyl)carbazolyl group,
   a (9-phenyl)phenylcarbazolyl group,
   a (9-naphthyl)carbazolyl group,
   a diphenylcarbazol-9-yl group,
   a phenylcarbazol-9-yl group,
   a methylbenzimidazolyl group,
   an ethylbenzimidazolyl group,
   a phenyltriazinyl group,
   a biphenylyltriazinyl group,
   a diphenyltriazinyl group,
   a phenylquinazolinyl group, and
   a biphenylylquinazolinyl group.
· Substituted heterocyclic group containing an oxygen atom (specific example group G2B2):
   a phenyldibenzofuranyl group,
   a methyldibenzofuranyl group,
   a t-butyldibenzofuranyl group, and
   a monovalent residue of spiro[9H-xanthene-9,9'-[9H]fluorene].
· Substituted heterocyclic group containing a sulfur atom (specific example group G2B3):
   a phenyldibenzothiophenyl group,
   a methyldibenzothiophenyl group,
   a t-butyldibenzothiophenyl group, and
   a monovalent residue of spiro[9H-thioxanthene-9,9'-[9H]fluorene].
· Group in which one or more hydrogen atoms of the monovalent heterocyclic group derived from the ring structures represented by any of the following general formulas (TEMP-16) to (TEMP-33) are substituted by a substituent (specific example group G2B4):

The "one or more hydrogen atoms of the monovalent heterocyclic group" means one or more hydrogen atoms selected from hydrogen atoms bonded with ring carbon atoms of the monovalent heterocyclic group, a hydrogen atom bonded with a nitrogen atom when at least one of X_{A} and Y_{A} is NH, and hydrogen atoms of a methylene group when one of X_{A} and Y_{A} is CH₂.

### · "Substituted or unsubstituted alkyl group"

Specific examples of the "substituted or unsubstituted alkyl group" (specific example group G3) described in this specification include the following unsubstituted alkyl groups (specific example group G3A) and the following substituted alkyl groups (specific example group G3B). (Here, the unsubstituted alkyl group refers to the case where the "substituted or unsubstituted alkyl group" is an " alkyl group unsubstituted by a substituent", and the substituted alkyl group refers to the case where the "substituted or unsubstituted alkyl group" is an " alkyl group substituted by a substituent".). In this specification, in the case where simply referred as an "alkyl group" includes both the "unsubstituted alkyl group" and the "substituted alkyl group."

The "substituted alkyl group" means a group in which one or more hydrogen atoms in the "unsubstituted alkyl group" are substituted by a substituent. Specific examples of the "substituted alkyl group" include groups in which one or more hydrogen atoms in the following "unsubstituted alkyl group" (specific example group G3A) are substituted by a substituent, the following substituted alkyl group (specific example group G3B), and the like. In this specification, the alkyl group in the "unsubstituted alkyl group" means a linear alkyl group. Thus, the "unsubstituted alkyl group" includes a straight-chain "unsubstituted alkyl group" and a branched-chain "unsubstituted alkyl group". It should be noted that the examples of the "unsubstituted alkyl group" and the examples of the "substituted alkyl group" enumerated in this specification are mere examples, and the "substituted alkyl group" described in this specification includes a group in which hydrogen atom of the alkyl group itself in the "substituted alkyl group" of the specific example group G3B is further substituted by a substituent, and a group in which hydrogen atom of a substituent in the "substituted alkyl group" of the specific example group G3B is further substituted by a substituent.
· Unsubstituted alkyl group (specific example group G3A):
   a methyl group,
   an ethyl group,
   a n-propyl group,
   an isopropyl group,
   a n-butyl group,
   an isobutyl group,
   a s-butyl group, and
   a t-butyl group.
· Substituted alkyl group (specific example group G3B):
   a heptafluoropropyl group (including isomers),
   a pentafluoroethyl group,
   a 2,2,2-trifluoroethyl group, and
   a trifluoromethyl group.

### · "Substituted or unsubstituted alkenyl group"

Specific examples of the "substituted or unsubstituted alkenyl group" described in this specification (specific example group G4) include the following unsubstituted alkenyl group (specific example group G4A), the following substituted alkenyl group (specific example group G4B), and the like. (Here, the unsubstituted alkenyl group refers to the case where the "substituted or unsubstituted alkenyl group" is a " alkenyl group unsubstituted by a substituent", and the "substituted alkenyl group" refers to the case where the "substituted or unsubstituted alkenyl group" is a " alkenyl group substituted by a substituent."). In this specification, in the case where simply referred as an "alkenyl group" includes both the "unsubstituted alkenyl group" and the "substituted alkenyl group."

The "substituted alkenyl group" means a group in which one or more hydrogen atoms in the "unsubstituted alkenyl group" are substituted by a substituent. Specific examples of the "substituted alkenyl group" include a group in which the following "unsubstituted alkenyl group" (specific example group G4A) has a substituent, the following substituted alkenyl group (specific example group G4B), and the like. It should be noted that the examples of the "unsubstituted alkenyl group" and the examples of the "substituted alkenyl group" enumerated in this specification are mere examples, and the "substituted alkenyl group" described in this specification includes a group in which a hydrogen atom of the alkenyl group itself in the "substituted alkenyl group" of the specific example group G4B is further substituted by a substituent, and a group in which a hydrogen atom of a substituent in the "substituted alkenyl group" of the specific example group G4B is further substituted by a substituent.
· Unsubstituted alkenyl group (specific example group G4A):
   a vinyl group,
   an allyl group,
   a 1-butenyl group,
   a 2-butenyl group, and
   a 3-butenyl group.
· Substituted alkenyl group (specific example group G4B):
   a 1,3-butanedienyl group,
   a 1-methylvinyl group,
   a 1-methylallyl group,
   a 1,1-dimethylallyl group,
   a 2-methylally group, and
   a 1,2-dimethylallyl group.

### · "Substituted or unsubstituted alkynyl group"

Specific examples of the "substituted or unsubstituted alkynyl group" described in this specification (specific example group G5) include the following unsubstituted alkynyl group (specific example group G5A) and the like. (Here, the unsubstituted alkynyl group refers to the case where the "substituted or unsubstituted alkynyl group" is an " alkynyl group unsubstituted by a substituent".). In this specification, in the case where simply referred as an "alkynyl group" includes both the "unsubstituted alkynyl group" and the "substituted alkynyl group."

The "substituted alkynyl group" means a group in which one or more hydrogen atoms in the "unsubstituted alkynyl group" are substituted by a substituent. Specific examples of the "substituted alkynyl group" include a group in which one or more hydrogen atoms in the following "unsubstituted alkynyl group" (specific example group G5A) are substituted by a substituent, and the like.

· Unsubstituted alkynyl group (specific example group G5A):
an ethynyl group.

### · "Substituted or unsubstituted cycloalkyl group"

Specific examples of the "substituted or unsubstituted cycloalkyl group" described in this specification (specific example group G6) include the following unsubstituted cycloalkyl group (specific example group G6A), the following substituted cycloalkyl group (specific example group G6B), and the like. (Here, the unsubstituted cycloalkyl group refers to the case where the "substituted or unsubstituted cycloalkyl group" is a " cycloalkyl group unsubstituted by a substituent", and the substituted cycloalkyl group refers to the case where the "substituted or unsubstituted cycloalkyl group" is a " cycloalkyl group substituted by a substituent".). In this specification, in the case where simply referred as a "cycloalkyl group" includes both the "unsubstituted cycloalkyl group" and the "substituted cycloalkyl group."

The "substituted cycloalkyl group" means a group in which one or more hydrogen atoms in the "unsubstituted cycloalkyl group" are substituted by a substituent. Specific examples of the "substituted cycloalkyl group" include a group in which one or more hydrogen atoms in the following "unsubstituted cycloalkyl group" (specific example group G6A) are substituted by a substituent, and examples of the following substituted cycloalkyl group (specific example group G6B), and the like. It should be noted that the examples of the "unsubstituted cycloalkyl group" and the examples of the "substituted cycloalkyl group" enumerated in this specification are mere examples, and the "substituted cycloalkyl group" in this specification includes a group in which one or more hydrogen atoms bonded with the carbon atom of the cycloalkyl group itself in the "substituted cycloalkyl group " of the specific example group G6B are substituted by a substituent, and a group in which a hydrogen atom of a substituent in the "substituted cycloalkyl group" of specific example group G6B is further substituted by a substituent.
· Unsubstituted cycloalkyl group (specific example group G6A):
   a cyclopropyl group,
   a cyclobutyl group,
   a cyclopentyl group,
   a cyclohexyl group,
   a 1-adamantyl group,
   a 2-adamantyl group,
   a 1-norbornyl group, and
   a 2-norbornyl group.
· Substituted cycloalkyl group (specific example group G6B):
   a 4-methylcyclohexyl group.

### · "Group represented by -Si (R₉₀₁)(R₉₀₂)(R₉₀₃)"

Specific examples of the group represented by -Si(R₉₀₁)(R₉₀₂)(R₉₀₃) described in this specification (specific example group G7) include:
- Si(G1)(G1)(G1),
- Si(G1)(G2)(G2),
- Si(G1)(G1)(G2),
- Si(G2)(G2)(G2),
- Si(G3)(G3)(G3), and
- Si(G6)(G6)(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

Plural G1's in -Si(G1)(G1)(G1) are the same or different.

Plural G2's in -Si(G1)(G2)(G2) are the same or different.

Plural G1's in -Si(G1)(G1)(G2) are the same or different.

Plural G2's in -Si(G2)(G2)(G2) are be the same or different.

Plural G3's in -Si(G3)(G3)(G3) are the same or different.

Plural G6's in -Si(G6)(G6)(G6) are be the same or different.

### · "Group represented by -O-(R₉₀₄)"

Specific examples of the group represented by -O-(R₉₀₄) in this specification (specific example group G8) include:
- O(G1),
- O(G2),
- O(G3), and
- O(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

### · "Group represented by -S-(R₉₀₅)"

Specific examples of the group represented by -S-(R₉₀₅) in this specification (specific example group G9) include:
- S(G1),
- S(G2),
- S(G3), and
- S(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

### · "Group represented by -N(R₉₀₆)(R₉₀₇)"

Specific examples of the group represented by -N(R₉₀₆)(R₉₀₇) in this specification (specific example group G10) include:
- N(G1)(G1),
- N(G2)(G2),
- N(G1)(G2),
- N(G3)(G3), and
- N(G6)(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

Plural G1's in -N(G1)(G1) are the same or different.

Plural G2's in -N(G2)(G2) are the same or different.

Plural G3's in -N(G3)(G3) are the same or different.

Plural G6's in -N(G6)(G6) are the same or different.

### · "Halogen atom"

Specific examples of the "halogen atom" described in this specification (specific example group G11) include a fluorine atom, a chlorine atom, a bromine atom, an iodine atom, and the like.

### · "Substituted or unsubstituted fluoroalkyl group"

The "substituted or unsubstituted fluoroalkyl group" described in this specification is a group in which at least one hydrogen atom bonded with a carbon atom constituting the alkyl group in the "substituted or unsubstituted alkyl group" is substituted by a fluorine atom, and includes a group in which all hydrogen atoms bonded with a carbon atom constituting the alkyl group in the "substituted or unsubstituted alkyl group" are substituted by a fluorine atom (a perfluoro group). The number of carbon atoms of the "unsubstituted fluoroalkyl group" is 1 to 50, preferably 1 to 30, more preferably 1 to 18, unless otherwise specified in this specification. The "substituted fluoroalkyl group" means a group in which one or more hydrogen atoms of the "fluoroalkyl group" are substituted by a substituent. The "substituted fluoroalkyl group" described in this specification also includes a group in which one or more hydrogen atoms bonded with a carbon atom of the alkyl chains in the "substituted fluoroalkyl group" are further substituted by a substituent, and a group in which one or more hydrogen atom of a substituent in the "substituted fluoroalkyl group" are further substituted by a substituent. Specific examples of the "unsubstituted fluoroalkyl group" include a group in which one or more hydrogen atoms in the "alkyl group" (specific group G3) are substituted by a fluorine atom, and the like.

### · "Substituted or unsubstituted haloalkyl group"

The "substituted or unsubstituted haloalkyl group" described in this specification is a group in which at least one hydrogen atom bonded with a carbon atom constituting the alkyl group in the "substituted or unsubstituted alkyl group" is substituted by a halogen atom, and also includes a group in which all hydrogen atoms bonded with a carbon atom constituting the alkyl group in the "substituted or unsubstituted alkyl group" are substituted by a halogen atom. The number of carbon atoms of the "unsubstituted haloalkyl group" is 1 to 50, preferably 1 to 30, more preferably 1 to 18, unless otherwise specified in this specification. The "substituted haloalkyl group" means a group in which one or more hydrogen atoms of the "haloalkyl group" are substituted by a substituent. The "substituted haloalkyl group" described in this specification also includes a group in which one or more hydrogen atoms bonded with a carbon atom of the alkyl chain in the "substituted haloalkyl group" are further substituted by a substituent, and a group in which one or more hydrogen atoms of a substituent in the "substituted haloalkyl group" are further substituted by a substituent. Specific examples of the "unsubstituted haloalkyl group" include a group in which one or more hydrogen atoms in the "alkyl group" (specific example group G3) are substituted by a halogen atom, and the like. A haloalkyl group is sometimes referred to as an alkyl halide group.

### · "Substituted or unsubstituted alkoxy group"

Specific examples of the "substituted or unsubstituted alkoxy group" described in this specification include a group represented by -O(G3), wherein G3 is the "substituted or unsubstituted alkyl group" described in the specific example group G3. The number of carbon atoms of the "unsubstituted alkoxy group" is 1 to 50, preferably 1 to 30, more preferably 1 to 18, unless otherwise specified in this specification.

### · "Substituted or unsubstituted alkylthio group"

Specific examples of the "substituted or unsubstituted alkylthio group" described in this specification include a group represented by -S(G3), wherein G3 is the "substituted or unsubstituted alkyl group" described in the specific example group G3. The number of carbon atoms of the "unsubstituted alkylthio group" is 1 to 50, preferably 1 to 30, more preferably 1 to 18, unless otherwise specified in this specification.

### · "Substituted or unsubstituted aryloxy group"

Specific examples of the "substituted or unsubstituted aryloxy group" described in this specification include a group represented by -O(G1), wherein G1 is the "substituted or unsubstituted aryl group" described in the specific example group G1. The number of ring carbon atoms of the "unsubstituted aryloxy group" is 6 to 50, preferably 6 to 30, more preferably 6 to 18, unless otherwise specified in this specification.

### · "Substituted or unsubstituted arylthio group"

Specific examples of the "substituted or unsubstituted arylthio group" described in this specification include a group represented by -S(G1), wherein G1 is a "substituted or unsubstituted aryl group" described in the specific example group G1. The number of ring carbon atoms of the "unsubstituted arylthio group" is 6 to 50, preferably 6 to 30, more preferably 6 to 18, unless otherwise specified in this specification.

### · "Substituted or unsubstituted trialkylsilyl group"

Specific examples of the "trialkylsilyl group" described in this specification include a group represented by -Si(G3)(G3)(G3), where G3 is the "substituted or unsubstituted alkyl group" described in the specific example group G3. Plural G3's in -Si(G3)(G3)(G3) are the same or different. The number of carbon atoms in each alkyl group of the "trialkylsilyl group" is 1 to 50, preferably 1 to 20, more preferably 1 to 6, unless otherwise specified in this specification.

### · "Substituted or unsubstituted aralkyl group"

Specific examples of the "substituted or unsubstituted aralkyl group" described in this specification is a group represented by -(G3)-(G1), wherein G3 is the "substituted or unsubstituted alkyl group" described in the specific example group G3, and G1 is the "substituted or unsubstituted aryl group" described in the specific example group G1. Therefore, the "aralkyl group" is a group in which a hydrogen atom of the "alkyl group" is substituted by an "aryl group" as a substituent, and is one form of the "substituted alkyl group." The "unsubstituted aralkyl group" is the "unsubstituted alkyl group" substituted by the "unsubstituted aryl group", and the number of carbon atoms of the "unsubstituted aralkyl group" is 7 to 50, preferably 7 to 30, more preferably 7 to 18, unless otherwise specified in this specification.

Specific examples of the "substituted or unsubstituted aralkyl group" include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, a 2-β-naphthylisopropyl group, and the like.

Unless otherwise specified in this specification, examples of the substituted or unsubstituted aryl group described in this specification preferably include a phenyl group, a p-biphenyl group, a m-biphenyl group, an o-biphenyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, 9,9-dimethylfluorenyl group, 9,9-diphenylfluorenyl group, and the like.

Unless otherwise specified in this specification, examples of the substituted or unsubstituted heterocyclic groups described in this specification preferably include a pyridyl group, a pyrimidinyl group, a triazinyl group, a quinolyl group, an isoquinolyl group, a quinazolinyl group, a benzimidazolyl group, a phenanthrolinyl group, a carbazolyl group (a 1-carbazolyl group, a 2-carbazolyl group, a 3-carbazolyl group, a 4-carbazolyl group, or a 9-carbazolyl group), a benzocarbazolyl group, an azacarbazolyl group, a diazacarbazolyl group, a dibenzofuranyl group, a naphthobenzofuranyl group, an azadibenzofuranyl group, a diazadibenzofuranyl group, a dibenzothiophenyl group, a naphthobenzothiophenyl group, an azadibenzothiophenyl group, a diazadibenzothiophenyl group, a (9-phenyl)carbazolyl group (a (9-phenyl)carbazol-1-yl group, a (9-phenyl)carbazol-2-yl group, a (9-phenyl)carbazol-3-yl group, or a (9-phenyl)carbazol-4-yl group), a (9-biphenylyl)carbazolyl group, a (9-phenyl)phenylcarbazolyl group, a diphenylcarbazol-9-yl group, a phenylcarbazol-9-yl group, a phenyltriazinyl group, a biphenylyltriazinyl group, a diphenyltriazinyl group, a phenyldibenzofuranyl group, a phenyldibenzothiophenyl group, and the like.

In this specification, the carbazolyl group is specifically any of the following groups, unless otherwise specified in this specification.

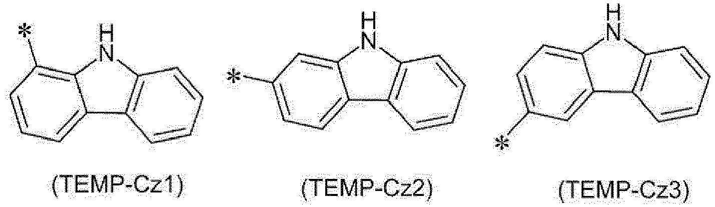

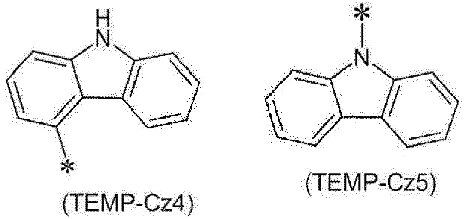

In this specification, the (9-phenyl)carbazolyl group is specifically any of the following groups, unless otherwise specified in this specification.

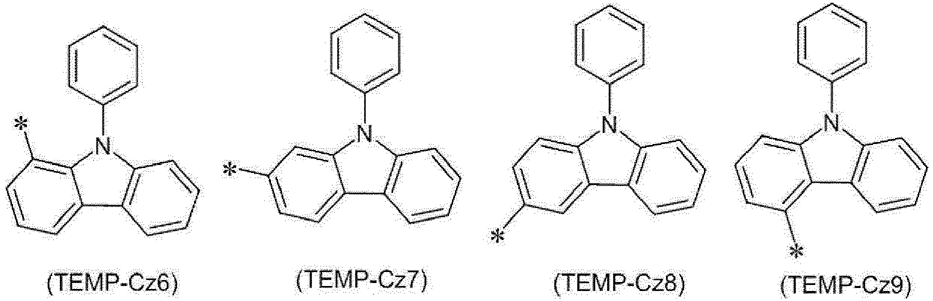

In the general formulas (TEMP-Cz1) to (TEMP-Cz9), * represents a bonding site.

In this specification, the dibenzofuranyl group and the dibenzothiophenyl group are specifically any of the following groups, unless otherwise specified in this specification.

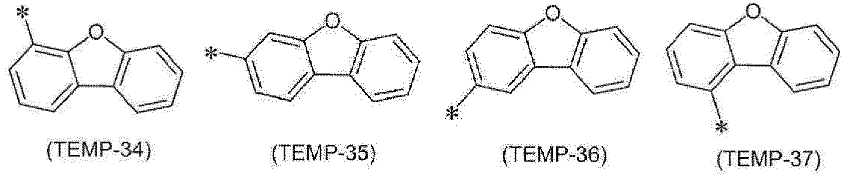

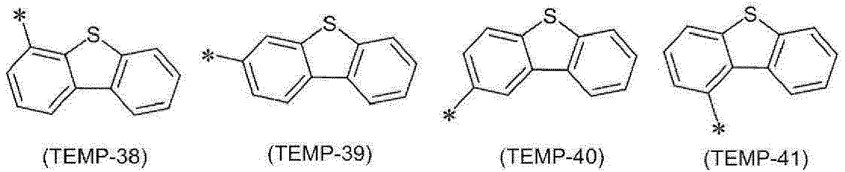

In the general formulas (TEMP-34) to (TEMP-41), * represents a bonding site.

The substituted or unsubstituted alkyl group described in this specification is preferably a methyl group, an ethyl group, a propyl group, an isopropyl group, a n-butyl group, an isobutyl group, a t-butyl group, or the like, unless otherwise specified in this specification.

### · "Substituted or unsubstituted arylene group"

The "substituted or unsubstituted arylene group" described in this specification is a divalent group derived by removing one hydrogen atom on the aryl ring of the "substituted or unsubstituted aryl group", unless otherwise specified. Specific examples of the "substituted or unsubstituted arylene group" (specific example group G12) include a divalent group derived by removing one hydrogen atom on the aryl ring of the "substituted or unsubstituted aryl group" described in the specific example group G1, and the like.

### · "Substituted or unsubstituted divalent heterocyclic group"

The "substituted or unsubstituted divalent heterocyclic group" described in this specification is a divalent group derived by removing one hydrogen atom on the heterocycle of the "substituted or unsubstituted heterocyclic group", unless otherwise specified. Specific examples of the "substituted or unsubstituted divalent heterocyclic group" (specific example group G13) include a divalent group derived by removing one hydrogen atom on the heterocycle of the "substituted or unsubstituted heterocyclic group" described in the specific example group G2, and the like.

### · "Substituted or unsubstituted alkylene group"

The "substituted or unsubstituted alkylene group" described in this specification is a divalent group derived by removing one hydrogen atom on the alkyl chain of the "substituted or unsubstituted alkyl group", unless otherwise specified. Specific examples of the "substituted or unsubstituted alkylene group" (specific example group G14) include a divalent group derived by removing one hydrogen atom on the alkyl chain of the "substituted or unsubstituted alkyl group" described in the specific example group G3, and the like.

The substituted or unsubstituted arylene group described in this specification is preferably any group of the following general formulas (TEMP-42) to (TEMP-68), unless otherwise specified in this specification.

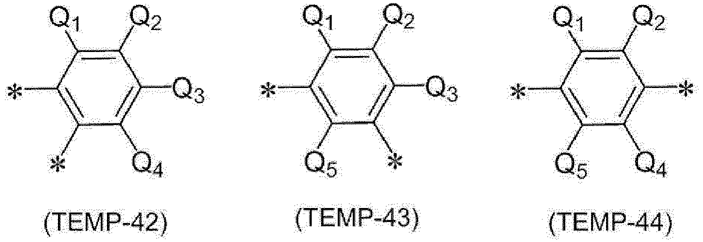

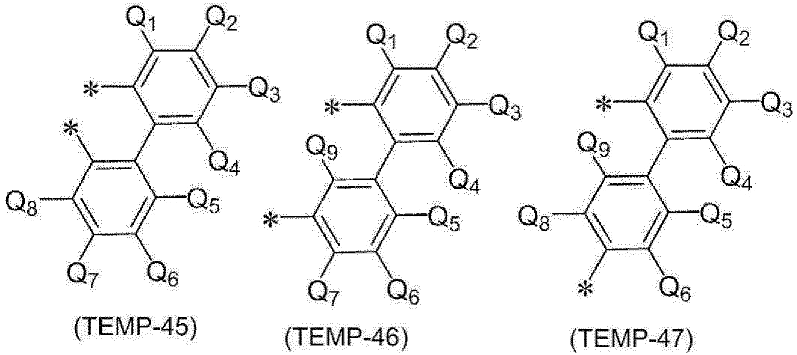

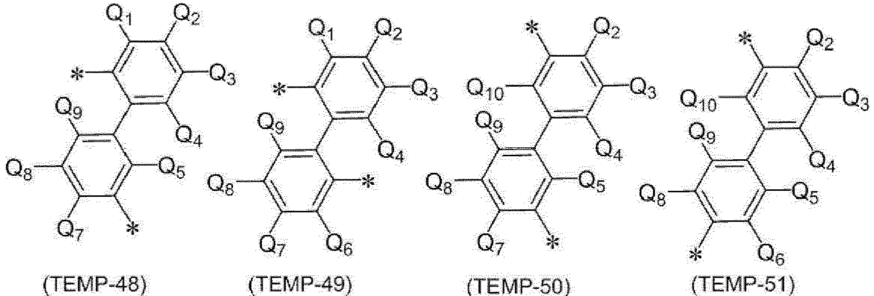

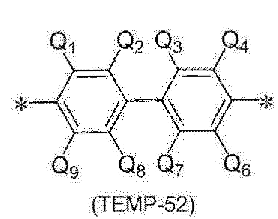

In the general formulas (TEMP-42) to (TEMP-52), Q₁ to Q₁₀ are independently a hydrogen atom or a substituent.

In the general formulas (TEMP-42) to (TEMP-52), * represents a bonding site.

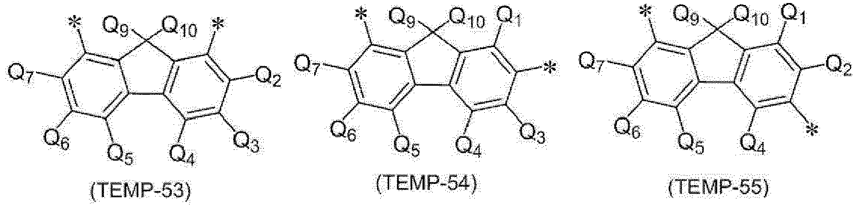

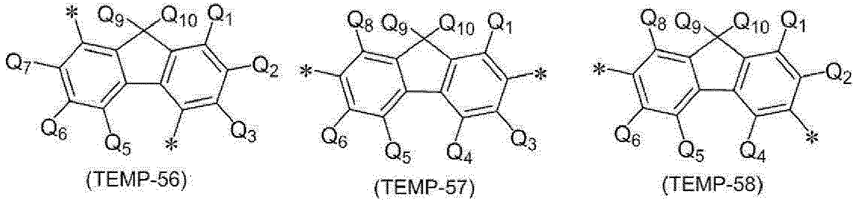

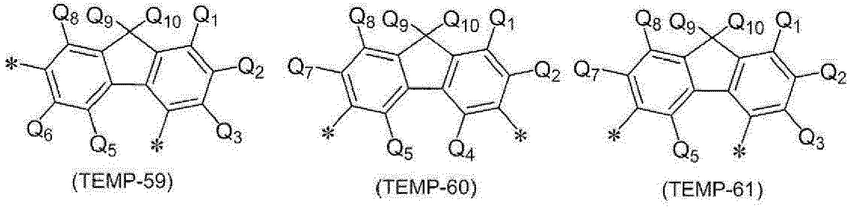

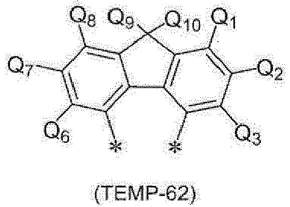

In the general formulas (TEMP-53) to (TEMP-62), Q₁ to Q₁₀ are independently a hydrogen atom or a substituent.

Q₉ and Q₁₀ may be bonded with each other via a single bond to form a ring.

In the general formulas (TEMP-53) to (TEMP-62), * represents a bonding site.

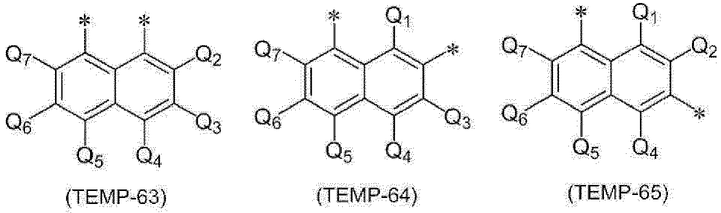

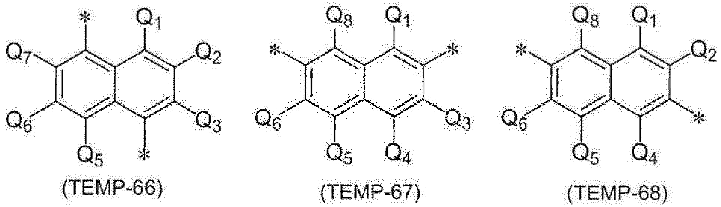

In the general formulas (TEMP-63) to (TEMP-68), Q₁ to Qa are independently a hydrogen atom or a substituent.

In the general formulas (TEMP-63) to (TEMP-68), * represents a bonding site.

The substituted or unsubstituted divalent heterocyclic group described in this specification is preferably any group of the following general formulas (TEMP-69) to (TEMP-102), unless otherwise specified in this specification.

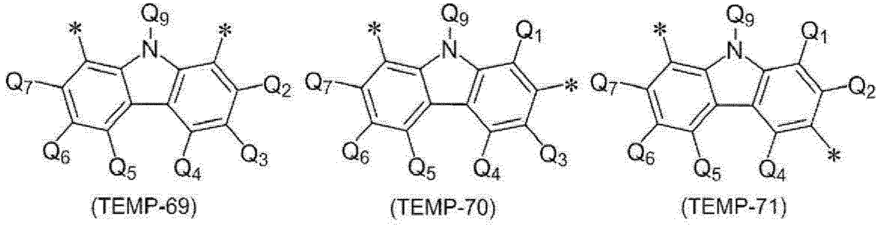

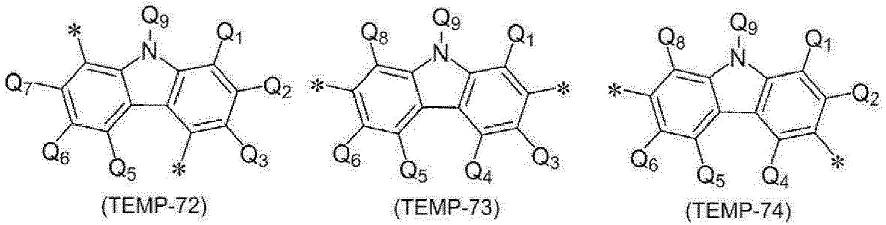

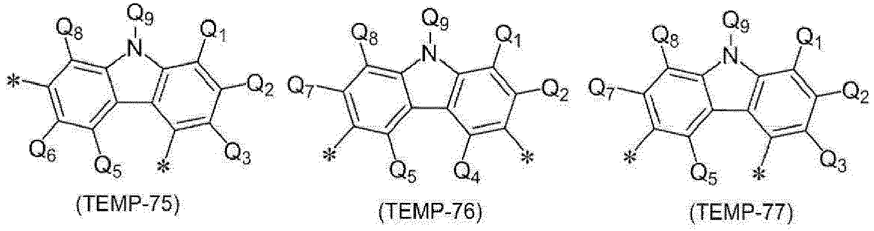

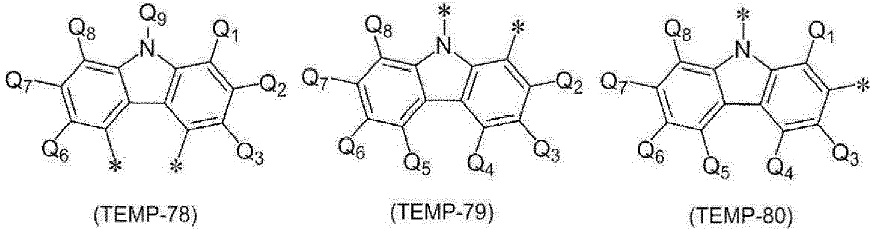

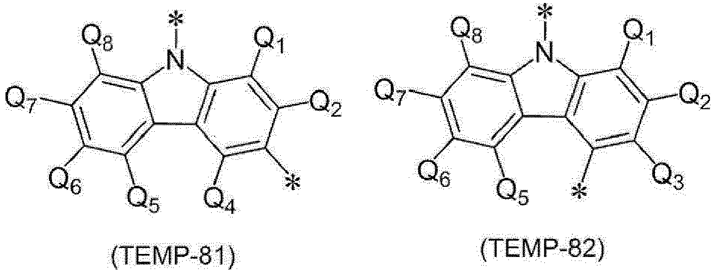

In the general formulas (TEMP-69) to (TEMP-82), Q₁ to Q₉ are independently a hydrogen atom or a substituent.

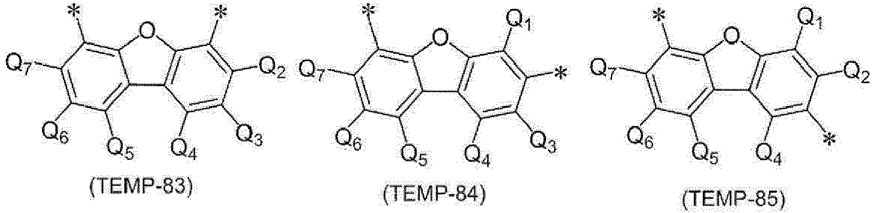

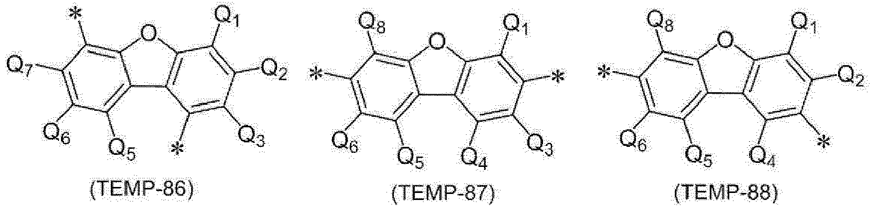

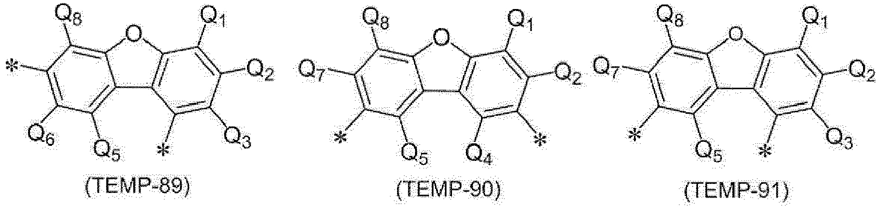

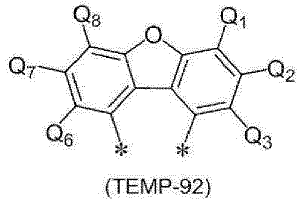

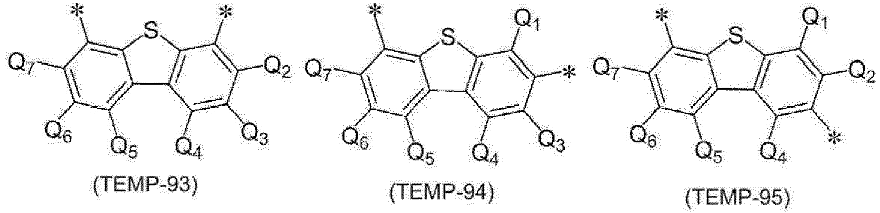

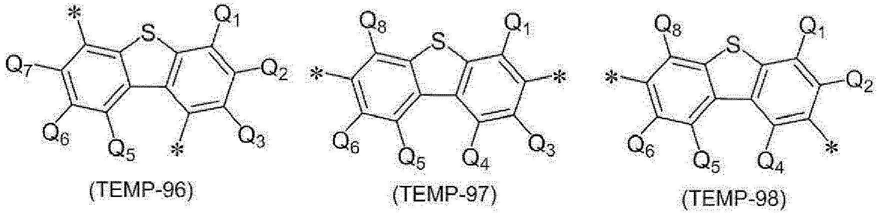

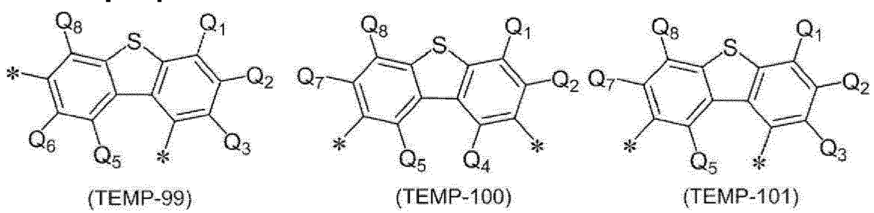

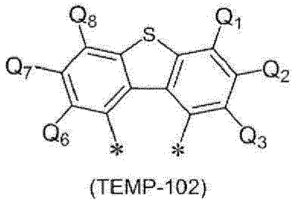

In the general formulas (TEMP-83) to (TEMP-102), Q₁ to Qa are independently a hydrogen atom or a substituent.

The above is the explanation of the "Substituent described in this specification."

### · "The case where bonded with each other to form a ring"

In this specification, the case where "one or more sets of adjacent two or more form a substituted or unsubstituted monocycle by bonding with each other, form a substituted or unsubstituted fused ring by bonding with each other, or do not bond with each other" means the case where "one or more sets of adjacent two or more form a substituted or unsubstituted monocycle by bonding with each other"; the case where "one or more sets of adjacent two or more form a substituted or unsubstituted fused ring by bonding with each other"; and the case where "one or more sets of adjacent two or more do not bond with each other."

The case where "one or more sets of adjacent two or more form a substituted or unsubstituted monocycle by bonding with each other" and the case where "one or more sets of adjacent two or more form a substituted or unsubstituted fused ring by bonding with each other" in this specification (these cases may be collectively referred to as "the case where forming a ring by bonding with each other") will be described below. The case of an anthracene compound represented by the following general formula (TEMP-103) in which the mother skeleton is an anthracene ring will be described as an example.

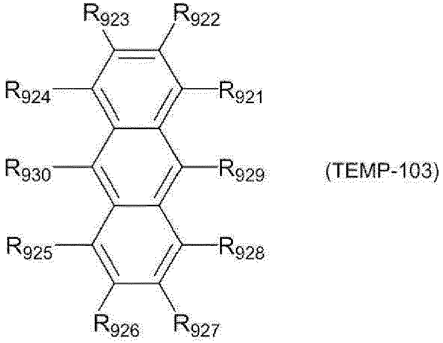

For example, in the case where "one or more sets of adjacent two or more among R₉₂₁ to R₉₃₀ form a ring by bonding with each other", the one set of adjacent two includes a pair of R₉₂₁ and R₉₂₂, a pair of R₉₂₂ and Rszs, a pair of R₉₂₃ and R₉₂₄, a pair of R₉₂₄ and R₉₃₀, a pair of R₉₃₀ and R₉₂₅, a pair of R₉₂₅ and R₉₂₆, a pair of R₉₂₆ and R₉₂₇, a pair of R₉₂₇ and R₉₂₈, a pair of R₉₂₈ and R₉₂₉, and a pair of R₉₂₉ and R₉₂₁.

The "one or more sets" means that two or more sets of the adjacent two or more sets may form a ring at the same time. For example, R₉₂₁ and R₉₂₂ form a ring Q_{A} by bonding with each other, and at the same, time R₉₂₅ and R₉₂₆ form a ring Q_{B} by bonding with each other, the anthracene compound represented by the general formula (TEMP-103) is represented by the following general formula (TEMP-104).

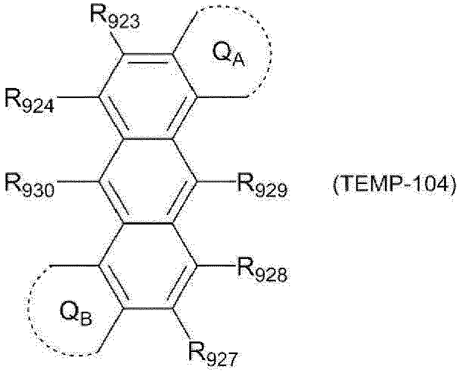

The case where the "set of adjacent two or more" form a ring includes not only the case where the set (pair) of adjacent "two" is bonded with as in the above-mentioned examples, but also the case where the set of adjacent "three or more" are bonded with each other. For example, it means the case where R₉₂₁ and R₉₂₂ form a ring Q_{A} by bonding with each other, and R₉₂₂ and R₉₂₃ form a ring Qc by bonding with each other, and adjacent three (R₉₂₁, R₉₂₂ and R₉₂₃) form rings by bonding with each other and together fused to the anthracene mother skeleton. In this case, the anthracene compound represented by the general formula (TEMP-103) is represented by the following general formula (TEMP-105). In the following general formula (TEMP-105), the ring Q_{A} and the ring Qc share R₉₂₂.

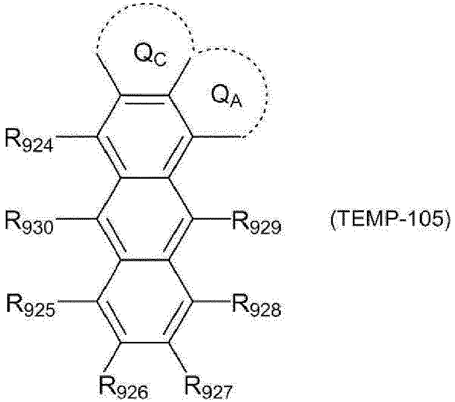

The "monocycle" or "fused ring" formed may be a saturated ring or an unsaturated ring, as a structure of the formed ring alone. Even when the "one pair of adjacent two" forms a "monocycle" or a "fused ring" , the "monocycle" or the "fused ring" may form a saturated ring or an unsaturated ring. For example, the ring Q_{A} and the ring Q_{B} formed in the general formula (TEMP-104) are independently a "monocycle" or a "fused ring." The ring Q_{A} and the ring Qc formed in the general formula (TEMP-105) are "fused ring." The ring Q_{A} and ring Qc of the general formula (TEMP-105) are fused ring by fusing the ring Q_{A} and the ring Qc together. When the ring Q_{A} of the general formula (TMEP-104) is a benzene ring, the ring Q_{A} is a monocycle. When the ring Q_{A} of the general formula (TMEP-104) is a naphthalene ring, the ring Q_{A} is a fused ring.

The "unsaturated ring" includes, in addition to an aromatic hydrocarbon ring and an aromatic heterocycle, an aliphatic hydrocarbon ring with an unsaturated bond, i.e., double and/or triple bonds in the ring structure (e.g., cyclohexene, cyclohexadiene, etc.), and a non-aromatic heterocycle with an unsaturated bond (e.g., dihydropyran, imidazoline, pyrazoline, quinolizine, indoline, isoindoline, etc.). The "saturated ring" includes an aliphatic hydrocarbon ring without an unsaturated bond and a non-aromatic heterocycle without ab unsaturated bond.

Specific examples of the aromatic hydrocarbon ring include a structure in which the group listed as a specific example in the specific example group G1 is terminated by a hydrogen atom.

Specific examples of the aromatic heterocycle include a structure in which the aromatic heterocyclic group listed as a specific example in the example group G2 is terminated by a hydrogen atom.

Specific examples of the aliphatic hydrocarbon ring include a structure in which the group listed as a specific example in the specific example group G6 is terminated by a hydrogen atom.

The term "to form a ring" means forming a ring only with plural atoms of the mother skeleton, or with plural atoms of the mother skeleton and one or more arbitrary atoms in addition. For example, the ring Q_{A} shown in the general formula (TEMP-104), which is formed by bonding R₉₂₁ and R₉₂₂ with each other, is a ring formed from the carbon atom of the anthracene skeleton with which R₉₂₁ is bonded, the carbon atom of the anthracene skeleton with which R₉₂₂ is bonded, and one or more arbitrary atoms. For example, in the case where the ring Q_{A} is formed with R₉₂₁ and R₉₂₂, when a monocyclic unsaturated ring is formed with the carbon atom of the anthracene skeleton with which R₉₂₁ is bonded, the carbon atom of the anthracene skeleton with which R₉₂₂ is bonded, and four carbon atoms, the ring formed with R₉₂₁ and R₉₂₂ is a benzene ring.

Here, the "arbitrary atom" is preferably at least one atom selected from the group consisting of a carbon atom, a nitrogen atom, an oxygen atom, and a sulfur atom, unless otherwise specified in this specification. In the arbitrary atom (for example, a carbon atom or a nitrogen atom), a bond which does not form a ring may be terminated with a hydrogen atom or the like, or may be substituted with "arbitrary substituent" described below. When an arbitrary atom other than a carbon atom is contained, the ring formed is a heterocycle.

The number of "one or more arbitrary atom(s)" constituting a monocycle or a fused ring is preferably 2 or more and 15 or less, more preferably 3 or more and 12 or less, and still more preferably 3 or more and 5 or less, unless otherwise specified in this specification.

The "monocycle" is preferable among the "monocycle" and the "fused ring", unless otherwise specified in this specification.

The "unsaturated ring" is preferable among the "saturated ring" and the "unsaturated ring", unless otherwise specified in this specification.

Unless otherwise specified in this specification, the "monocycle " is preferably a benzene ring.

Unless otherwise specified in this specification, the "unsaturated ring" is preferably a benzene ring.

Unless otherwise specified in this specification, when "one or more sets of adjacent two or more" are "bonded with each other to form a substituted or unsubstituted monocycle" or "bonded with each other to form a substituted or unsubstituted fused ring", this specification, one or more sets of adjacent two or more are preferably bonded with each other to form a substituted or unsubstituted "unsaturated ring" from plural atoms of the mother skeleton and one or more and 15 or less atoms which is at least one kind selected from a carbon atom, a nitrogen atom, an oxygen atom, and a sulfur atom.

The substituent in the case where the above-mentioned "monocycle" or "fused ring" has a substituent is, for example, an "arbitrary substituent" described below. Specific examples of the substituent which the above-mentioned "monocycle" or "fused ring" has include the substituent described above in the "Substituent described in this specification" section.

The substituent in the case where the above-mentioned "saturated ring" or "unsaturated ring" has a substituent is, for example, an "arbitrary substituent" described below. Specific examples of the substituent which the above-mentioned "monocycle" or "fused ring" has include the substituent described above in the "Substituent described in this specification" section.

The foregoing describes the case where "one or more sets of adjacent two or more form a substituted or unsubstituted monocycle by bonding with each other" and the case where "one or more sets of adjacent two or more form a substituted or unsubstituted fused ring by bonding with each other" (the case where "forming a ring by bonding with each other").

### · Substituent in the case of "substituted or unsubstituted"

In one embodiment in this specification, the substituent (in this specification, sometimes referred to as an "arbitrary substituent") in the case of "substituted or unsubstituted" is, for example, a group selected from the group consisting of:
an unsubstituted alkyl group including 1 to 50 carbon atoms,
an unsubstituted alkenyl group including 2 to 50 carbon atoms,
an unsubstituted alkynyl group including 2 to 50 carbon atoms,
an unsubstituted cycloalkyl group including 3 to 50 ring carbon atoms,
-Si(R₉₀₁)(R₉₀₂)(R₉₀₃),
-O-(R₉₀₄),
-S-(R905),
-N(R₉₀₆)(R₉₀₇),
a halogen atom, a cyano group, a nitro group,
an unsubstituted aryl group including 6 to 50 ring carbon atoms, and an unsubstituted heterocyclic group including 5 to 50 ring atoms,
wherein, R₉₀₁ to R₉₀₇ are independently
a hydrogen atom,
a substituted or unsubstituted alkyl group including 1 to 50 carbon atoms,
a substituted or unsubstituted cycloalkyl group including 3 to 50 ring carbon atoms,
a substituted or unsubstituted aryl group including 6 to 50 ring carbon atoms, or
a substituted or unsubstituted heterocyclic group including 5 to 50 ring atoms.

When two or more R₉₀₁'s are present, the two or more R₉₀₁'s may be the same or different.

When two or more R₉₀₂'s are present, the two or more R₉₀₂'s may be the same or different.

When two or more R₉₀₃'s are present, the two or more R₉₀₃'s may be the same or different.

When two or more R₉₀₄'s are present, the two or more R₉₀₄'s may be the same or different.

When two or more R₉₀₅'s are present, the two or more R₉₀₅'s may be the same or different.

When two or more R₉₀₆'s are present, the two or more R₉₀₆'s may be the same or different.

When two or more R₉₀₇'s are present, the two or more R₉₀₇'s may be the same or different.

In one embodiment, the substituent in the case of "substituted or unsubstituted" is a group selected from the group consisting of:
an alkyl group including 1 to 50 carbon atoms,
an aryl group including 6 to 50 ring carbon atoms, and
a heterocyclic group including 5 to 50 ring atoms.

In one embodiment, the substituent in the case of "substituted or unsubstituted" is a group selected from the group consisting of:
an alkyl group including 1 to 18 carbon atoms,
an aryl group including 6 to 18 ring carbon atoms, and
a heterocyclic group including 5 to 18 ring atoms.

Specific examples of each of the arbitrary substituents include specific examples of substituent described in the section "Substituent described in this specification" above.

Unless otherwise specified in this specification, adjacent arbitrary substituents may form a "saturated ring" or an "unsaturated ring", preferably form a substituted or unsubstituted saturated 5-membered ring, a substituted or unsubstituted saturated 6-membered ring, a substituted or unsubstituted unsaturated 5-membered ring, or a substituted or unsubstituted unsaturated 6-membered ring, more preferably form a benzene ring.

Unless otherwise specified in this specification, the arbitrary substituent may further have a substituent. The substituent which the arbitrary substituent further has is the same as that of the above-mentioned arbitrary substituent.

In this specification, the numerical range represented by "AA to BB" means the range including the numerical value AA described on the front side of "AA to BB" as the lower limit and the numerical value BB described on the rear side of "AAto BB" as the upper limit.

### [Compound]

A compound according to an aspect of the present invention is represented by the following formula (1): 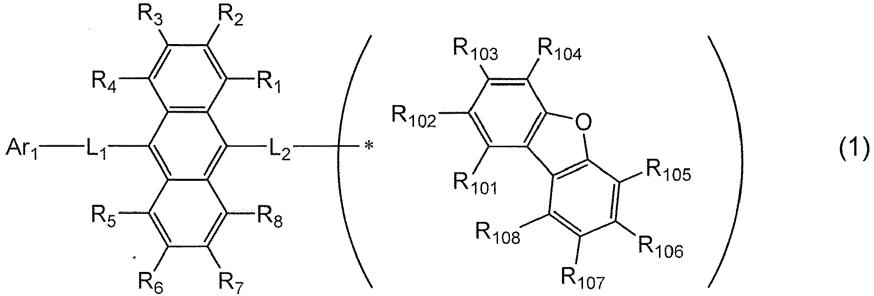 wherein in the formula (1),
R₁ to R₈ are independently a hydrogen atom, or a substituent R;
L₁ and L₂ are independently
a single bond,
a substituted or unsubstituted phenylene group,
a substituted or unsubstituted naphthylene group, or
a divalent group in which these two groups are combined;
Ar₁ is
a substituted or unsubstituted phenyl group,
a substituted or unsubstituted p-biphenyl group,
a substituted or unsubstituted m-biphenyl group,
a substituted or unsubstituted o-biphenyl group,
a substituted or unsubstituted 1-naphthyl group,
a substituted or unsubstituted 2-naphthyl group, or
a substituted or unsubstituted phenanthryl group;
the adjacent two of R₁₀₁ to R₁₀₈ form a substituted or unsubstituted benzene ring by bonding with each other, or do not bond with each other;
one of, R₁₀₁ to R₁₀₈ which do not form the substituted or unsubstituted benzene ring, and carbon atoms in the substituted or unsubstituted benzene ring in the case where the adjacent two of R₁₀₁ to R₁₀₈ form a substituted or unsubstituted benzene ring by bonding with each other is bonded with L₂ via a single bond;
R₁₀₁ to R₁₀₈ which do not form the substituted or unsubstituted benzene ring and which are not bonded with L₂ are independently a hydrogen atom, or a substituent R;
the substituent R is selected from the group consisting of
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
-Si(R₉₀₁)(R₉₀₂)(R₉₀₃),
-O-(R₉₀₄),
-S-(R905),
-N(R₉₀₆)(R₉₀₇)
(wherein R₉₀₁ to R₉₀₇ are independently
a hydrogen atom,
a substituted or unsubstituted alkyl group having 1 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
a halogen atom, a cyano group, a nitro group,
a substituted or unsubstituted aryl group having 6 to 50 ring carbon atoms, and
a substituted or unsubstituted monovalent heterocyclic group having 5 to 50 ring atoms;
provided that the compound represented by the formula (1) satisfies all of the following conditions 1 to 3;
condition 1: R₁ to R₈ which are hydrogen atoms are not a deuterium atom;
condition 2: one or more of, hydrogen atoms possessed by L₁, L₂ and Ar₁, hydrogen atoms possessed by the substituent in the case where L₁, L₂ and Ar₁ are substituted, hydrogen atoms possessed by R₁ to R₈ which are the substituents R, R₁₀₁ to R₁₀₈ which are hydrogen atoms, hydrogen atoms possessed by R₁₀₁ to R₁₀₈ which are the substituents R, and hydrogen atoms possessed by the substituted or unsubstituted benzene ring in the case where the adjacent two of R₁₀₁ to R₁₀₈ form a substituted or unsubstituted benzene ring by bonding with each other are a deuterium atom; and
condition 3: when L₁ is a single bond, Ar₁ is not a phenyl group.

When L₁ is a single bond in the formula (1), Ar₁ is directly bonded with the anthracene skeleton. When L₂ is a single bond in the formula (1), the structure in the above parentheses is directly bonded with the anthracene skeleton at the bondable position of the structure (one of, R₁₀₁ to R₁₀₈ which do not form the substituted or unsubstituted benzene ring, and carbon atoms in the substituted or unsubstituted benzene ring).

The expression "R₁ to R₈ which are hydrogen atoms are not a deuterium atom" will be described.

The expression "hydrogen atoms are not a deuterium atom" in the present specification means that the amount of the deuterium atom in the hydrogen atoms is below the natural abundance based on the total amount of the protium atom and the deuterium atom. That is, the expression means that deuterium atoms can be included in the hydrogen atoms to the extent of the natural abundance thereof. The natural abundance of deuterium atom is, for example, 0.015% or less.

It can be confirmed by using a nuclear magnetic resonator that the amount of the deuterium atom in the hydrogen atoms is below the natural abundance based on the total amount of the protium atom and the deuterium atom.

The expression "one or more of, hydrogen atoms possessed by L₁, L₂ and Ar₁, hydrogen atoms possessed by the substituent in the case where L₁, L₂ and Ar₁ are substituted, hydrogen atoms possessed by R₁ to R₈ which are the substituents R, R₁₀₁ to R₁₀₈ which are hydrogen atoms, hydrogen atoms possessed by R₁₀₁ to R₁₀₈ which are the substituents R, and hydrogen atoms possessed by the substituted or unsubstituted benzene ring are a deuterium atom" will be described.

The expression "hydrogen atoms are a deuterium atom" in the present specification means that the amount of the deuterium atom in the hydrogen atoms based on the total amount of the protium atom and the deuterium atom is larger than the natural abundance thereof. It can be confirmed by using a nuclear magnetic resonator that the amount of the deuterium atom based on the total amount of the protium atom and the deuterium atom is larger than the natural abundance thereof.

In one embodiment, the adjacent two of R₁₀₁ to R₁₀₈ form a substituted or unsubstituted benzene ring by bonding with each other.

In one embodiment, R₁₀₁ is bonded with L₂ via a single bond.

In one embodiment, the compound represented by the formula (1) is a compound represented by the following formula (1-1): 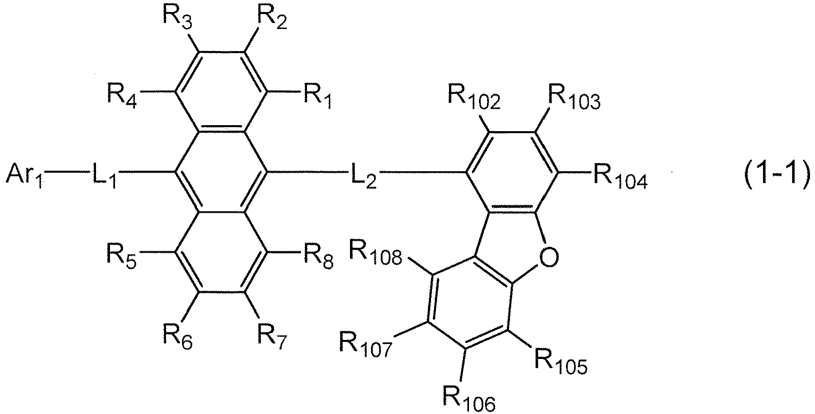 wherein in the formula (1-1), R₁ to R₈, R₁₀₂ to R₁₀₈, L₁, L₂ and Ar₁ are the same as defined in the formula (1).

In one embodiment, the adjacent two of R₁₀₅ to R₁₀₈ form a substituted or unsubstituted benzene ring by bonding with each other.

In one embodiment, the compound represented by the formula (1) is a compound represented by any one of the following formulas (1-11) to (1-13): 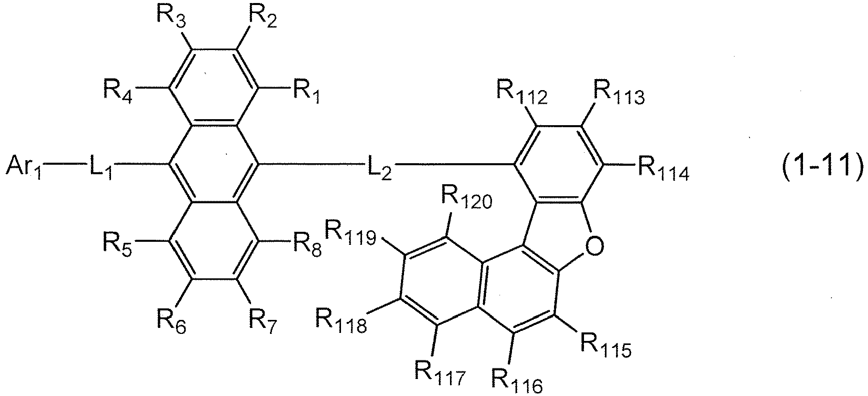 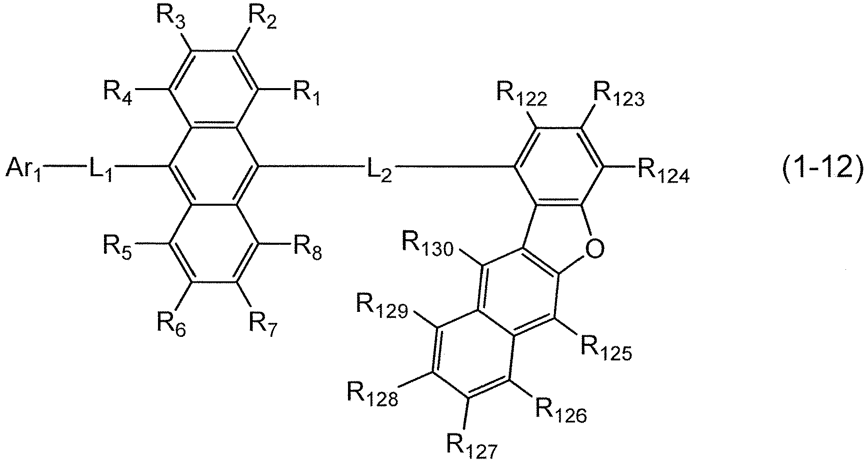 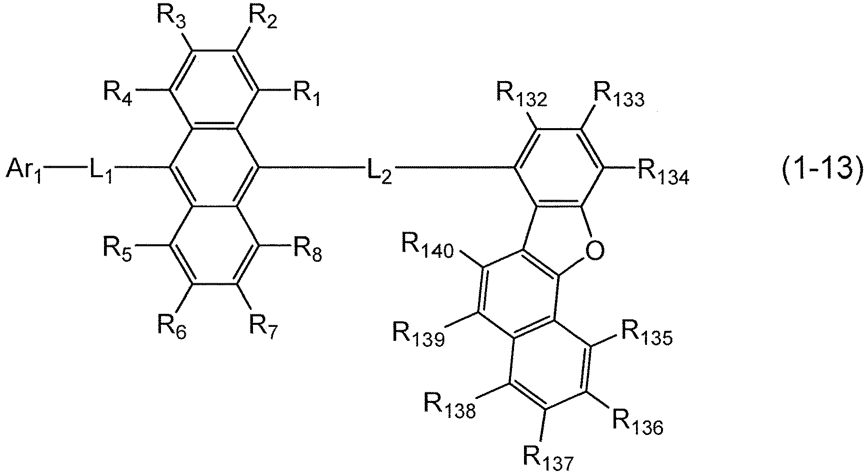 wherein in the formulas (1-11) to (1-13), R₁ to R₈, L₁, L₂ and Ar₁ are the same as defined in the formula (1);

in the formula (1-11), R₁₁₂ to R₁₂₀ are independently a hydrogen atom, or a substituent R; in the formula (1-12), R₁₂₂ to R₁₃₀ are independently a hydrogen atom, or a substituent R;

in the formula (1-13), R₁₃₂ to R₁₄₀ are independently a hydrogen atom, or a substituent R; and the substituent R is the same as defined in the formula (1).

In one embodiment, L₁ is
a substituted or unsubstituted phenylene group, or
a substituted or unsubstituted naphthylene group; and
one or more of hydrogen atoms possessed by L₁ are a deuterium atom.

In one embodiment, the compounds represented by the formulas (1-11) to (1-13) are a compound represented by any one of the following formulas (1-21) to (1-23): 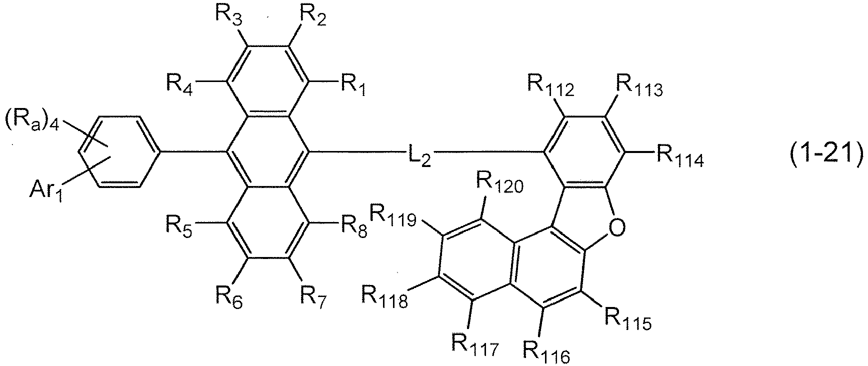 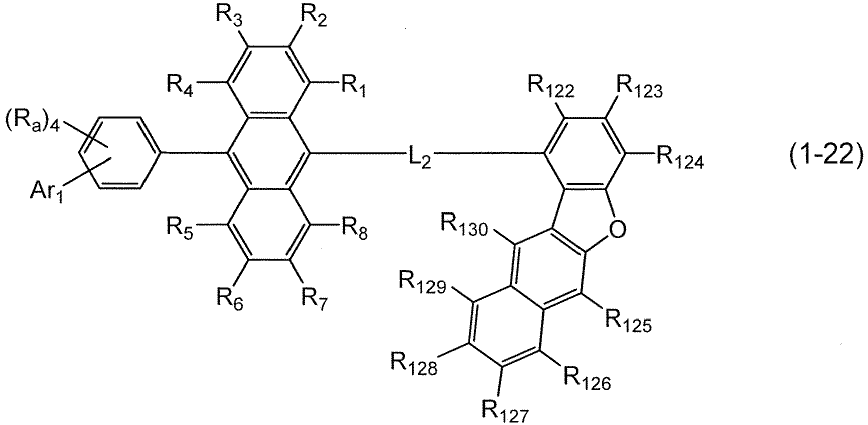 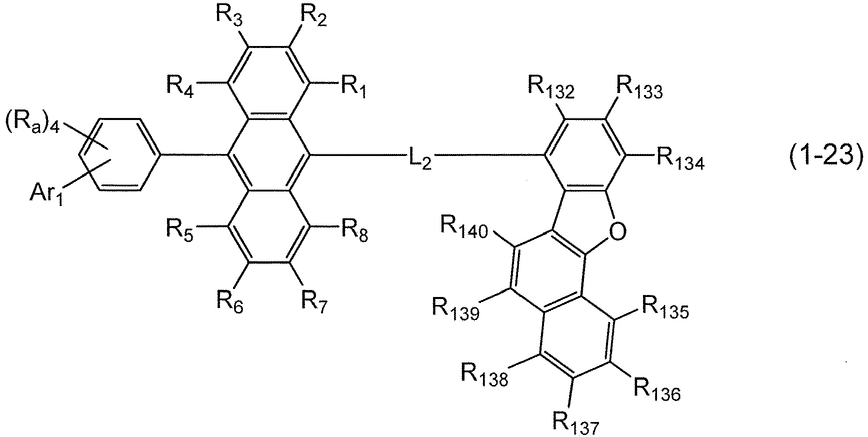 wherein in the formulas (1-21) to (1-23), R₁ to R₈, R₁₁₂ to R₁₂₀, R₁₂₂ to R₁₃₀, R₁₃₂ to R₁₄₀, L₂ and Ar₁ are the same as defined in the formulas (1-11) to (1-13);
four Rₐ's are independently a hydrogen atom, or a substituent R;
provided that one or more of the four Rₐ's are a deuterium atom; and
the substituent R is the same as defined in the formula (1).

It is preferable in terms of enhancing improving effects of device lifetime in the formula (1) that a compound in which one or more of hydrogen atoms possessed by L₁ are a deuterium atom and hydrogen atoms possessed by Ar₁ are not a deuterium atom, or a compound in which one or more of hydrogen atoms possessed by L₁ are a deuterium atom and one or more of hydrogen atoms possessed by Ar₁ are a deuterium atom be used.

It will be specifically described below using a compound in which all of deuterium atoms in the formula (1) are replaced with a protium atom (hereinafter, frequently referred to as "protium compound"), a compound having the same structure as the protium compound, except that one or more of hydrogen atoms possessed by L₁ are a deuterium atom (hereinafter, frequently referred to as "deuterium compound α"), a compound having the same structure as the protium compound, except that one or more of hydrogen atoms possessed by Ar₁ are a deuterium atom (hereinafter, frequently referred to as "deuterium compound β"), and a compound having the same structure as the protium compound, except that one or more of hydrogen atoms possessed by L₁ are a deuterium atom and one or more of hydrogen atoms possessed by Ar₁ are a deuterium atom (hereinafter, frequently referred to as "deuterium compound γ") as an example. Examples of the "protium compound", the "deuterium compound α", the "deuterium compound β" and the "deuterium compound γ" include the following compounds.

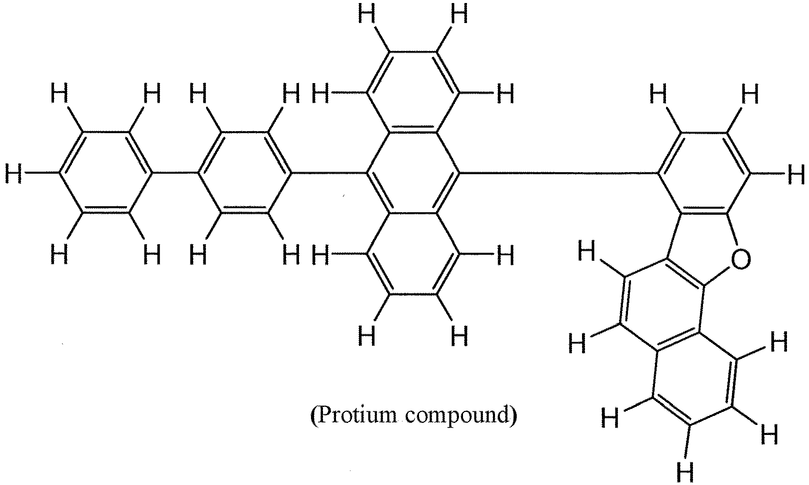

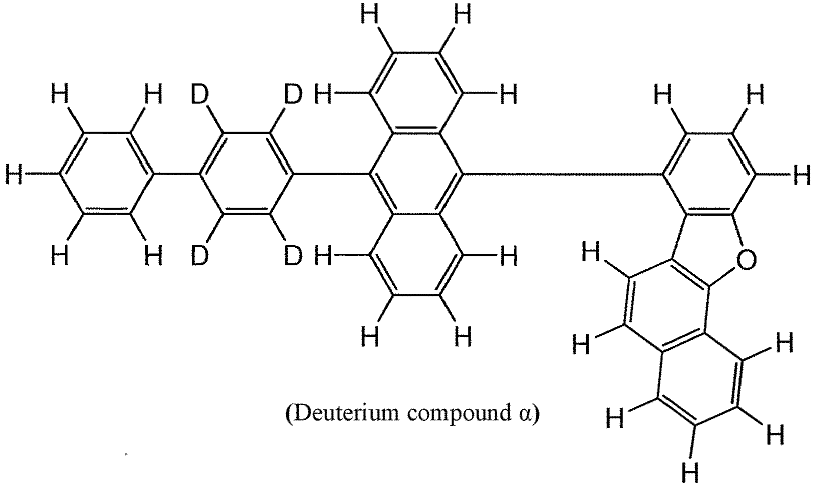

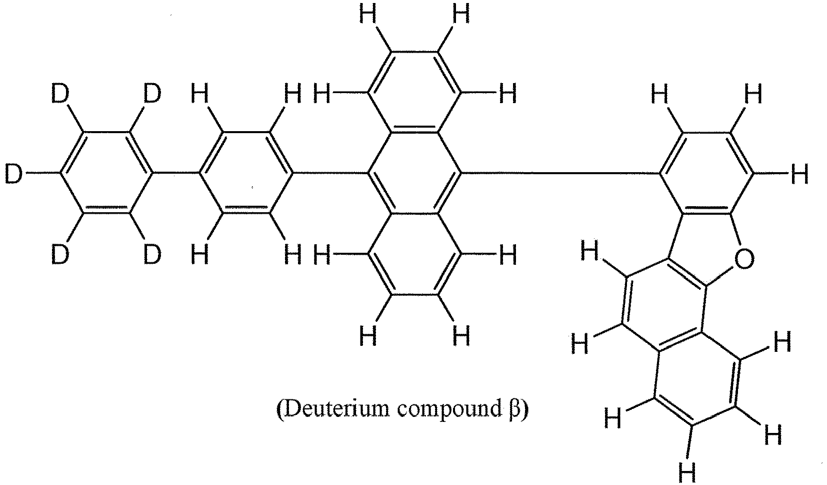

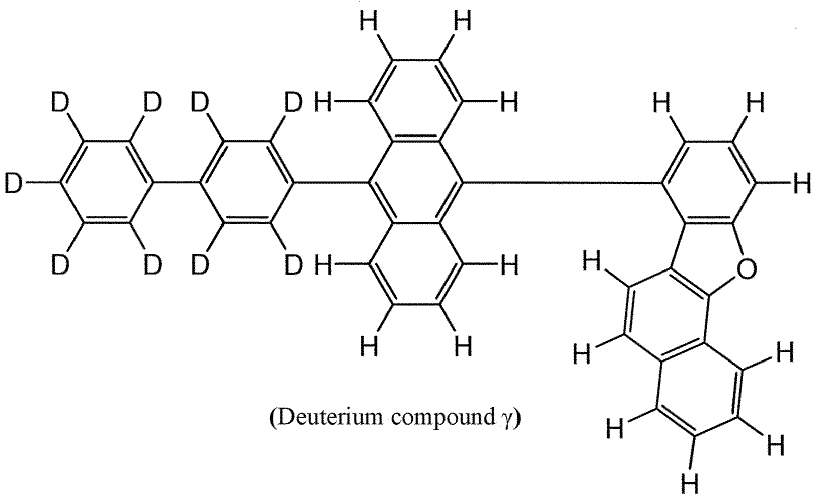

When increasing rates of the lifetimes of, a device in which the deuterium compound a is used, a device in which the deuterium compound β is used, and a device in which the deuterium compound γ is used, is compared based on the lifetime of a device in which the protium compound is used, the device in which the deuterium compound a or the deuterium compound γ is used has larger increasing rate of the lifetime than that of the device in which the deuterium compound β. That is, the case where the deuterium compound a or the deuterium compound γ is used is preferable as compared to the case where the deuterium compound β is used, since improving effects of device lifetime are enhanced.

In one embodiment, hydrogen atoms possessed by An are not a deuterium atom.

In one embodiment, a substituent in the case of "substituted or unsubstituted" in the formula (1) is selected from the group consisting of
an alkyl group having 1 to 50 carbon atoms,
a haloalkyl group having 1 to 50 carbon atoms,
an alkenyl group having 2 to 50 carbon atoms,
an alkynyl group having 2 to 50 carbon atoms,
a cycloalkyl group having 3 to 50 ring carbon atoms,
an alkoxy group having 1 to 50 carbon atoms,
an alkylthio group having 1 to 50 carbon atoms,
an aryloxy group having 6 to 50 ring carbon atoms,
an arylthio group having 6 to 50 ring carbon atoms,
an aralkyl group having 7 to 50 carbon atoms,
-Si(R₄₁)(R₄₂)(R₄₃),
-C(=O)R₄₄, -COOR₄₅,
-S(=O)₂R₄₆,
-P(=O)(R₄₇)(R₄₈),
-Ge(R₄₉)(R₅₀)(R₅₁),
-N(R₅₂)(R₅₃) (wherein, R₄₁ to R₅₃ are independently a hydrogen atom, an alkyl group having 1 to 50 carbon atoms, an aryl group having 6 to 50 ring carbon atoms, or a monovalent heterocyclic group having 5 to 50 ring atoms; when two or more of each of R₄₁ to R₅₃ are present, the two or more of each of R₄₁ to R₅₃ may be the same as or different from each other),
a hydroxy group,
a halogen atom,
a cyano group,
a nitro group,
an aryl group having 6 to 50 ring carbon atoms, and
a monovalent heterocyclic group having 5 to 50 ring atoms.

In one embodiment, a substituent in the case of "substituted or unsubstituted" in the formula (1) is selected from the group consisting of
an alkyl group having 1 to 18 carbon atoms,
an aryl group having 6 to 18 ring carbon atoms, and
a monovalent heterocyclic group having 5 to 18 ring atoms.

The compound according to an aspect of the present invention can be synthesized in accordance with Examples by using known alternative reactions or raw materials adapted to the target compound.

Specific examples of the compound according to an aspect of the present invention (the compound represented by the formula (1)) will be described below, but these are merely examples, and the compound according to an aspect of the present invention is not limited to the following specific examples.

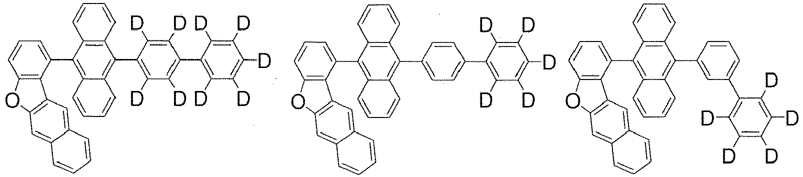

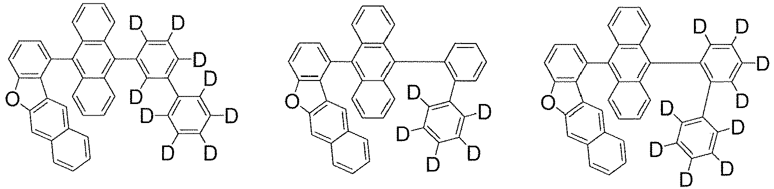

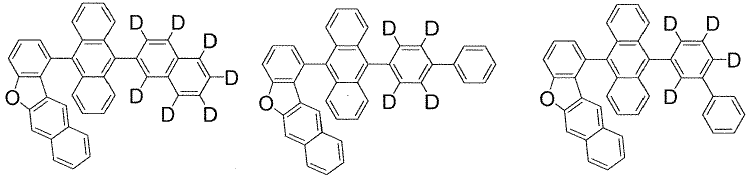

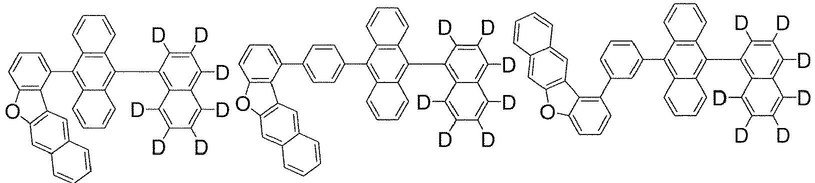

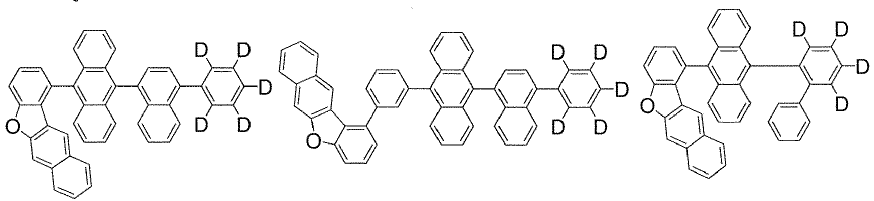

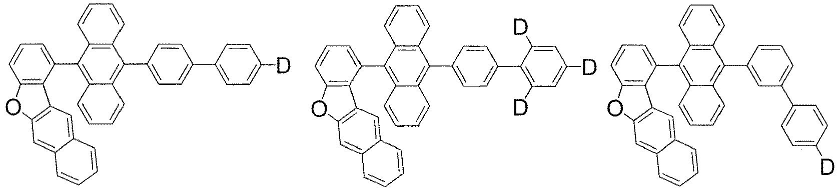

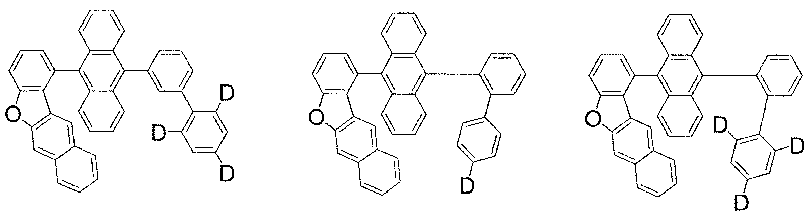

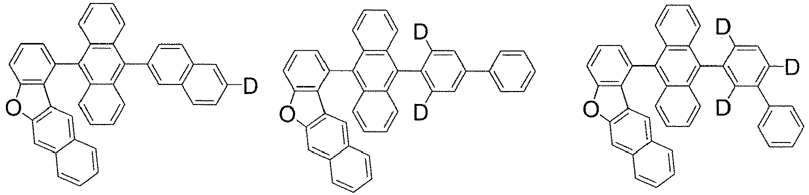

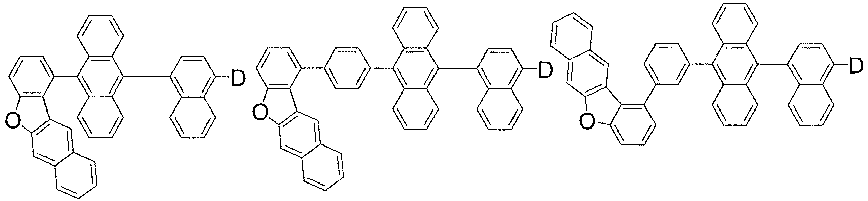

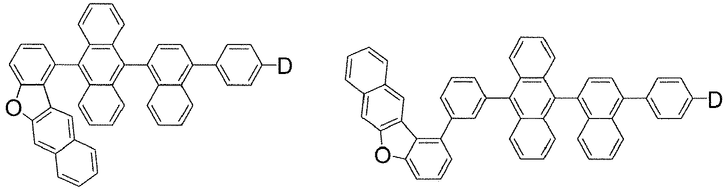

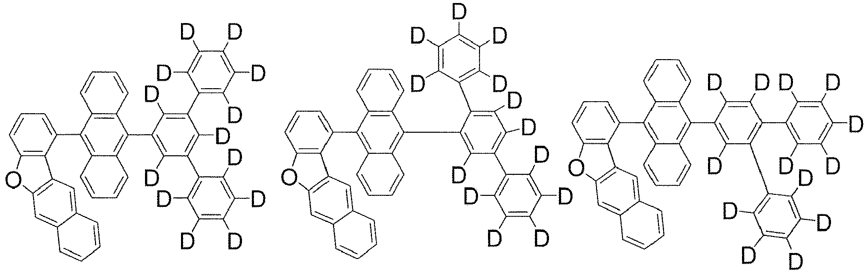

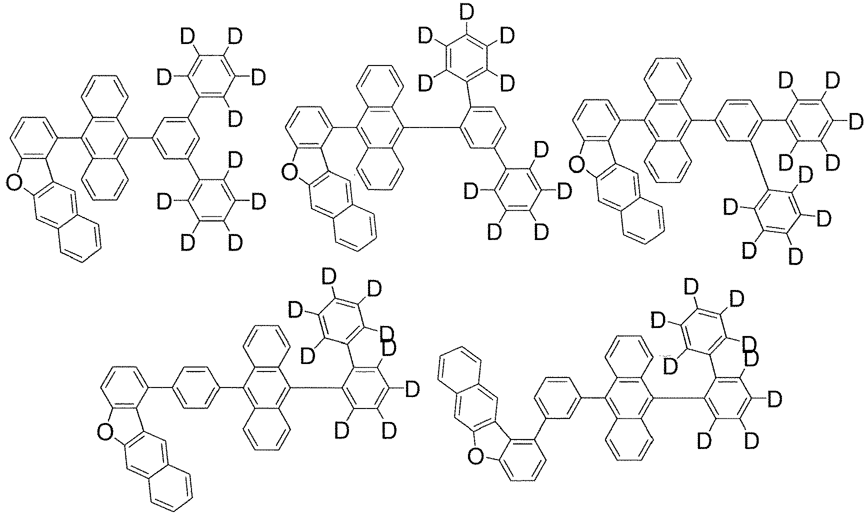

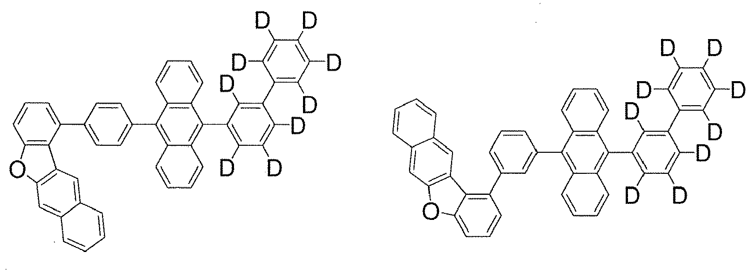

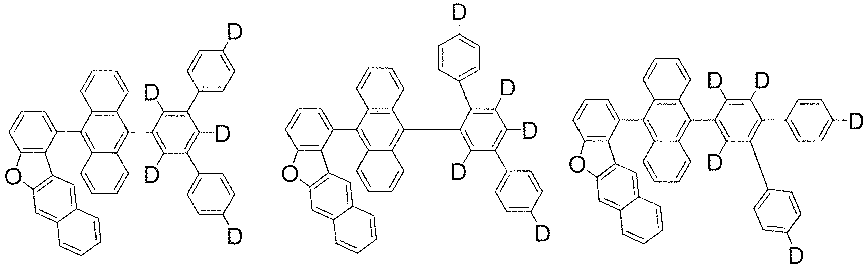

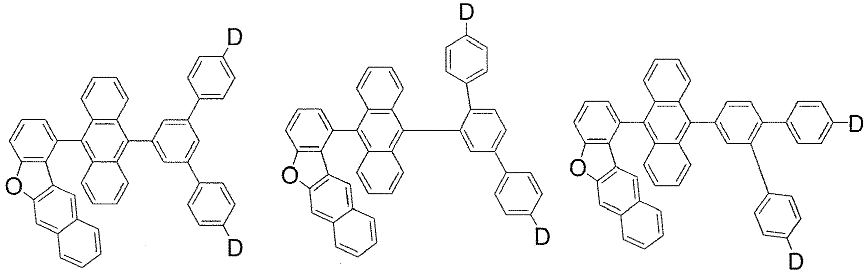

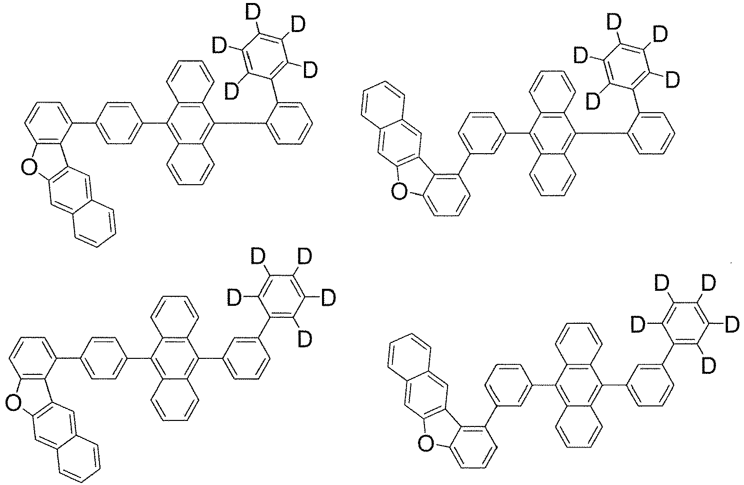

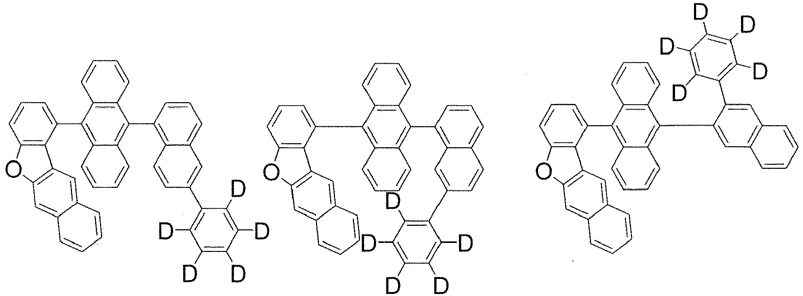

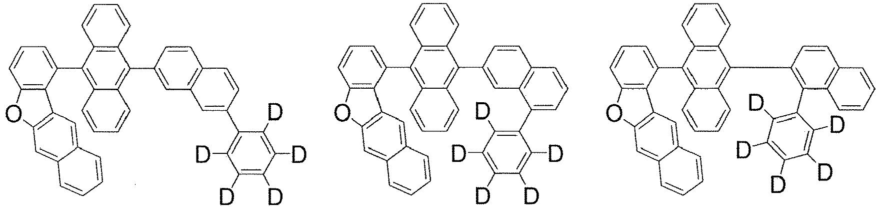

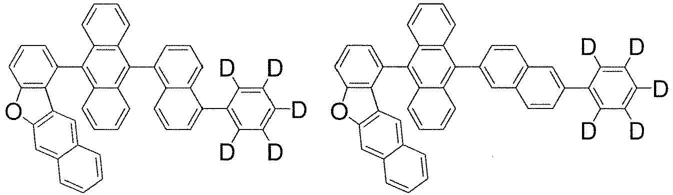

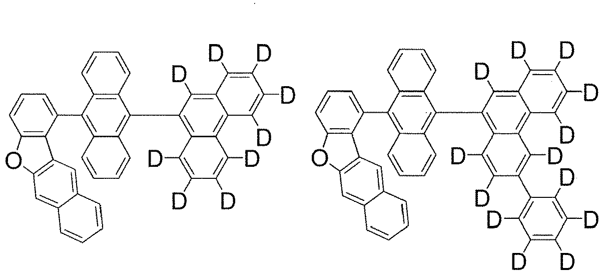

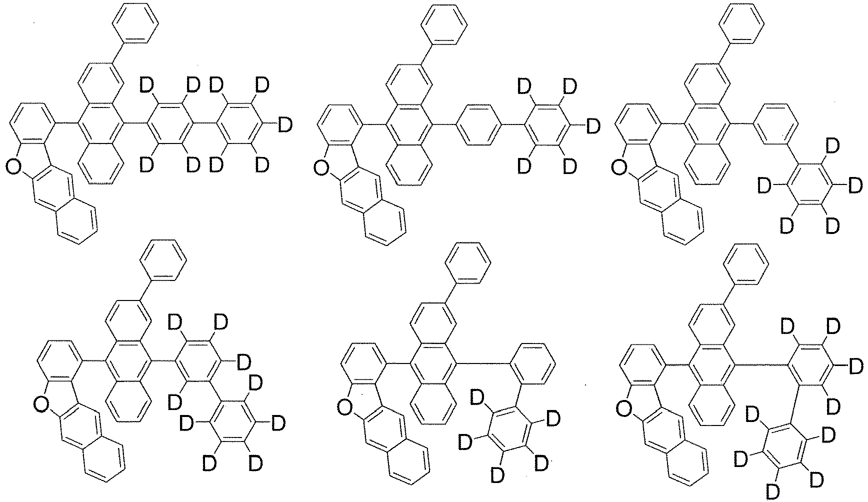

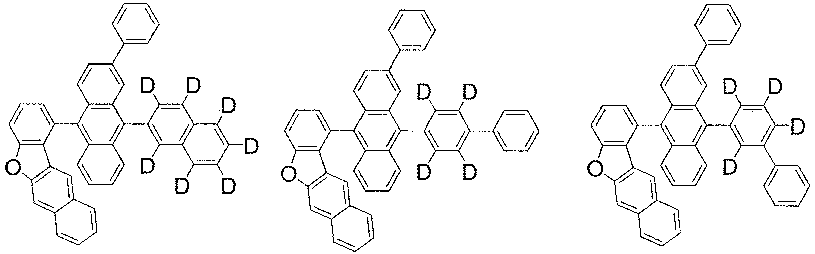

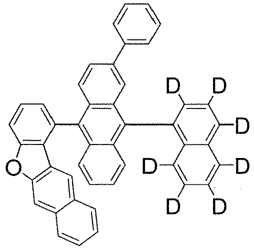

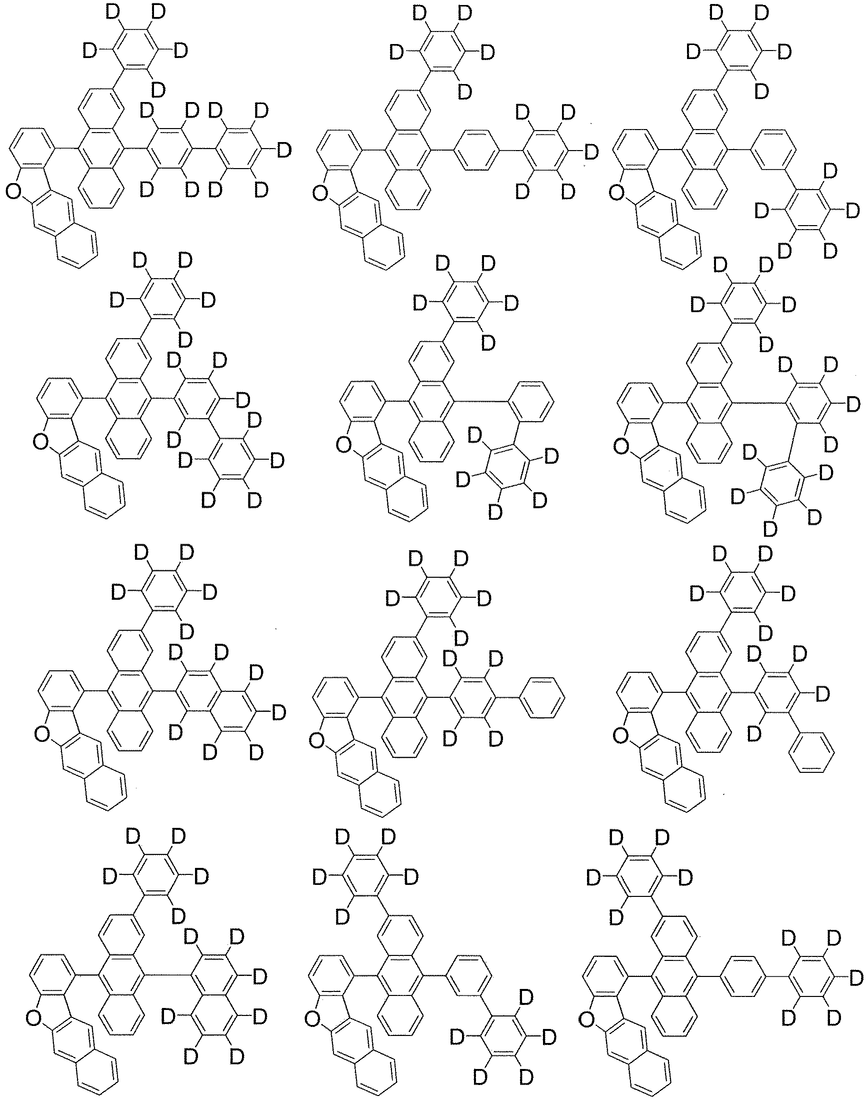

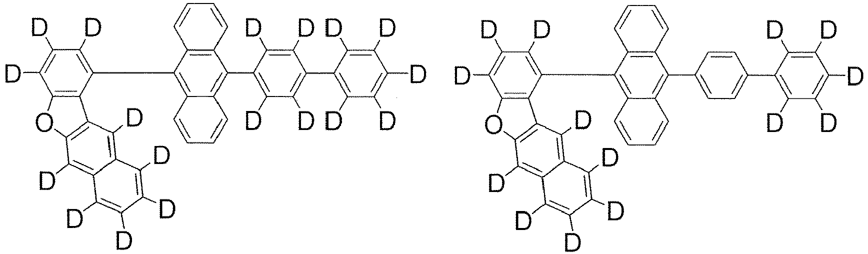

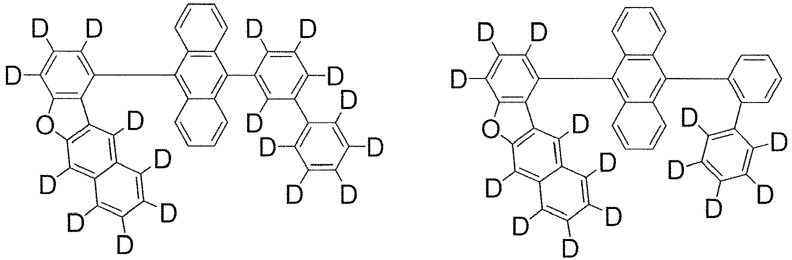

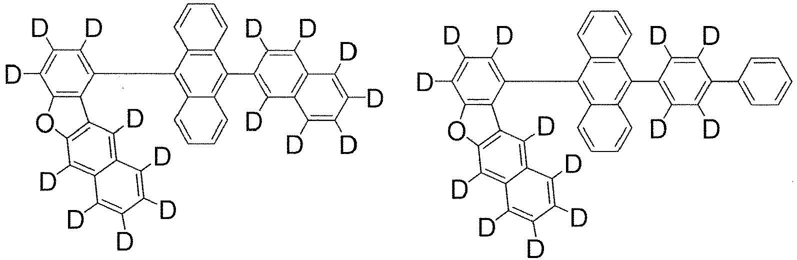

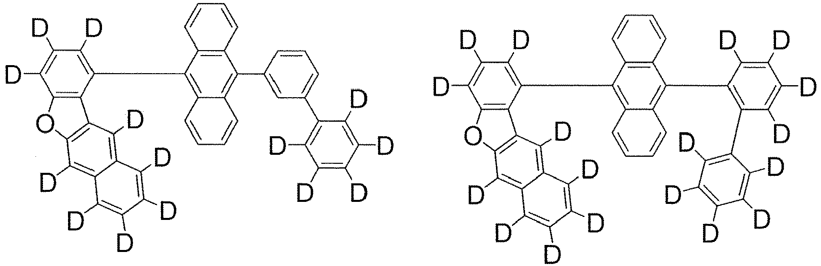

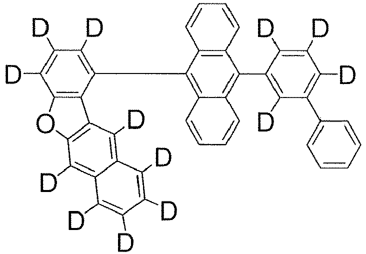

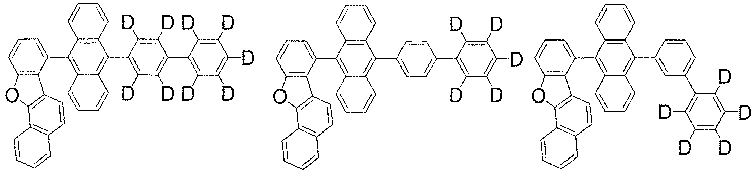

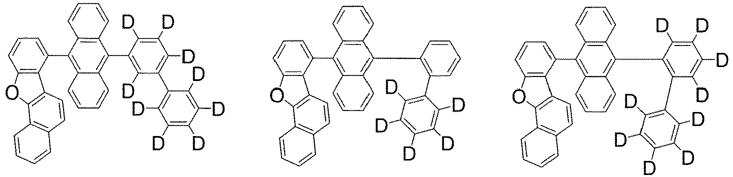

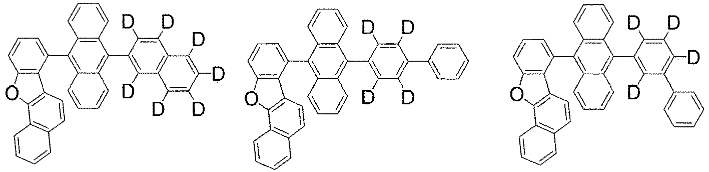

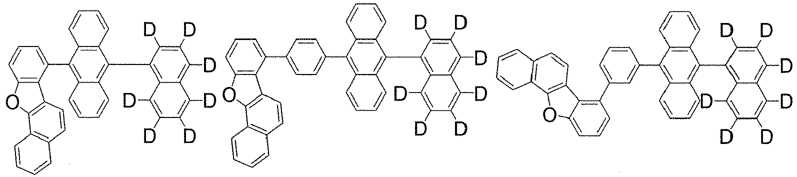

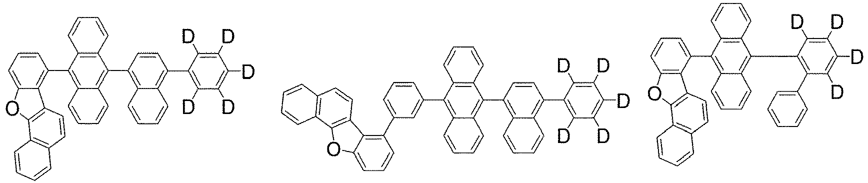

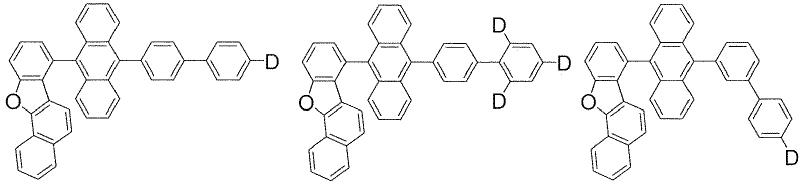

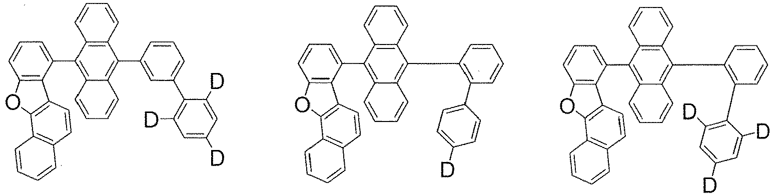

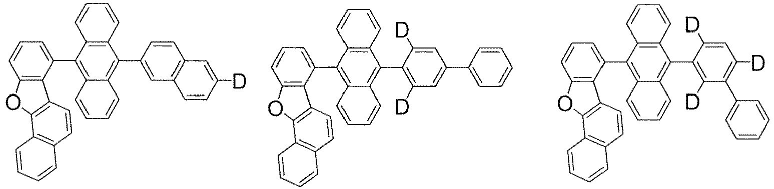

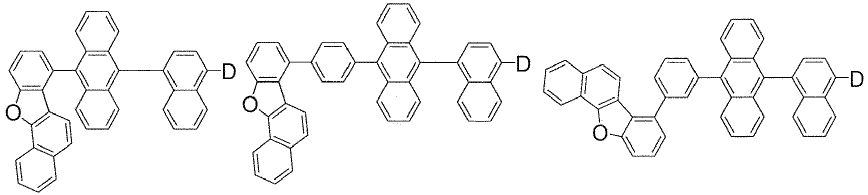

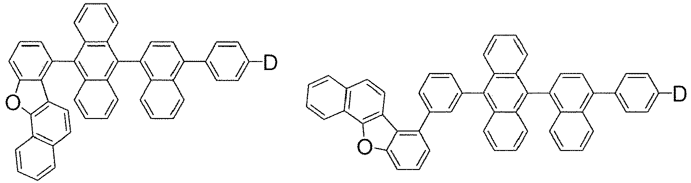

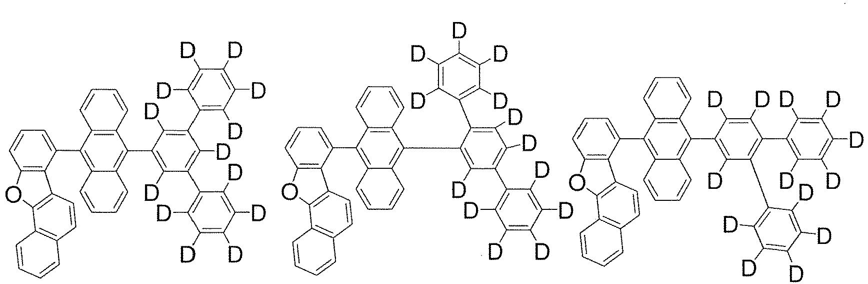

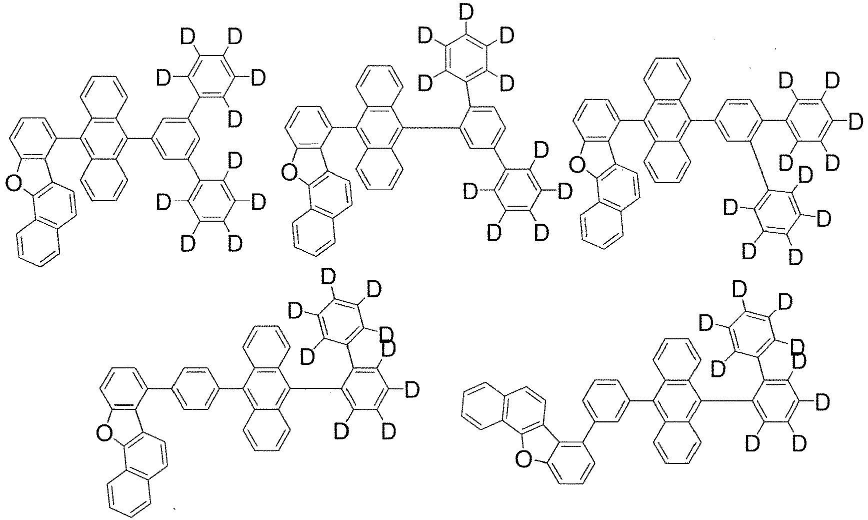

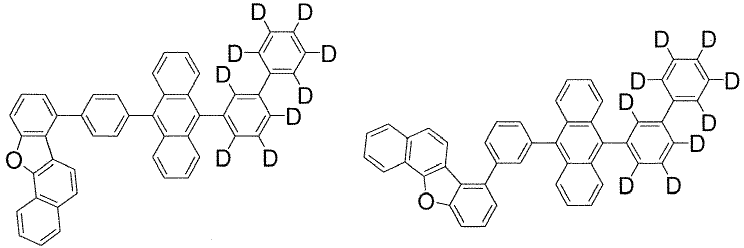

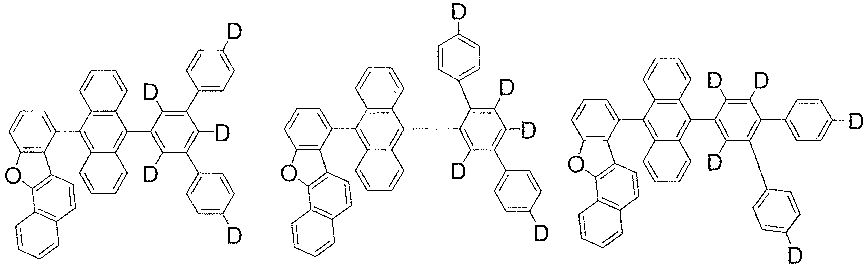

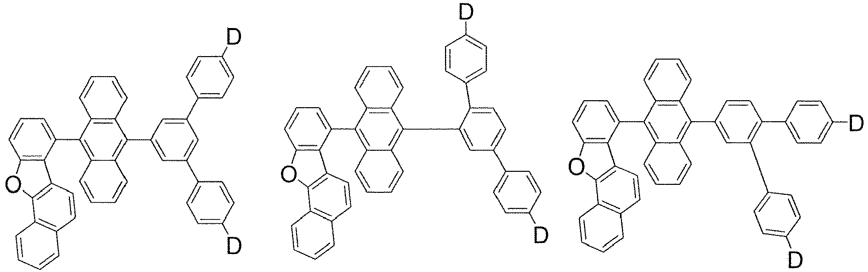

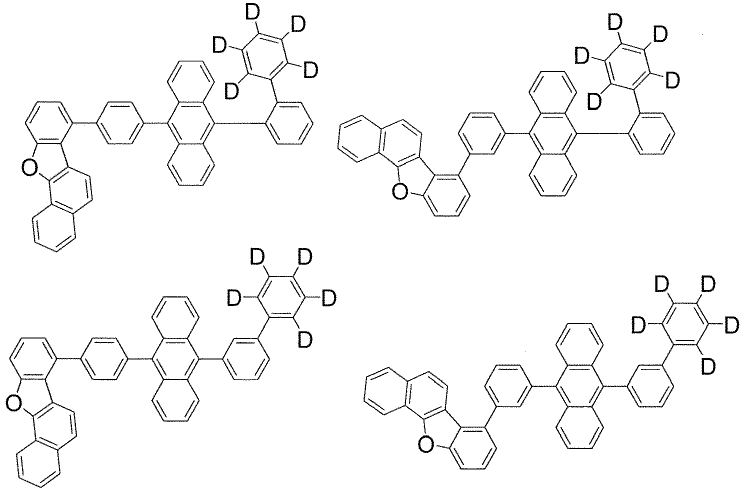

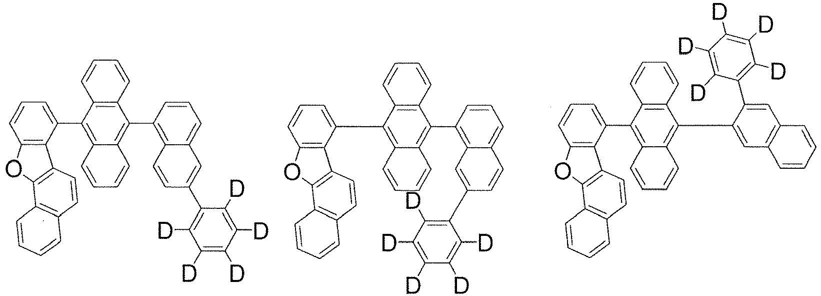

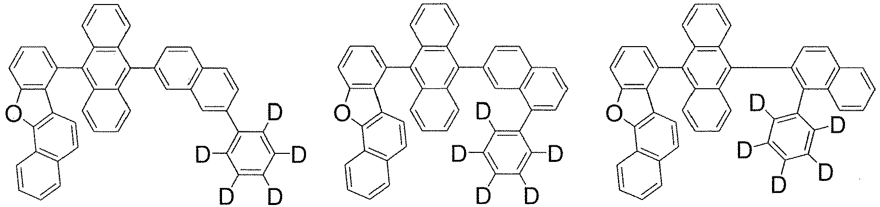

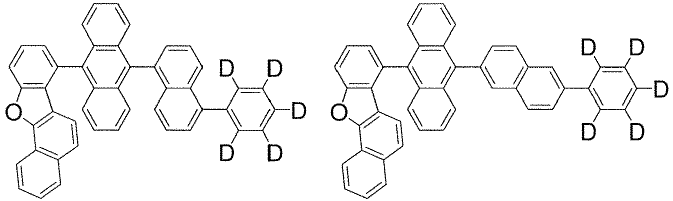

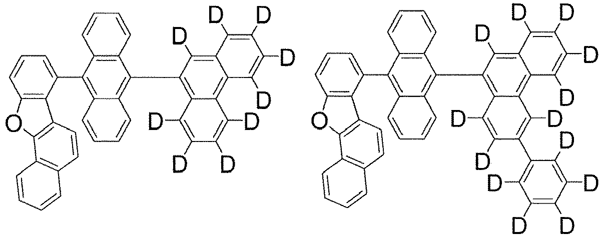

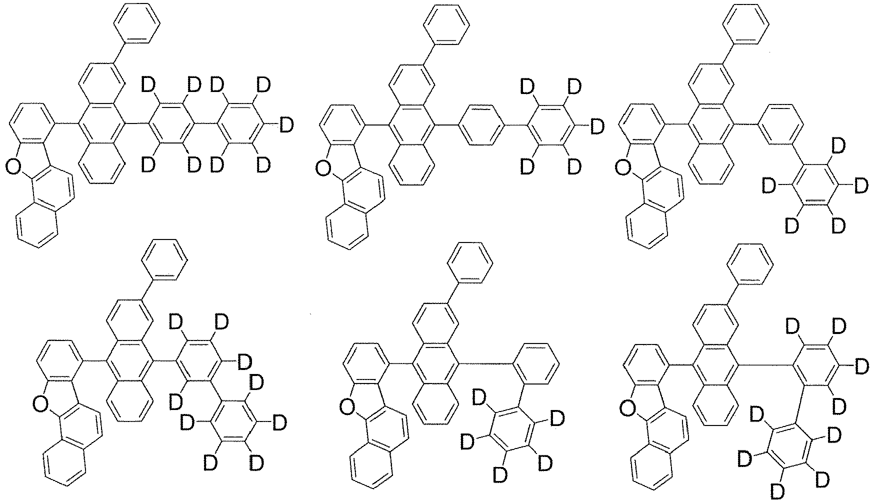

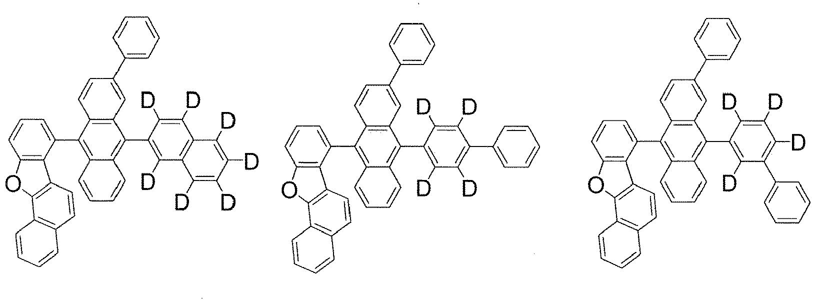

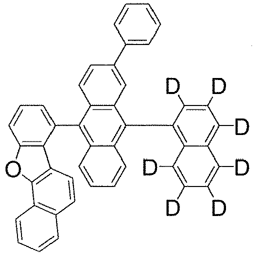

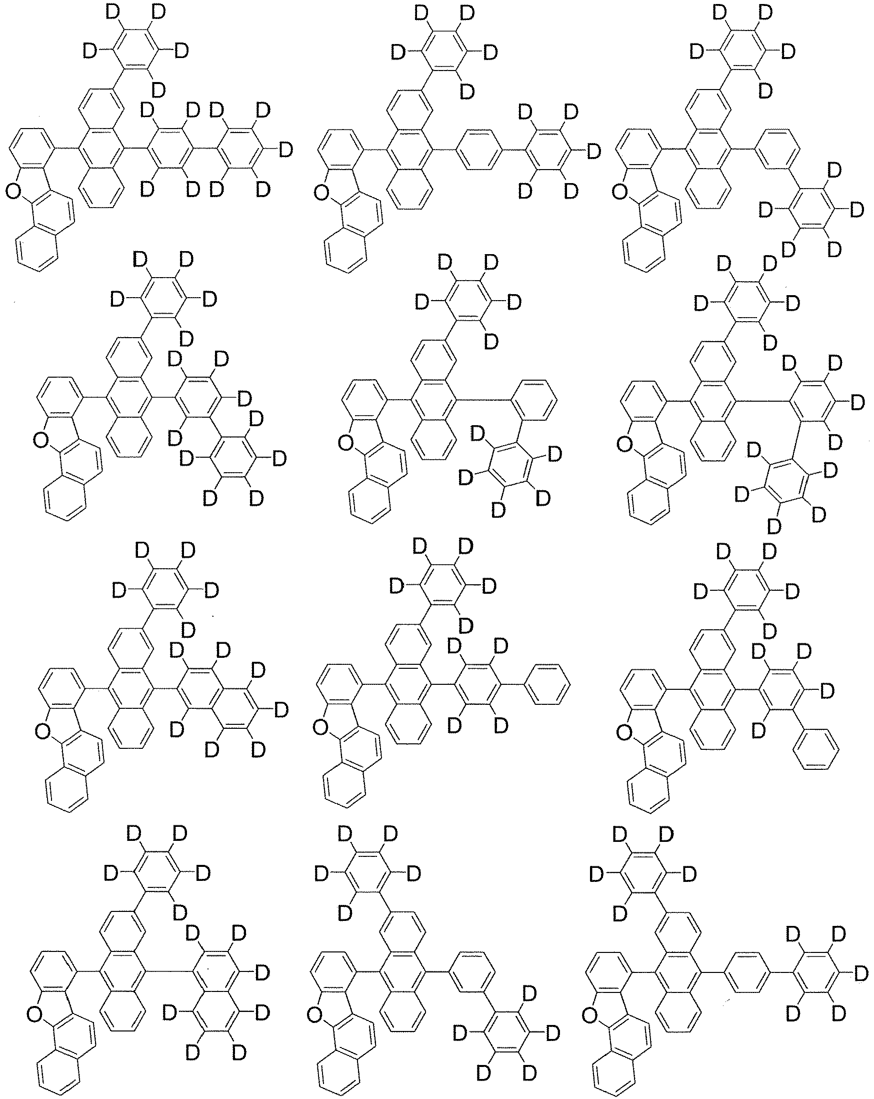

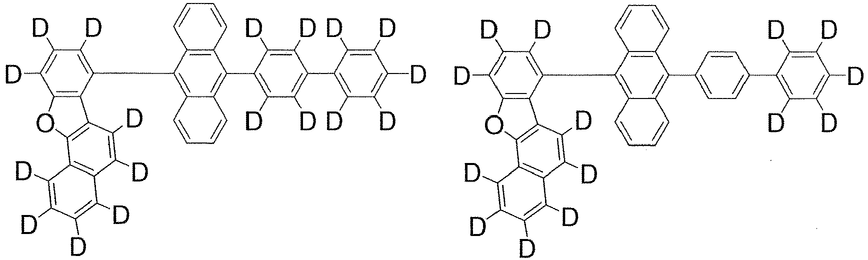

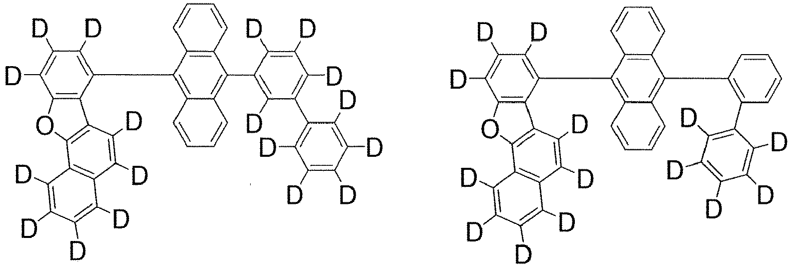

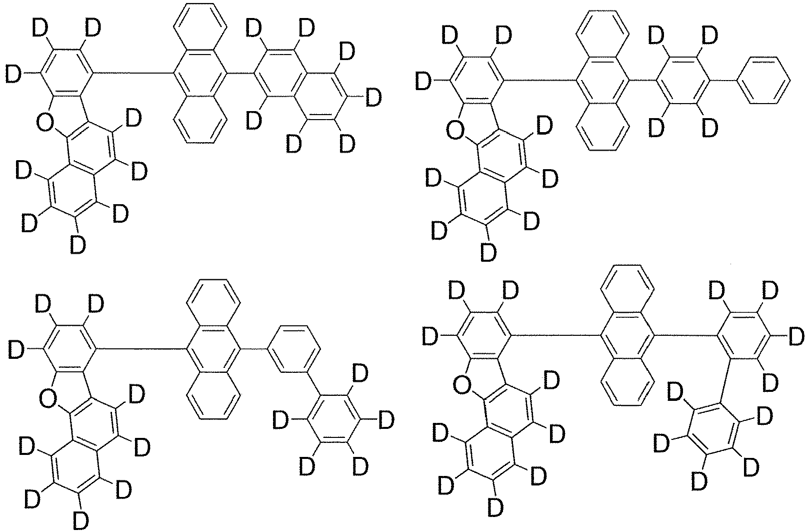

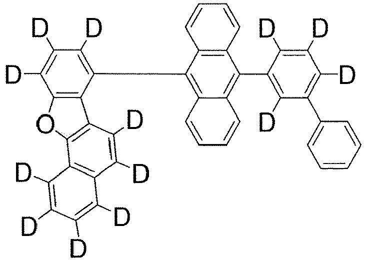

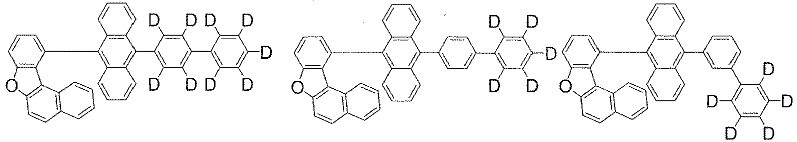

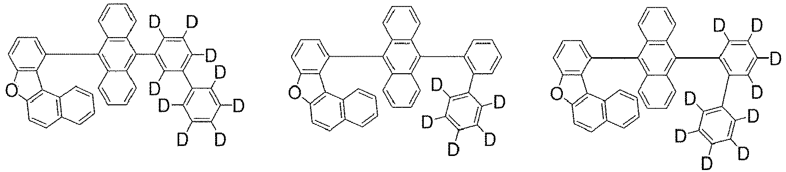

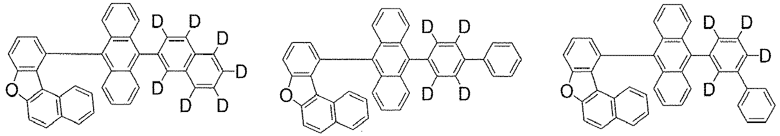

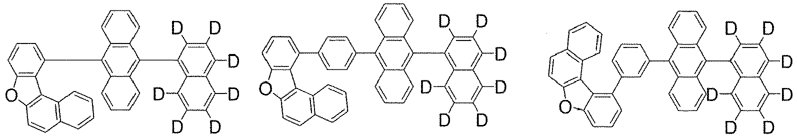

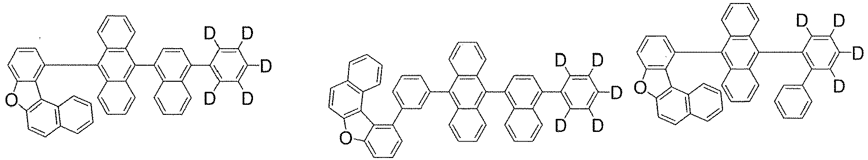

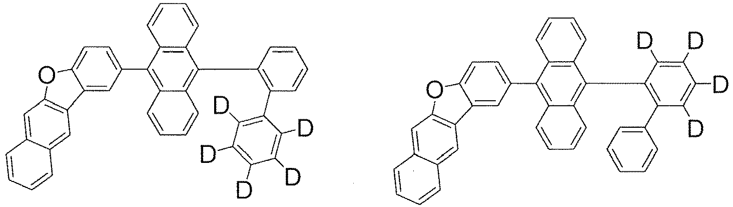

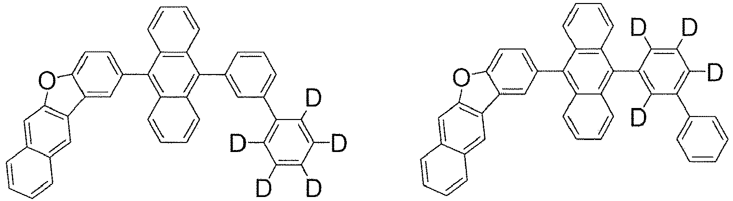

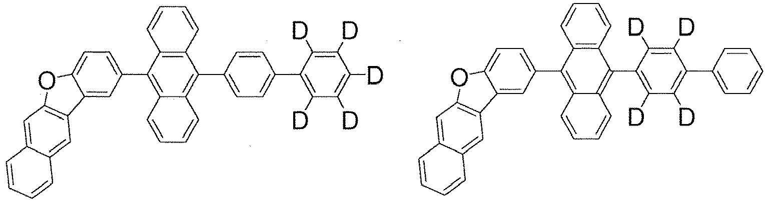

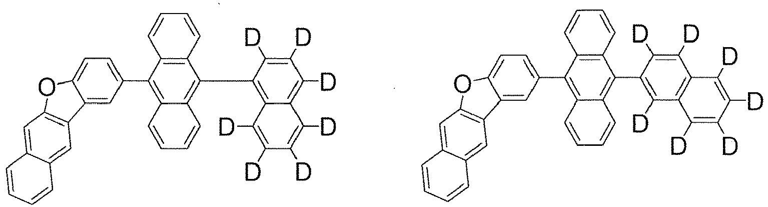

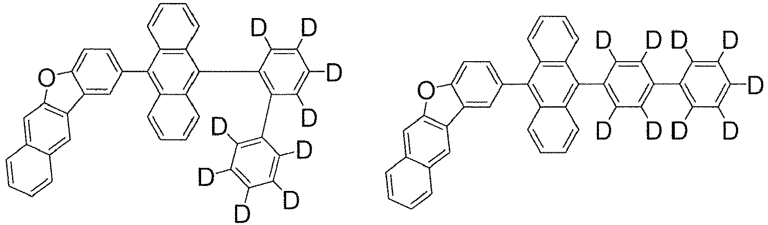

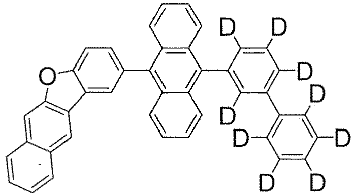

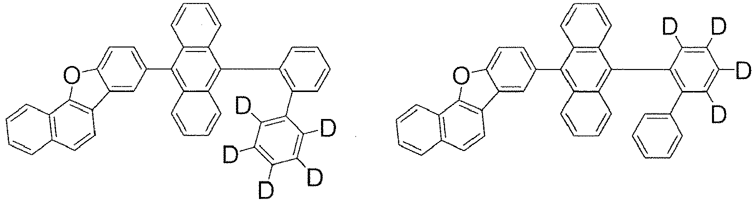

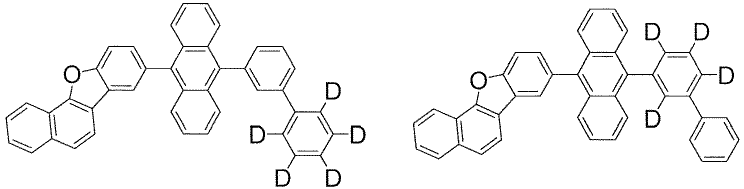

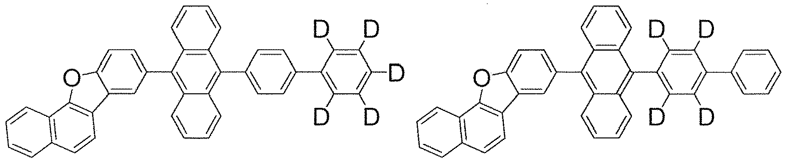

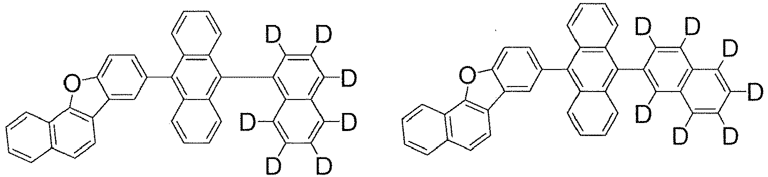

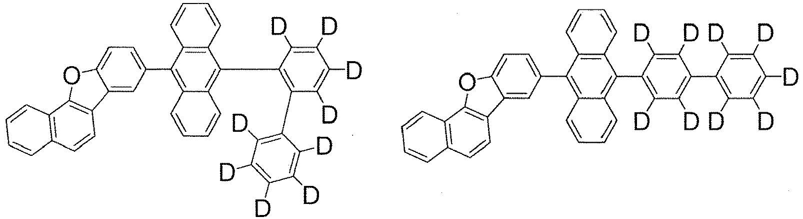

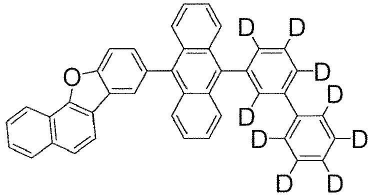

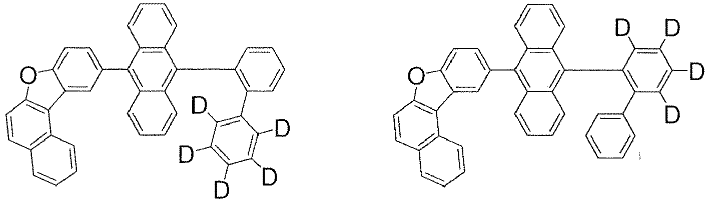

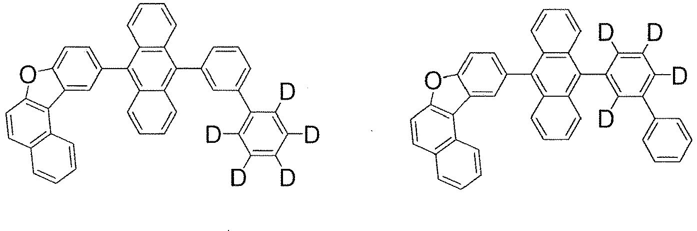

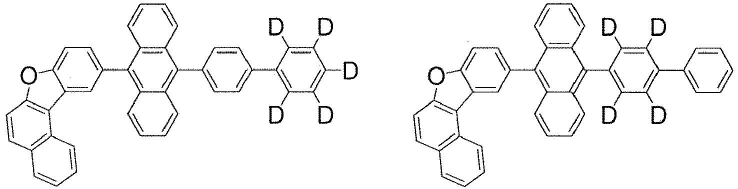

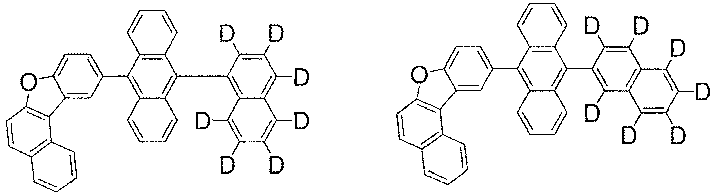

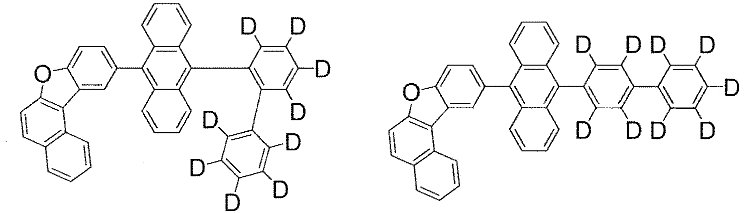

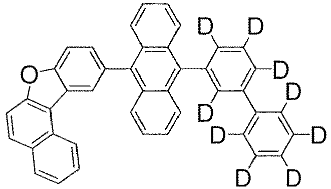

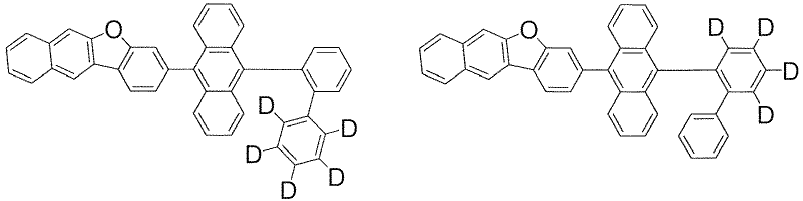

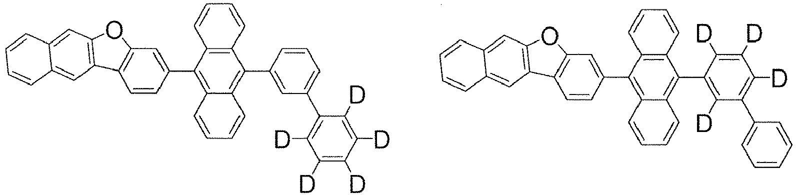

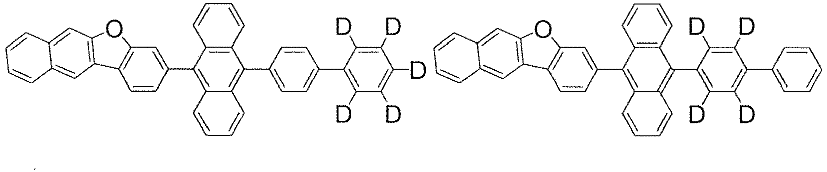

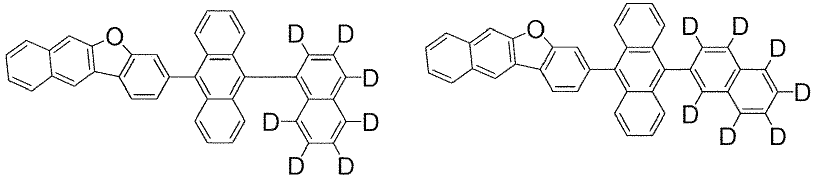

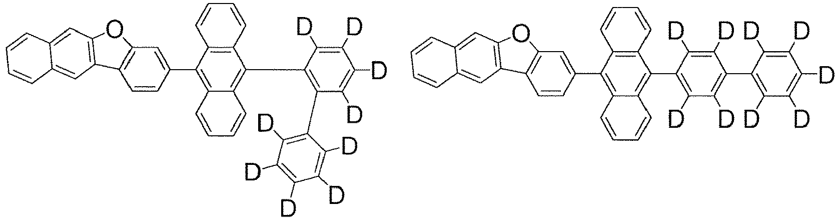

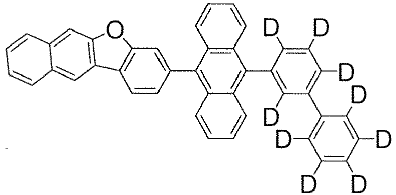

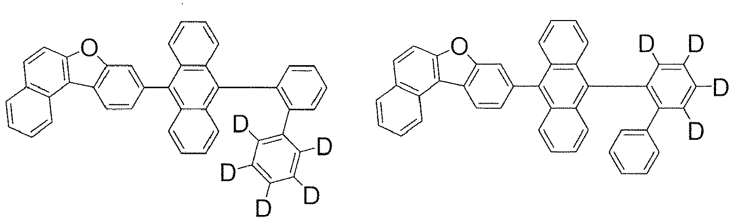

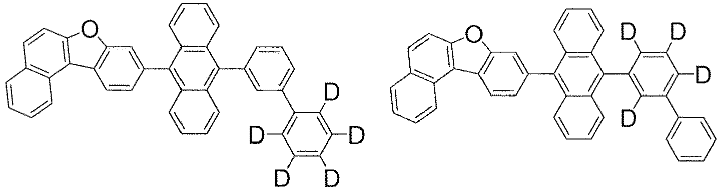

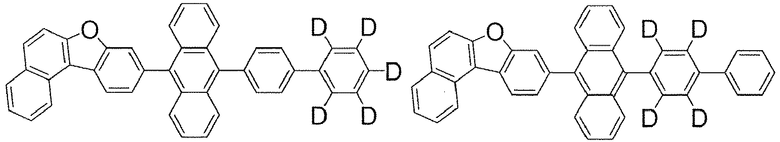

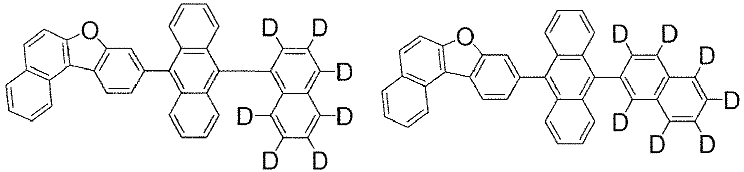

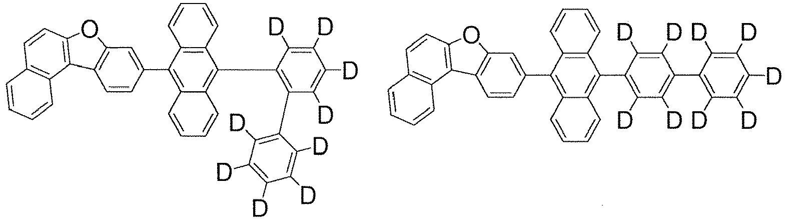

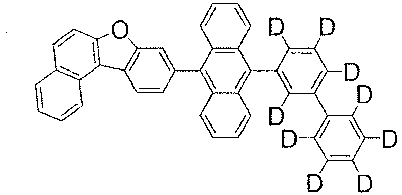

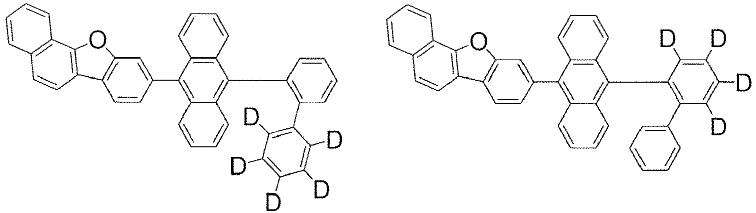

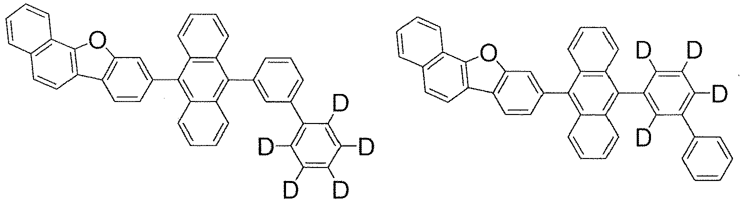

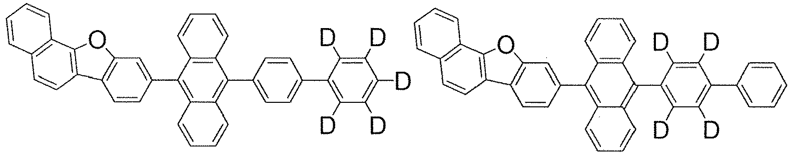

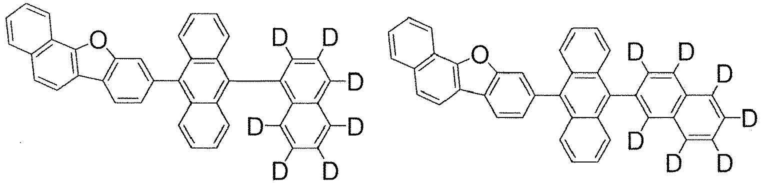

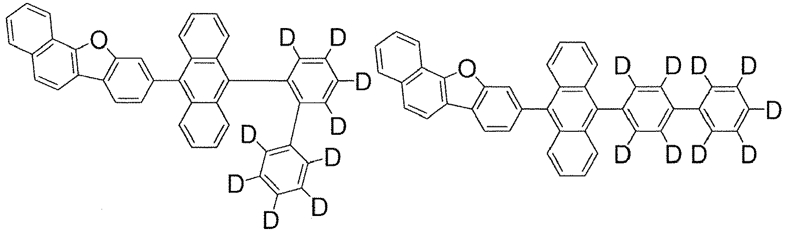

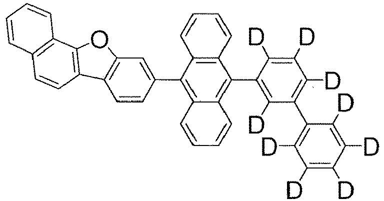

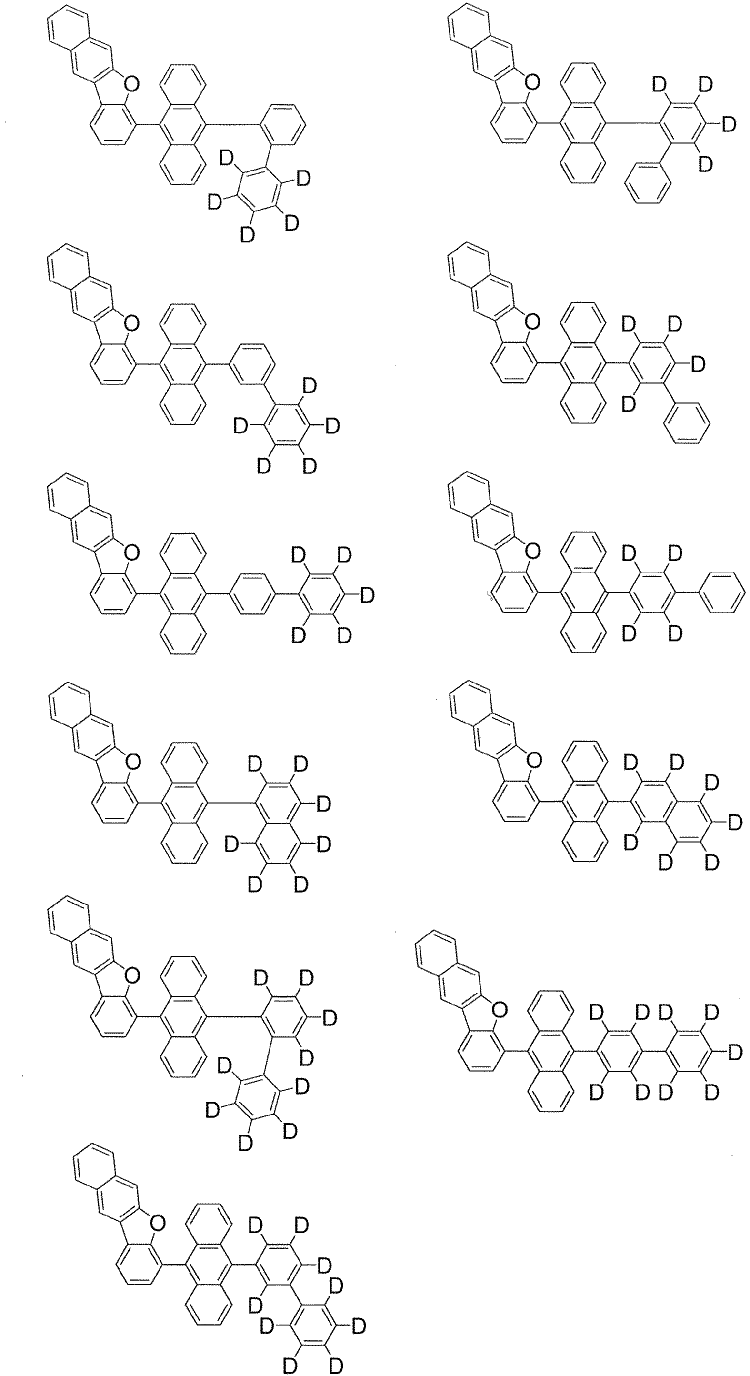

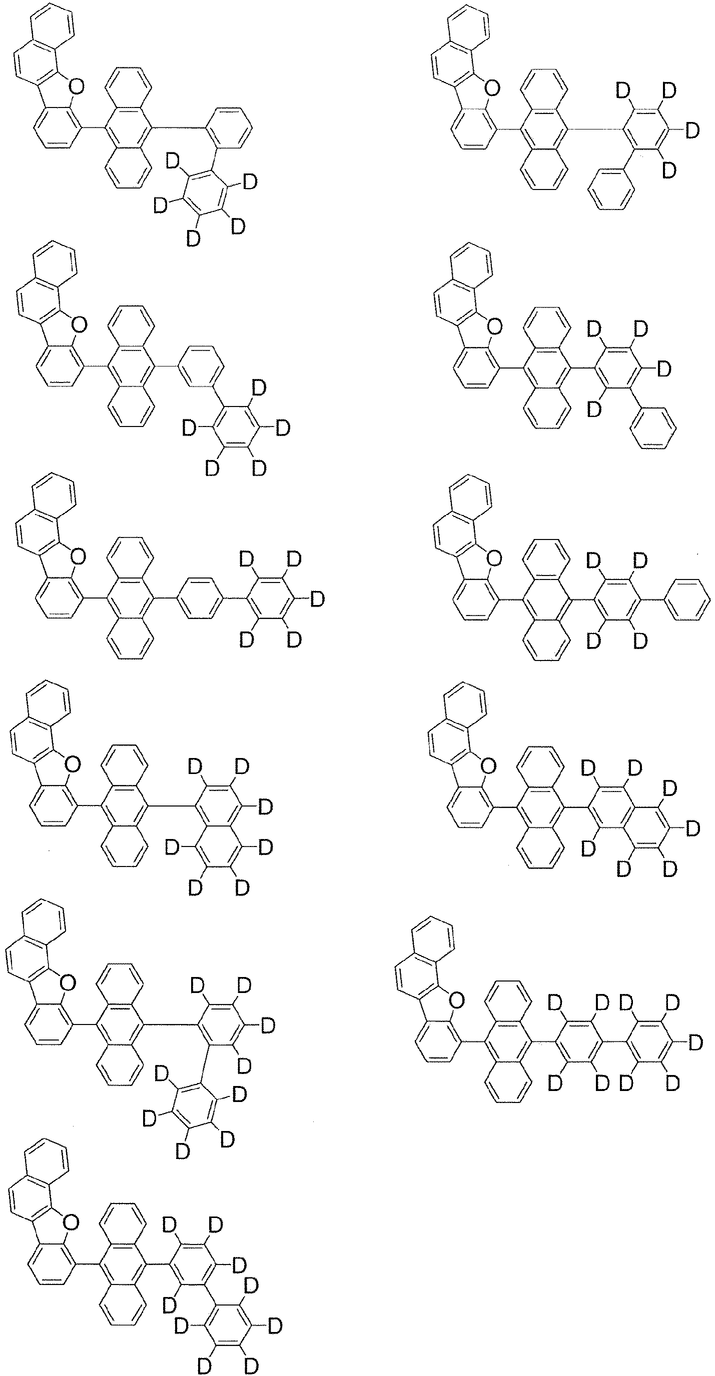

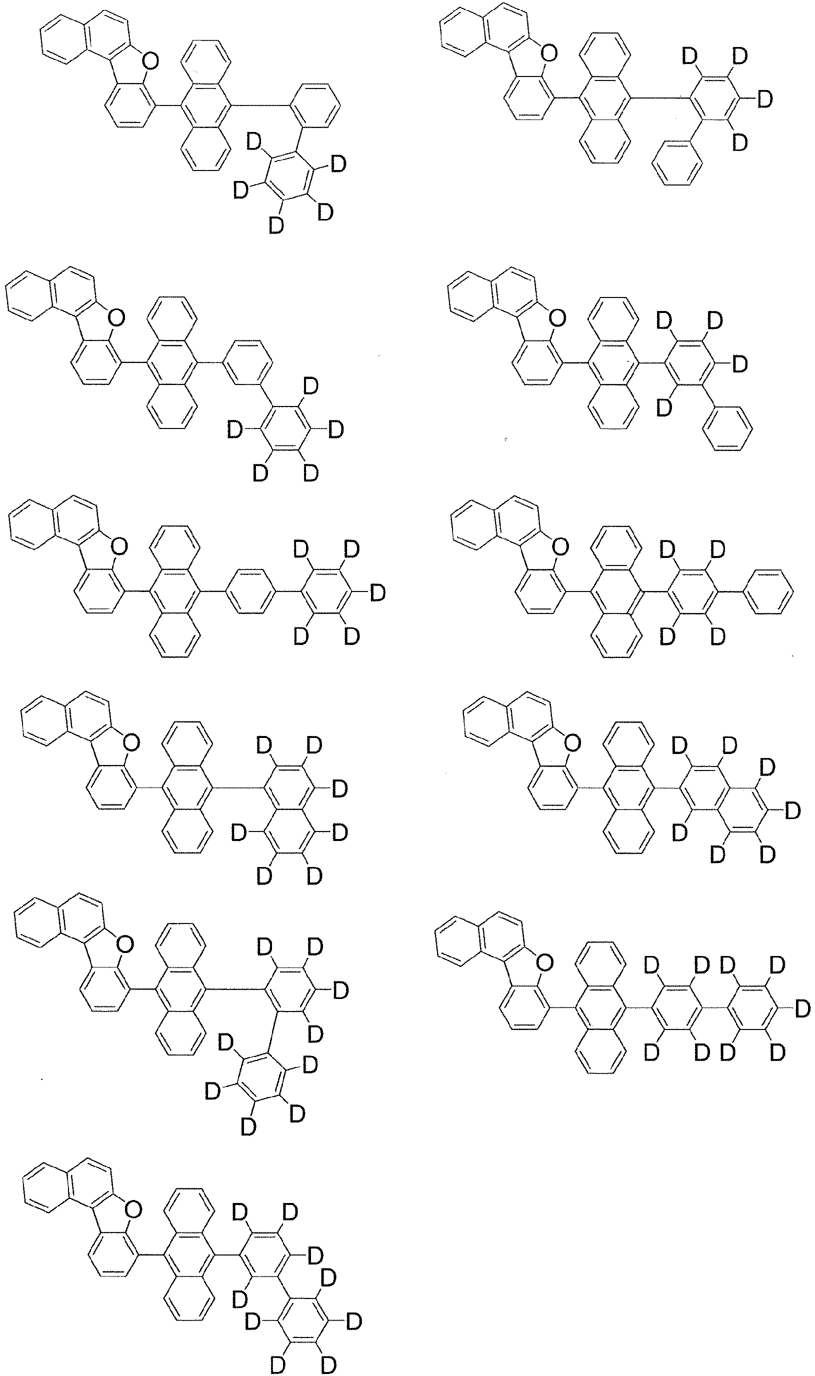

### [First organic electroluminescence device]

An organic EL device according to an aspect of the present invention includes a cathode; an anode; and one or more emitting layers arranged between the cathode and the anode, wherein at least one layer of the one or more emitting layers includes a compound represented by the formula (1A) described later, and a compound represented by the formula (41) described later.

### [Second organic electroluminescence device]

An organic EL device according to an aspect of the present invention includes a cathode; an anode; and one or more emitting layers arranged between the cathode and the anode, wherein at least one layer of the one or more emitting layers includes a compound represented by the formula (1B) described later, and a compound represented by the formula (41) described later.

When the organic EL device according to an aspect of the present invention includes the configurations described above, the lifetime of the device can be improved.

Each compound will be described in detail below.

### [Compound represented by formula (1A)]

A compound represented by the formula (1A) is shown as follows: 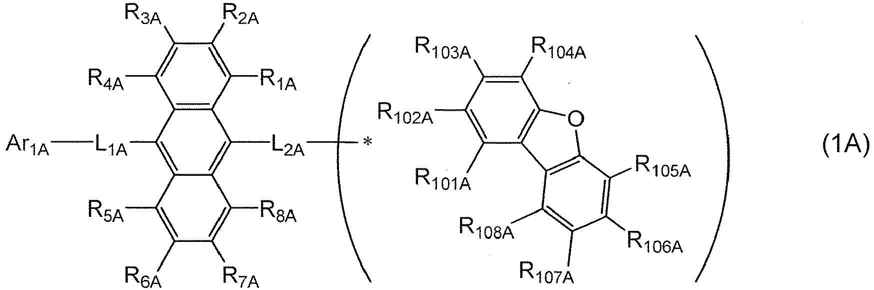 wherein in the formula (1A),
R_{1A} to R_{8A} are independently a hydrogen atom, or a substituent R;
L_{1A} and L_{2A} are independently
a single bond,
a substituted or unsubstituted arylene group having 6 to 18 ring carbon atoms, or
a substituted or unsubstituted divalent heterocyclic group having 5 to 18 ring atoms;
Ar_{1A} is a substituted or unsubstituted phenyl group,
a substituted or unsubstituted p-biphenyl group,
a substituted or unsubstituted m-biphenyl group,
a substituted or unsubstituted o-biphenyl group,
a substituted or unsubstituted 1-naphthyl group,
a substituted or unsubstituted 2-naphthyl group, or
a substituted or unsubstituted phenanthryl group;
one of R_{101A} to R_{104A} is bonded with L_{2A} via a single bond;
R_{101A} to R_{104A} which are not bonded with L_{2A} are independently
a hydrogen atom,
a substituted or unsubstituted aryl group having 6 to 12 ring carbon atoms, or
a substituted or unsubstituted monovalent heterocyclic group having 5 to 12 ring atoms;
R_{105A} to R_{108A} are hydrogen atoms;
the substituent R is selected from the group consisting of
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
-Si(R₉₀₁)(R₉₀₂)(R₉₀₃),
-O-(R₉₀₄),
-S-(R₉₀₅),
-N(R₉₀₆)(R₉₀₇)
(wherein R₉₀₁ to R₉₀₇ are independently
a hydrogen atom,
a substituted or unsubstituted alkyl group having 1 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
a halogen atom, a cyano group, a nitro group,
a substituted or unsubstituted aryl group having 6 to 50 ring carbon atoms, and
a substituted or unsubstituted monovalent heterocyclic group having 5 to 50 ring atoms;
provided that the compound represented by the formula (1A) satisfies the following conditions A1 and A2;
condition A1: R_{1A} to R_{8A} which are hydrogen atoms are not a deuterium atom; and
condition A2: one or more of, hydrogen atoms possessed by L_{1A}, L_{2A} and Ar_{1A}; hydrogen atoms possessed by the substituent in the case where L_{1A}, L_{2A} and Ar_{1A} are substituted; hydrogen atoms possessed by R_{1A} to R_{8A} which are the substituents R; R_{101A} to R_{108A} which are hydrogen atoms; and hydrogen atoms possessed by R_{101A} to R_{104A} which are a substituted or unsubstituted aryl group having 6 to 12 ring carbon atoms, or a substituted or unsubstituted monovalent heterocyclic group having 5 to 12 ring atoms are a deuterium atom.

When L_{1A} is a single bond in the formula (1A), Ar_{1A} is directly bonded with the anthracene skeleton.

When L_{2A} is a single bond in the formula (1A), the structure in the above parentheses is directly bonded with the anthracene skeleton at the bondable position of the structure (one of R_{101A} to R_{104A}).

In one embodiment, the compound represented by the formula (1A) is a compound represented by the following formula (1A-1): 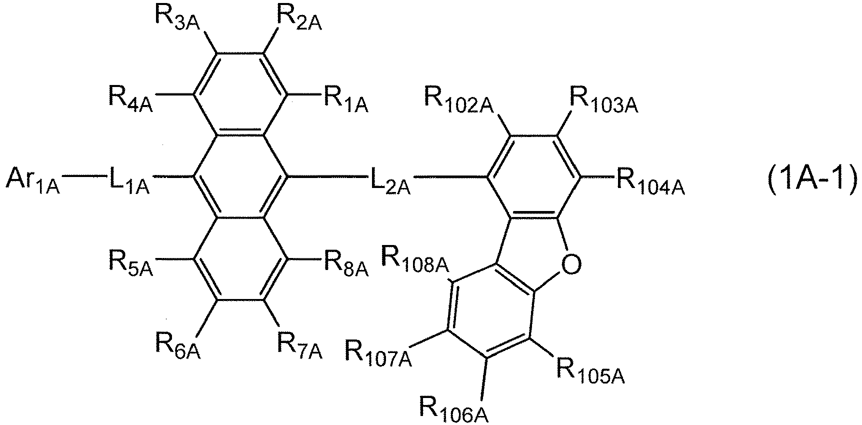 wherein in the formula (1A-1), R_{1A} to R_{8A}, R_{102A} to R_{108A}, L_{1A}, L_{2A} and Ar_{1A} are the same as defined in the formula (1).

In one embodiment, the compound represented by the formula (1A) is a compound represented by any one of the following formulas (1A-11) to (1A-14): 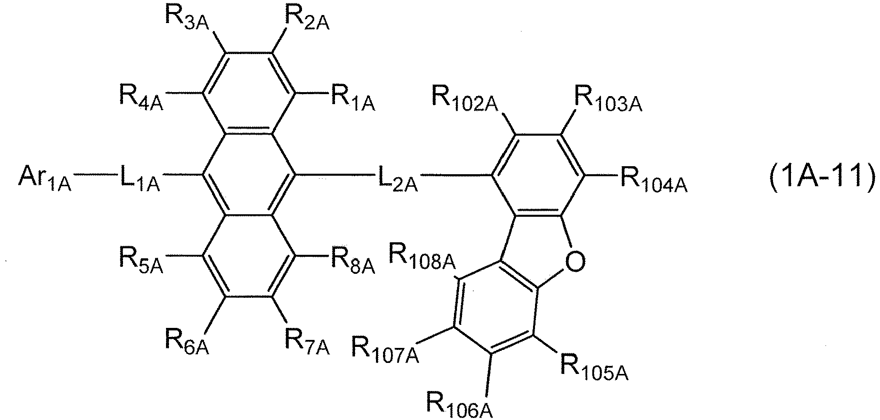 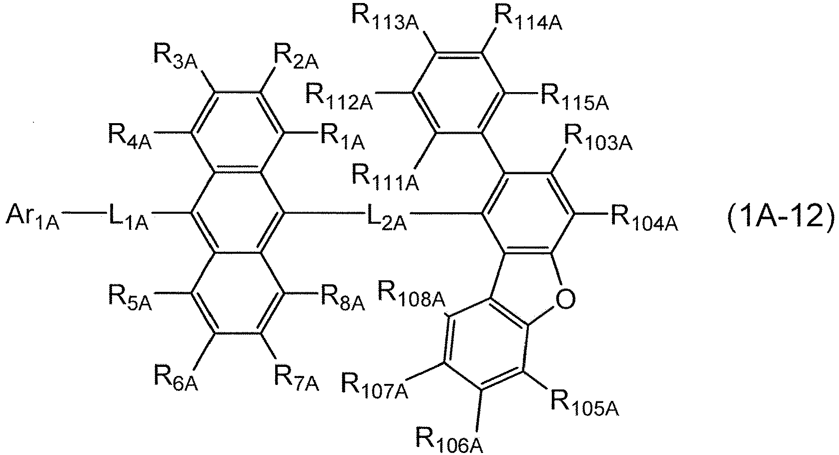 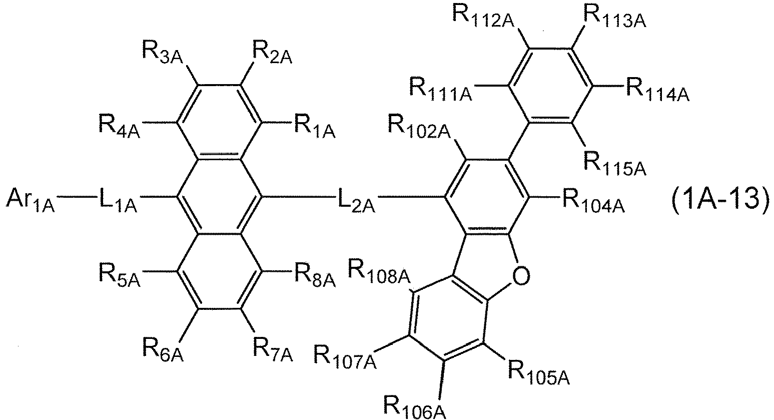 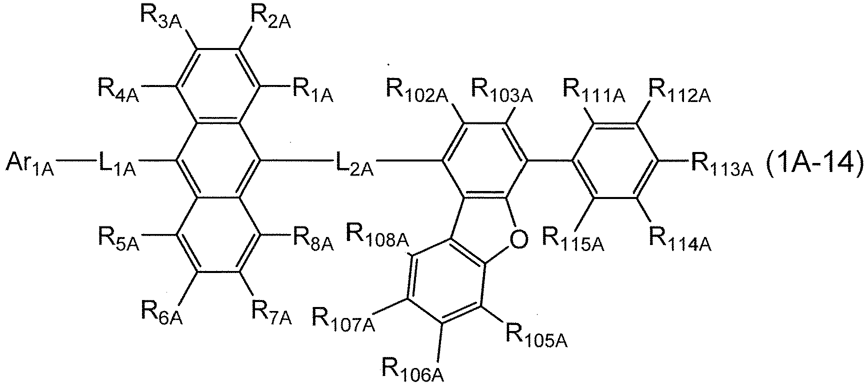 wherein in the formulas (1A-11) to (1A-14), R_{1A} to R_{8A}, R_{105A} to R_{108A}, L_{1A}, L_{2A} and Ar_{1A} are the same as defined in the formula (1A);
in the formula (1A-11), R_{102A} to R_{104A} are a hydrogen atom;
in the formula (1A-12), R_{103A}, R_{104A} and R_{111A} to R_{115A} are a hydrogen atom;
in the formula (1A-13), R_{102A}, R_{104A} and R_{111A} to R_{115A} are a hydrogen atom; and
in the formula (1A-14), R_{102A}, R_{103A} and R_{111A} to R_{115A} are a hydrogen atom.

In one embodiment, L_{1A} is
a substituted or unsubstituted phenylene group, or
a substituted or unsubstituted naphthylene group; and
one or more of hydrogen atoms possessed by L_{1A} are a deuterium atom.

In one embodiment, the compounds represented by the formulas (1A-11) to (1A-14) are a compound represented by any one of the following formulas (1A-21) to (1A-24): 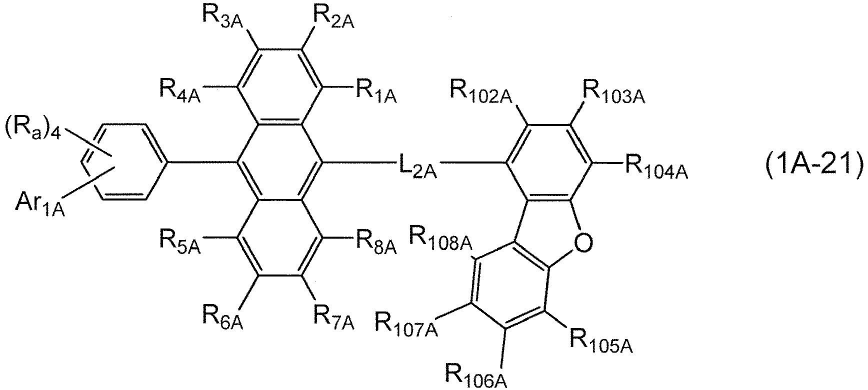 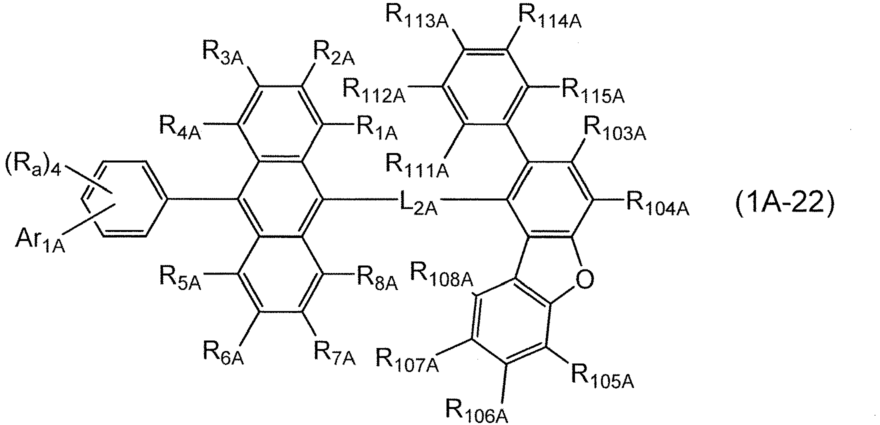 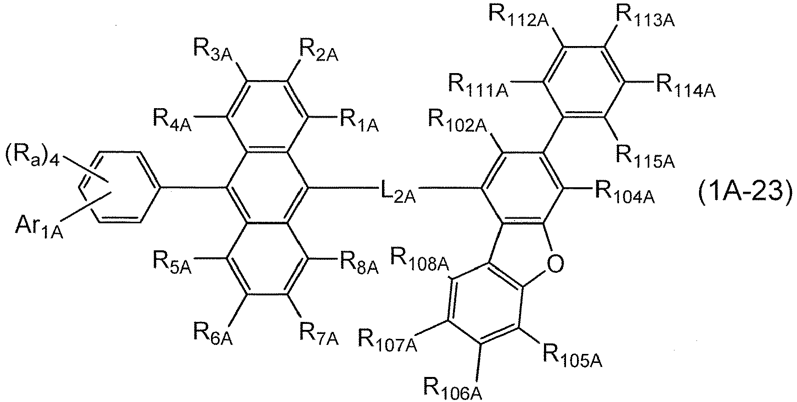 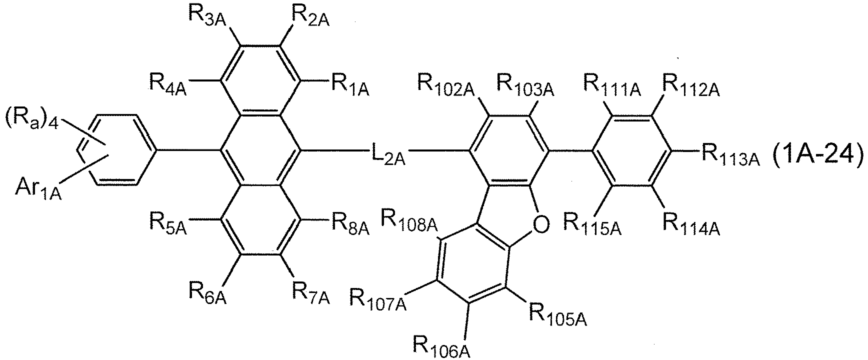 wherein in the formulas (1A-21) to (1A-24), R_{1A} to R_{8A}, R_{102A} to R_{108A}, R_{111A} to R_{115A}, L_{2A} and Ar_{1A} are the same as defined in the formulas (1A-11) to (1A-14);
four Rₐ's are independently a hydrogen atom, or a substituent R;
provided that one or more of the four Rₐ's are a deuterium atom; and
the substituent R is the same as defined in the formula (1A).

In one embodiment, hydrogen atoms possessed by Ar_{1A} are not a deuterium atom.

It is preferable in terms of enhancing improving effects of device lifetime in the formula (1A) that a compound in which one or more of hydrogen atoms possessed by L_{1A} are a deuterium atom and hydrogen atoms possessed by Ar_{1A} are not a deuterium atom be used.

It will be specifically described below using a compound in which all of deuterium atoms in the formula (1A) are replaced with a protium atom (hereinafter, frequently referred to as "protium compound"), a compound having the same structure as the protium compound, except that one or more of hydrogen atoms possessed by L_{1A} are a deuterium atom (hereinafter, frequently referred to as "deuterium compound a"), and a compound having the same structure as the protium compound, except that one or more of hydrogen atoms possessed by Ar_{1A} are a deuterium atom (hereinafter, frequently referred to as "deuterium compound β") as an example. Examples of the "protium compound", the "deuterium compound a" and the "deuterium compound β" include the following compounds.

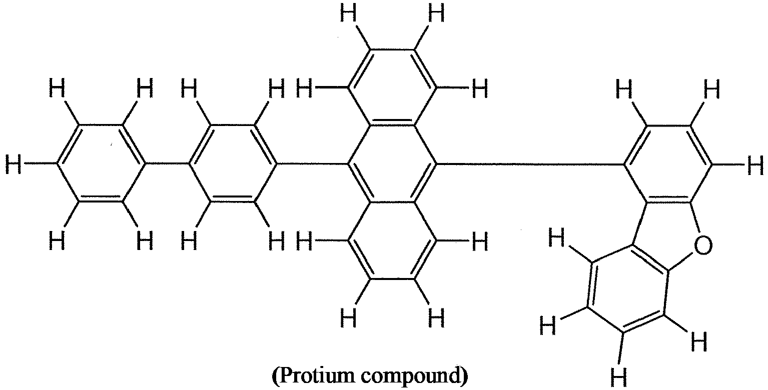

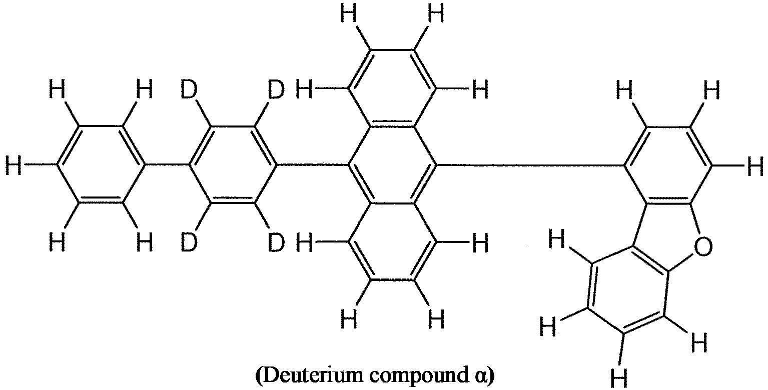

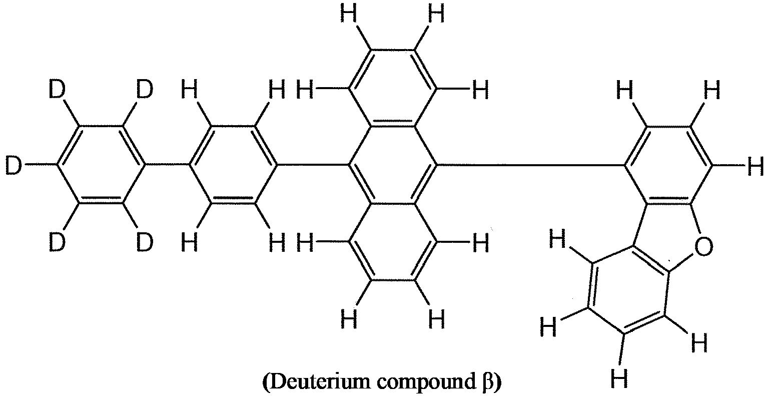

When increasing rates of the lifetimes of, a device in which the deuterium compound a is used, and a device in which the deuterium compound β is used is used, is compared based on the lifetime of a device in which the protium compound is used, the device in which the deuterium compound a is used has larger increasing rate of the lifetime. That is, the case where the deuterium compound a is used is preferable as compared to the case where the deuterium compound β is used, since improving effects of device lifetime are enhanced.

In one embodiment, the compound represented by the formula (1A) is the compound represented by the formula (1A-13).

In one embodiment, a substituent in the case of "substituted or unsubstituted" in the formula (1A) is selected from the group consisting of
an alkyl group having 1 to 50 carbon atoms,
a haloalkyl group having 1 to 50 carbon atoms,
an alkenyl group having 2 to 50 carbon atoms,
an alkynyl group having 2 to 50 carbon atoms,
a cycloalkyl group having 3 to 50 ring carbon atoms,
an alkoxy group having 1 to 50 carbon atoms,
an alkylthio group having 1 to 50 carbon atoms,
an aryloxy group having 6 to 50 ring carbon atoms,
an arylthio group having 6 to 50 ring carbon atoms,
an aralkyl group having 7 to 50 carbon atoms,
-Si(R₄₁)(R₄₂)(R₄₃),
-C(=O)R₄₄, -COOR₄₅,
-S(=O)₂R₄₆,
-P(=O)(R₄₇)(R₄₈),
-Ge(R₄₉)(R₅₀)(R₅₁),
-N(R₅₂)(R₅₃) (wherein, R₄₁ to R₅₃ are independently a hydrogen atom, an alkyl group having 1 to 50 carbon atoms, an aryl group having 6 to 50 ring carbon atoms, or a monovalent heterocyclic group having 5 to 50 ring atoms; when two or more of each of R₄₁ to R₅₃ are present, the two or more of each of R₄₁ to R₅₃ may be the same as or different from each other),
a hydroxy group,
a halogen atom,
a cyano group,
a nitro group,
an aryl group having 6 to 50 ring carbon atoms, and
a monovalent heterocyclic group having 5 to 50 ring atoms.

In one embodiment, a substituent in the case of "substituted or unsubstituted" in the formula (1A) is selected from the group consisting of
an alkyl group having 1 to 18 carbon atoms,
an aryl group having 6 to 18 ring carbon atoms, and
a monovalent heterocyclic group having 5 to 18 ring atoms.

The compound represented by the formula (1A) can be synthesized by using known alternative reactions or raw materials adapted to the target compound.

Specific examples of the compound represented by the formula (1A) will be described below, but these are merely examples, and the compound represented by the formula (1A) is not limited to the following specific examples.

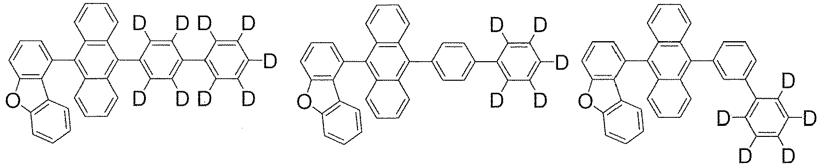

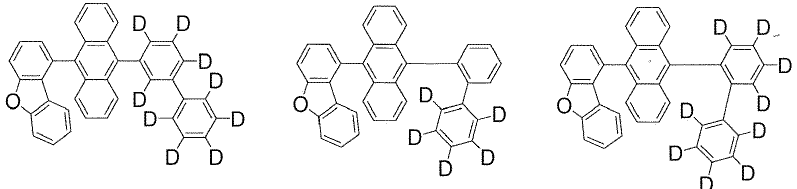

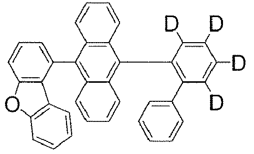

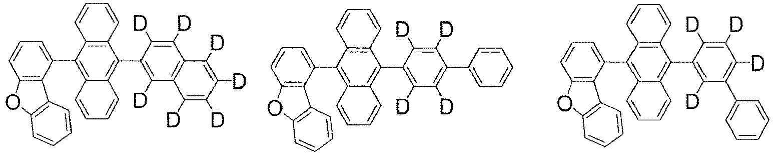

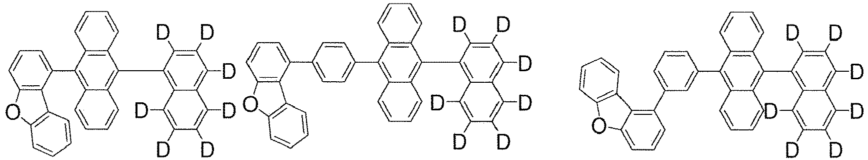

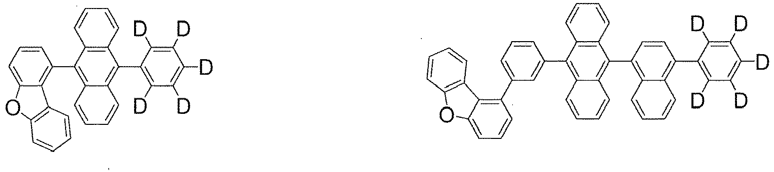

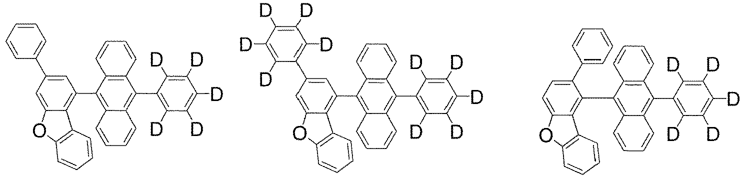

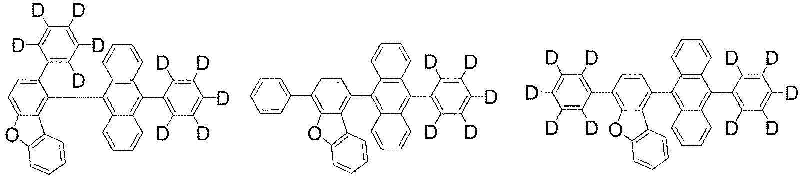

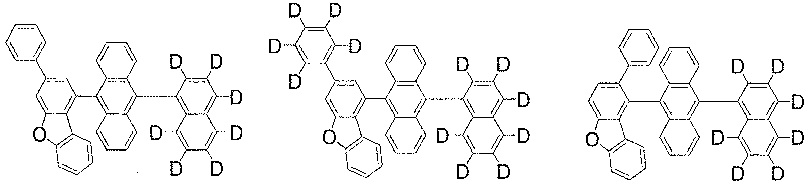

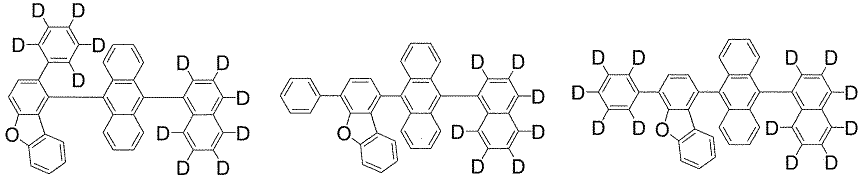

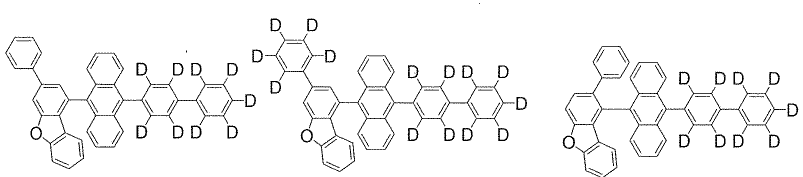

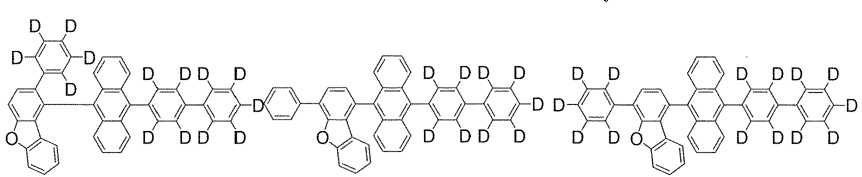

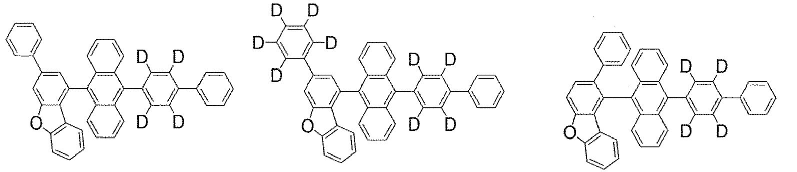

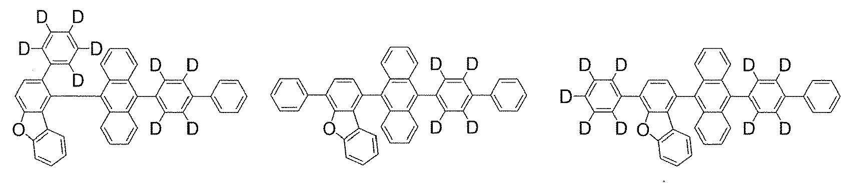

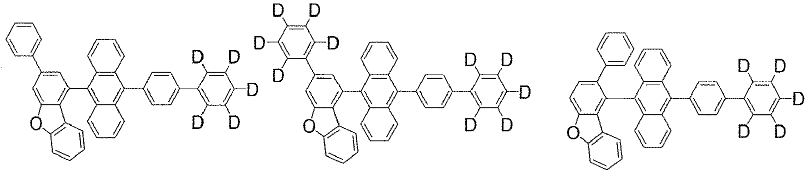

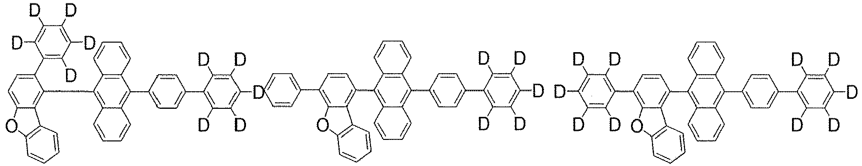

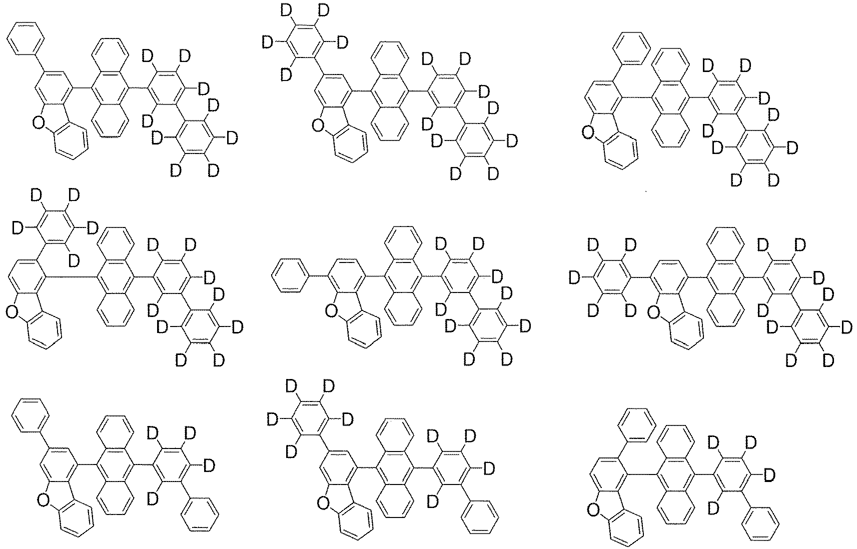

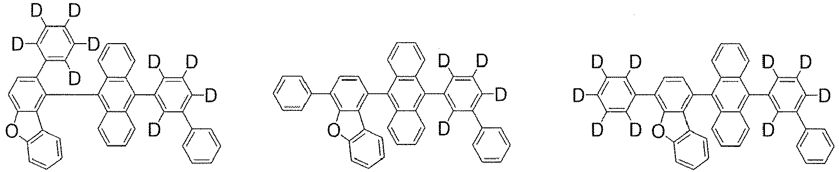

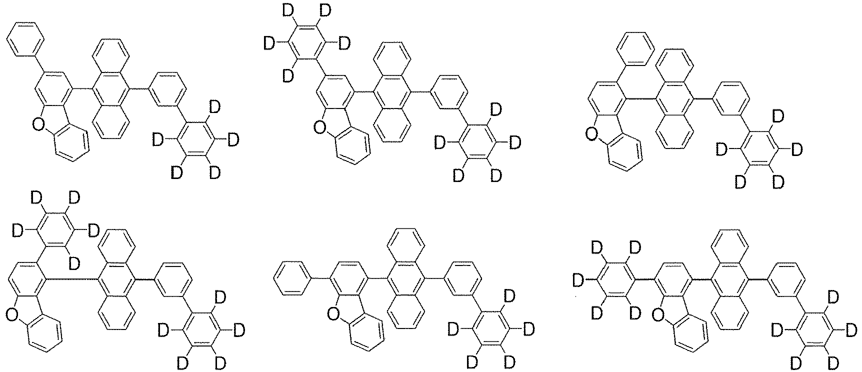

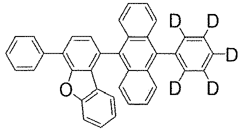

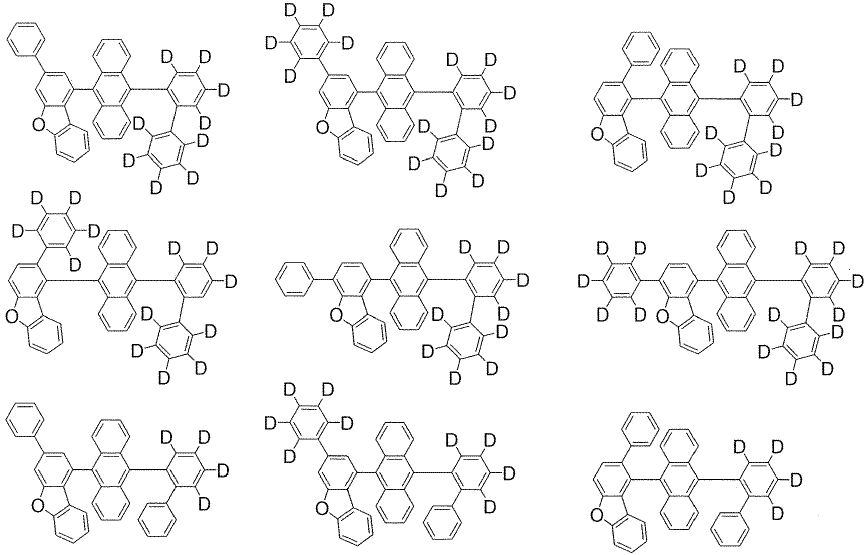

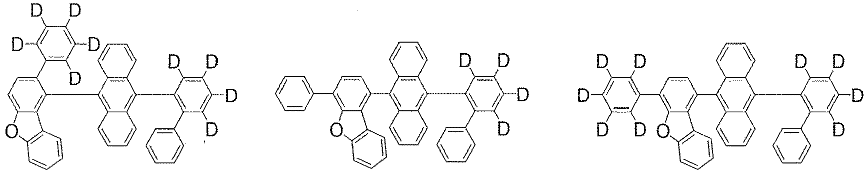

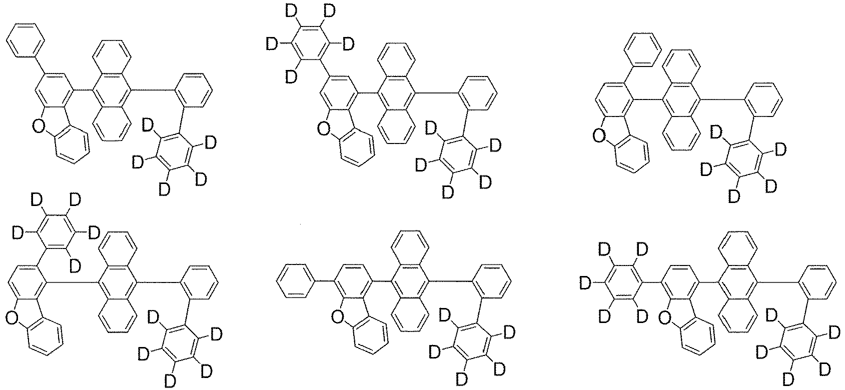

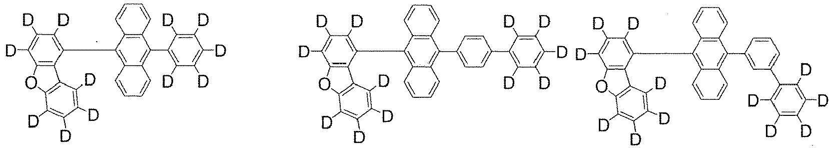

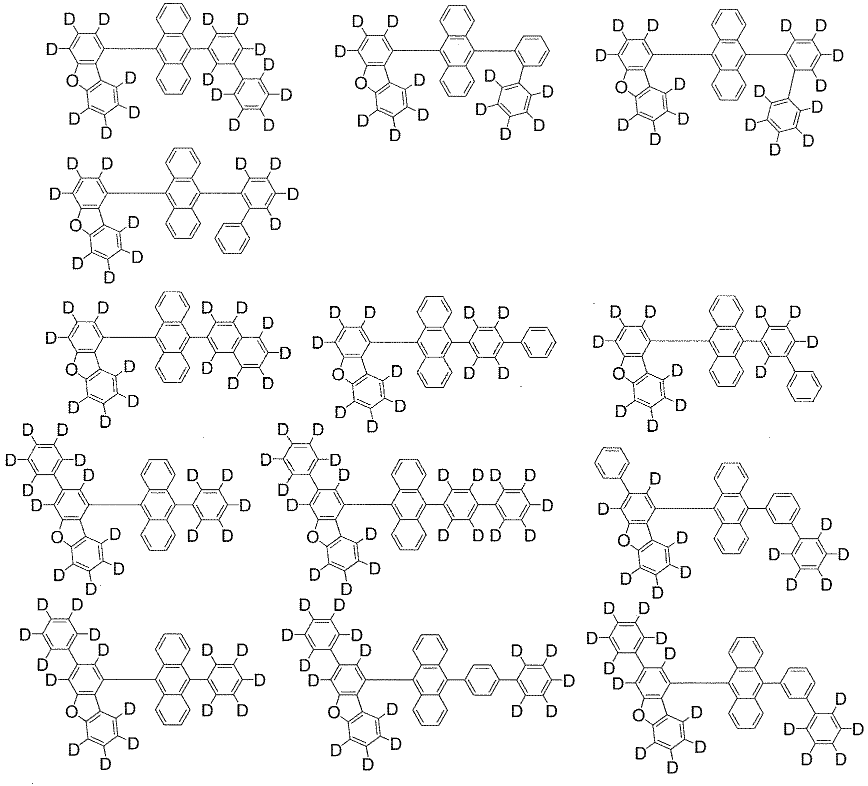

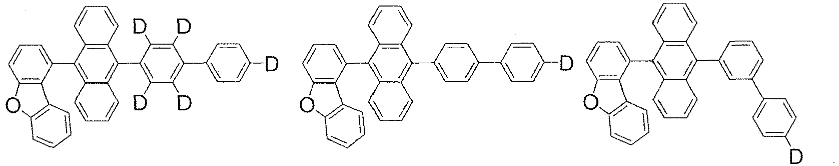

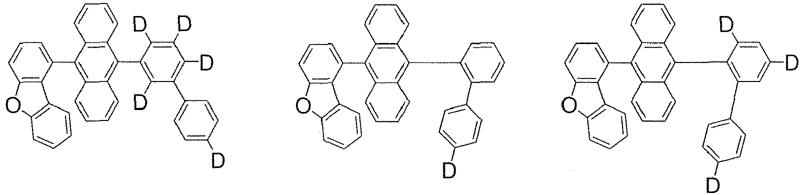

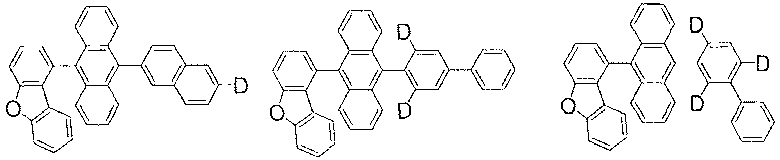

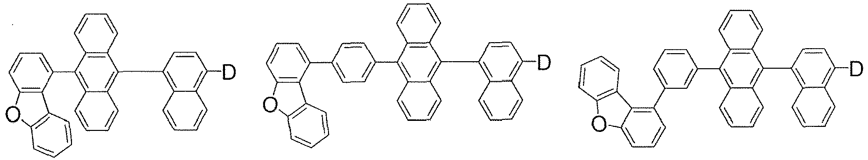

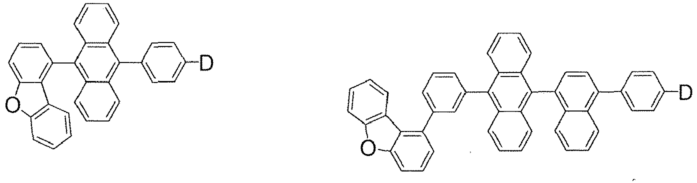

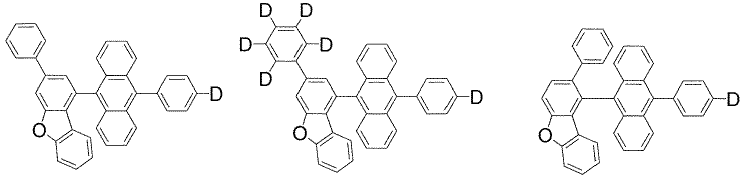

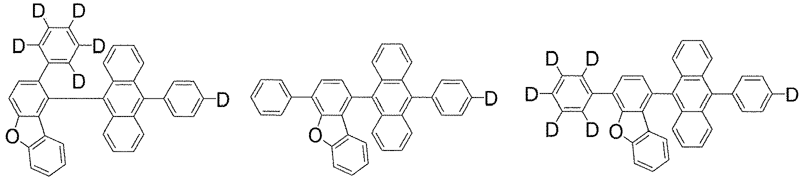

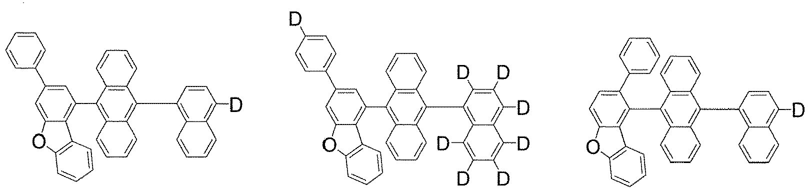

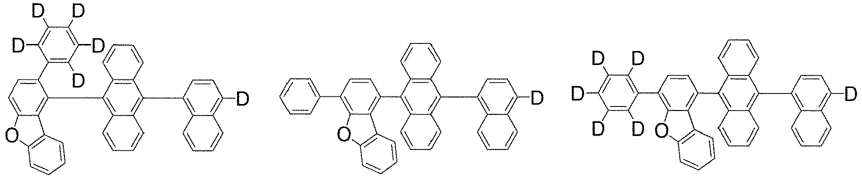

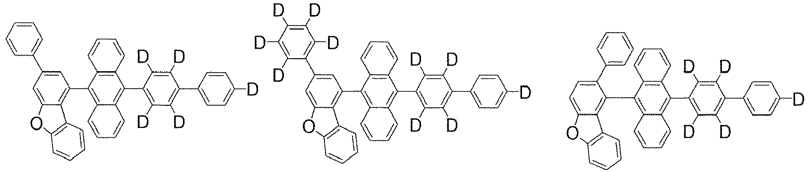

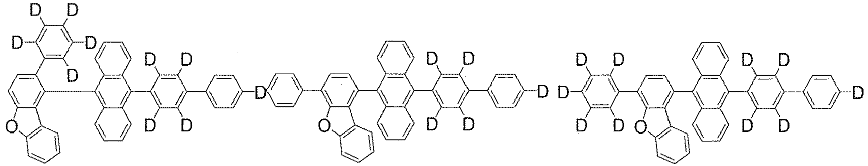

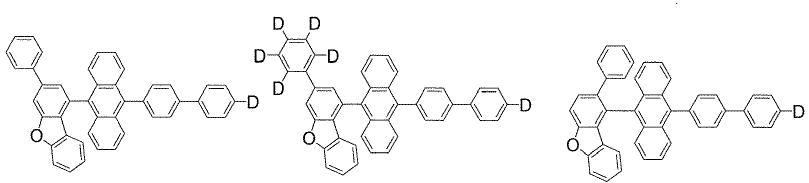

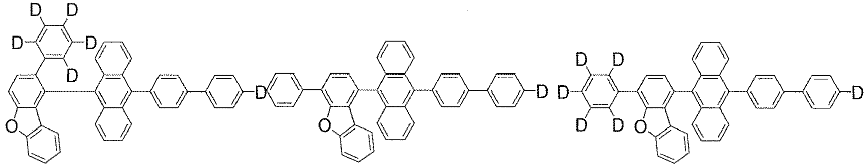

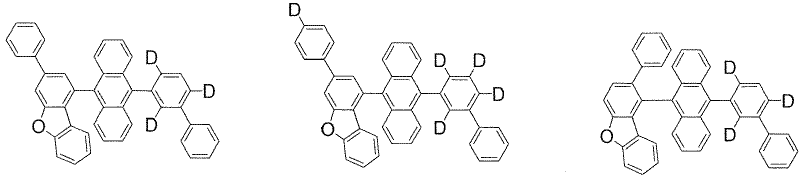

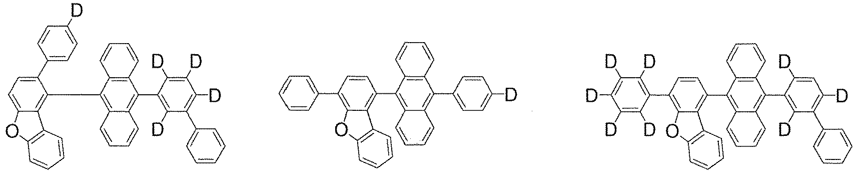

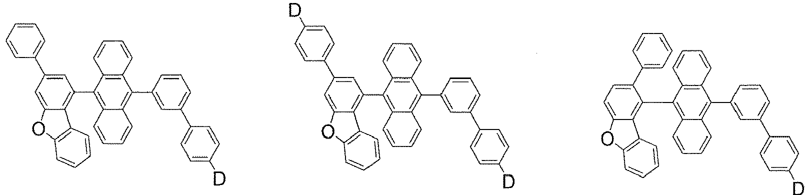

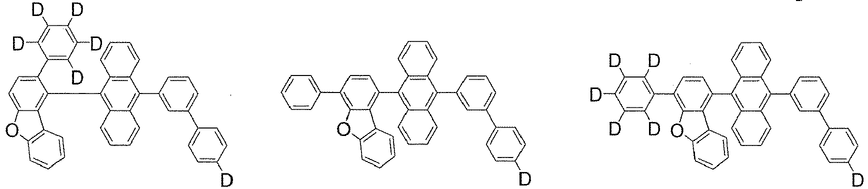

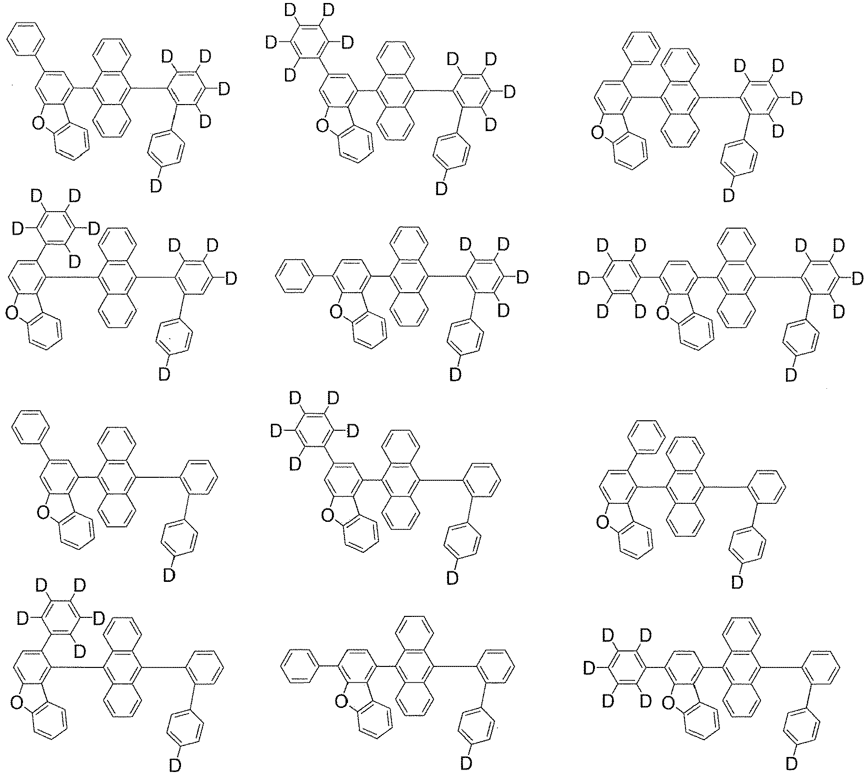

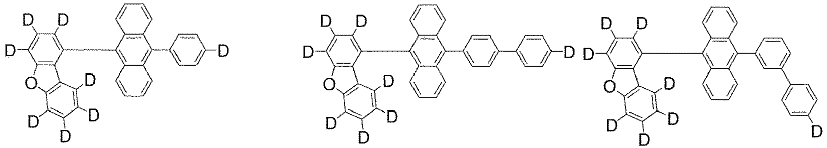

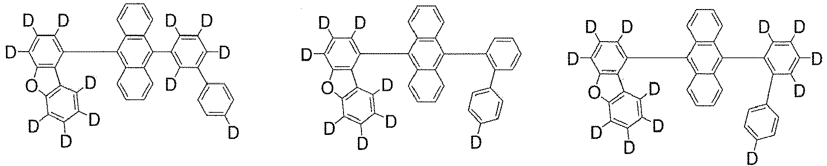

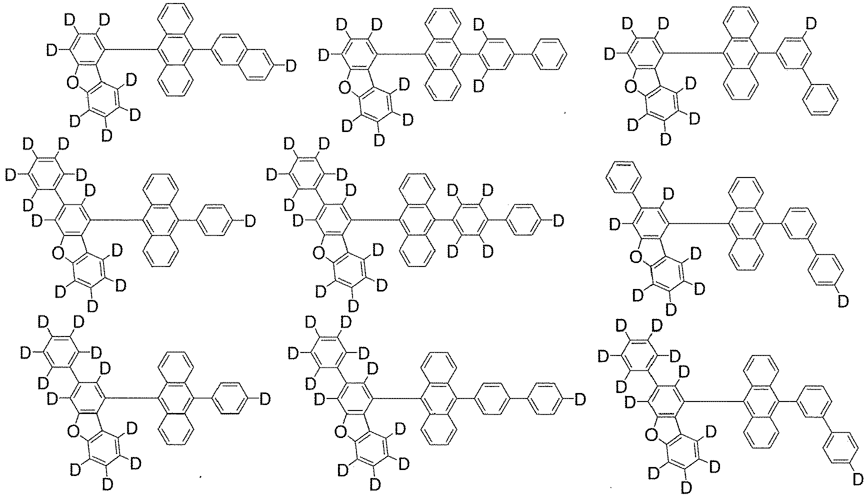

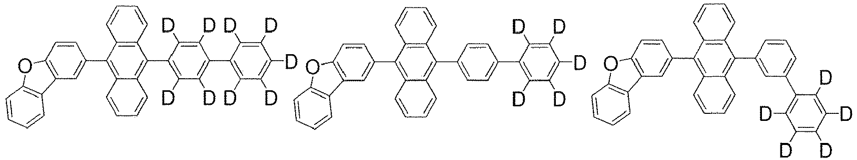

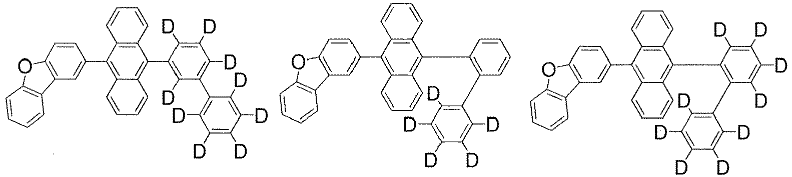

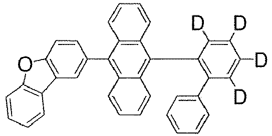

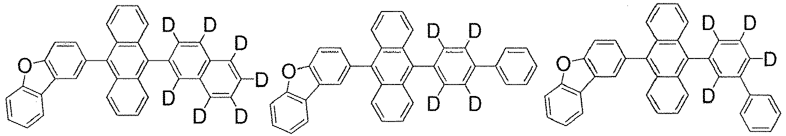

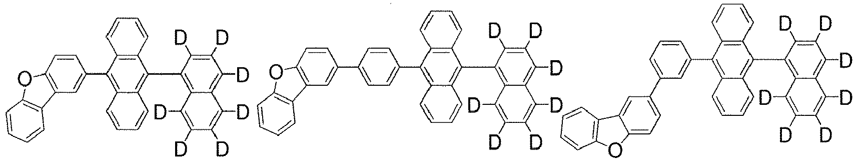

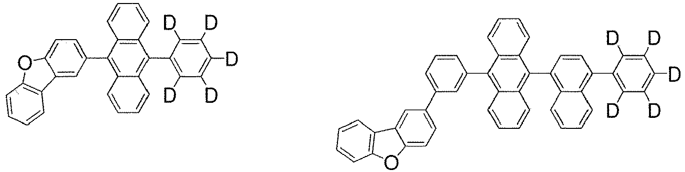

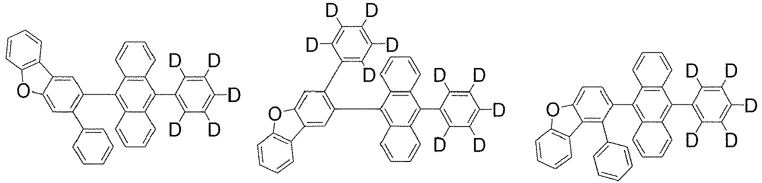

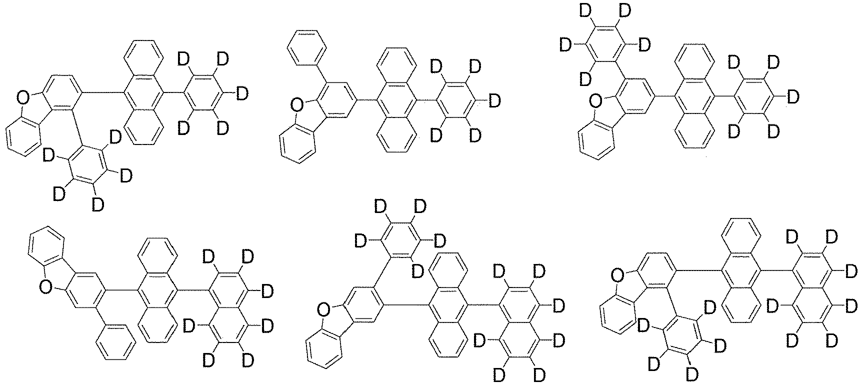

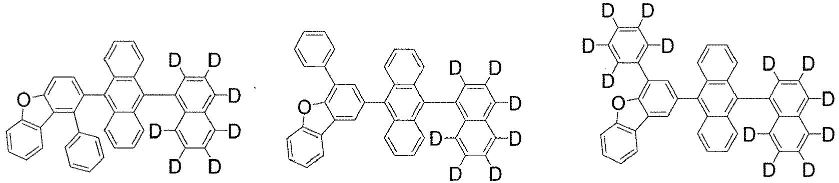

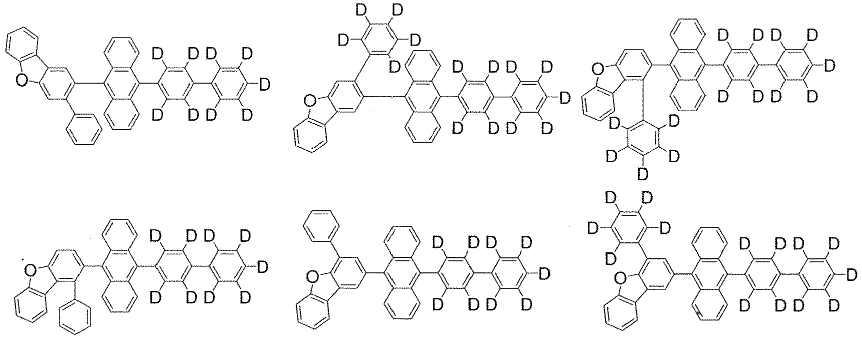

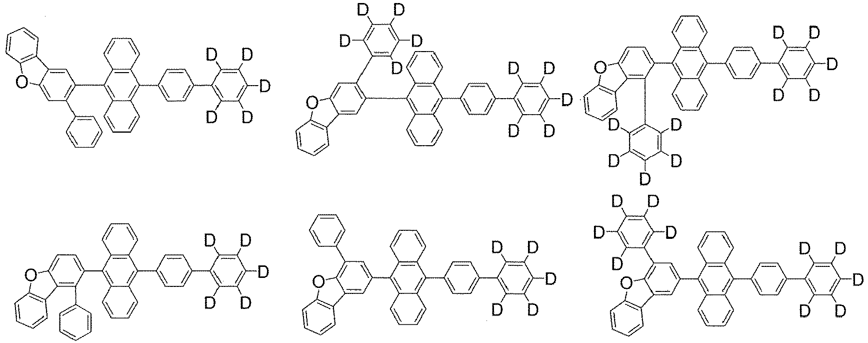

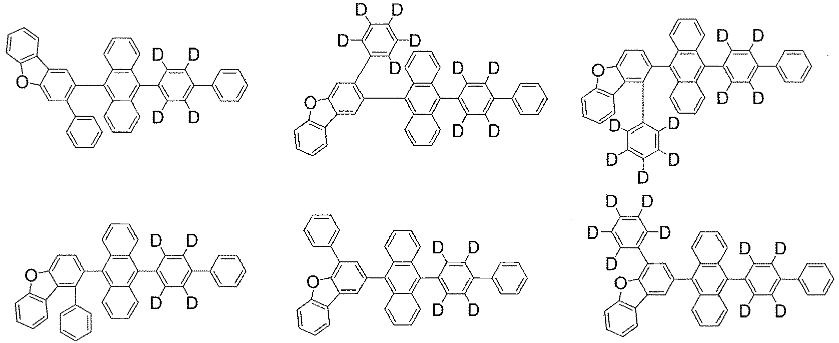

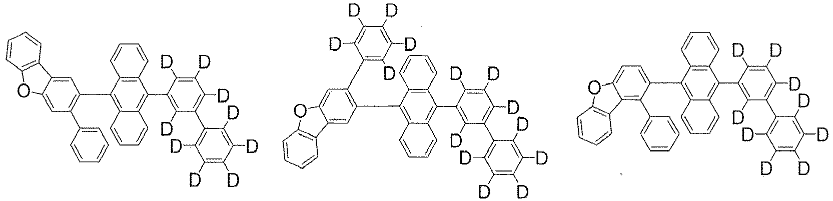

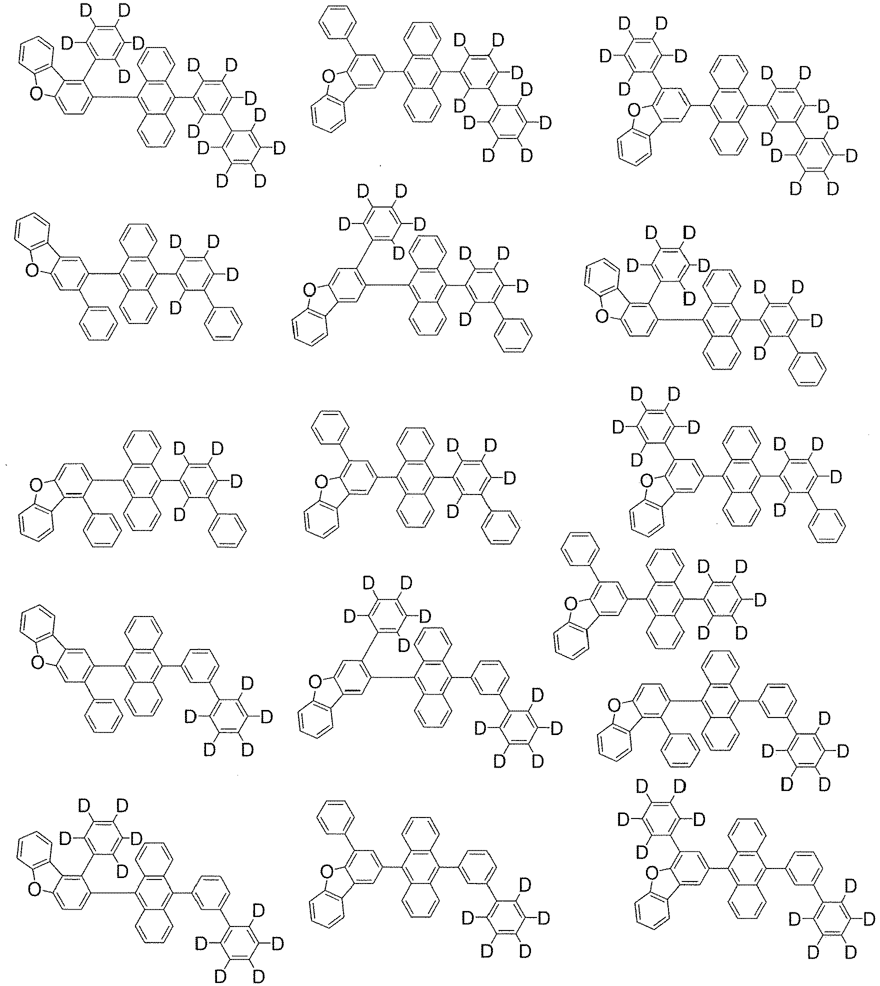

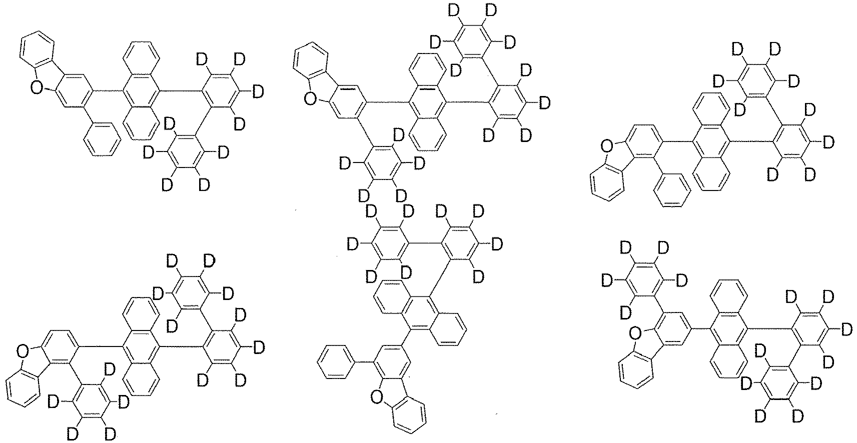

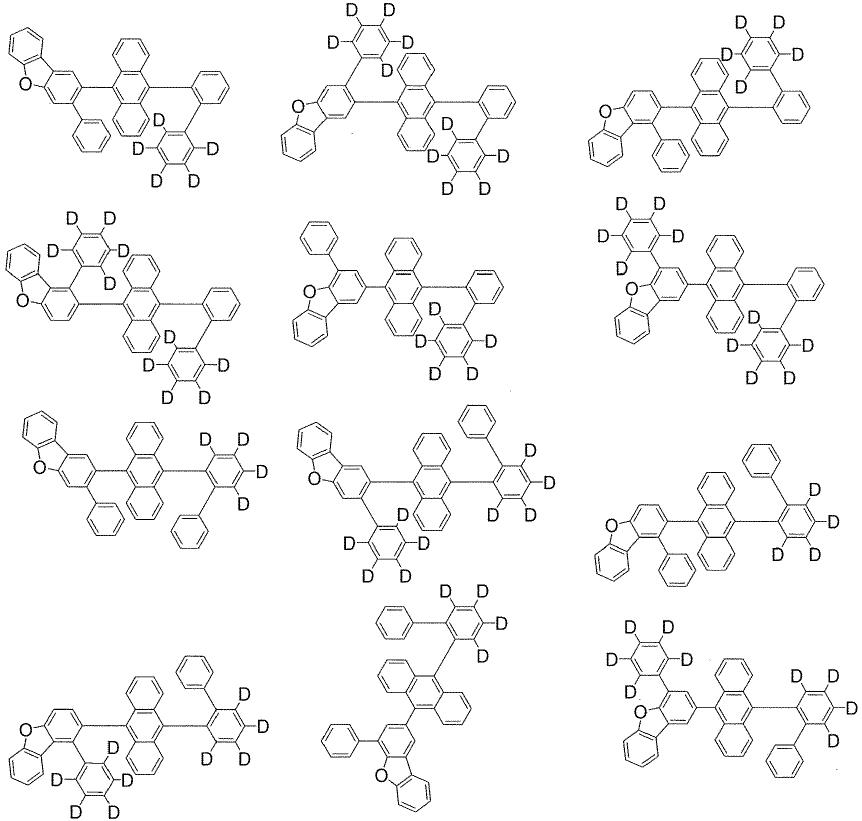

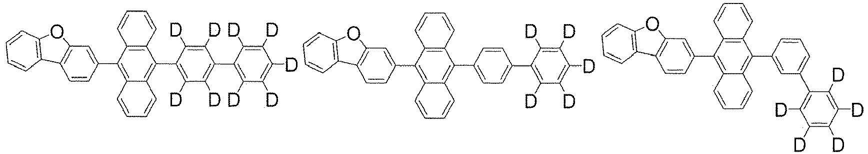

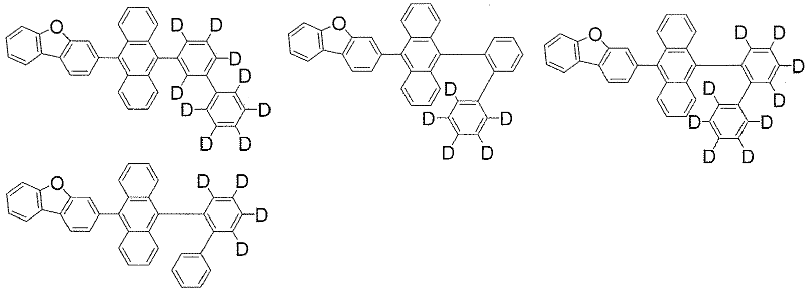

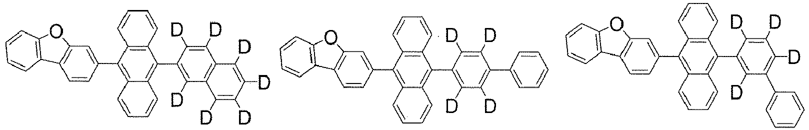

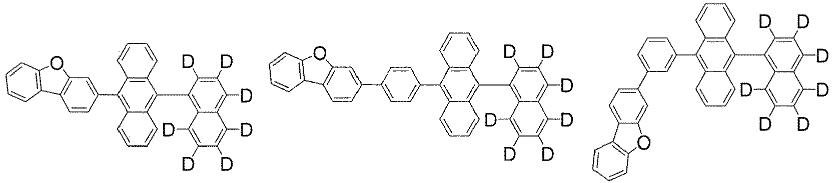

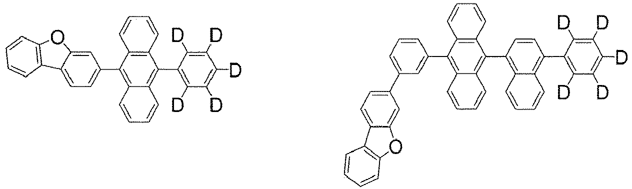

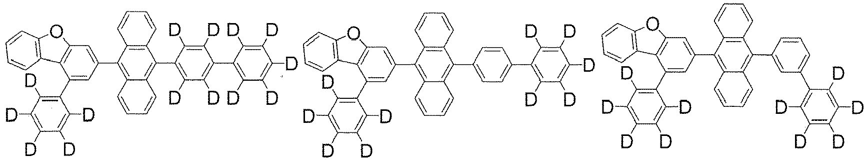

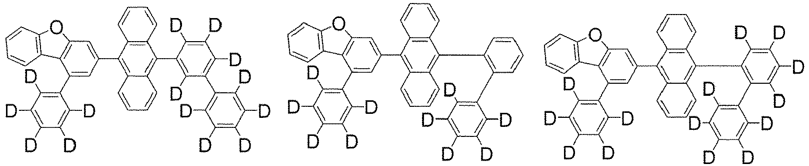

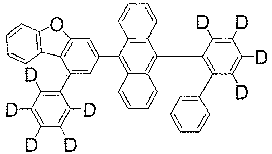

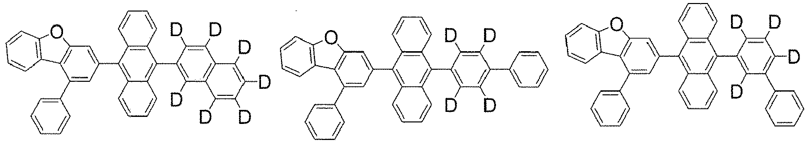

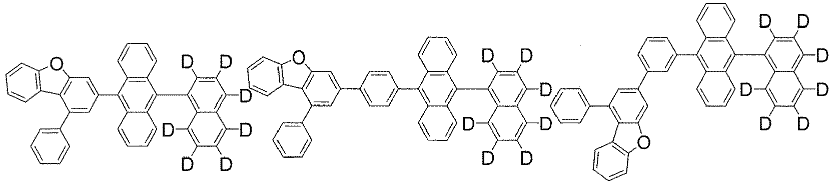

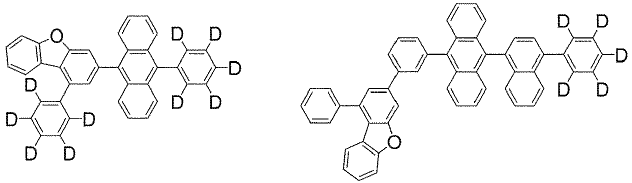

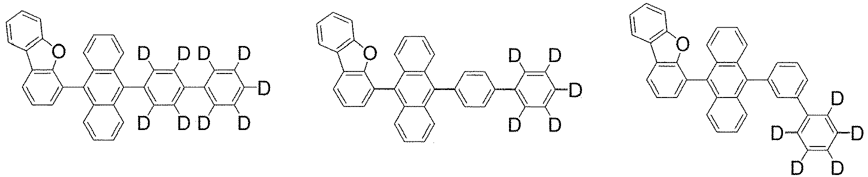

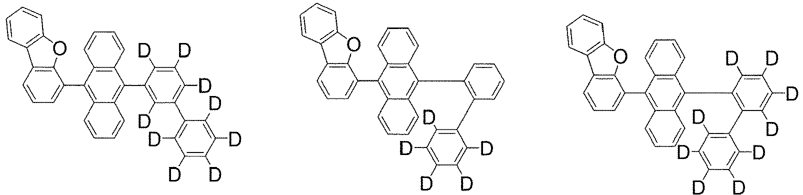

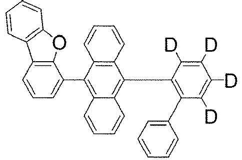

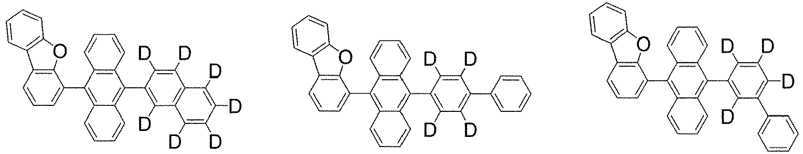

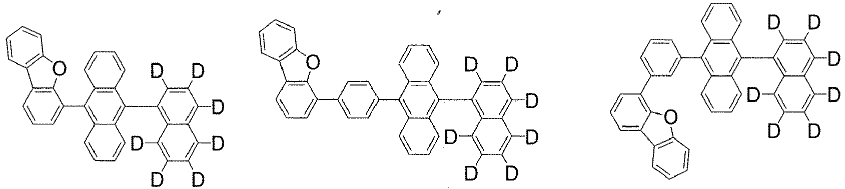

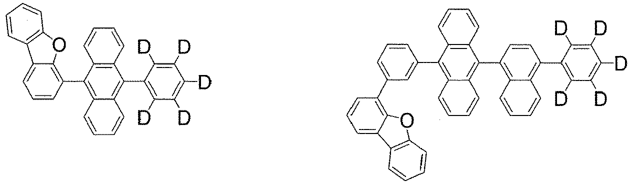

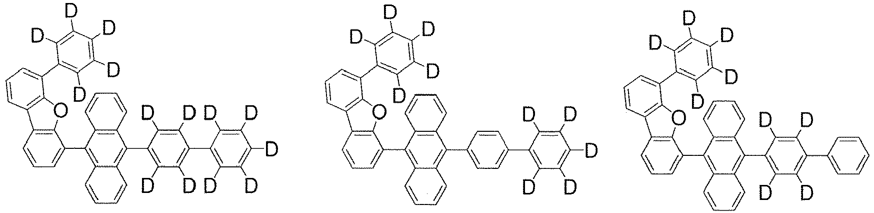

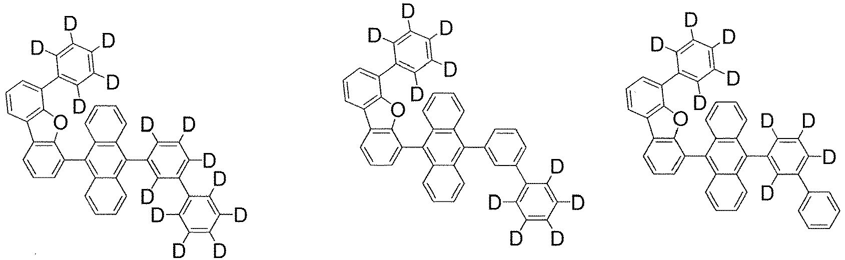

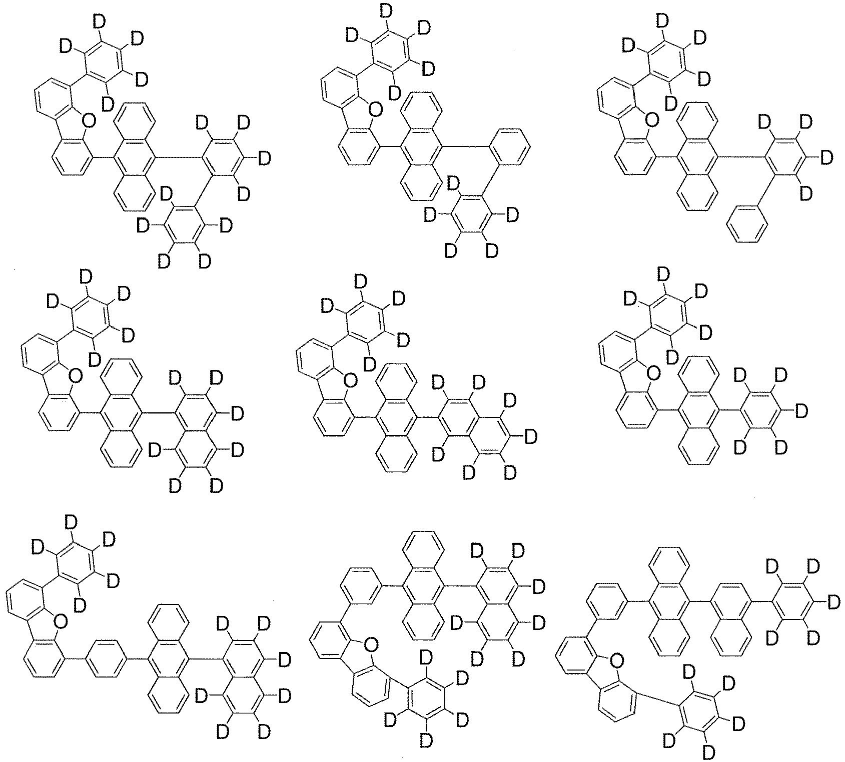

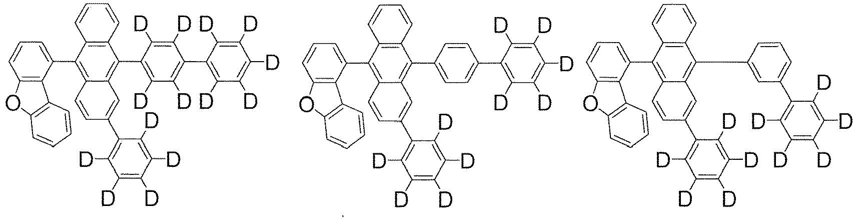

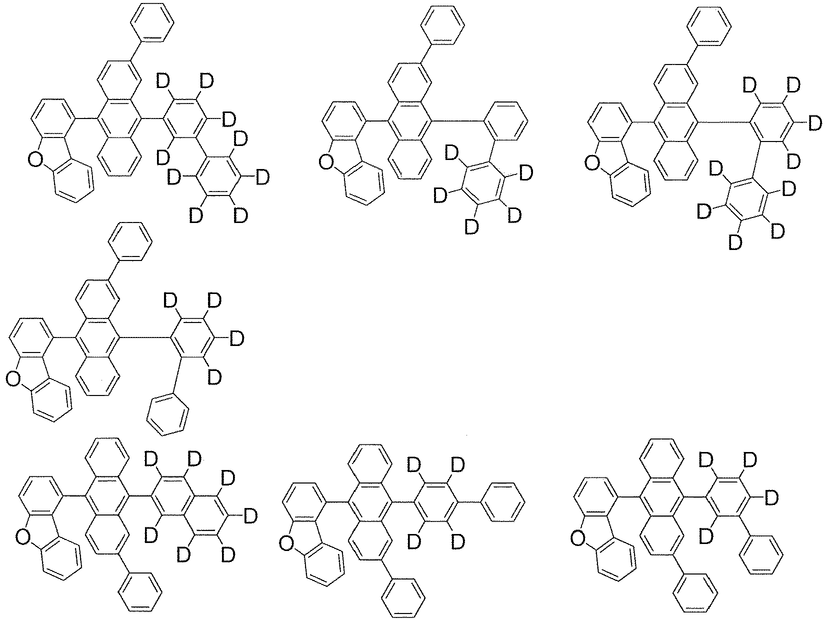

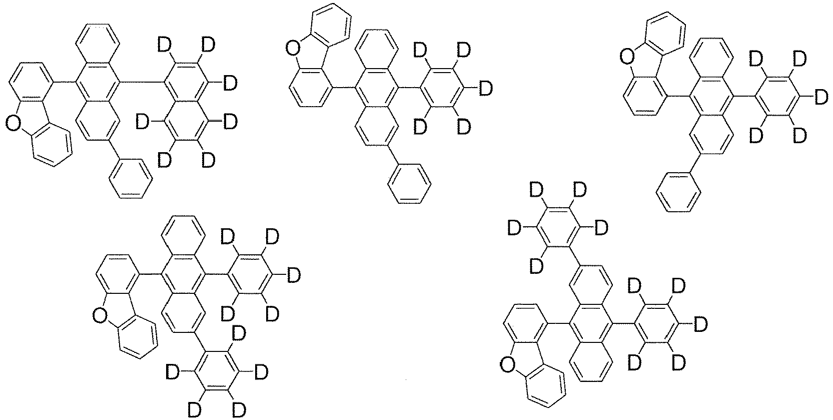

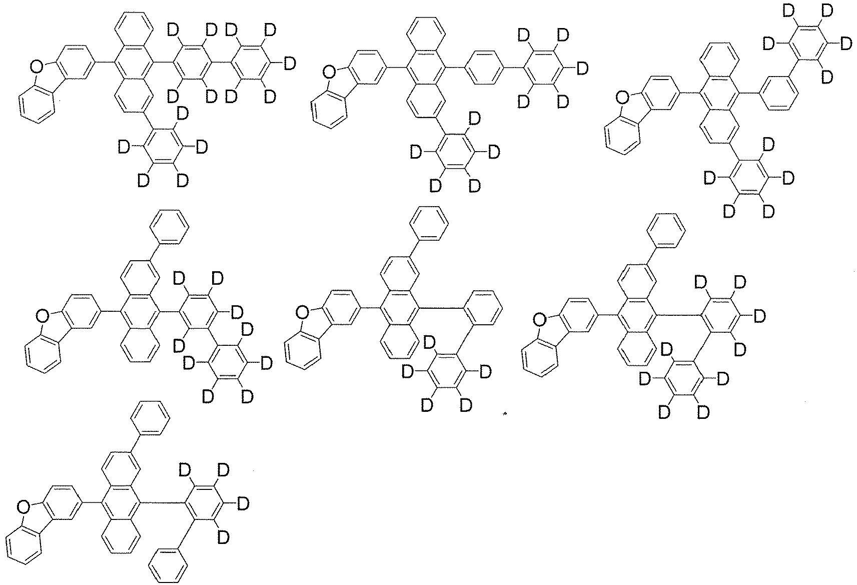

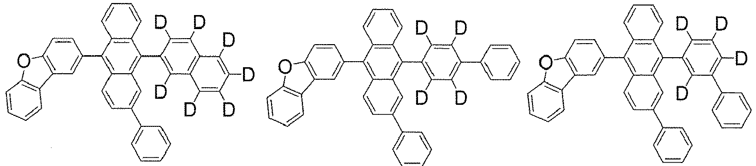

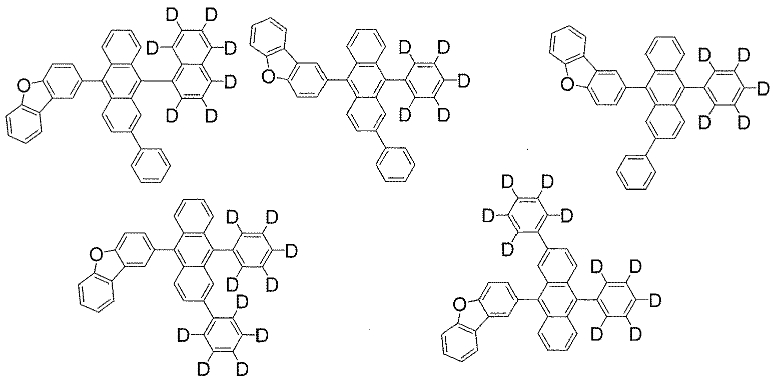

### [Compound represented by formula (1B)]

A compound represented by the formula (1B) is shown as follows: 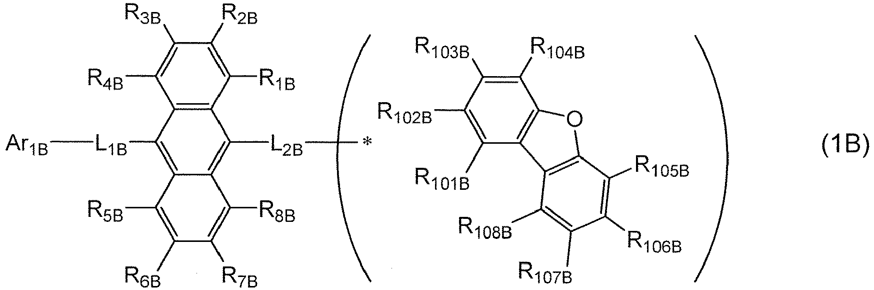 wherein in the formula (1B),
R_{1B} to R_{8B} are independently a hydrogen atom, or a substituent R;
L_{1B} and L_{2B} are independently
a single bond,
a substituted or unsubstituted arylene group having 6 to 18 ring carbon atoms, or
a substituted or unsubstituted divalent heterocyclic group having 5 to 18 ring atoms;
Ar_{1B} is
a substituted or unsubstituted phenyl group,
a substituted or unsubstituted p-biphenyl group,
a substituted or unsubstituted m-biphenyl group,
a substituted or unsubstituted o-biphenyl group,
a substituted or unsubstituted 1-naphthyl group,
a substituted or unsubstituted 2-naphthyl group, or
a substituted or unsubstituted phenanthryl group;
the adjacent two of R_{101B} to R_{108B} form a substituted or unsubstituted benzene ring by bonding with each other;
one of, R_{101B} to R_{108B} which do not form the substituted or unsubstituted benzene ring, and carbon atoms in the substituted or unsubstituted benzene ring is bonded with L_{2B} via a single bond;
R_{101B} to R_{108B} which do not form the substituted or unsubstituted benzene ring and which are not bonded with L_{2B} are independently a hydrogen atom, or a substituent R;
the substituent R is selected from the group consisting of
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
-Si(R₉₀₁)(R₉₀₂)(R₉₀₃),
-O-(R₉₀₄),
-S-(R₉₀₅),
-N(R₉₀₆)(R₉₀₇)
(wherein R₉₀₁ to R₉₀₇ are independently
a hydrogen atom,
a substituted or unsubstituted alkyl group having 1 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
a halogen atom, a cyano group, a nitro group,
a substituted or unsubstituted aryl group having 6 to 50 ring carbon atoms, and
a substituted or unsubstituted monovalent heterocyclic group having 5 to 50 ring atoms;
provided that the compound represented by the formula (1B) satisfies the following conditions B1 and B2;
condition B1: R_{1B} to R_{8B} which are hydrogen atoms are not a deuterium atom; and
condition B2: one or more of, hydrogen atoms possessed by L_{1B}, L_{2B} and Ar_{1B}, hydrogen atoms possessed by the substituent in the case where L_{1B}, L_{2B} and Ar_{1B} are substituted, hydrogen atoms possessed by R_{1B} to R_{8B} which are the substituents R, R_{101B} to R_{108B} which are hydrogen atoms, hydrogen atoms possessed by R_{101B} to R_{108B} which are the substituents R, and hydrogen atoms possessed by the substituted or unsubstituted benzene ring are a deuterium atom.

When L_{1B} is a single bond in the formula (1B), Ar_{1B} is directly bonded with the anthracene skeleton.

When L_{2B} is a single bond in the formula (1B), the structure in the above parentheses is directly bonded with the anthracene skeleton at the bondable position of the structure (one of, R_{101B} to R_{108B} which do not form the substituted or unsubstituted benzene ring, and carbon atoms in the substituted or unsubstituted benzene ring).

In one embodiment, R_{101B} is bonded with L_{2B} via a single bond.

In one embodiment, the compound represented by the formula (1B) is a compound represented by the following formula (1B-1): 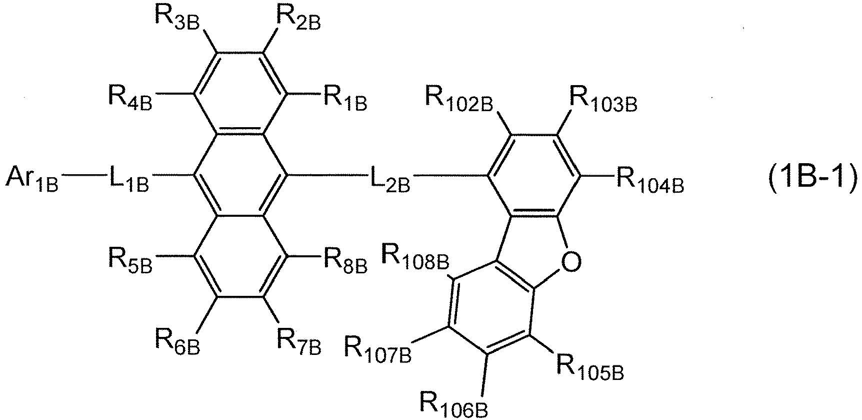 wherein in the formula (1B-1), R_{1B} to R_{8B}, R_{102B} to R_{108B}, L_{1B}, L_{2B} and Ar_{1B} are the same as defined in the formula (1B).

In one embodiment, in the formula (1B), one or more sets of the adjacent two or more of R_{105B} to R_{108B} form a substituted or unsubstituted benzene ring by bonding with each other.

In one embodiment, the compound represented by the formula (1B) is a compound represented by any one of the following formulas (1B-11) to (1B-13): 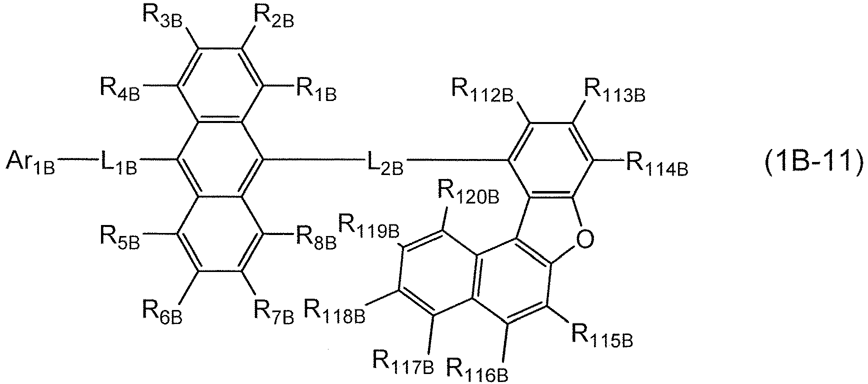 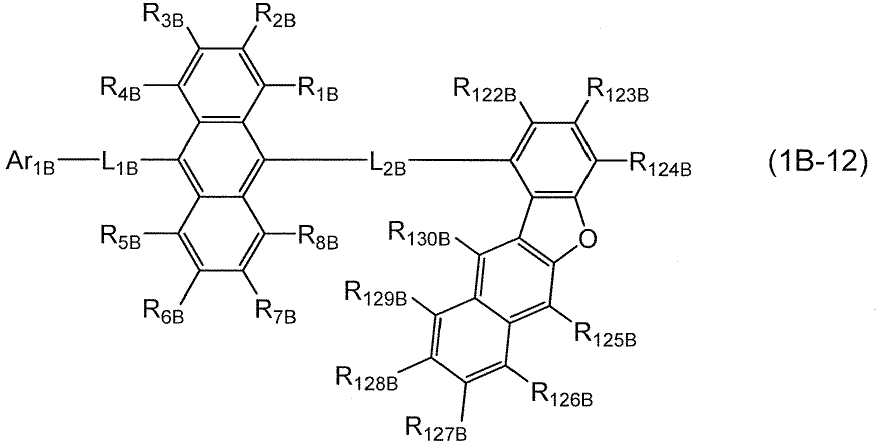 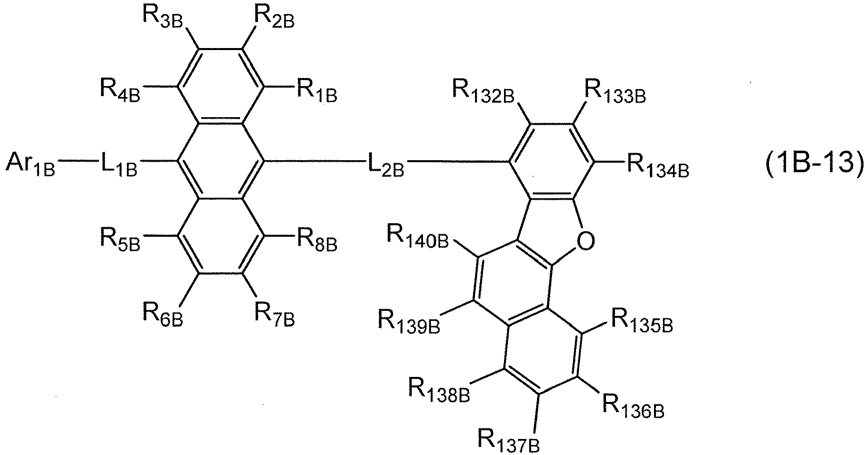 wherein in the formulas (1B-11) to (1B-13), R_{1B} to R_{8B}, L_{1B}, L_{2B} and Ar_{1B} are the same as defined in the formula (1B);
in the formula (1B-11), R_{112B} to R_{120B} are independently a hydrogen atom, or a substituent R;
in the formula (1B-12), R_{122B} to R_{130B} are independently a hydrogen atom, or a substituent R;
in the formula (1B-13), R_{132B} to R_{140B} are independently a hydrogen atom, or a substituent R; and
the substituent R is the same as defined in the formula (1B).

In one embodiment, L_{1B} is
a substituted or unsubstituted phenylene group, or
a substituted or unsubstituted naphthylene group; and
one or more of hydrogen atoms possessed by L_{1B} are a deuterium atom.

In one embodiment, the compounds represented by the formulas (1B-11) to (1B-13) are a compound represented by any one of the following formulas (1B-21) to (1B-23): 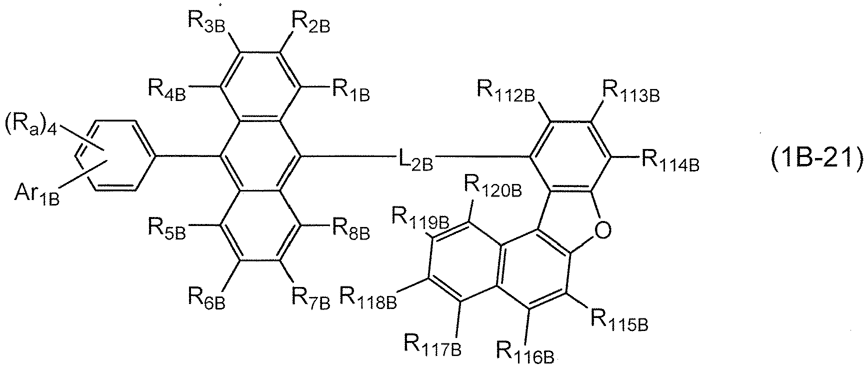 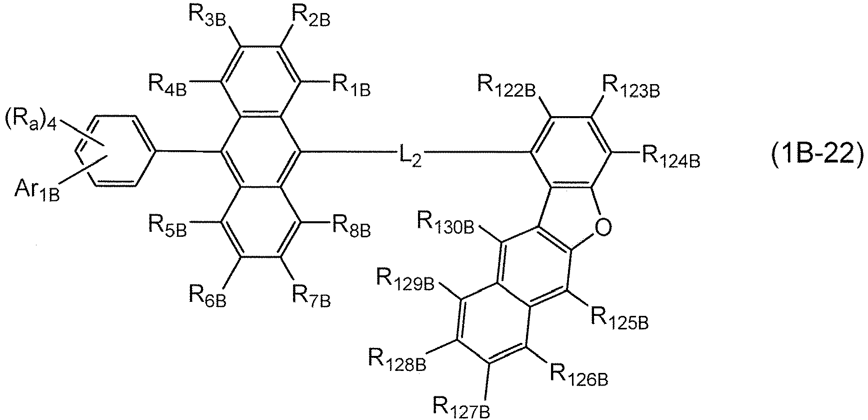 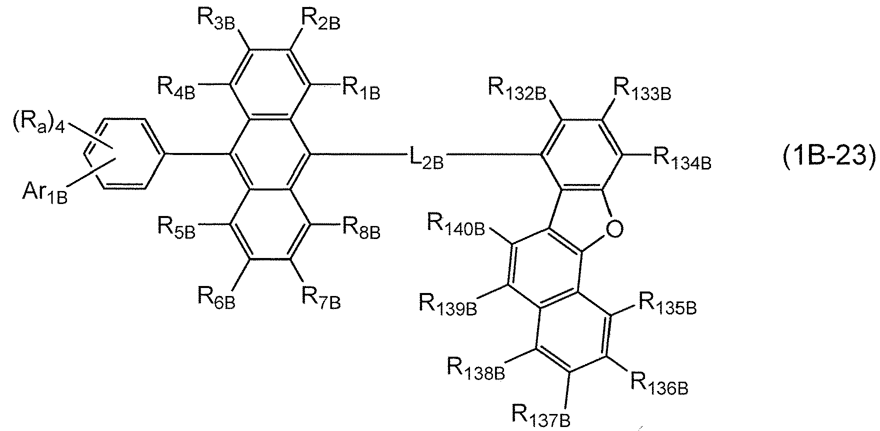 wherein in the formulas (1B-21) to (1B-23), R_{1B} to R_{8B}, R_{112B} to R_{120B}, R_{122B} to R_{130B}, R_{132B} to R_{140B}, L_{2B} and Ar_{1B} are the same as defined in the formulas (1B-11) to (1B-13);
four Rₐ's are independently a hydrogen atom, or a substituent R;
provided that one or more of the four Rₐ's are a deuterium atom; and
the substituent R is the same as defined in the formula (1B).

In one embodiment, hydrogen atoms possessed by Ar_{1B} are not a deuterium atom.

It is preferable in terms of enhancing improving effects of device lifetime in the formula (1B) that a compound in which one or more of hydrogen atoms possessed by L_{1B} are a deuterium atom and hydrogen atoms possessed by Ar_{1B} are not a deuterium atom be used.

It will be specifically described below using a compound in which all of deuterium atoms in the formula (1B) are replaced with a protium atom (hereinafter, frequently referred to as "protium compound"), a compound having the same structure as the protium compound, except that one or more of hydrogen atoms possessed by L_{1B} are a deuterium atom (hereinafter, frequently referred to as "deuterium compound a"), and a compound having the same structure as the protium compound, except that one or more of hydrogen atoms possessed by Ar_{1B} are a deuterium atom (hereinafter, frequently referred to as "deuterium compound β") as an example. Examples of the "protium compound", the "deuterium compound a" and the "deuterium compound β" include the following compounds.

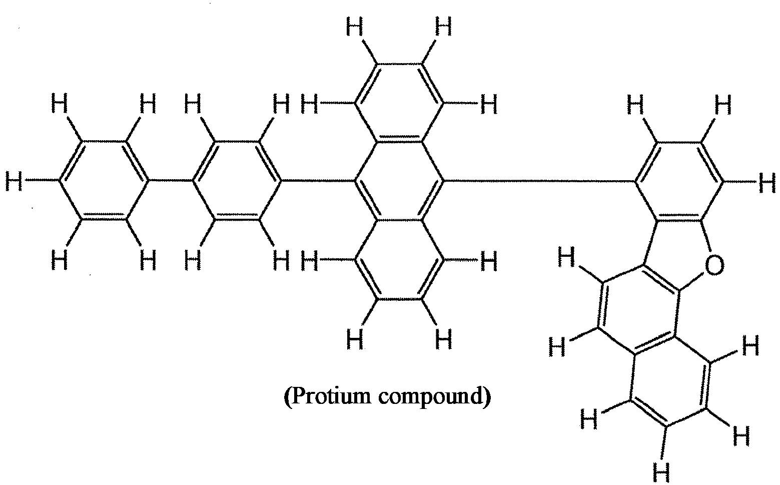

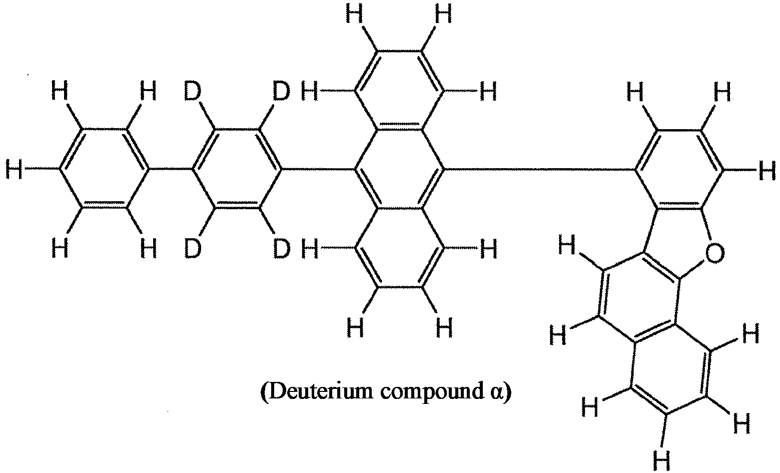

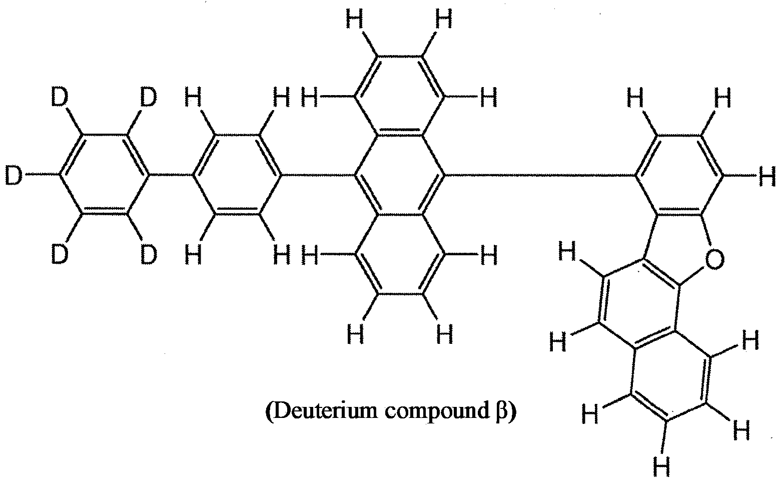

When increasing rates of the lifetimes of, a device in which the deuterium compound a is used, and a device in which the deuterium compound β is used is used, is compared based on the lifetime of a device in which the protium compound is used, the device in which the deuterium compound a is used has larger increasing rate of the lifetime. That is, the case where the deuterium compound a is used is preferable as compared to the case where the deuterium compound β is used, since improving effects of device lifetime are enhanced.

In one embodiment, the compound represented by the formula (1B) is a compound represented by the following formula (1B-121): 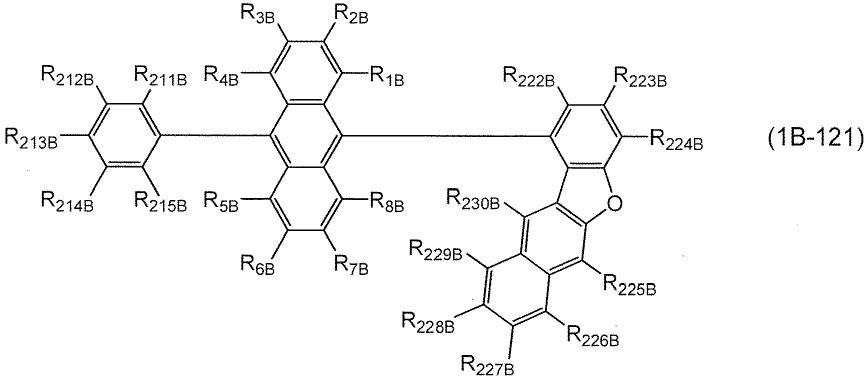 wherein in the formula (1B-121), R_{1B} to R_{8B} are the same as defined in the formula (1B);
R_{211B} to R_{215B} are independently a hydrogen atom, or a substituent R;
R_{222B} to R_{230B} are independently a hydrogen atom, or a substituent R;
the substituent R is the same as defined in the formula (1);
provided that the compound represented by the formula (1B-121) satisfies the following conditions B1-1 and B2-1;
condition B1-1: at least one of R_{211B} to R_{215B} is a deuterium atom; and
condition B2-1: R_{222B} to R_{230B} which are hydrogen atoms are not a deuterium atom.

In one embodiment, R_{211B} to R_{215B} are hydrogen atoms.

In one embodiment, R_{1B} to R_{8B} are hydrogen atoms.

In one embodiment, a substituent in the case of "substituted or unsubstituted" in the formula (1B) is selected from the group consisting of
an alkyl group having 1 to 50 carbon atoms,
a haloalkyl group having 1 to 50 carbon atoms,
an alkenyl group having 2 to 50 carbon atoms,
an alkynyl group having 2 to 50 carbon atoms,
a cycloalkyl group having 3 to 50 ring carbon atoms,
an alkoxy group having 1 to 50 carbon atoms,
an alkylthio group having 1 to 50 carbon atoms,
an aryloxy group having 6 to 50 ring carbon atoms,
an arylthio group having 6 to 50 ring carbon atoms,
an aralkyl group having 7 to 50 carbon atoms,
-Si(R₄₁)(R₄₂)(R₄₃),
-C(=O)R₄₄, -COOR₄₅,
-S(=O)₂R₄₆,
-P(=O)(R₄₇)(R₄₈),
-Ge(R₄₉)(R₅₀)(R₅₁),
-N(R₅₂)(R₅₃) (wherein, R₄₁ to R₅₃ are independently a hydrogen atom, an alkyl group having 1 to 50 carbon atoms, an aryl group having 6 to 50 ring carbon atoms, or a monovalent heterocyclic group having 5 to 50 ring atoms; when two or more of each of R₄₁ to R₅₃ are present, the two or more of each of R₄₁ to R₅₃ may be the same as or different from each other),
a hydroxy group,
a halogen atom,
a cyano group,
a nitro group,
an aryl group having 6 to 50 ring carbon atoms, and
a monovalent heterocyclic group having 5 to 50 ring atoms.

In one embodiment, a substituent in the case of "substituted or unsubstituted" in the formula (1B) is selected from the group consisting of
an alkyl group having 1 to 18 carbon atoms,
an aryl group having 6 to 18 ring carbon atoms, and
a monovalent heterocyclic group having 5 to 18 ring atoms.

The compound represented by the formula (1B) can be synthesized by using known alternative reactions or raw materials adapted to the target compound.

Specific examples of the compound represented by the formula (1B) will be described below, but these are merely examples, and the compound represented by the formula (1B) is not limited to the following specific examples.

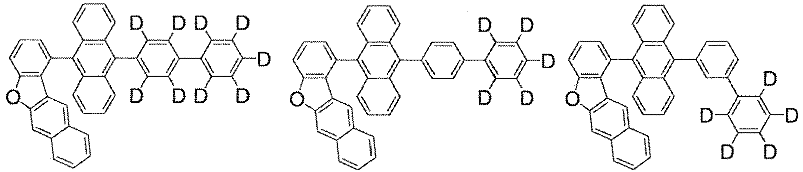

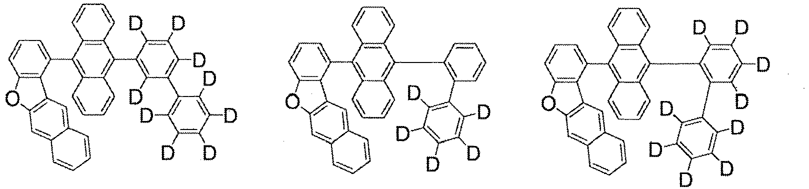

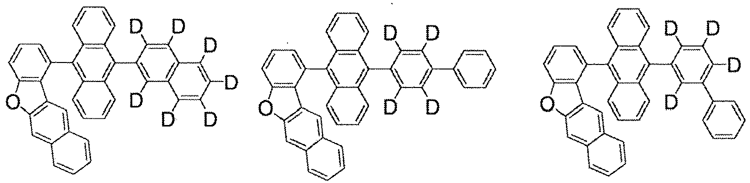

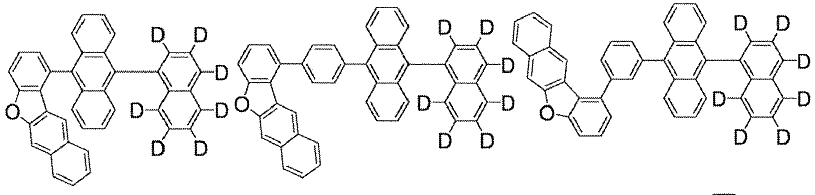

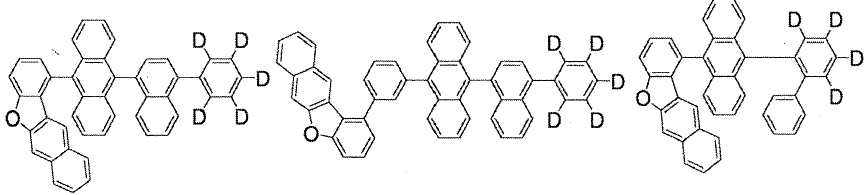

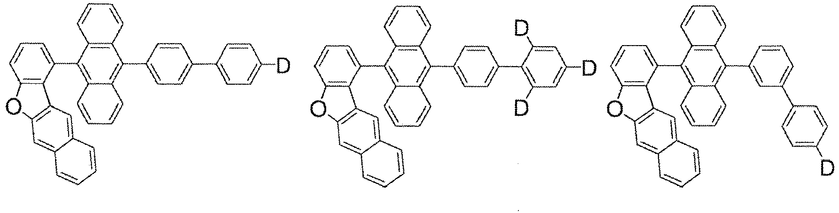

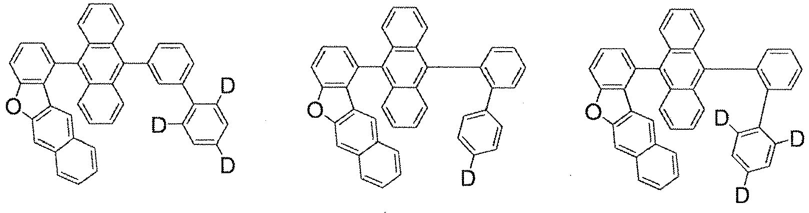

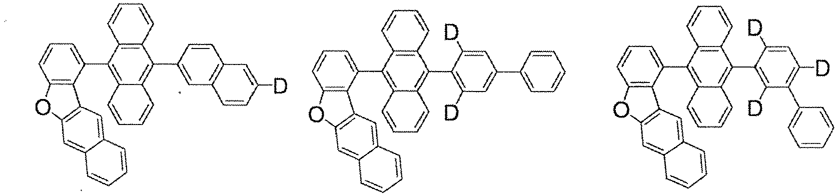

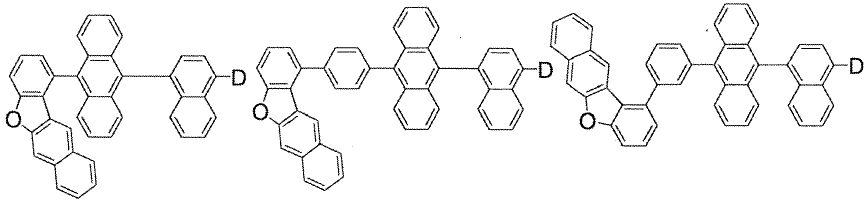

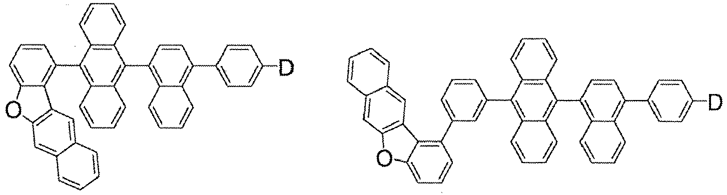

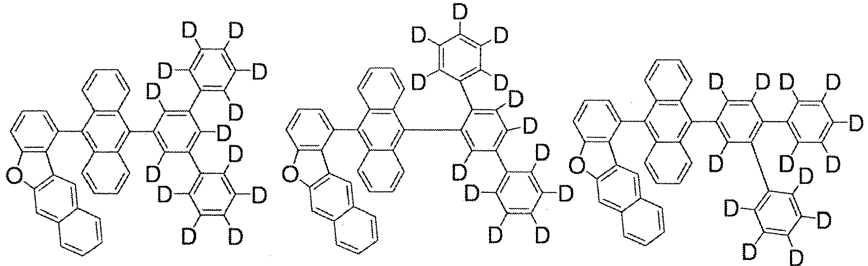

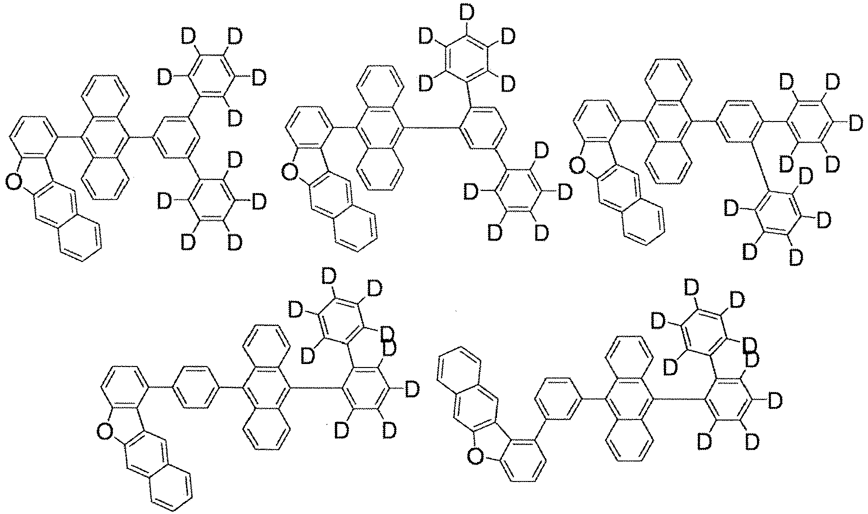

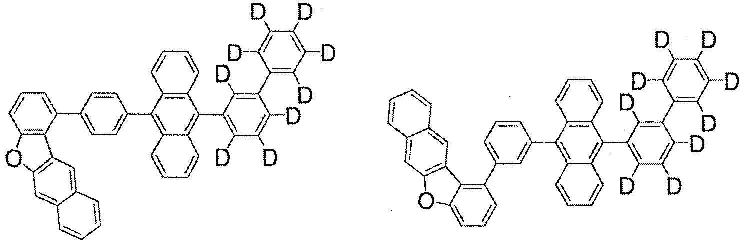

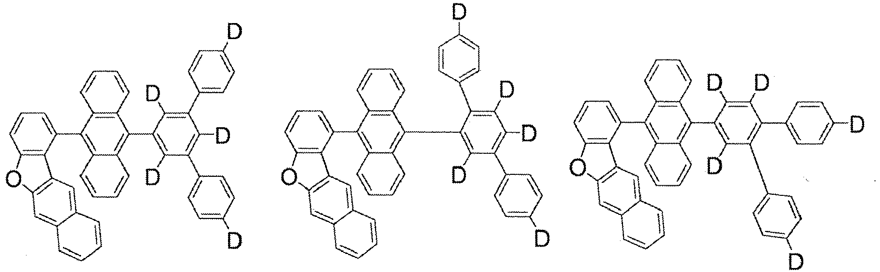

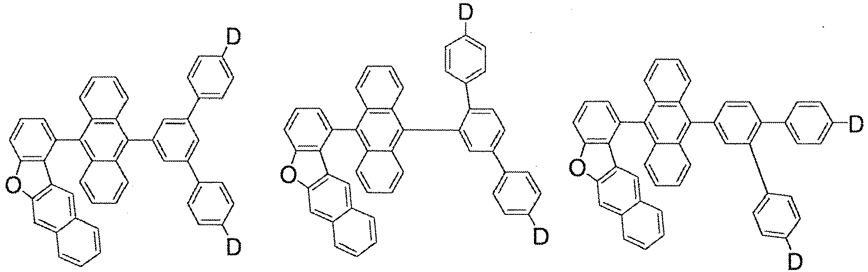

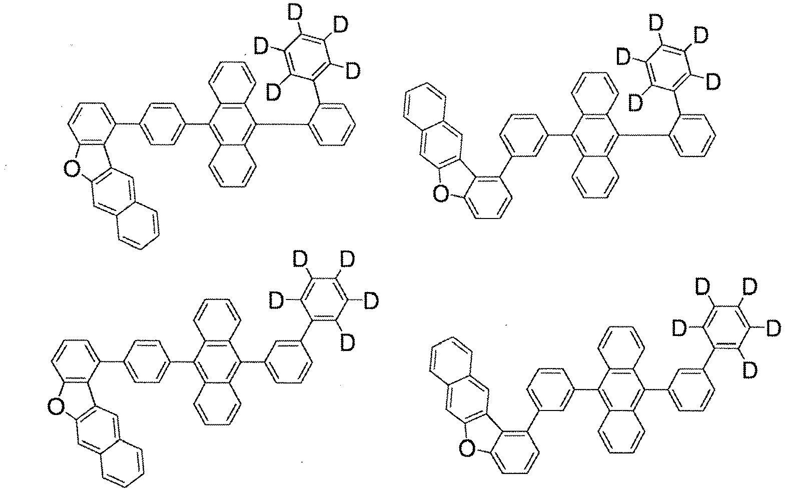

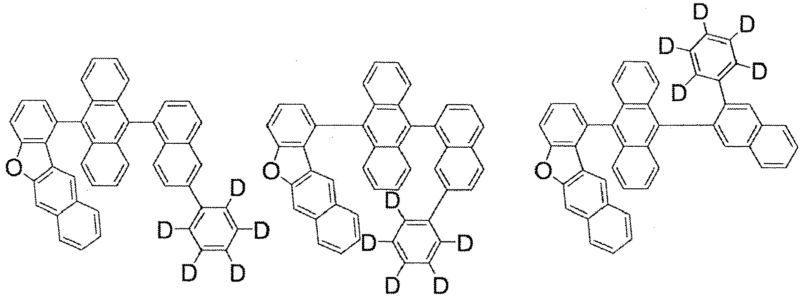

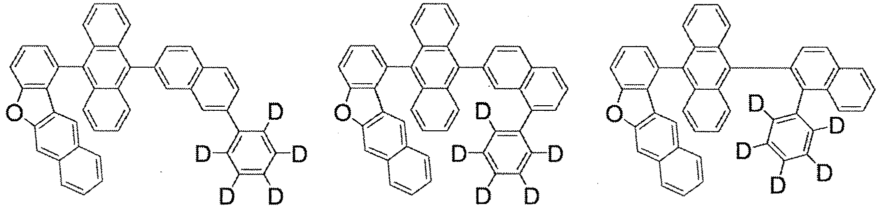

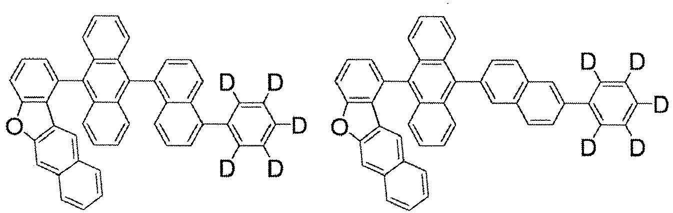

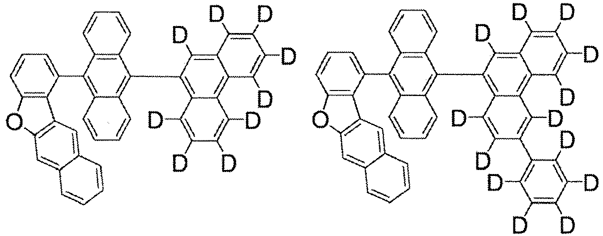

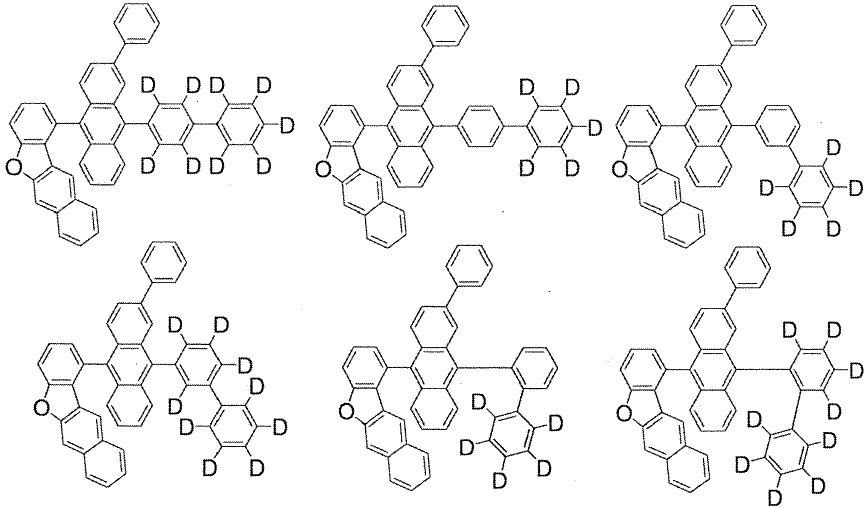

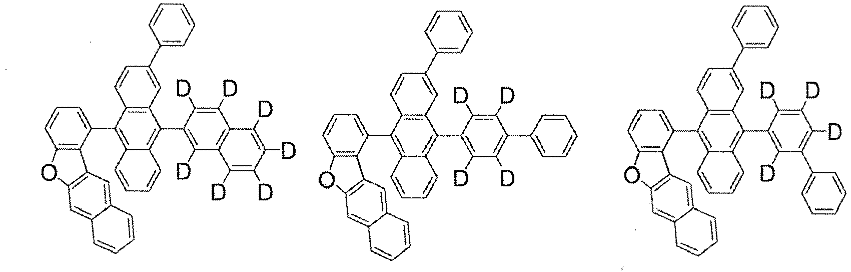

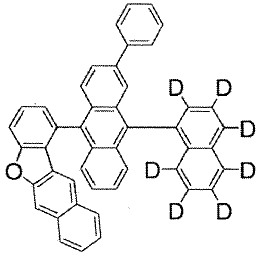

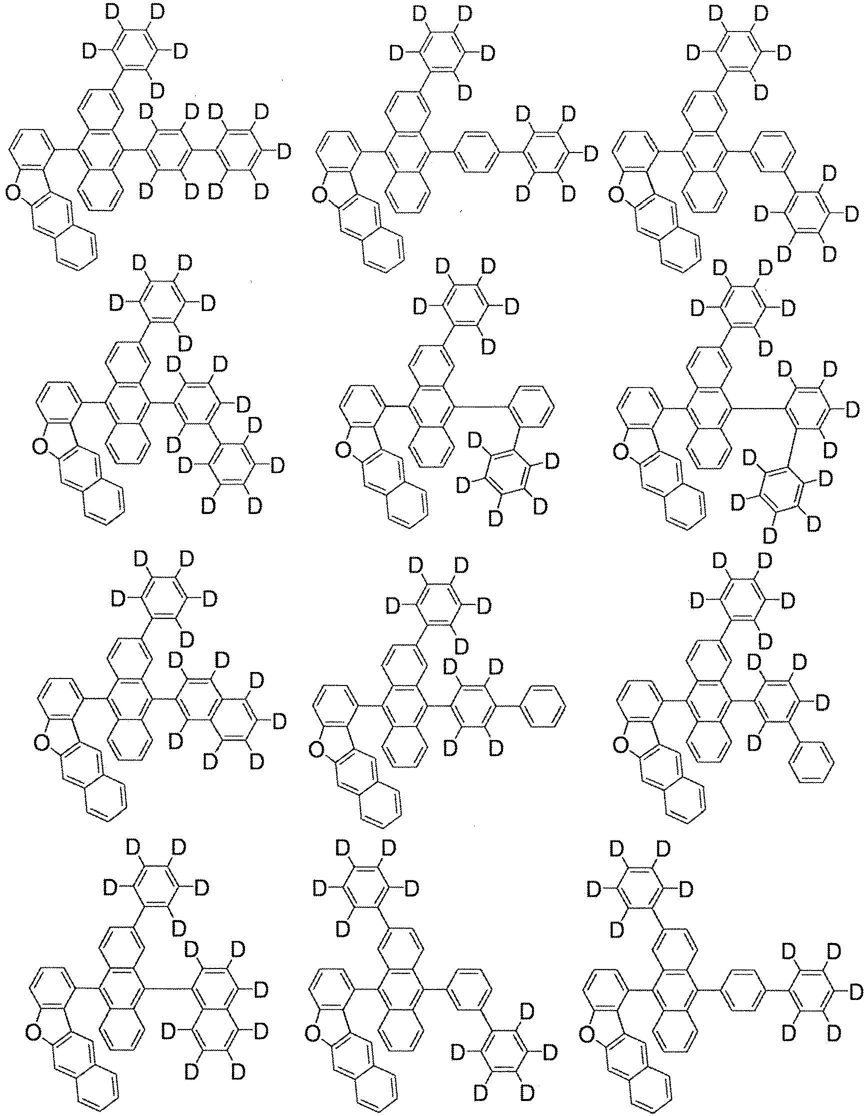

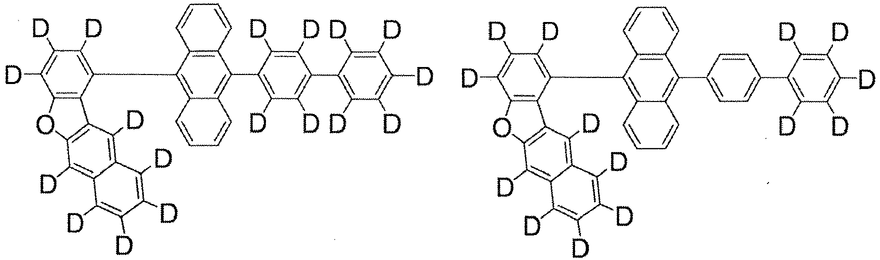

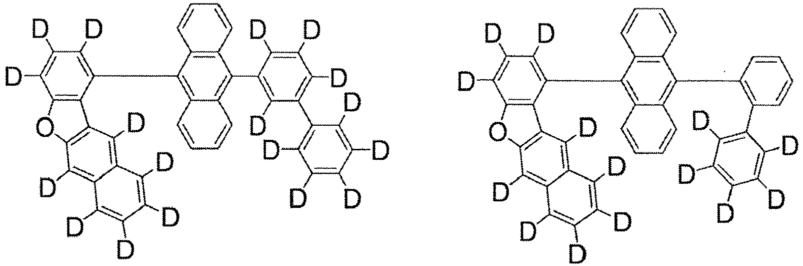

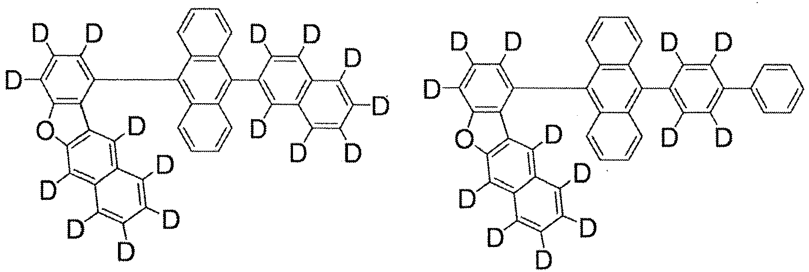

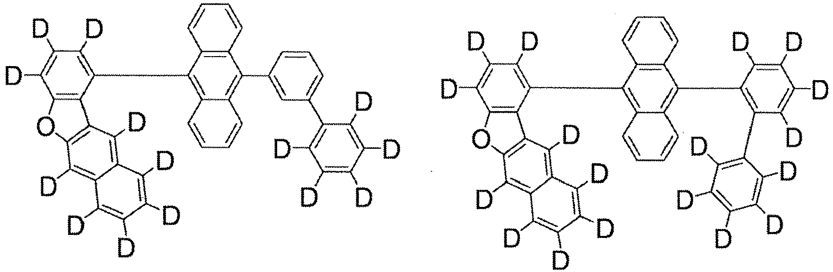

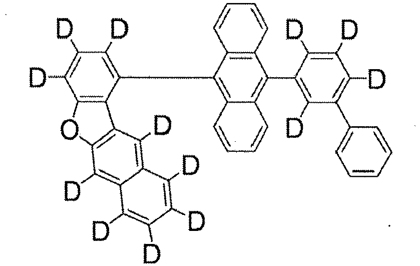

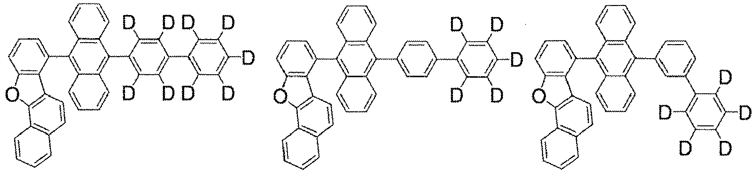

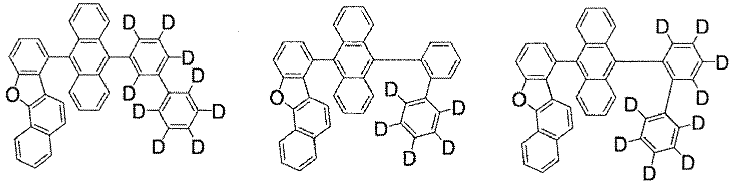

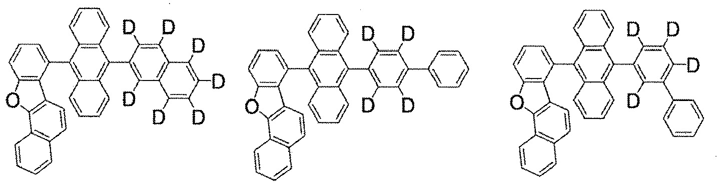

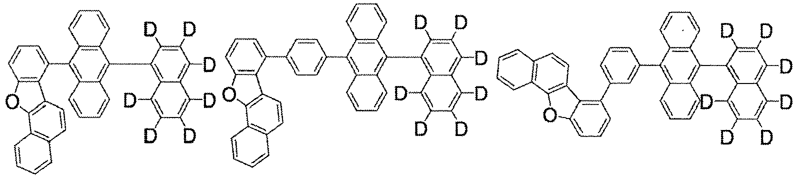

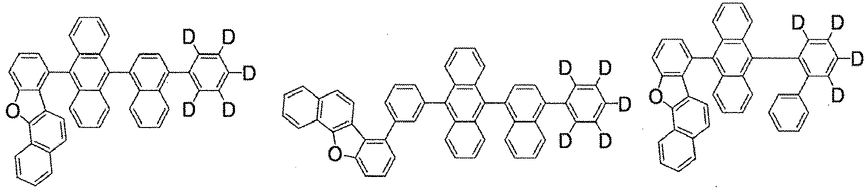

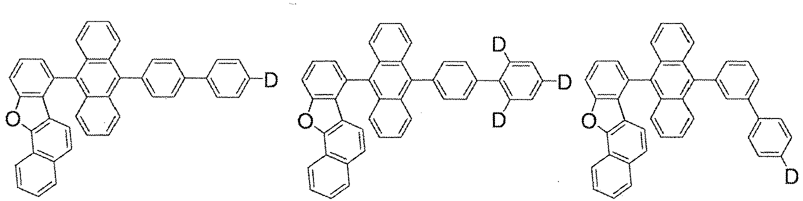

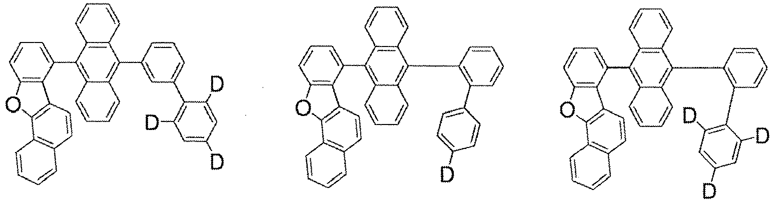

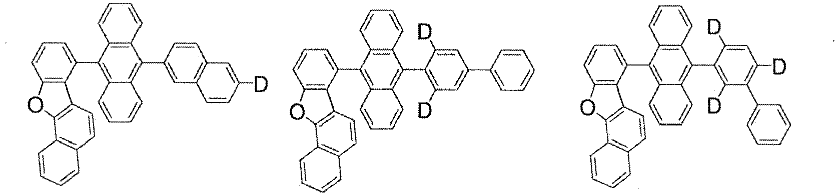

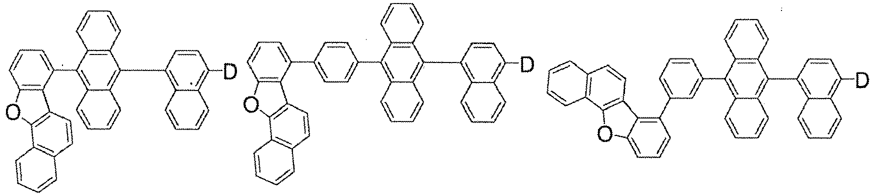

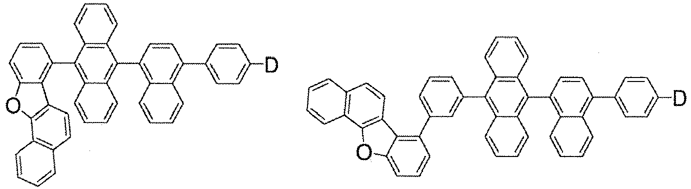

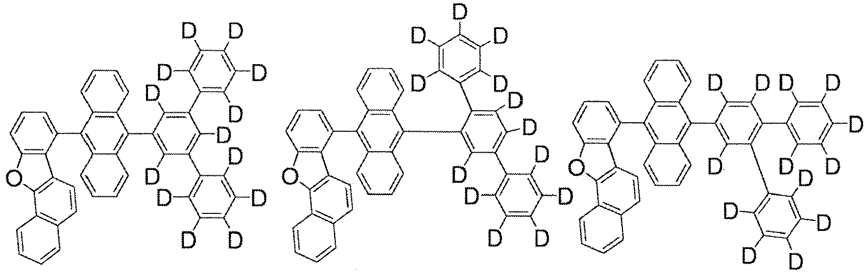

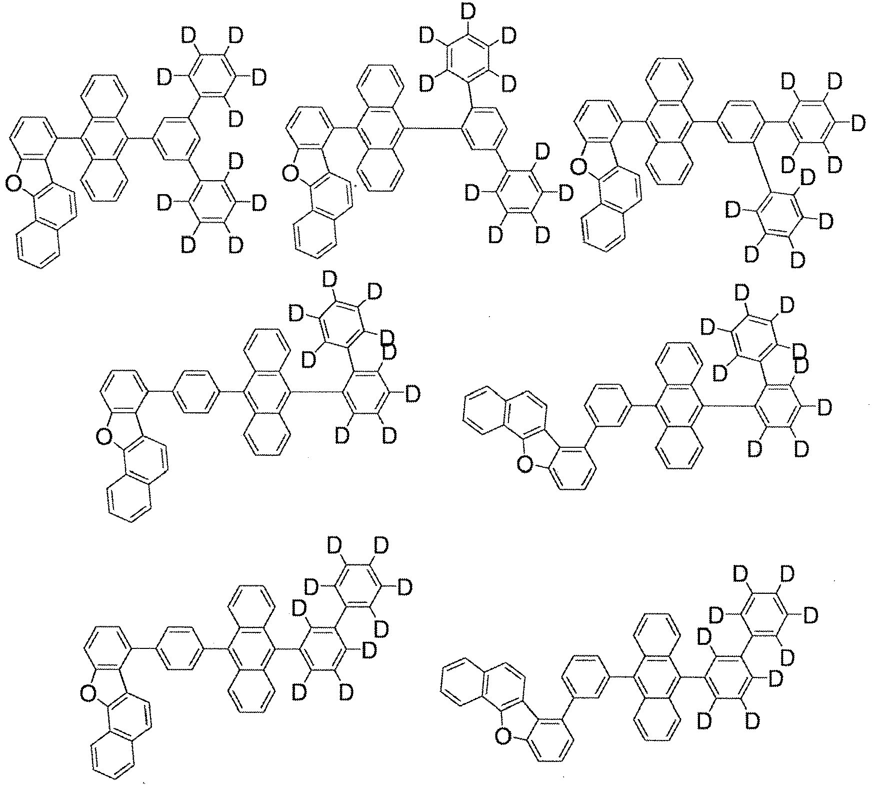

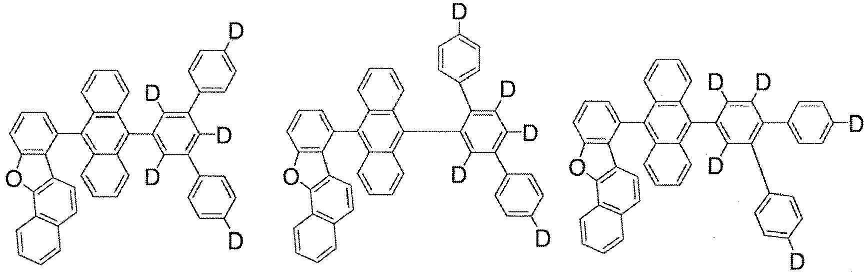

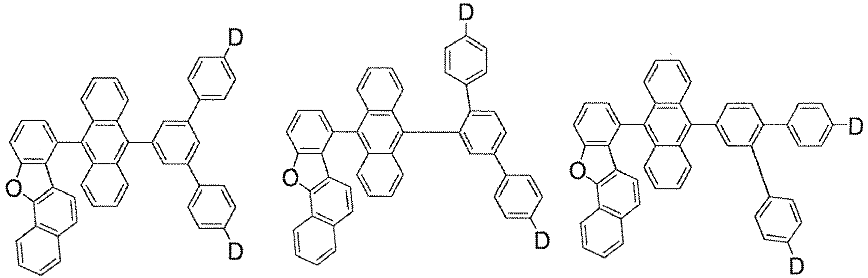

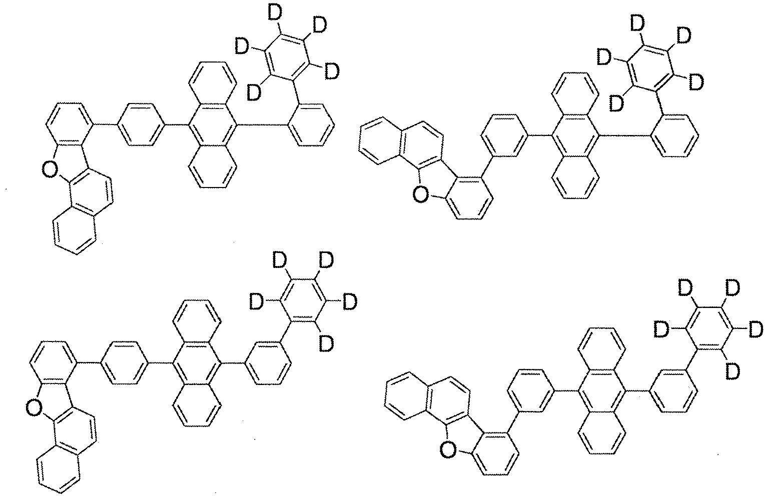

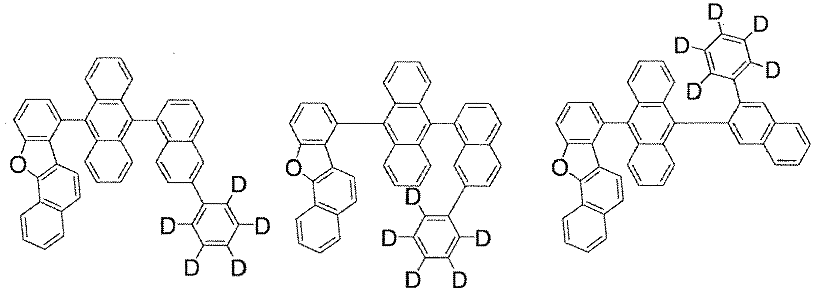

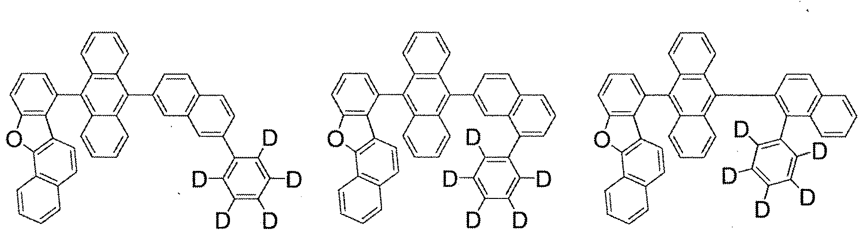

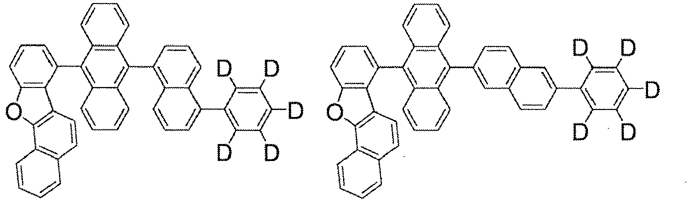

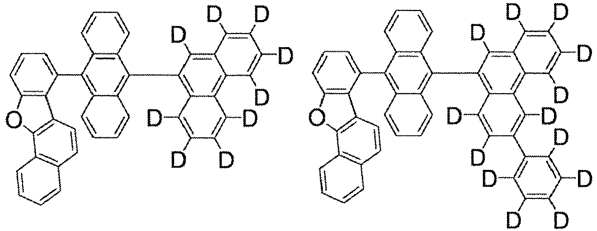

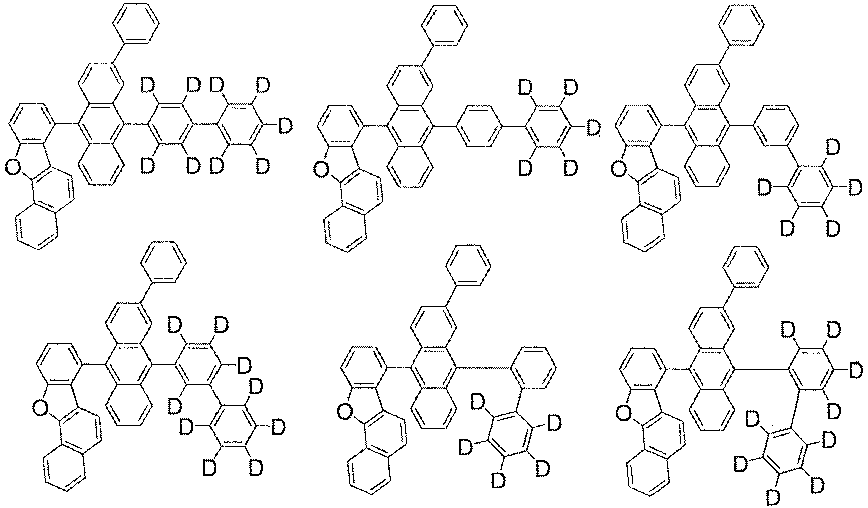

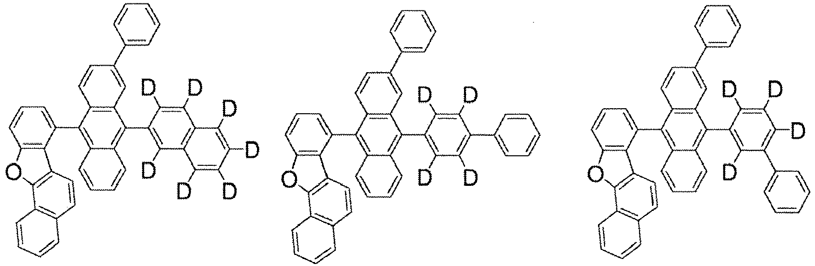

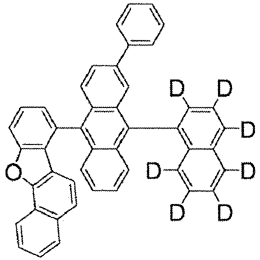

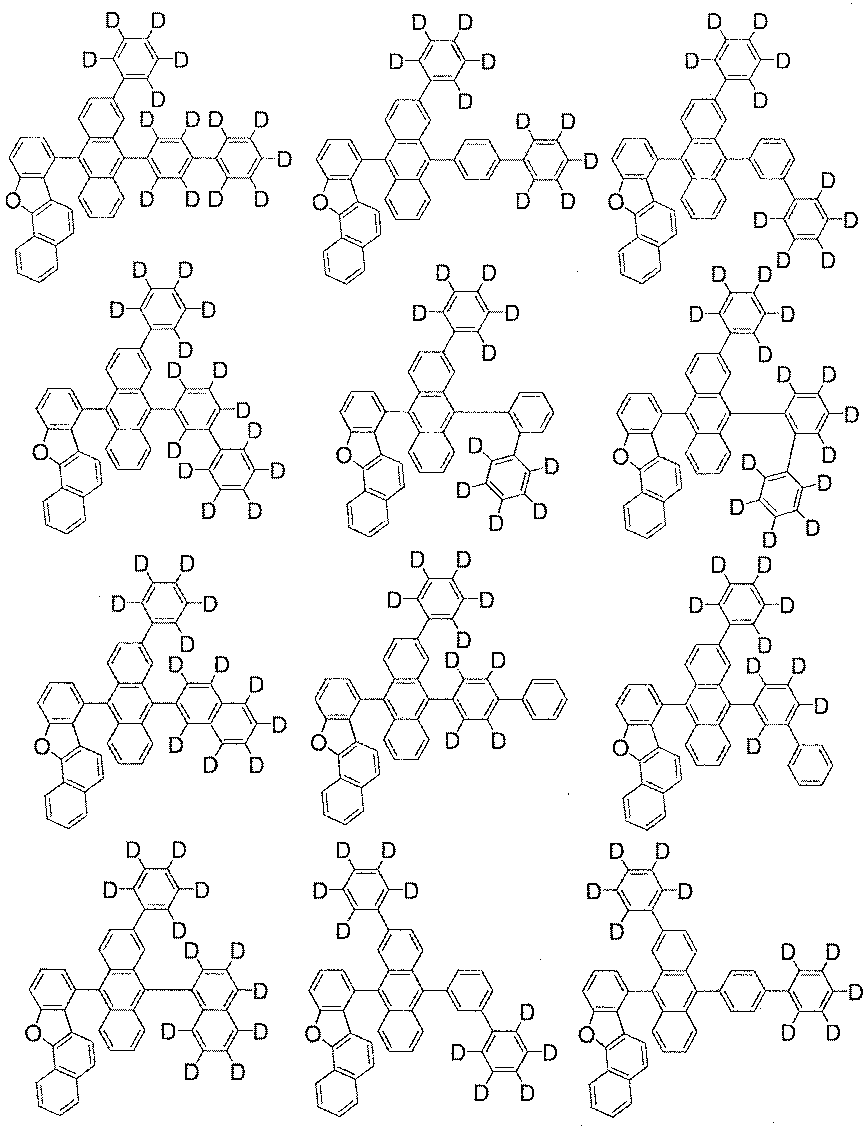

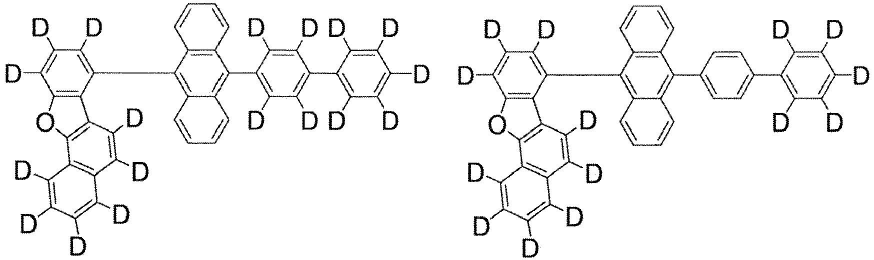

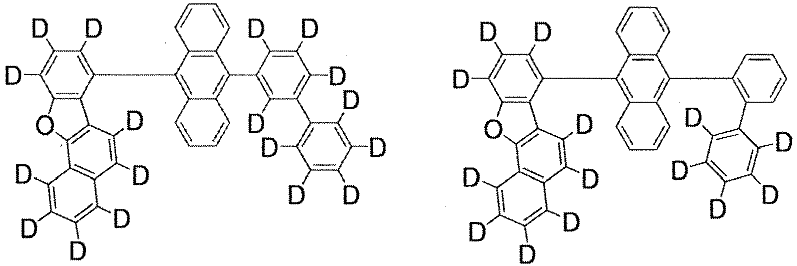

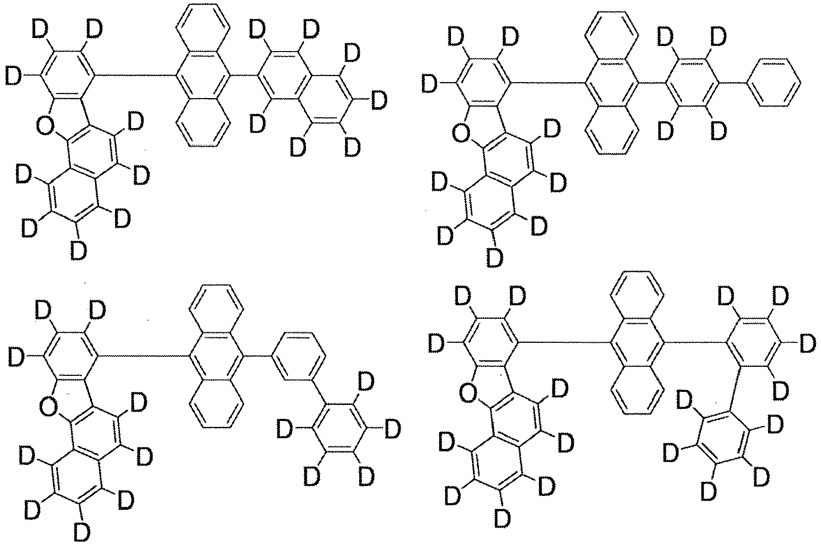

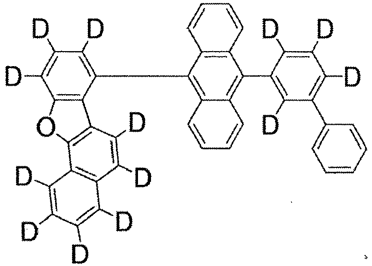

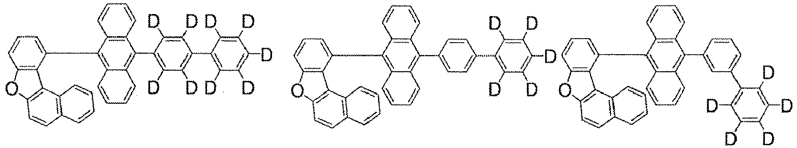

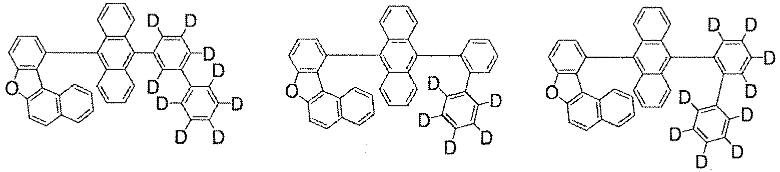

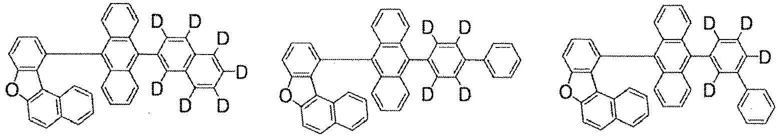

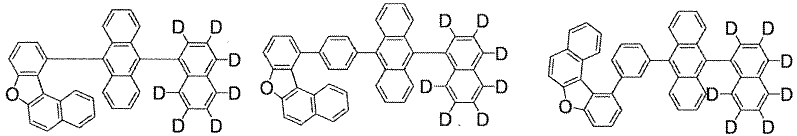

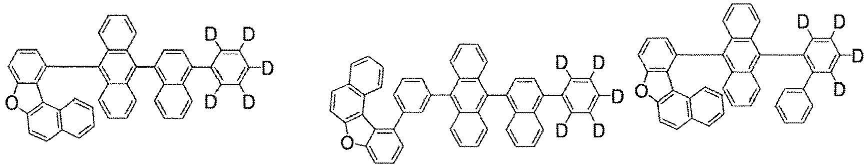

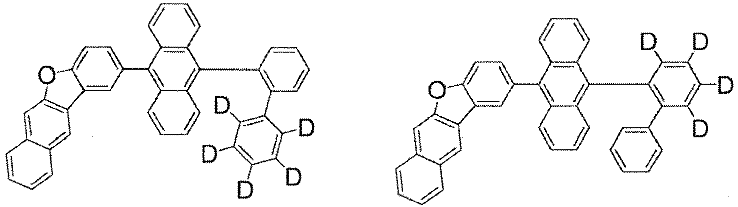

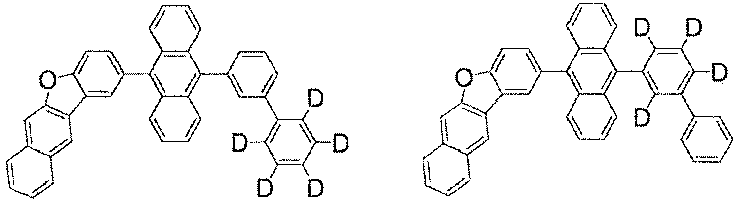

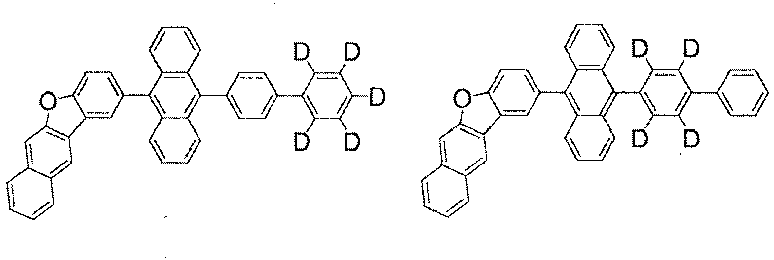

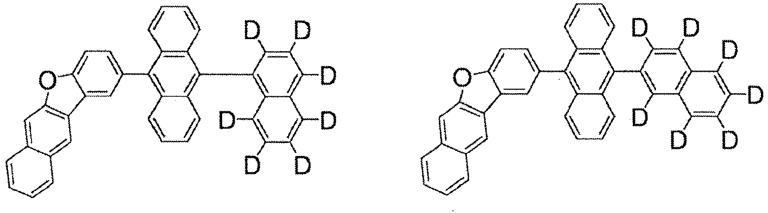

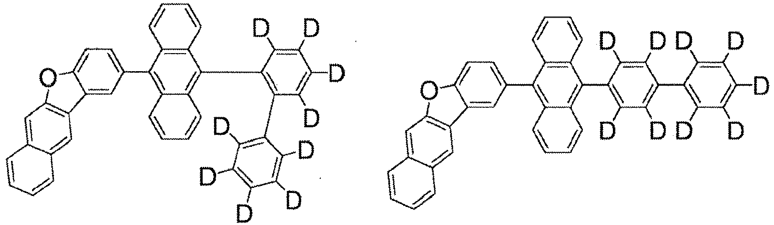

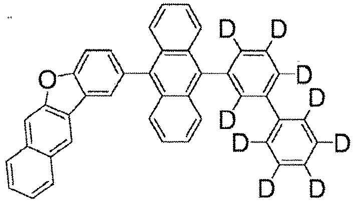

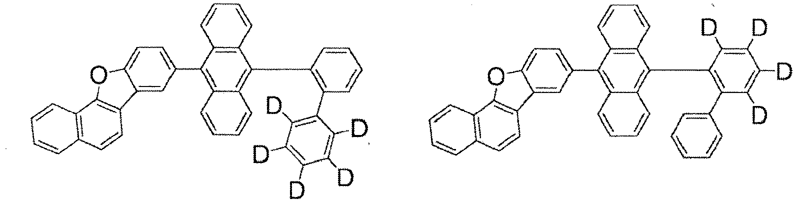

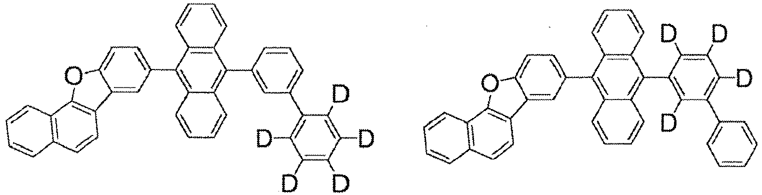

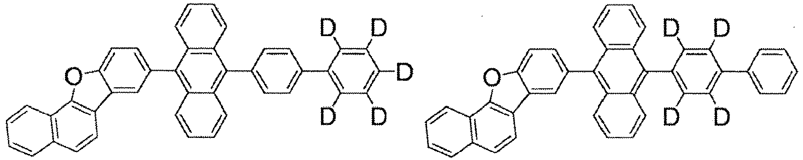

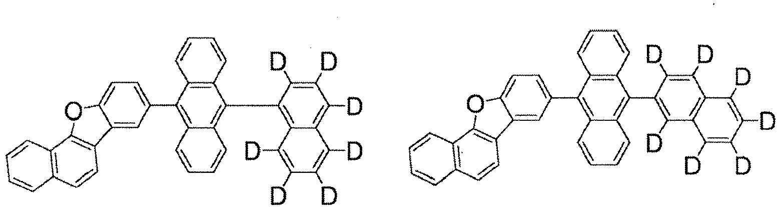

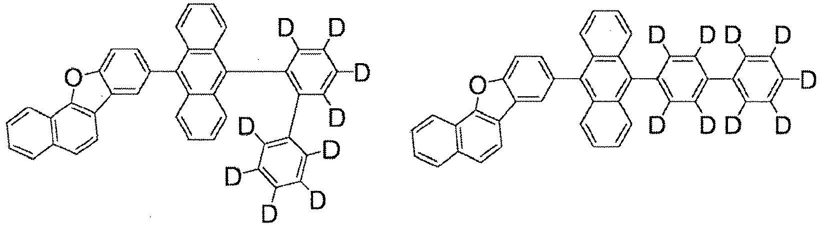

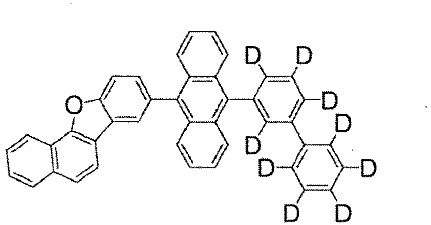

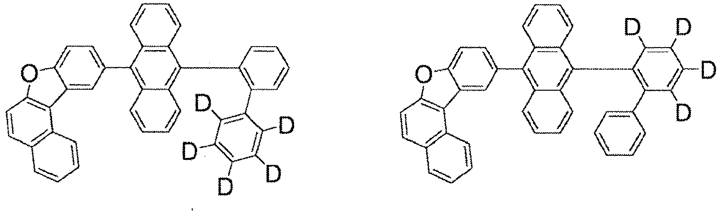

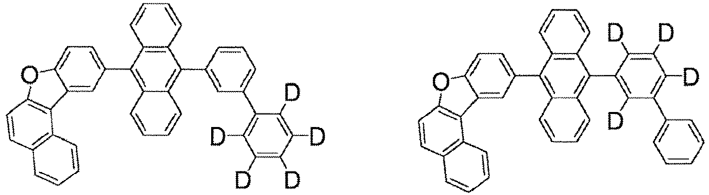

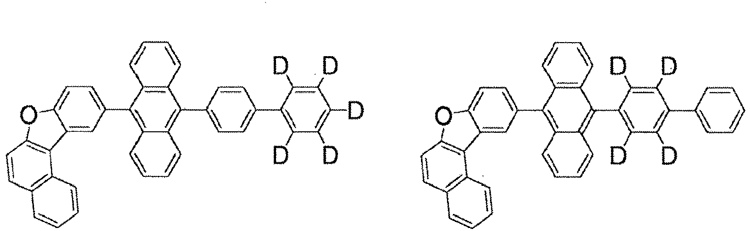

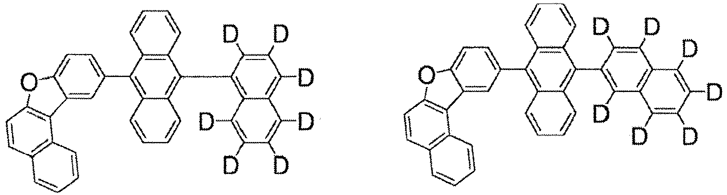

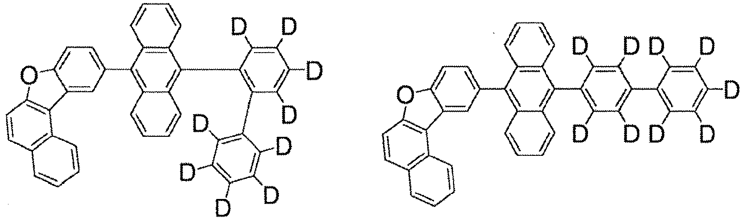

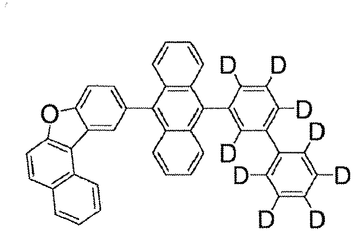

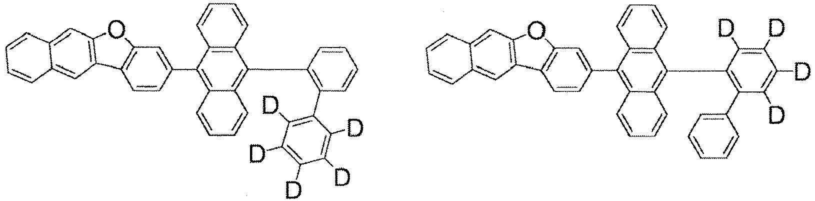

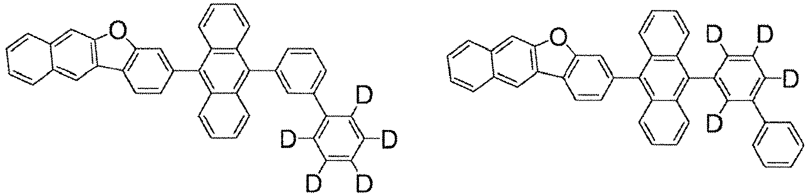

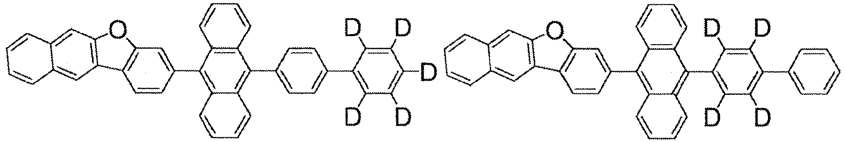

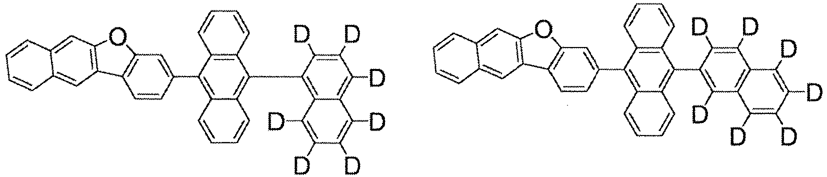

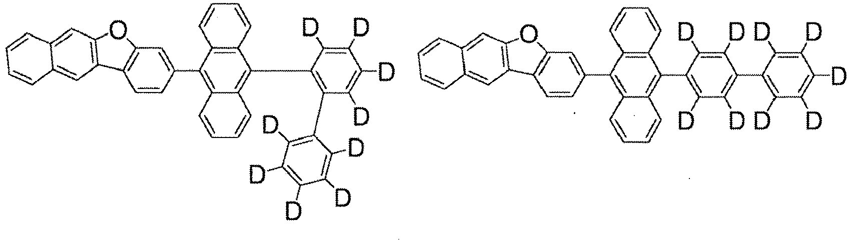

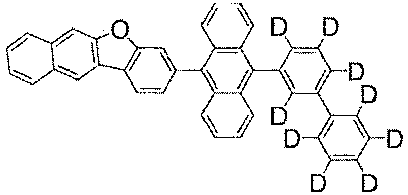

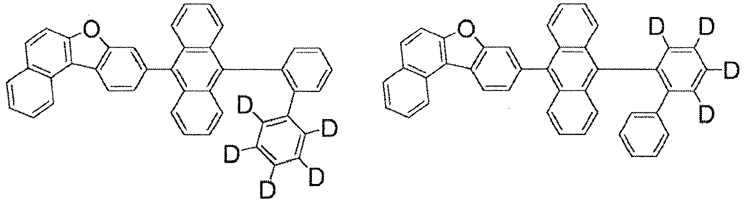

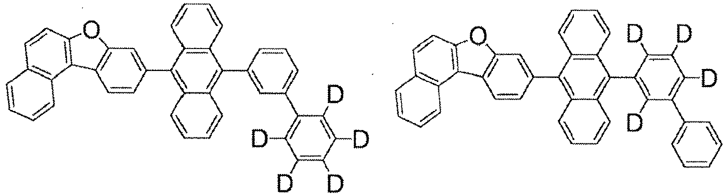

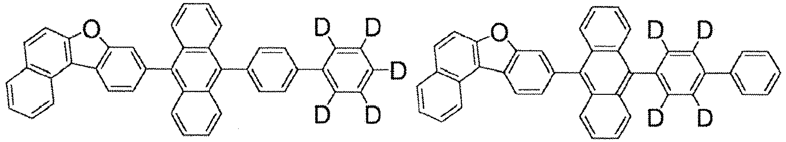

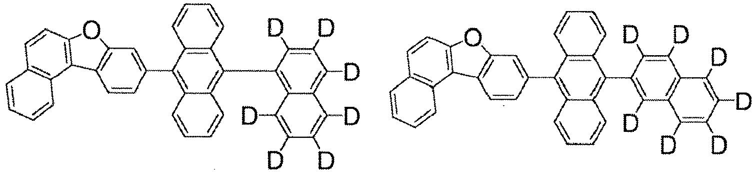

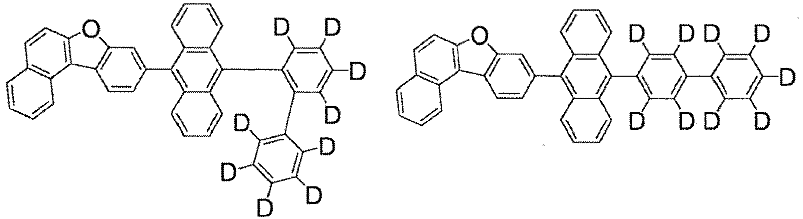

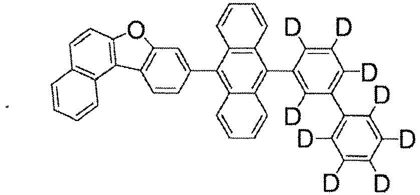

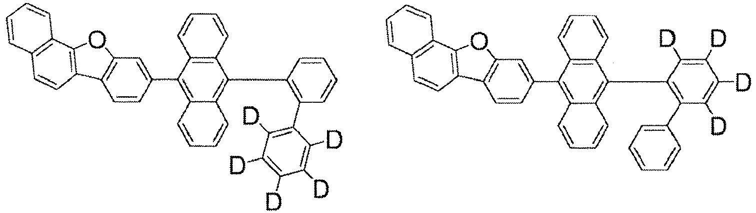

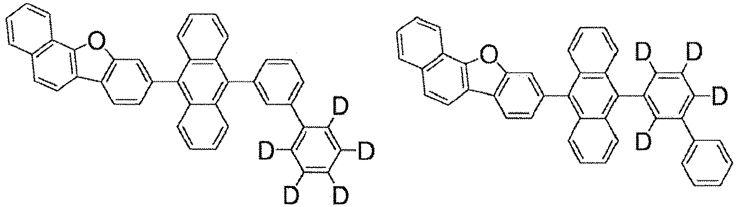

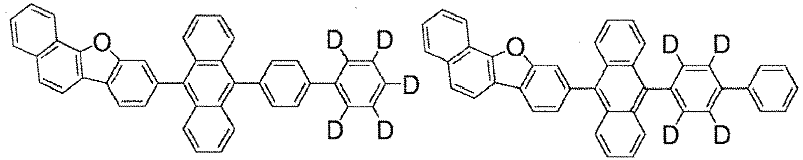

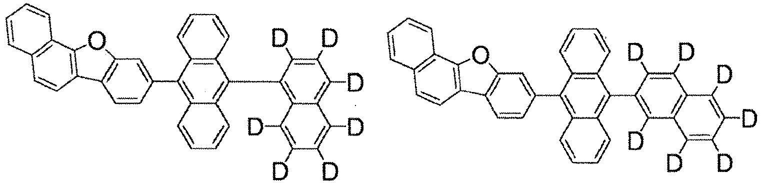

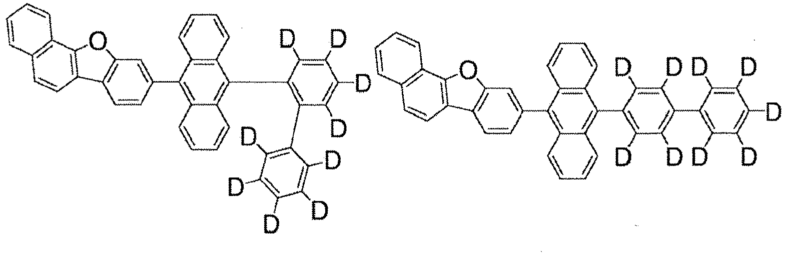

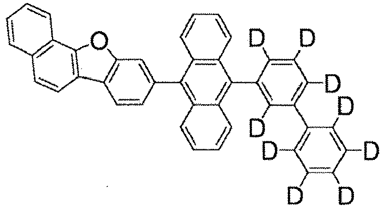

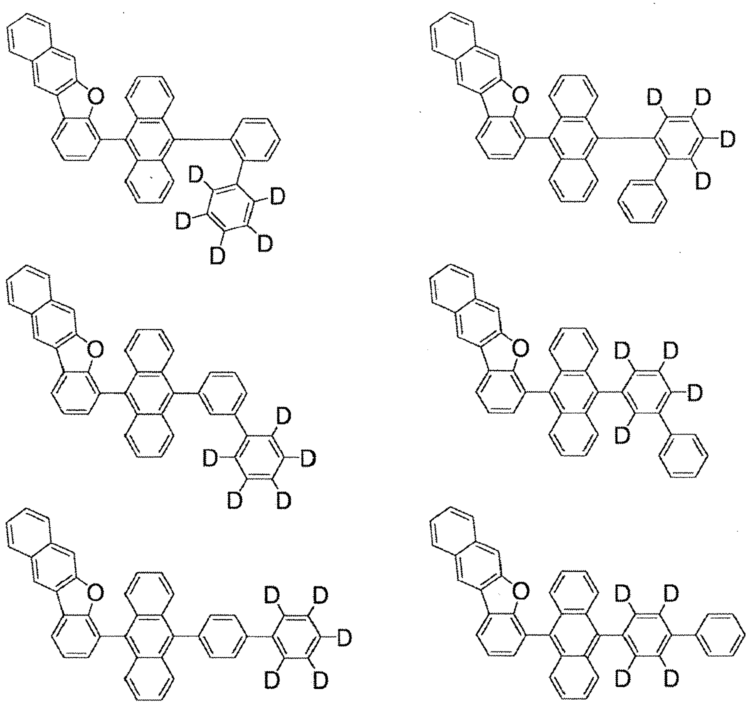

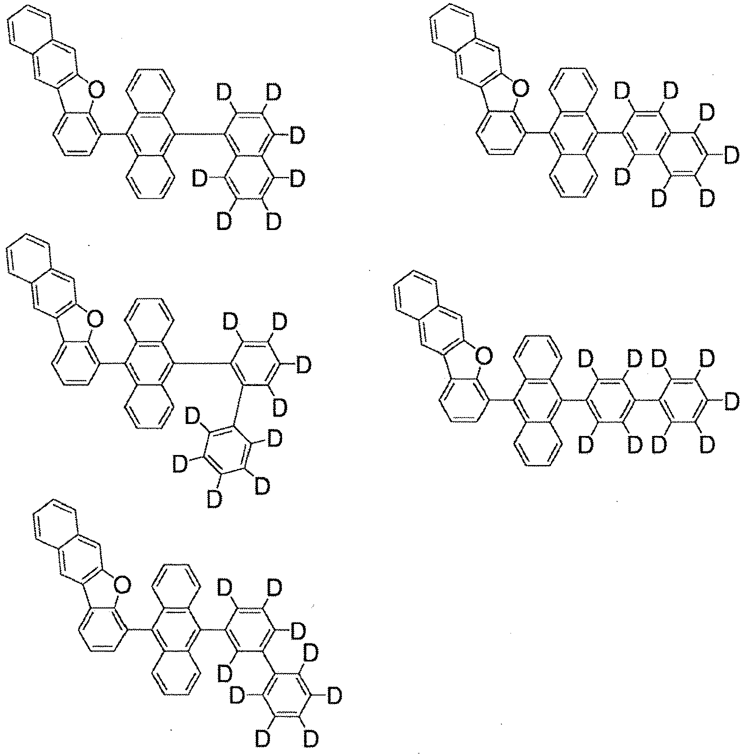

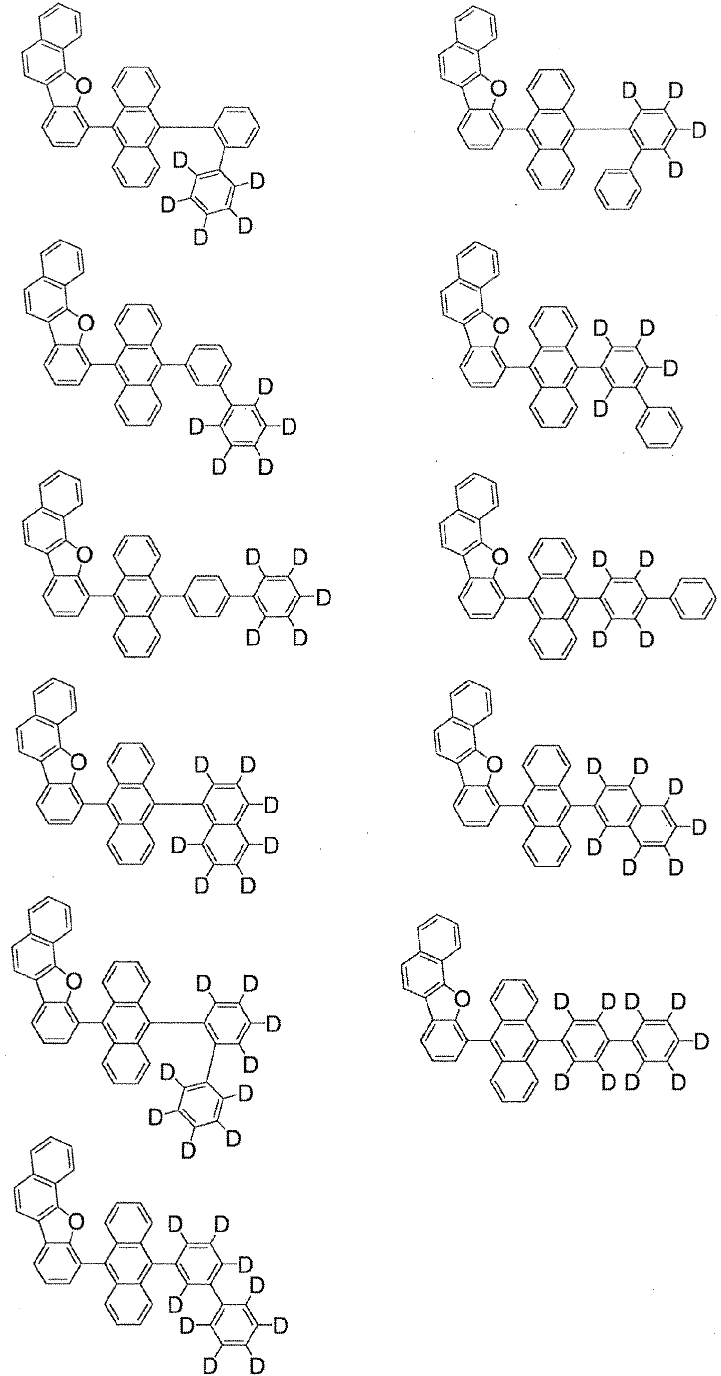

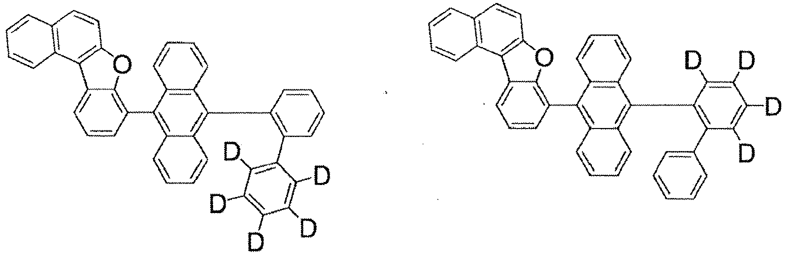

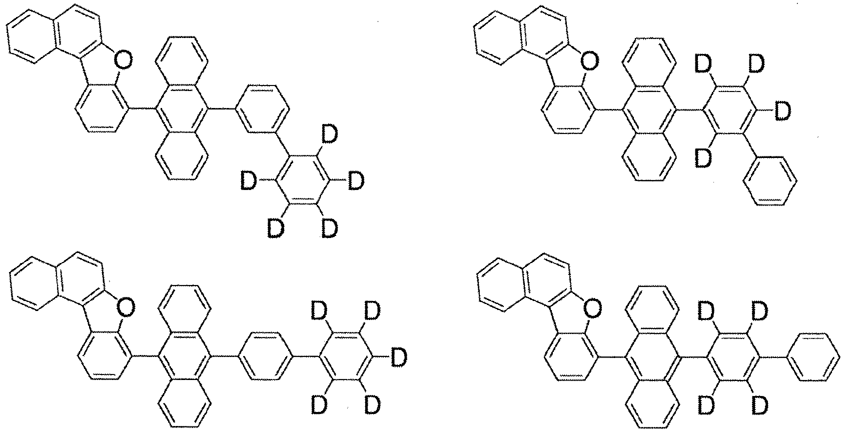

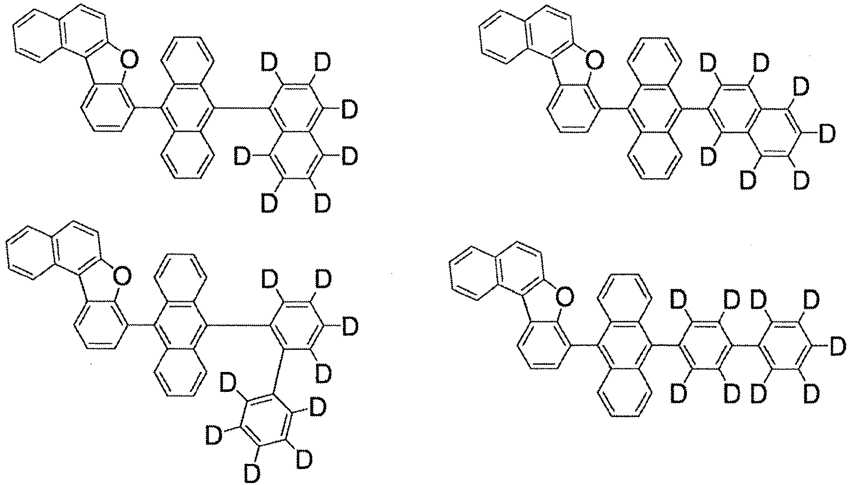

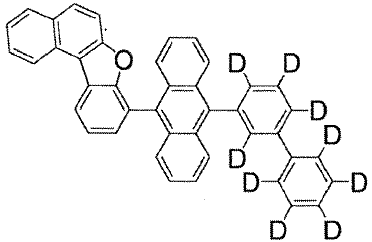

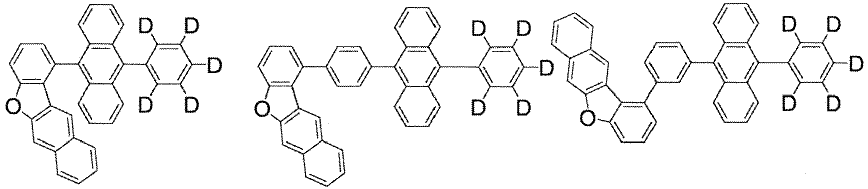

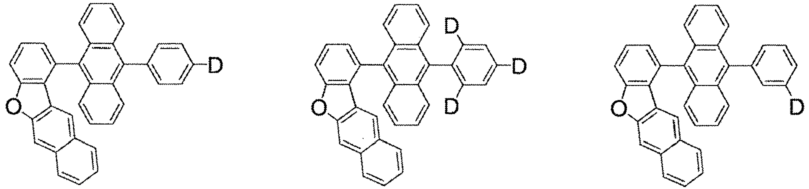

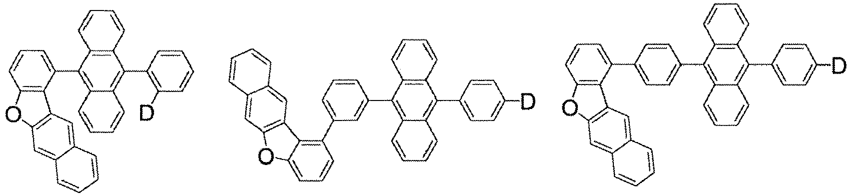

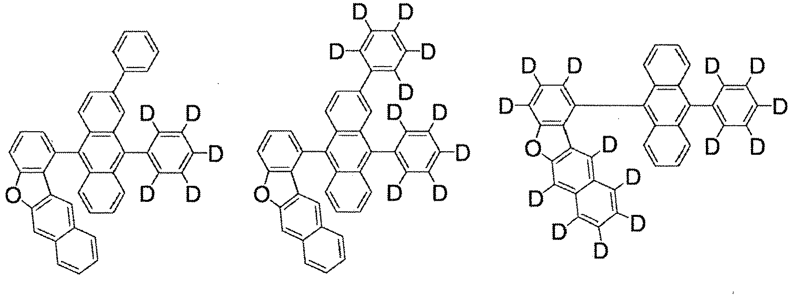

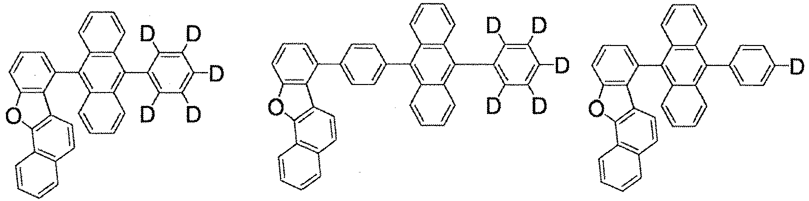

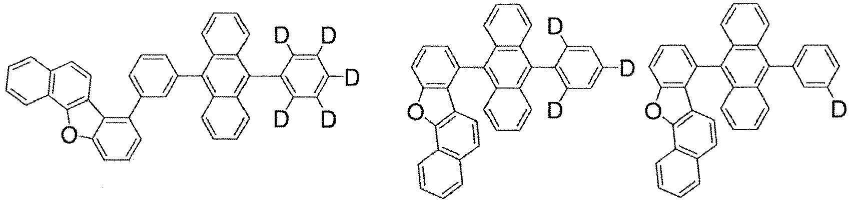

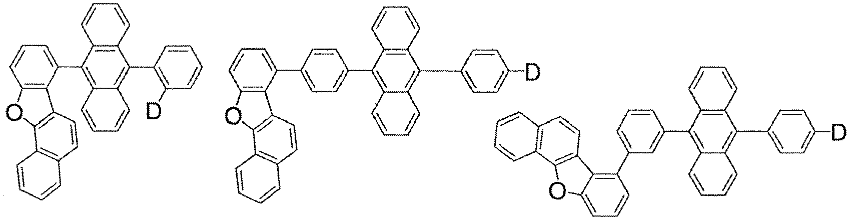

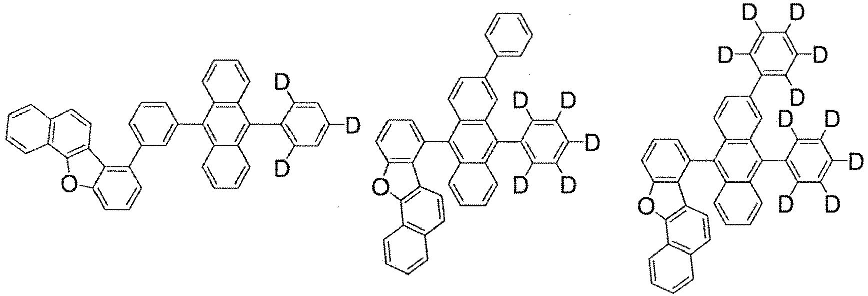

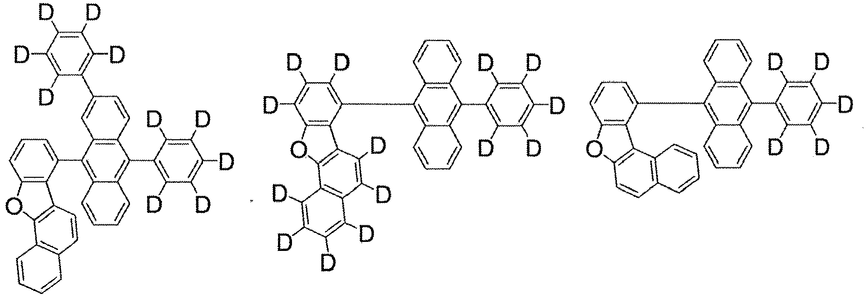

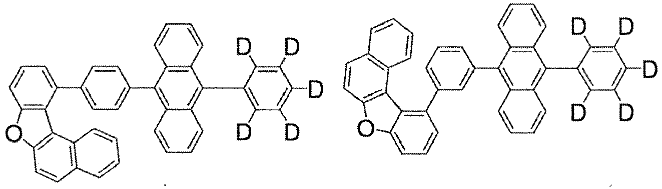

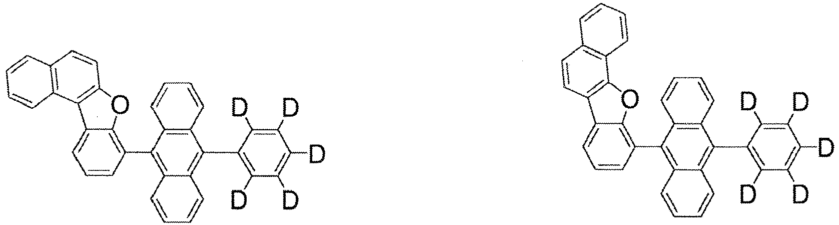

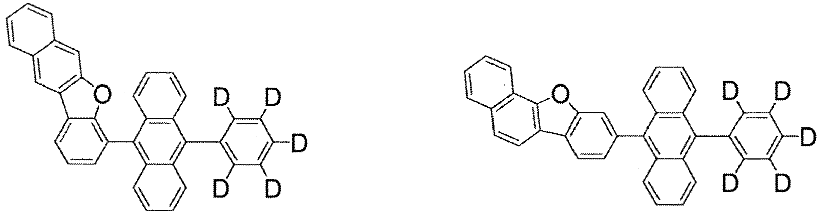

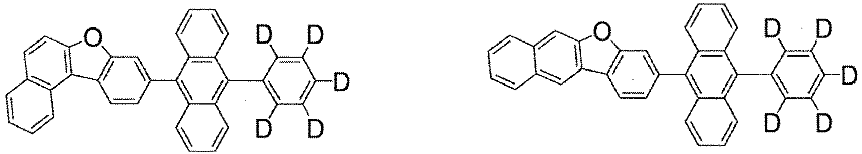

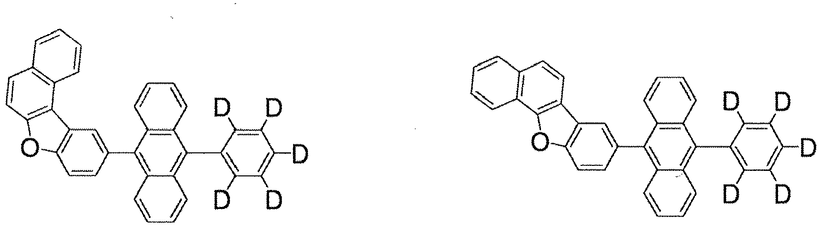

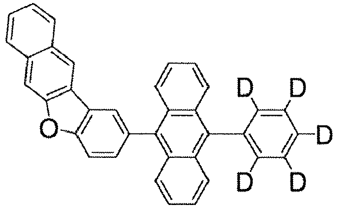

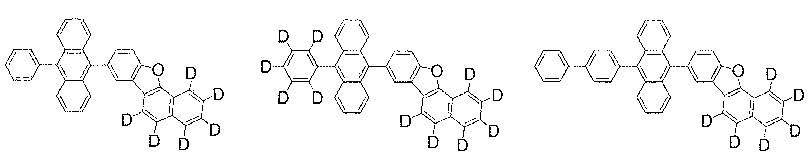

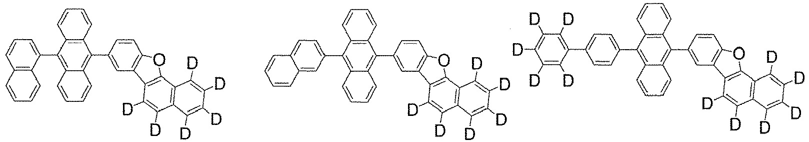

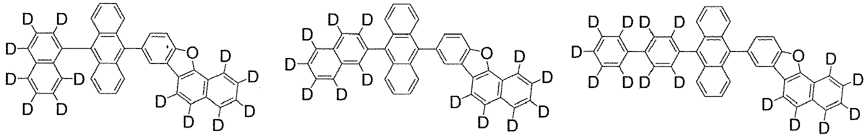

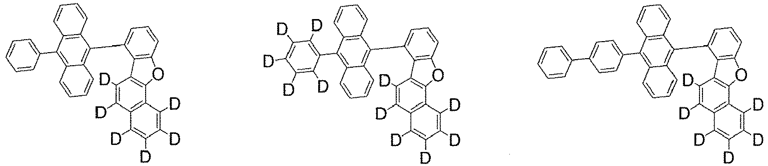

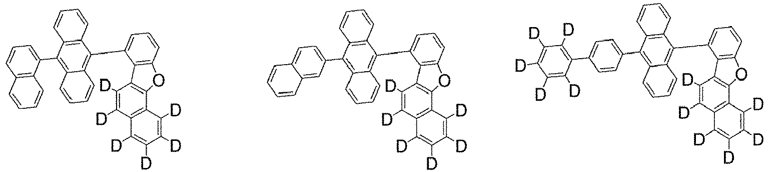

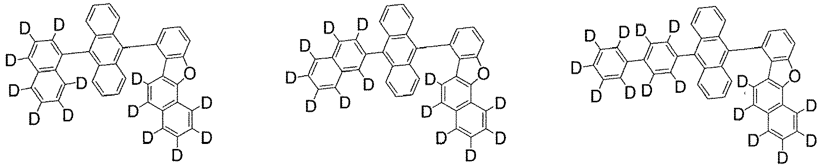

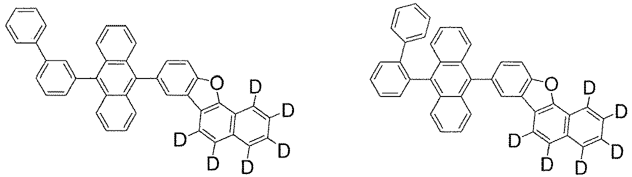

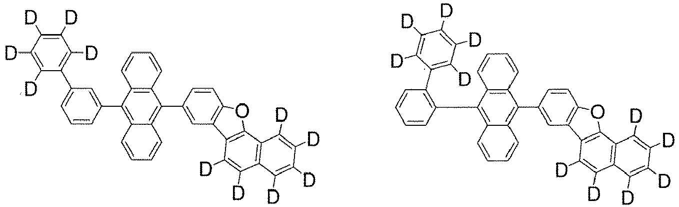

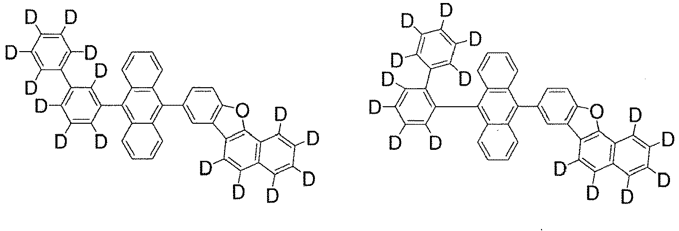

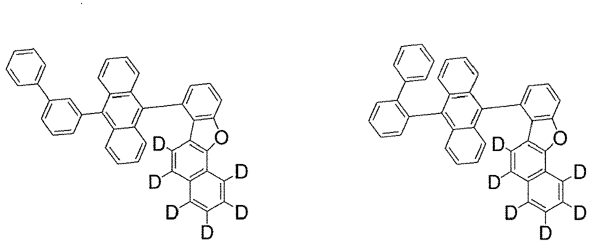

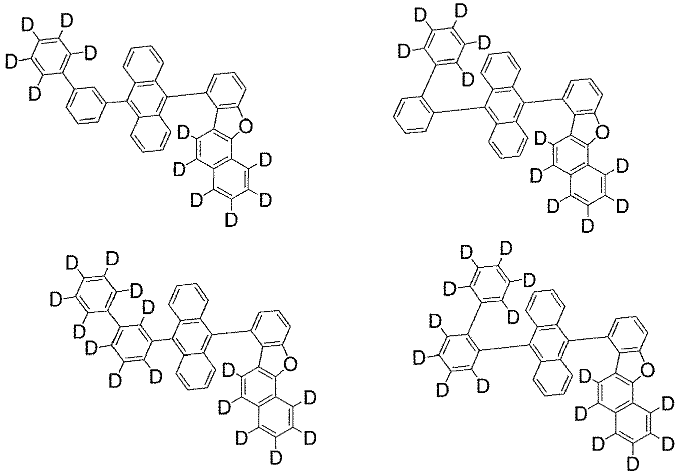

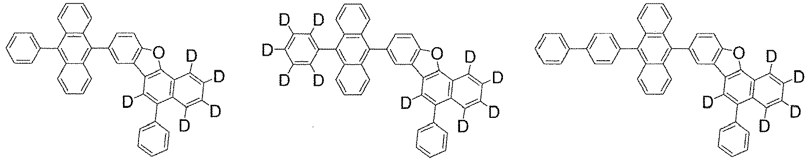

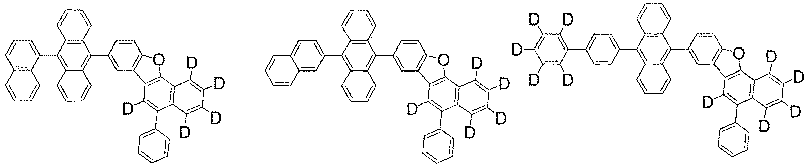

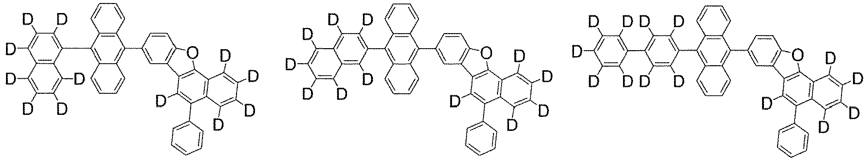

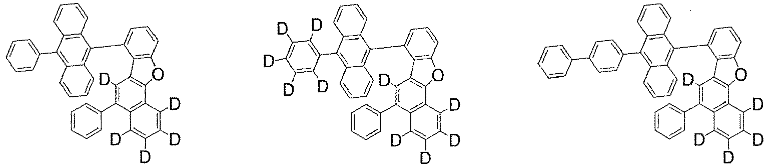

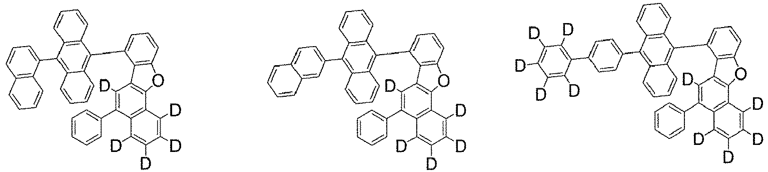

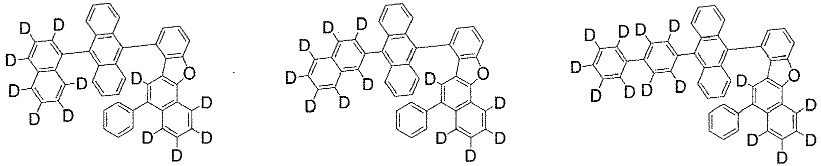

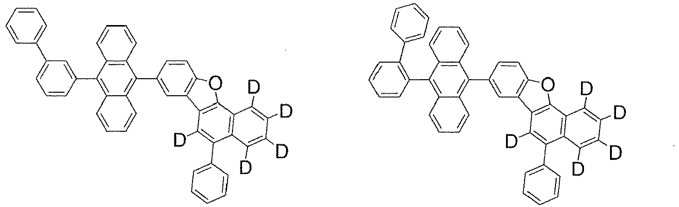

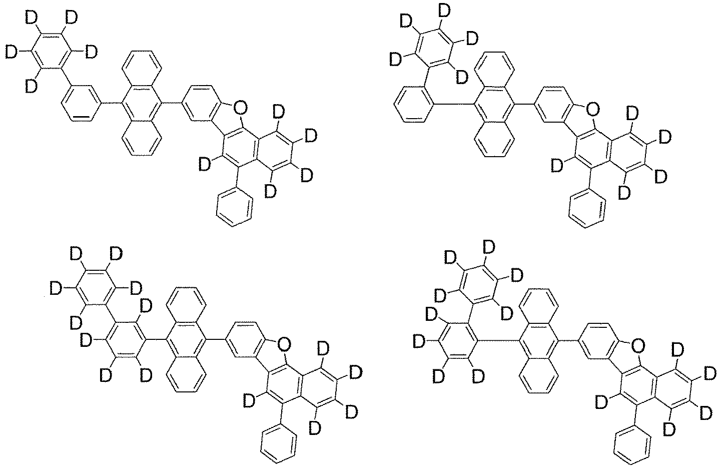

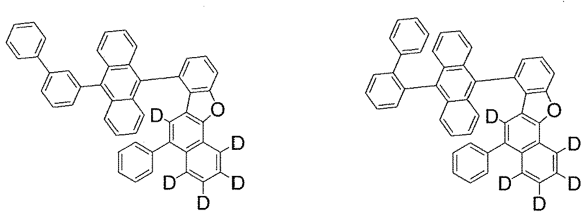

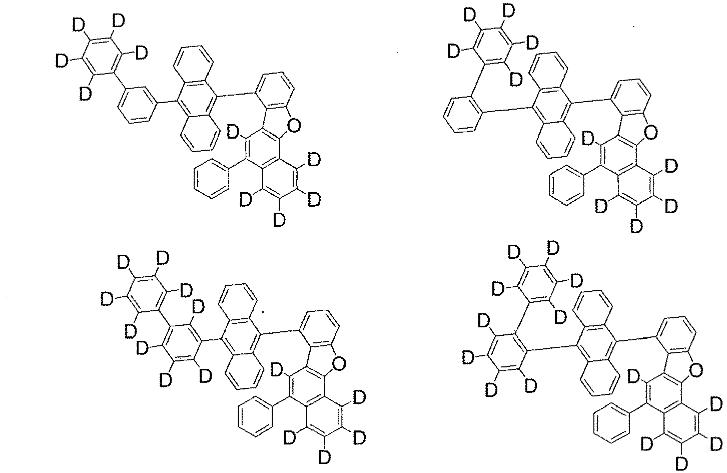

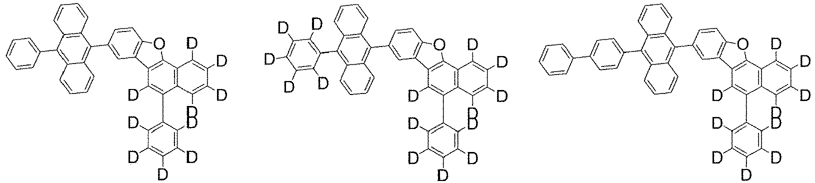

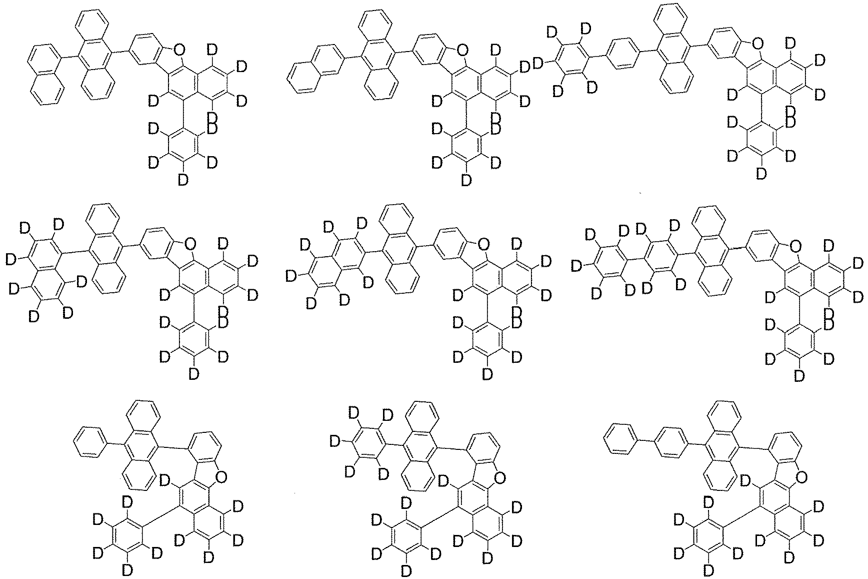

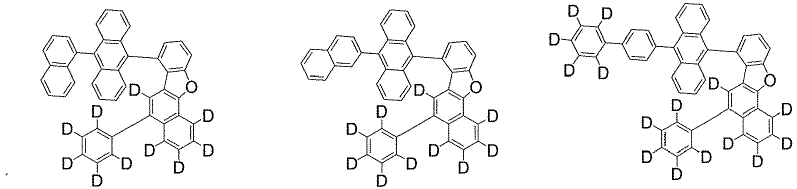

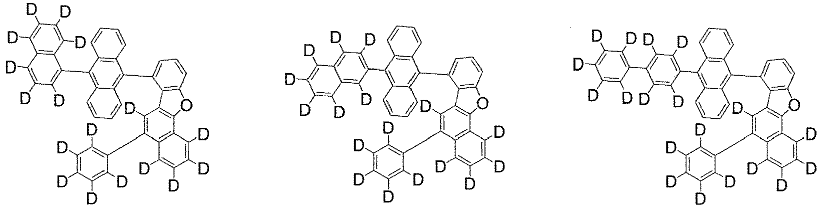

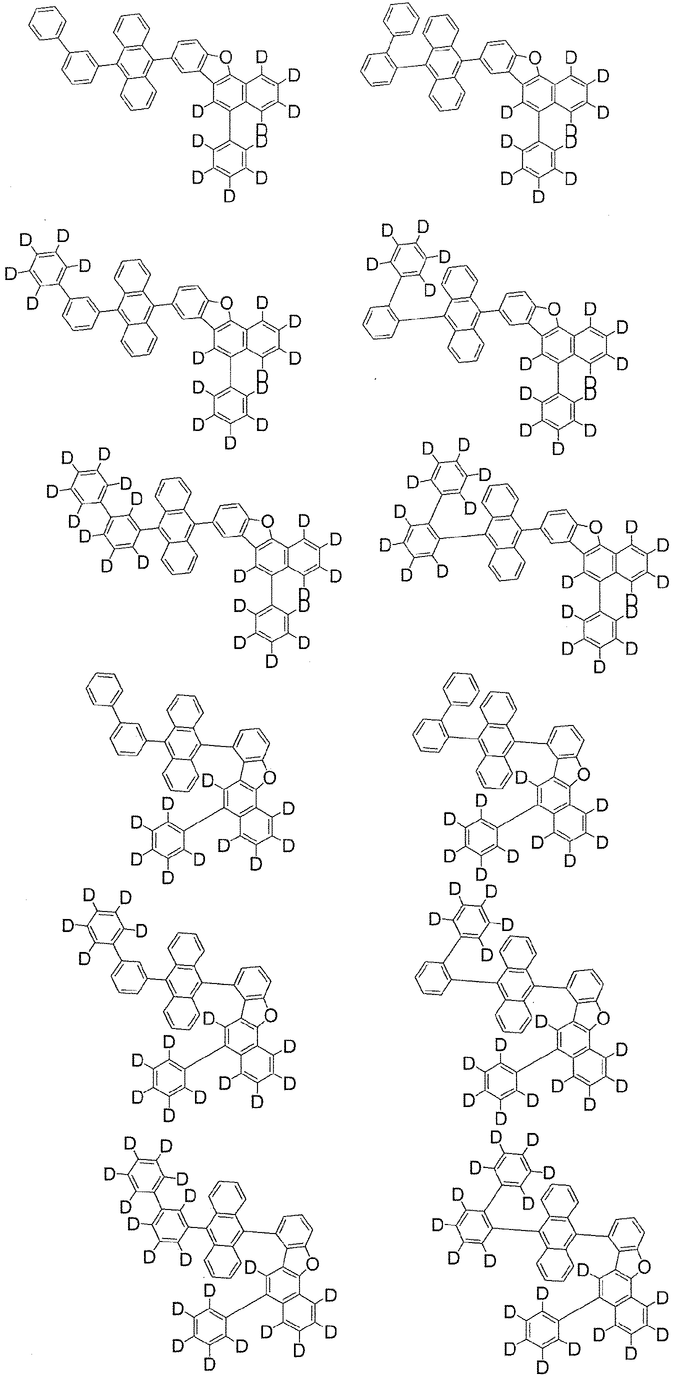

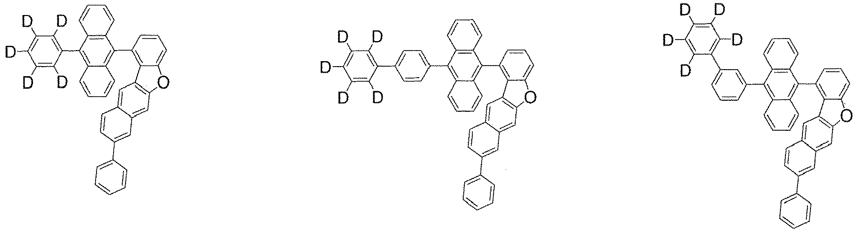

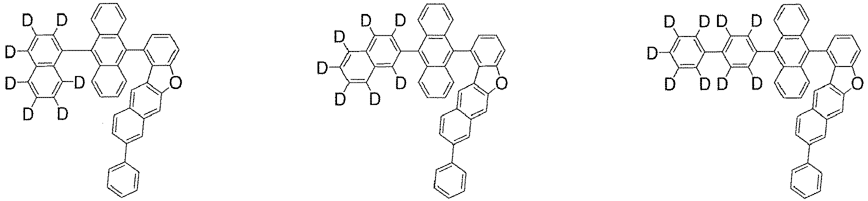

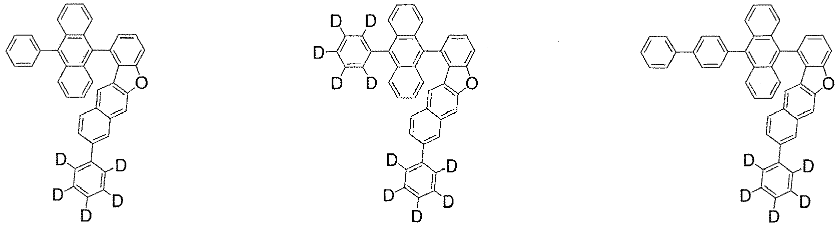

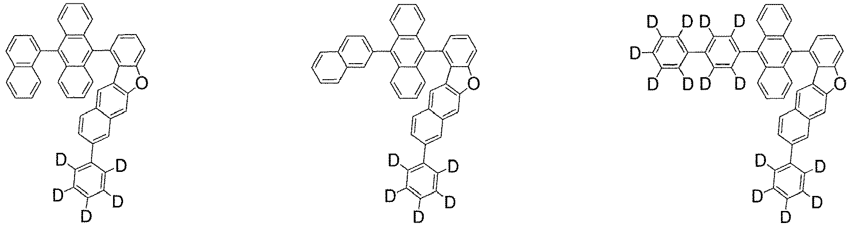

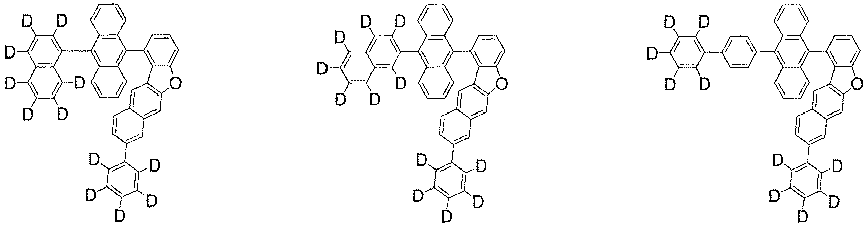

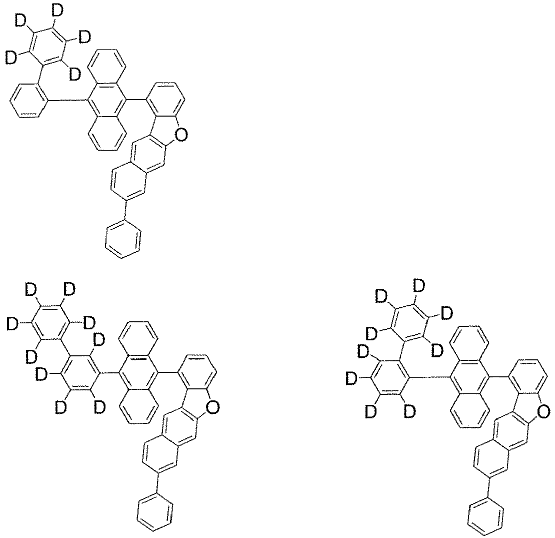

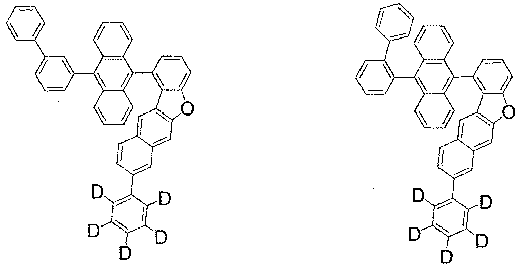

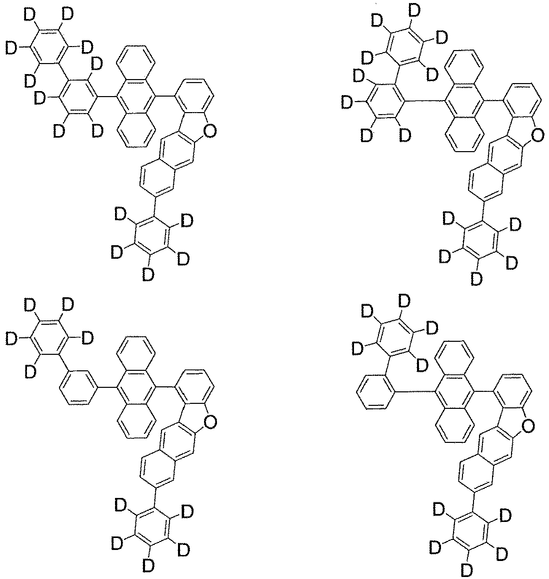

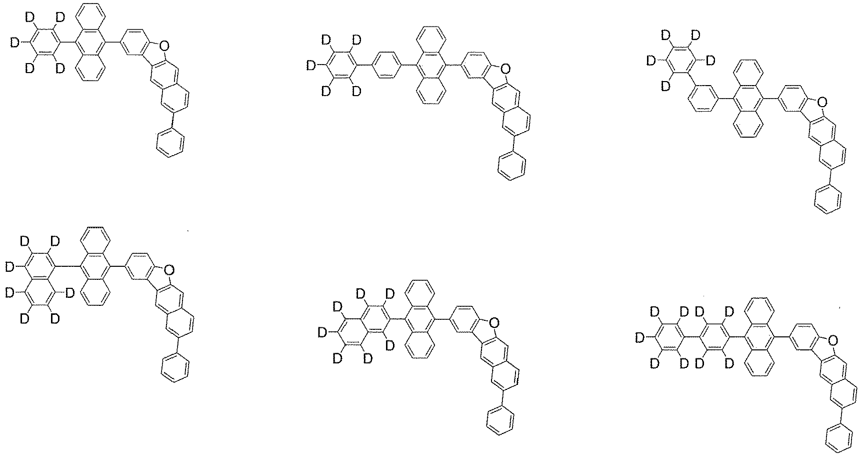

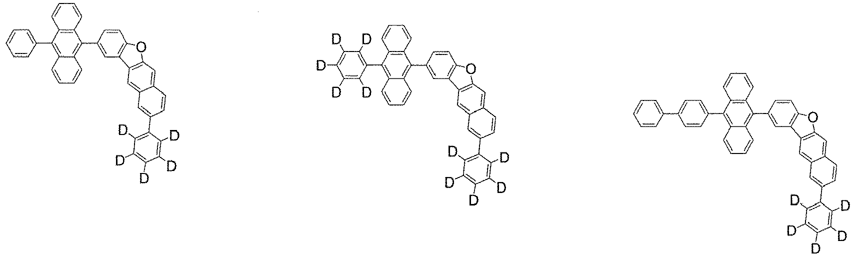

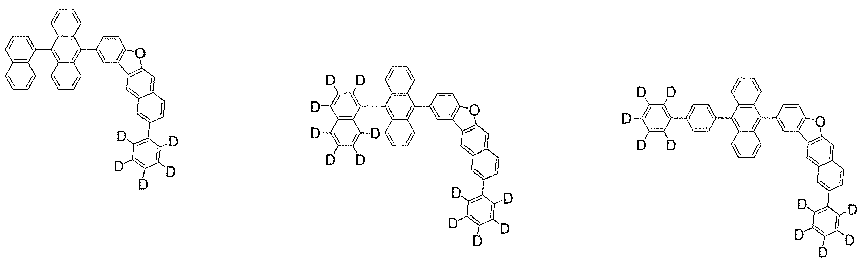

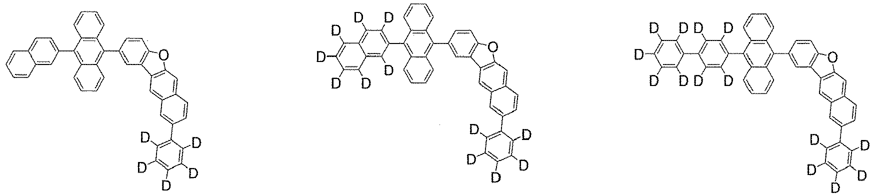

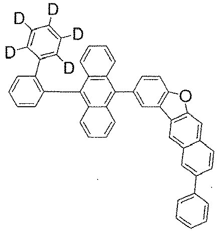

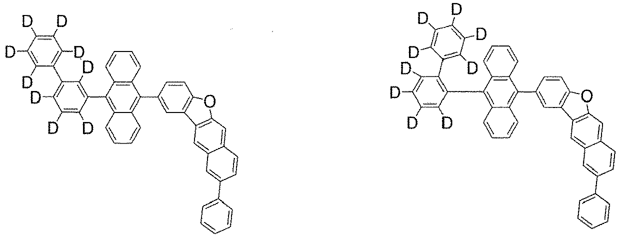

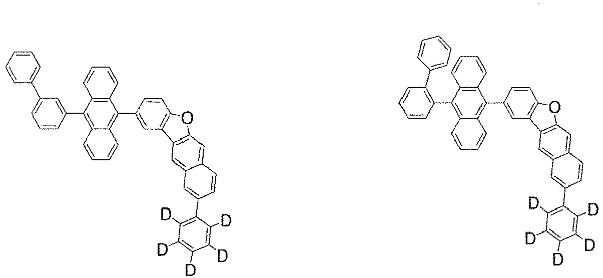

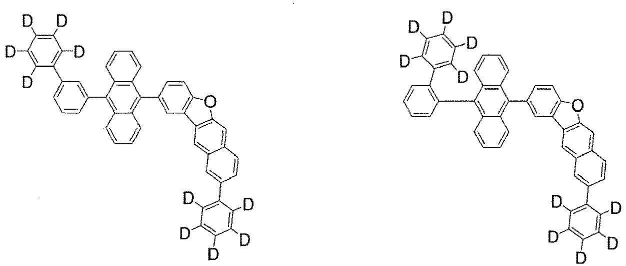

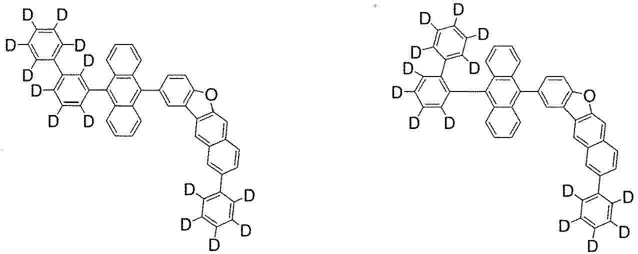

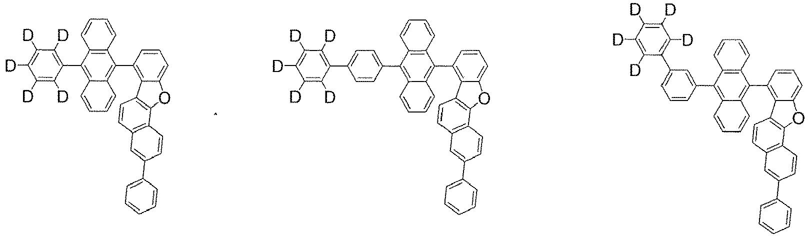

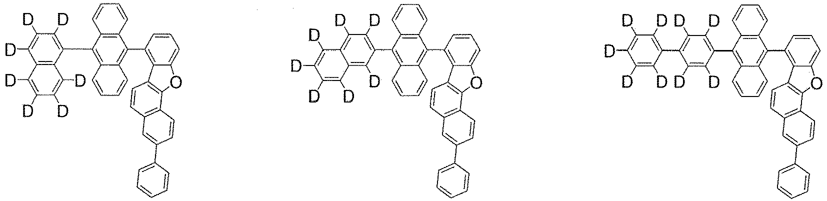

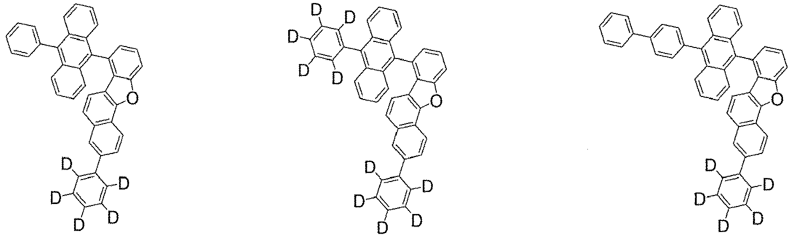

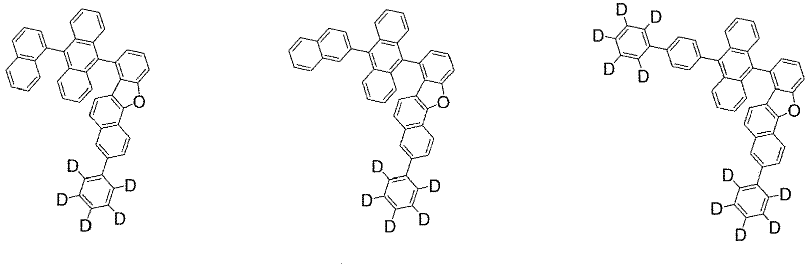

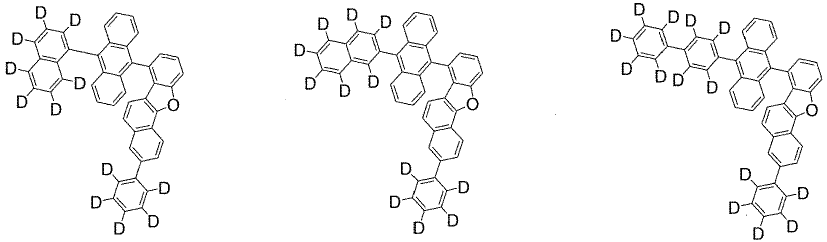

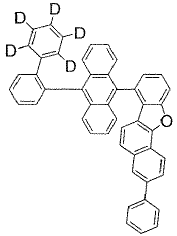

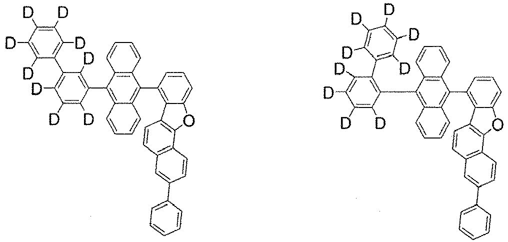

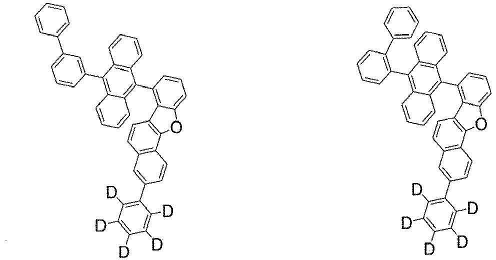

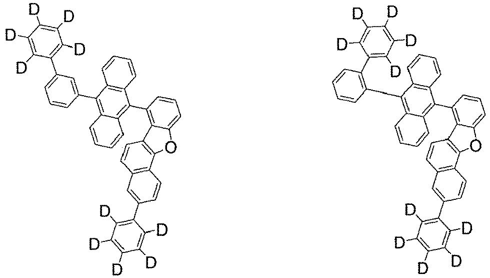

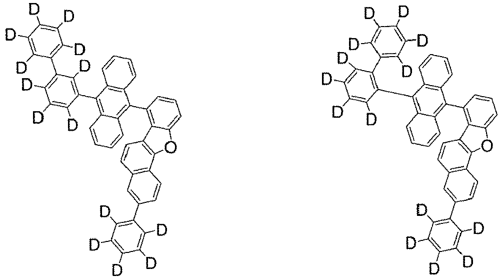

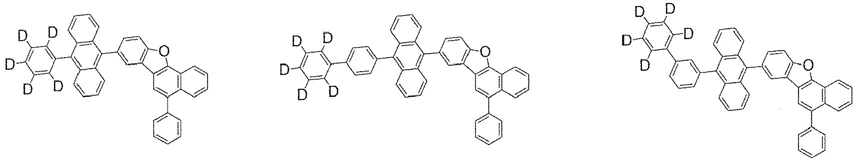

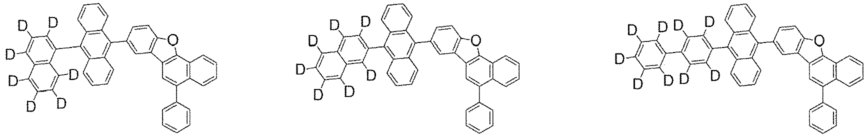

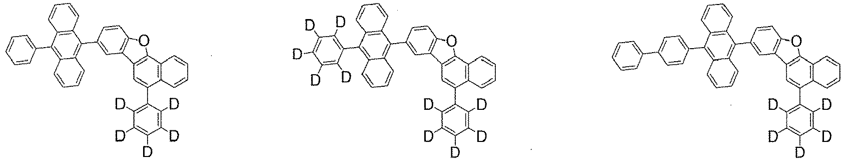

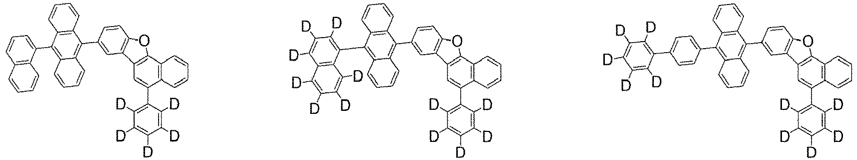

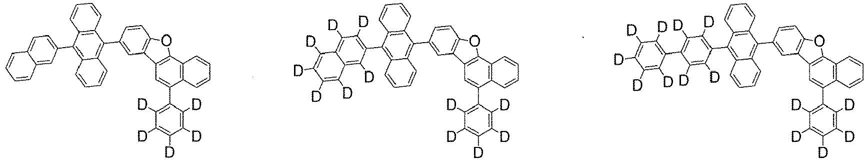

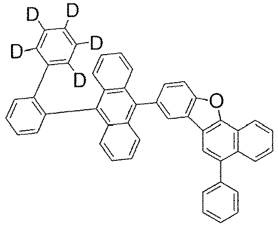

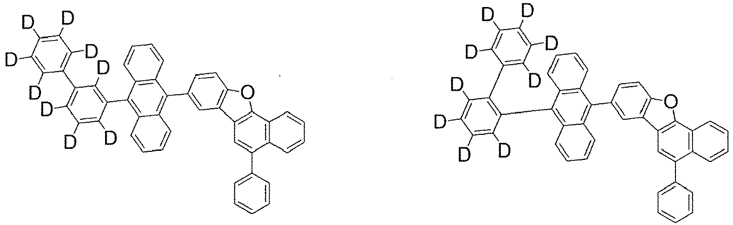

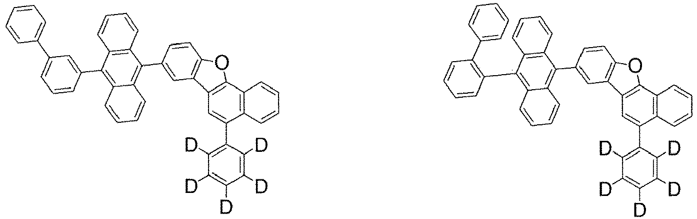

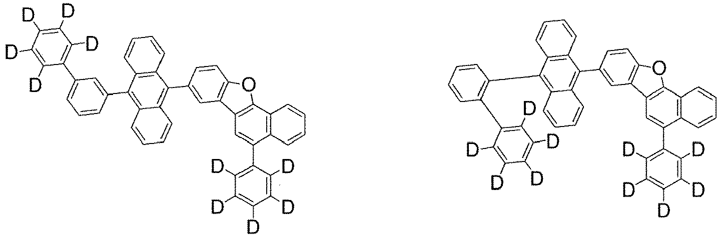

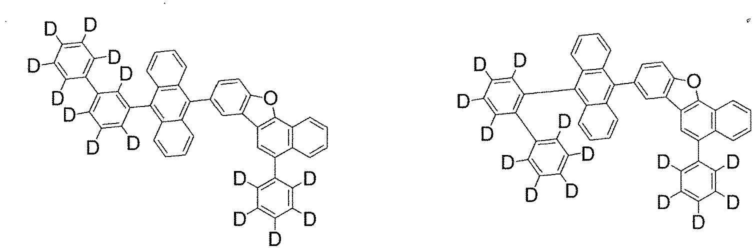

### [Compound represented by formula (41)]

A compound represented by the formula (41) is shown as follows: 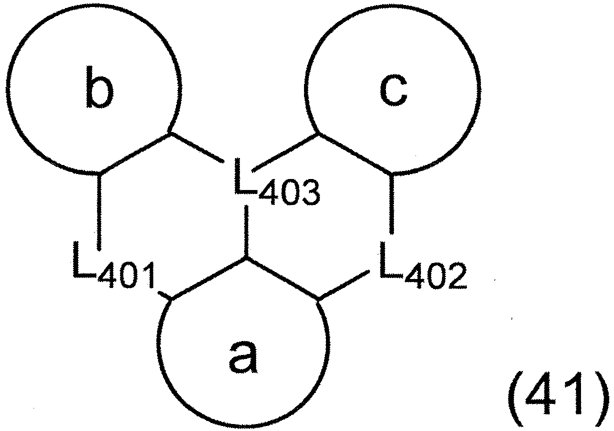 wherein in the formula (41),
a ring a, a ring b and a ring c are independently
a substituted or unsubstituted aromatic hydrocarbon ring having 6 to 50 ring carbon atoms, or
a substituted or unsubstituted heterocycle having 5 to 50 ring atoms;
L₄₀₁ and L₄₀₂ are independently O, S, Se, NR₄₀₁, C(R₄₀₂)(R₄₀₃), or Si(R₄₀₄)(R₄₀₅);
(wherein R₄₀₁ to R₄₀₅ form a substituted or unsubstituted, saturated or unsaturated ring by bonding with the ring a, the ring b or the ring c, or do not form the substituted or unsubstituted, saturated or unsaturated ring;
R₄₀₂ and R₄₀₃ form a substituted or unsubstituted, saturated or unsaturated ring by bonding with each other, or do not form the substituted or unsubstituted, saturated or unsaturated ring;
R₄₀₄ and R₄₀₅ form a substituted or unsubstituted, saturated or unsaturated ring by bonding with each other, or do not form the substituted or unsubstituted, saturated or unsaturated ring;
R₄₀₁ to R₄₀₅ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
when two or more of each of R₄₀₁ to R₄₀₅ are present, the two or more of each of R₄₀₁ to R₄₀₅ may be the same as or different from each other; and
L₄₀₃ is B, P, or P=O.

The compound represented by the formula (41) can be used as a guest (dopant) material in the emitting layer of the organic EL device. The guest material can be used in a constitution in which it is dispersed in another substance (host material). Since the compound represented by the formula (41) has high molecular planarity, it generally has high orientation to obtain effects that extracting efficiency of emission is enhanced. On the other hand, when it is dispersed in a host material having too high planarity, interaction of host materials with each other is increased to hardly cause energy transfer from the host material to the guest material and to be difficult to improve luminous efficiency.

Since the compound represented by the formula (1A) and the compound represented by the formula (1B) do not have too high molecular planarity as the host material, they can sufficiently ensure the energy transfer from the host material to the guest material when they are used in a constitution in which the compound represented by the formula (41) is dispersed in them. As a result, they can improve luminous efficiency and improve device lifetime.

It is preferable in terms of enhancing molecular planarity that one or more of the ring b and the ring c be a ring including a five-membered ring, and the five-membered ring be fused with a six-membered ring containing L₄₀₁ and L₄₀₃ or a six-membered ring containing L₄₀₂ and L₄₀₃. In such a case, since it suppresses an overlap of hydrogen atoms in the ring b and hydrogen atoms in the ring c, and planarity thereof is maintained, molecular planarity thereof is enhaced. As a result, when it is used in a constitution in which the compound represented by the formula (41) as the guest material is dispersed in the compound represented by the formula (1A) or (1B) as the host material, it is expected that luminous efficiency is improved and improving effects of device lifetime are enhanced.

The following figure is shown as an example where both of the ring b and the ring c are a benzofuran ring including a five-membered ring, and the five-membered ring (furan ring) is fused with each of a six-membered ring containing L₄₀₁ and L₄₀₃ and a six-membered ring containing L₄₀₂ and L₄₀₃.

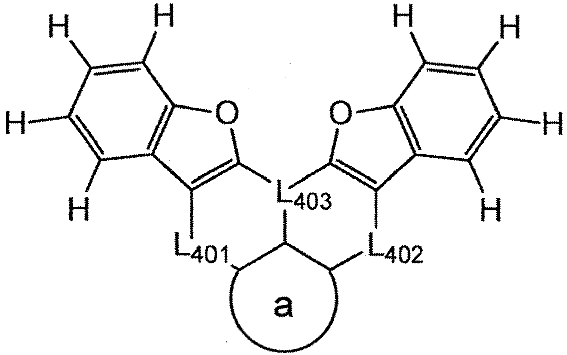

In one embodiment, the compound represented by the formula (41) is selected from the group consisting of compounds represented by the following formulas (41-1) to (41-6): 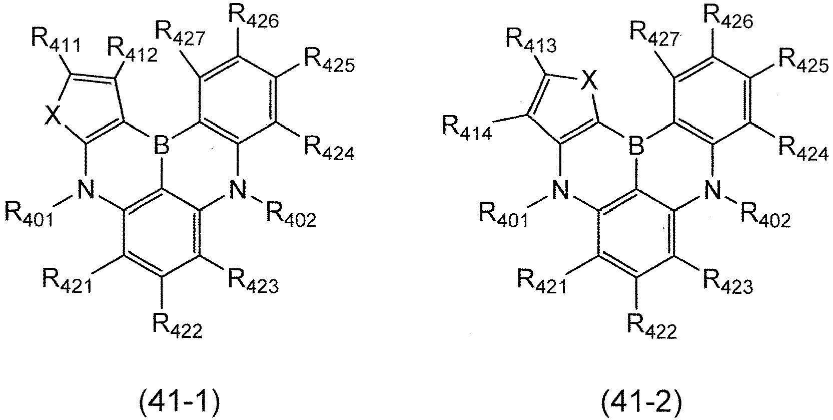 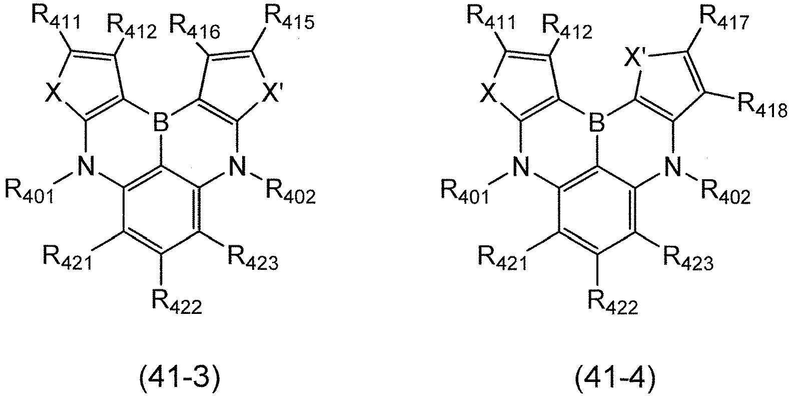 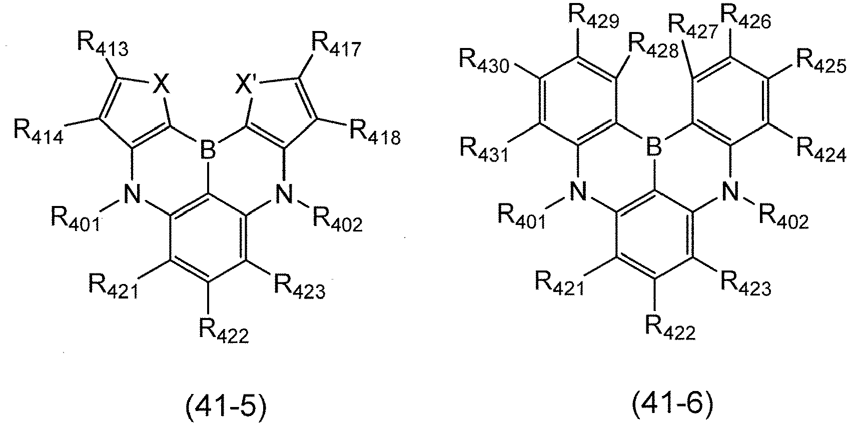 wherein in the formula (41-1),
X is O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₂₇ and R₄₁₂; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, and R₄₂₁ to R₄₂₇ which do not form the substituted or unsubstituted, saturated or unsaturated ring are independently a hydrogen atom or a substituent R;
in the formula (41-2),
X is O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₁₃ and R₄₁₄; and R₄₁₄ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or a substituted or unsubstituted monovalent heterocyclic group having 5 to 50 ring atoms;
R₄₀₃ to R₄₀₅, and R₄₁₃, R₄₁₄, and R₄₂₁ to R₄₂₇ which do not form the substituted or unsubstituted, saturated or unsaturated ring are independently a hydrogen atom or a substituent R;
in the formula (41-3),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₁₅ and R₄₁₆; R₄₁₆ and R₄₁₂; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, R₄₁₅, R₄₁₆, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-4),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₁₈; R₄₁₈ and R₄₁₇; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or a substituted or unsubstituted monovalent heterocyclic group having 5 to 50 ring atoms;
R₄₀₃ to R₄₀₅, and R₄₁₁, R₄₁₂, R₄₁₇, R₄₁₈, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-5),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₁₈; R₄₁₈ and R₄₁₇; R₄₁₃ and R₄₁₄; and R₄₁₄ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or a substituted or unsubstituted monovalent heterocyclic group having 5 to 50 ring atoms;
R₄₀₃ to R₄₀₅, and R₄₁₁, R₄₁₂, R₄₁₇, R₄₁₈, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-6),
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₂₇ and R₄₂₈; the adjacent two or more of Raza to R₄₃₁; and R₄₃₁ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or a substituted or unsubstituted monovalent heterocyclic group having 5 to 50 ring atoms;
R₄₂₁ to R₄₃₁ which do not form the substituted or unsubstituted, saturated or unsaturated ring are independently a hydrogen atom or a substituent R; and
the substituent R is the same as defined in the formula (1A).

In one embodiment, the compound represented by the formula (41) is selected from the group consisting of compounds represented by the formulas (41-1) to (41-5).

In one embodiment, the compound represented by the formula (41) is a compound represented by the following formula (42-2): 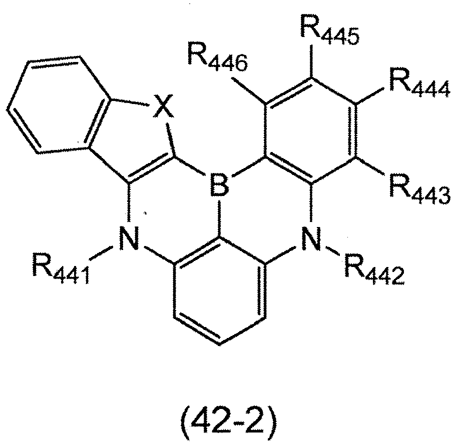 wherein in the formula (42-2),
R₄₄₁ and R₄₄₂ are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
Raas to R₄₄₆ are independently a hydrogen atom or a substituent R;
X is O, or S; and
the substituent R is the same as defined in the formula (1A).

In one embodiment, a substituent in the case of "substituted or unsubstituted" in the formula (41) is selected from the group consisting of
an alkyl group having 1 to 50 carbon atoms,
a haloalkyl group having 1 to 50 carbon atoms,
an alkenyl group having 2 to 50 carbon atoms,
an alkynyl group having 2 to 50 carbon atoms,
a cycloalkyl group having 3 to 50 ring carbon atoms,
an alkoxy group having 1 to 50 carbon atoms,
an alkylthio group having 1 to 50 carbon atoms,
an aryloxy group having 6 to 50 ring carbon atoms,
an arylthio group having 6 to 50 ring carbon atoms,
an aralkyl group having 7 to 50 carbon atoms,
-Si(R₄₁)(R₄₂)(R₄₃),
-C(=O)R₄₄, -COOR₄₅,
-S(=O)₂R₄₆,
-P(=O)(R₄₇)(R₄₈),
-Ge(R₄₉)(R₅₀)(R₅₁),
-N(R₅₂)(R₅₃) (wherein, R₄₁ to R₅₃ are independently a hydrogen atom, an alkyl group having 1 to 50 carbon atoms, an aryl group having 6 to 50 ring carbon atoms, or a monovalent heterocyclic group having 5 to 50 ring atoms; when two or more of each of R₄₁ to R₅₃ are present, the two or more of each of R₄₁ to R₅₃ may be the same as or different from each other),
a hydroxy group,
a halogen atom,
a cyano group,
a nitro group,
an aryl group having 6 to 50 ring carbon atoms, and
a monovalent heterocyclic group having 5 to 50 ring atoms.

In one embodiment, a substituent in the case of "substituted or unsubstituted" in the formula (41) is selected from the group consisting of
an alkyl group having 1 to 18 carbon atoms,
an aryl group having 6 to 18 ring carbon atoms, and
a monovalent heterocyclic group having 5 to 18 ring atoms.

The compound represented by the formula (41) can be synthesized by using known alternative reactions or raw materials adapted to the target compound.

Specific examples of the compound represented by the formula (41) will be described below, but these are merely examples, and the compound represented by the formula (41) is not limited to the following specific examples.

In the following specific examples, "Me" represents a methyl group, "tBu" represents a tertiary butyl group, and "Ph" represents a phenyl group.

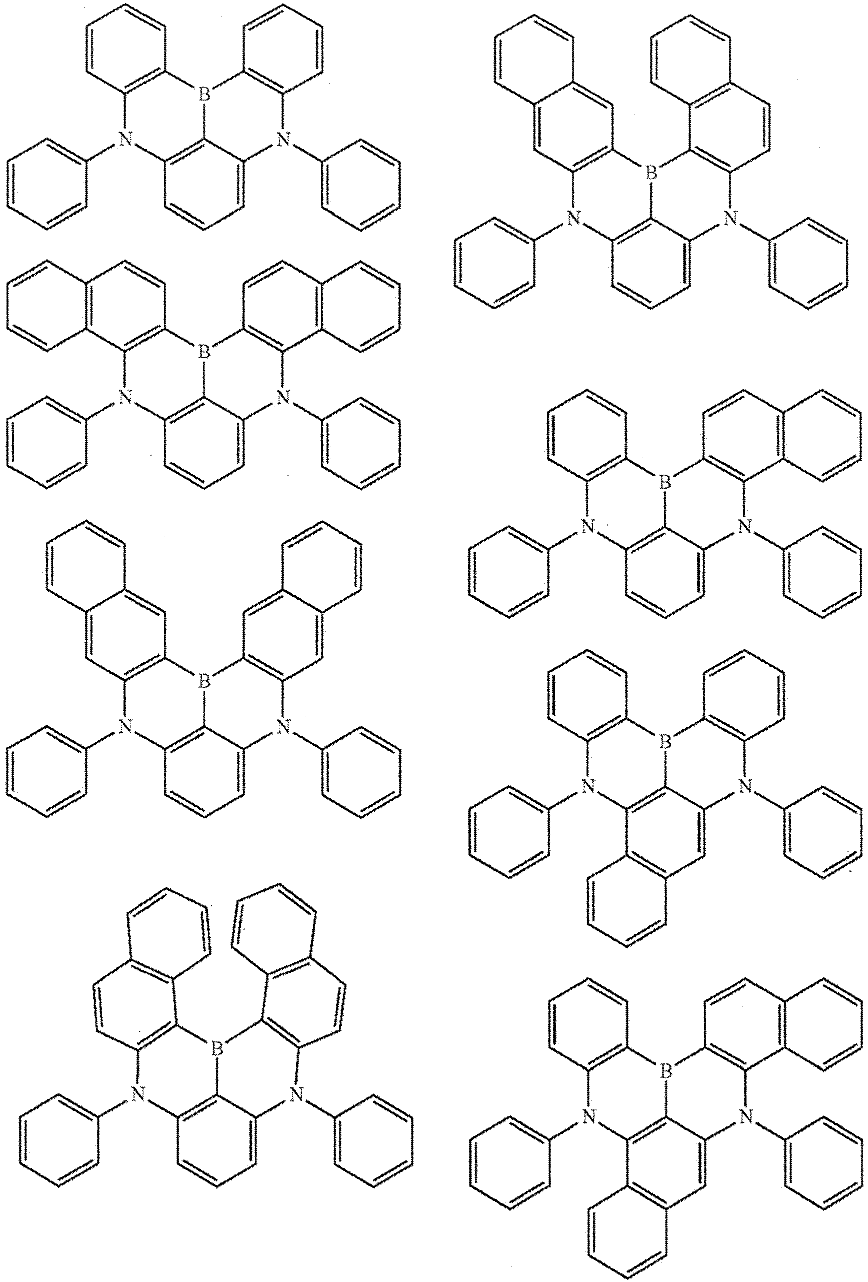

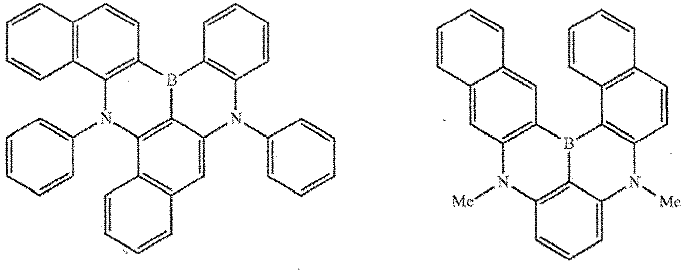

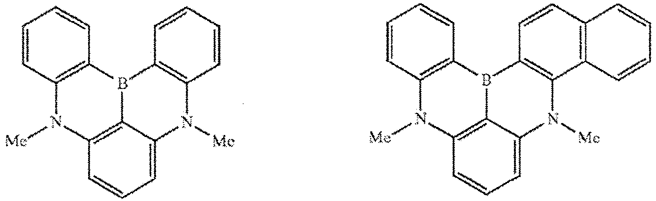

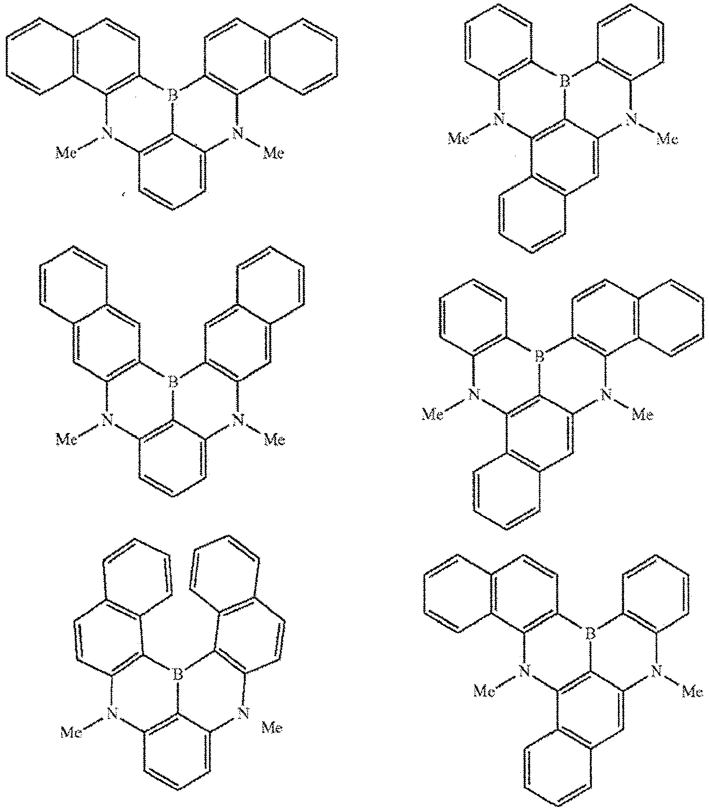

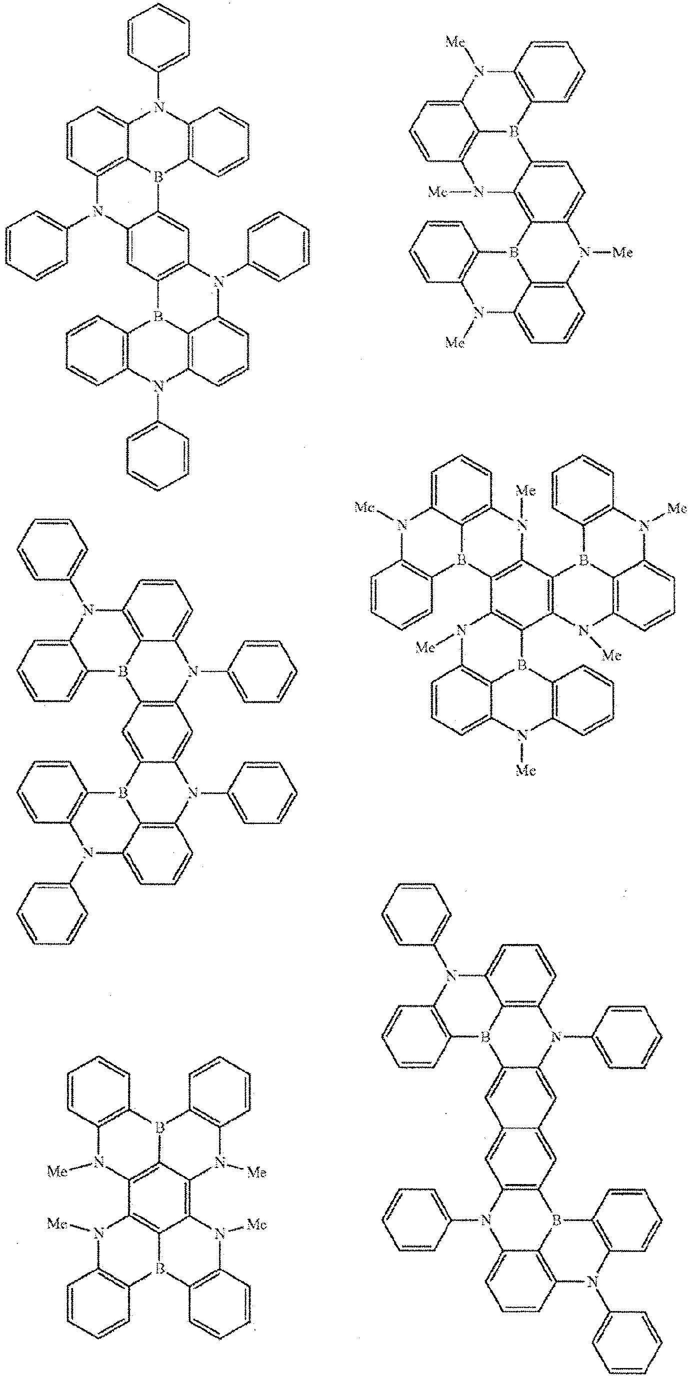

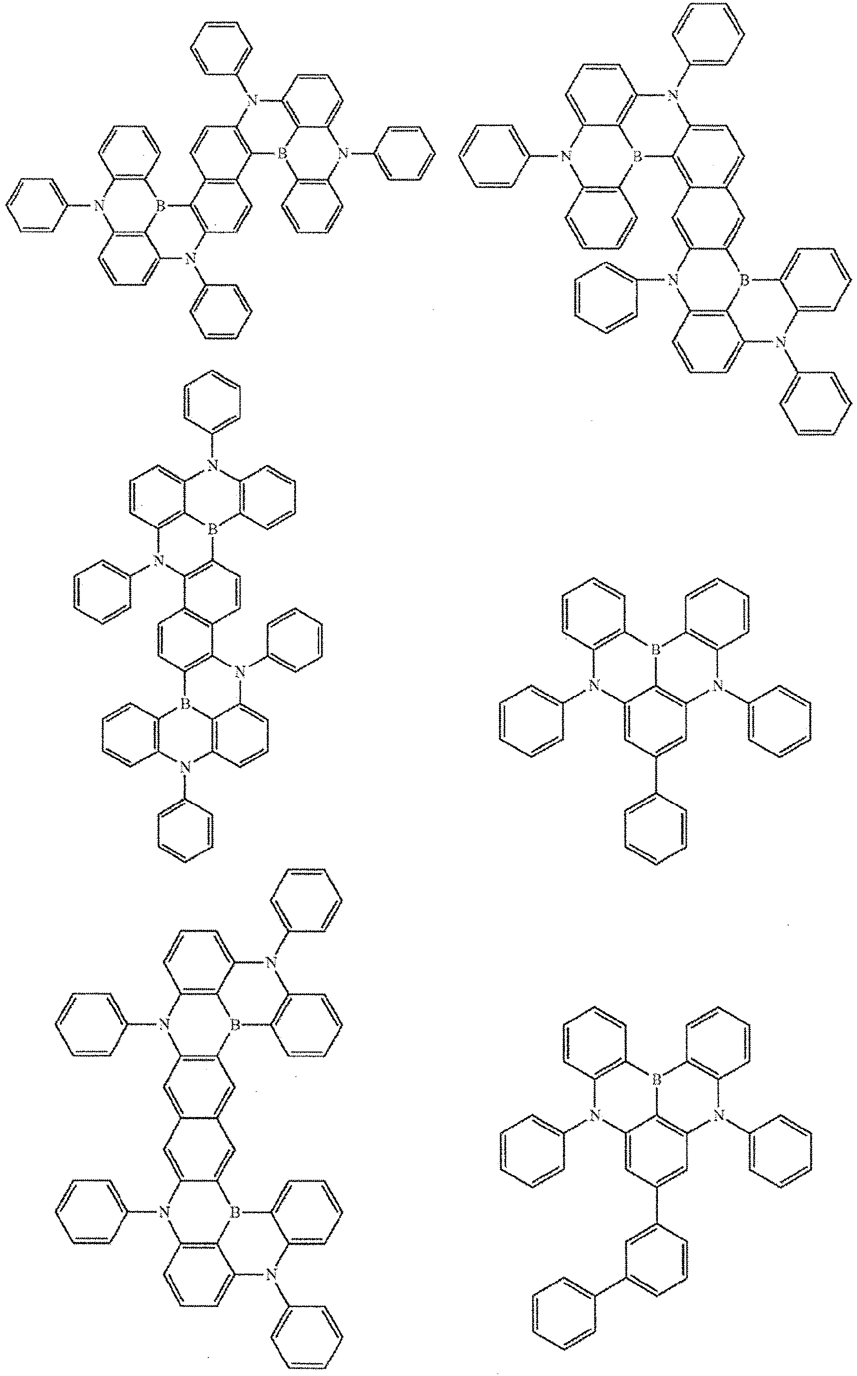

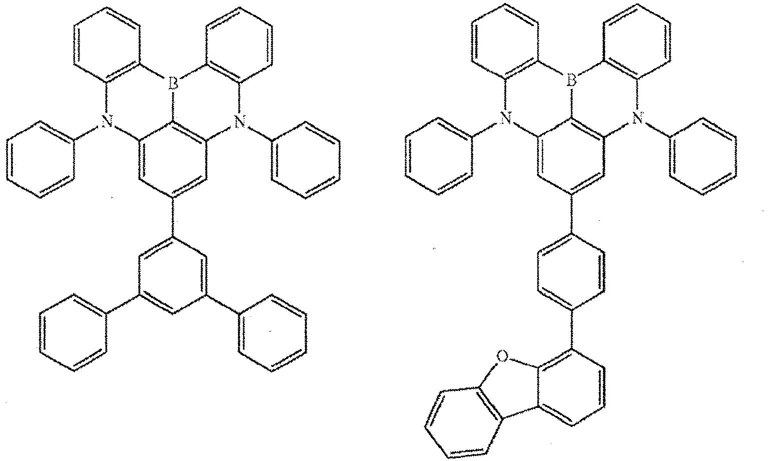

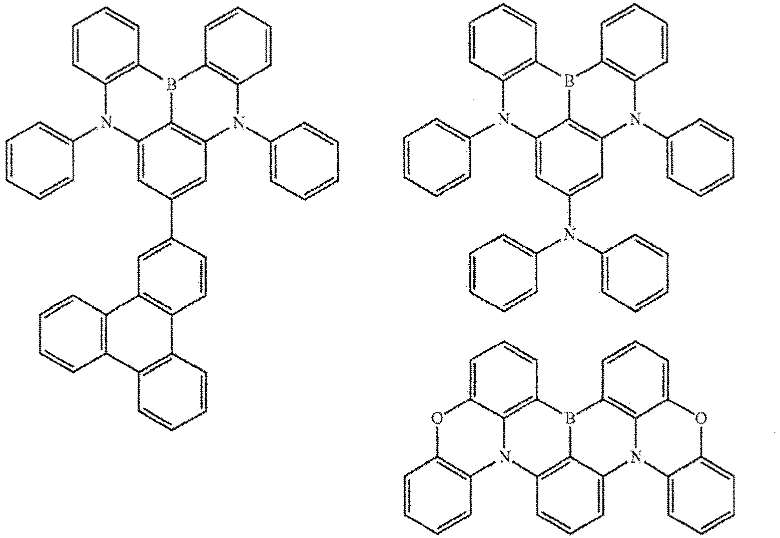

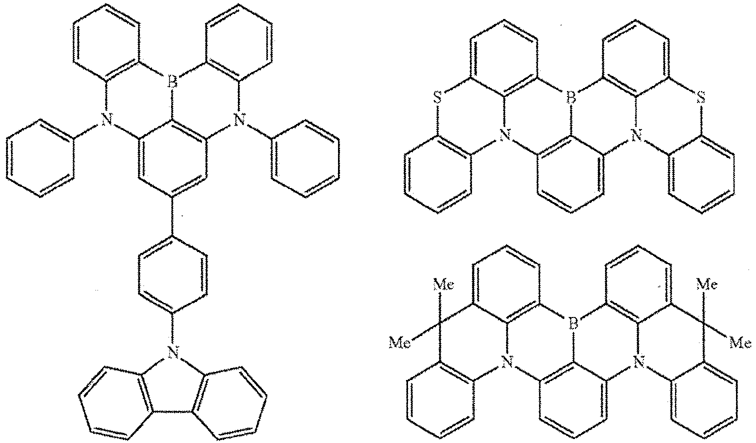

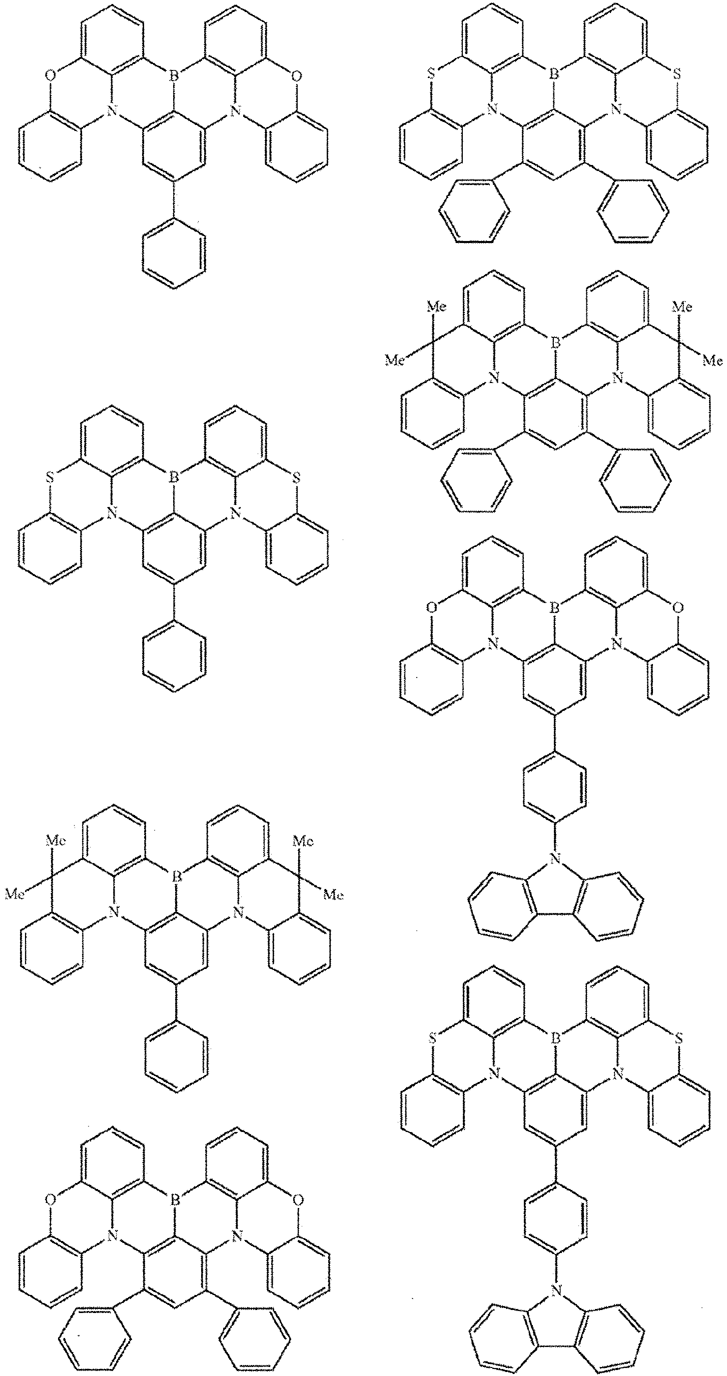

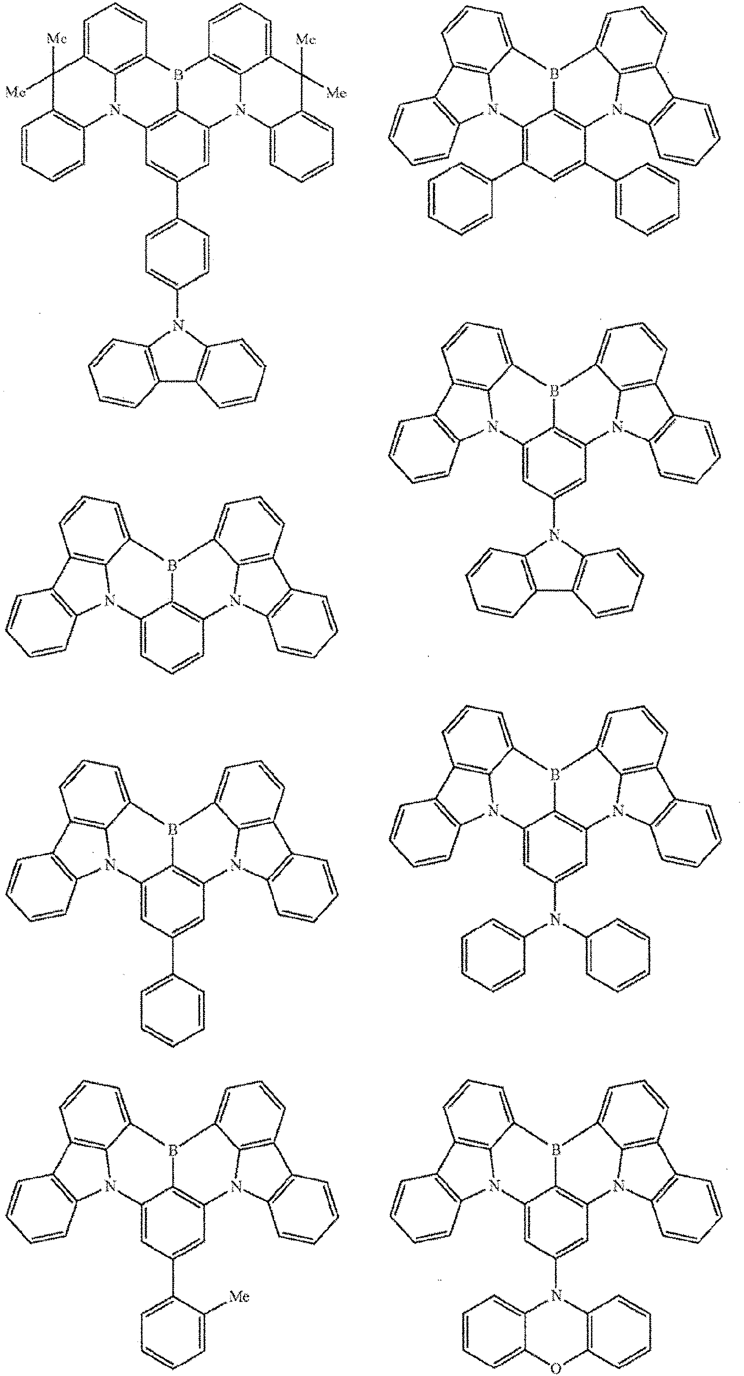

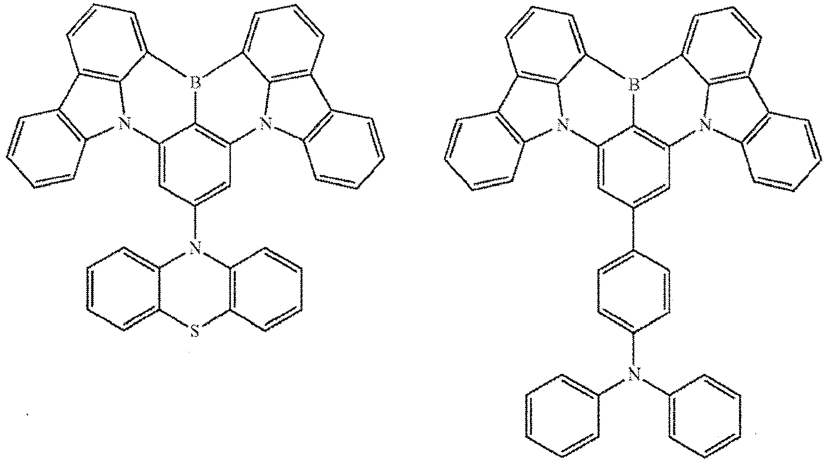

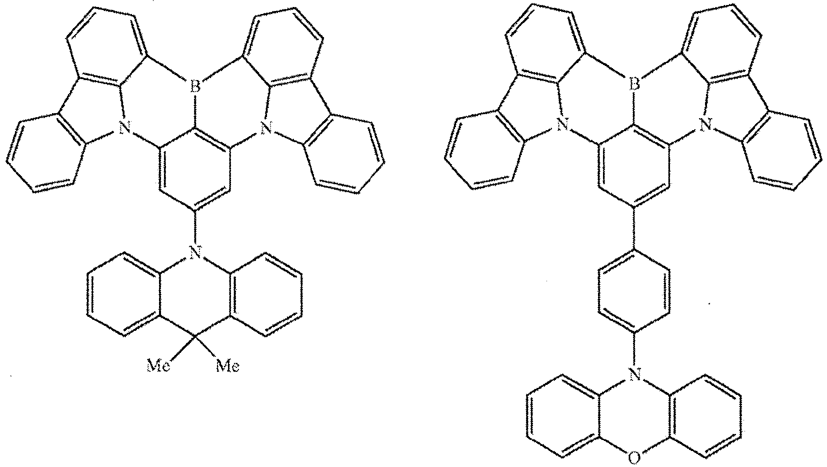

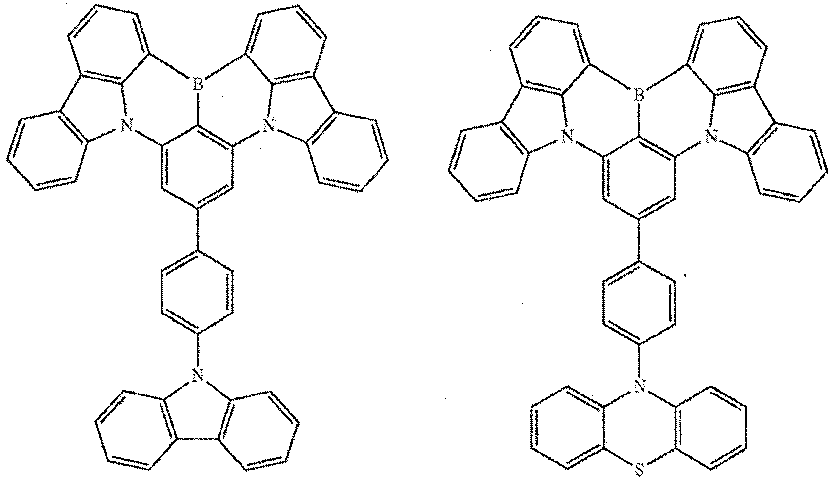

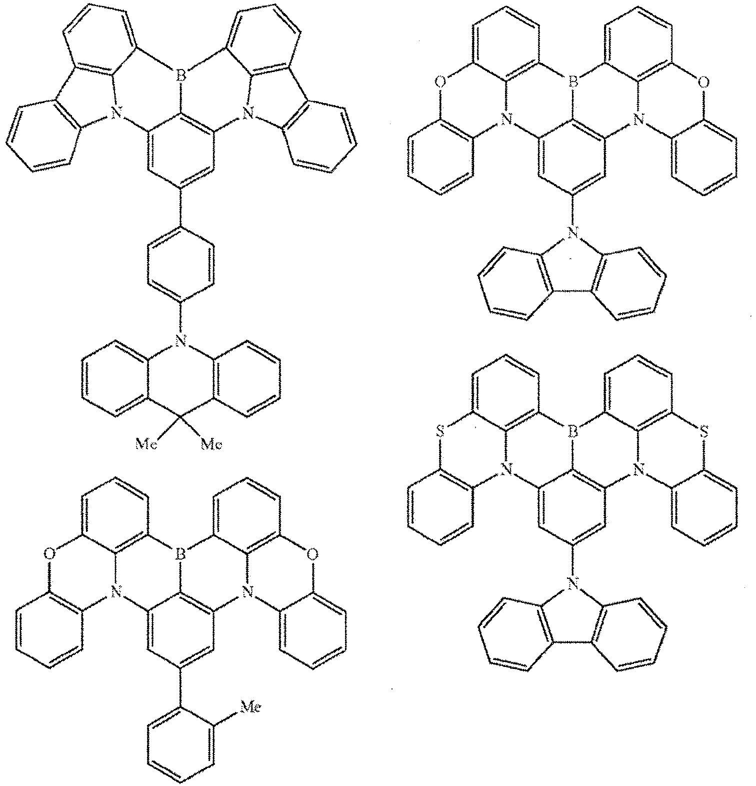

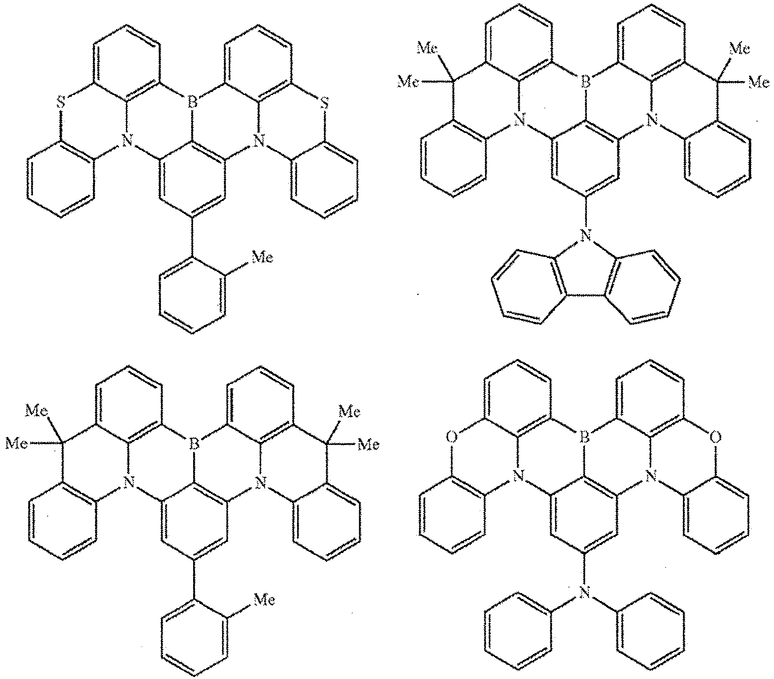

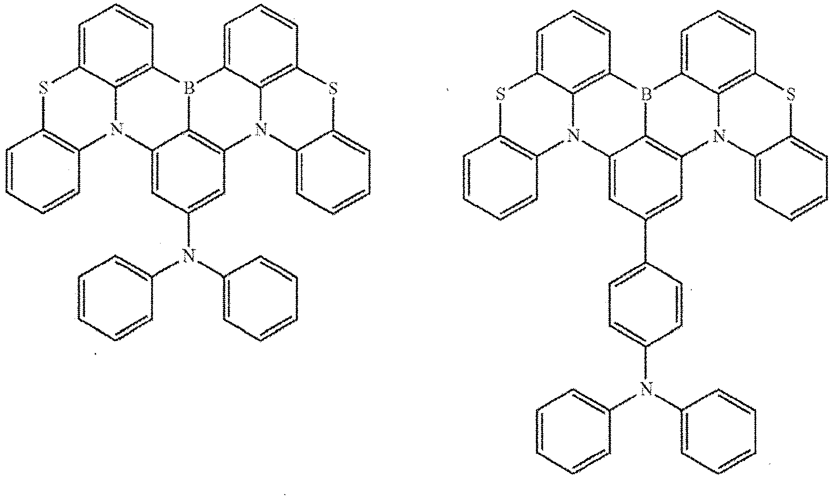

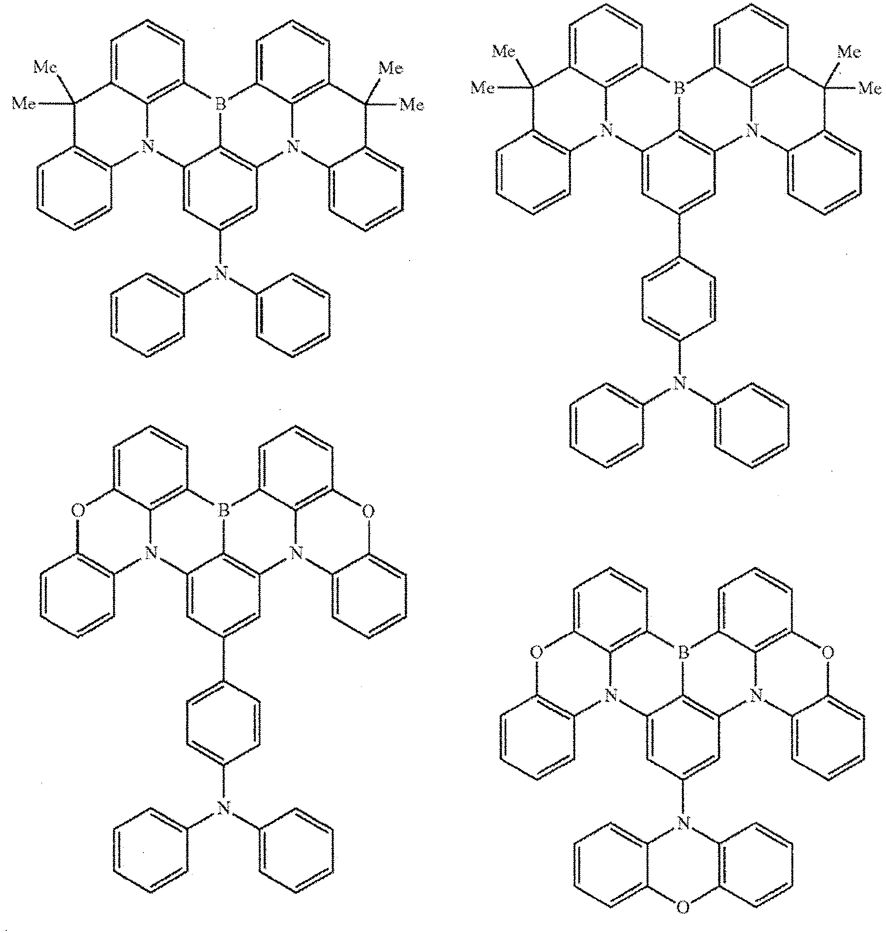

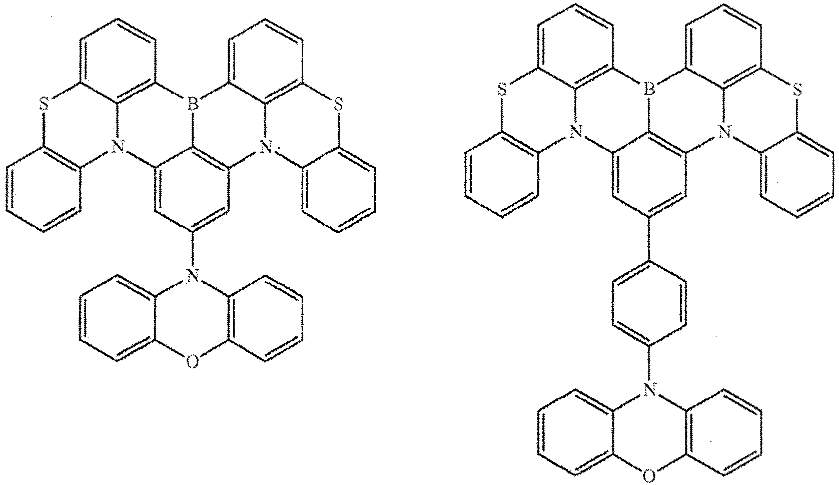

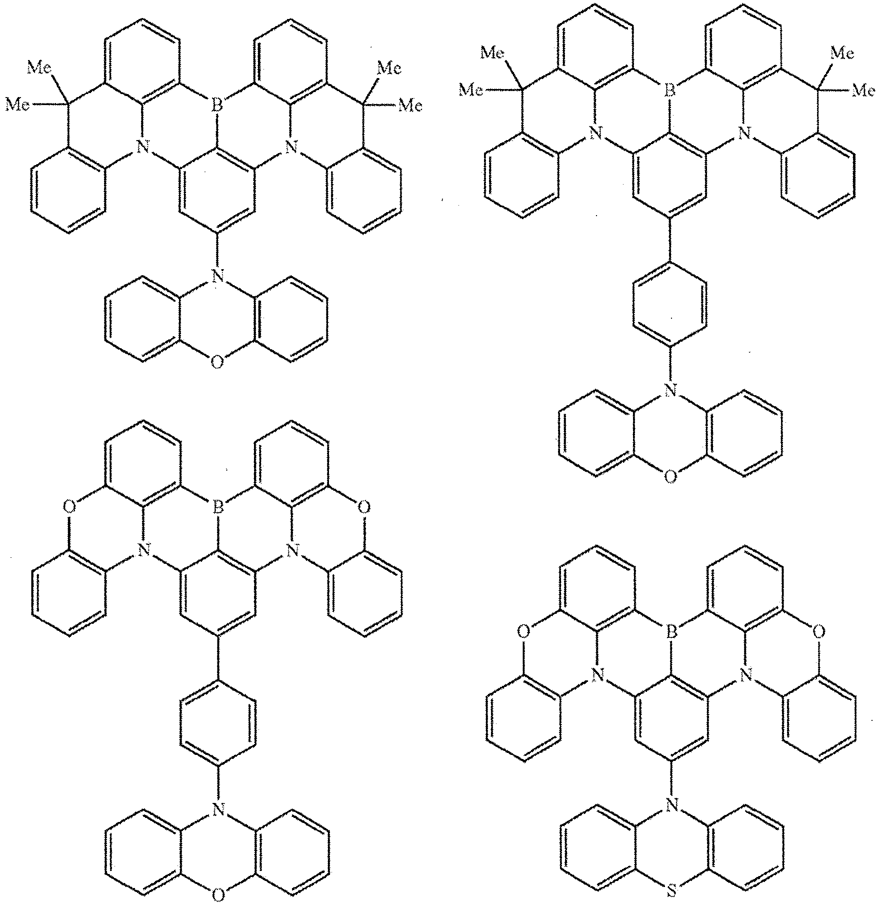

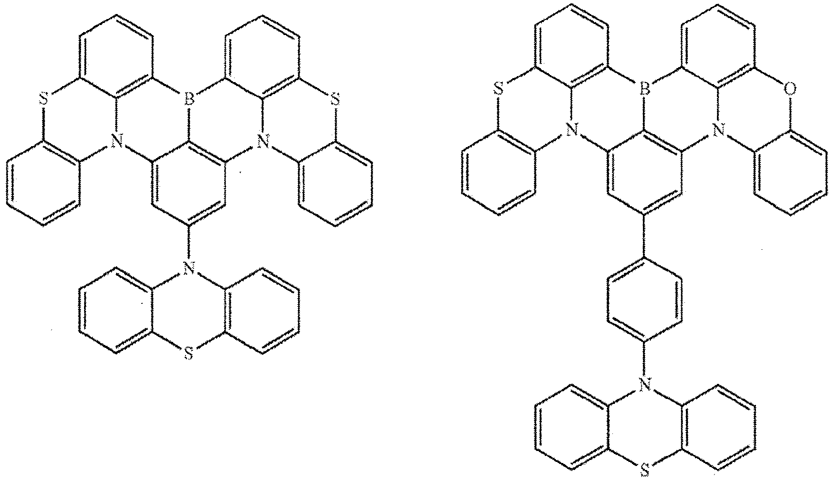

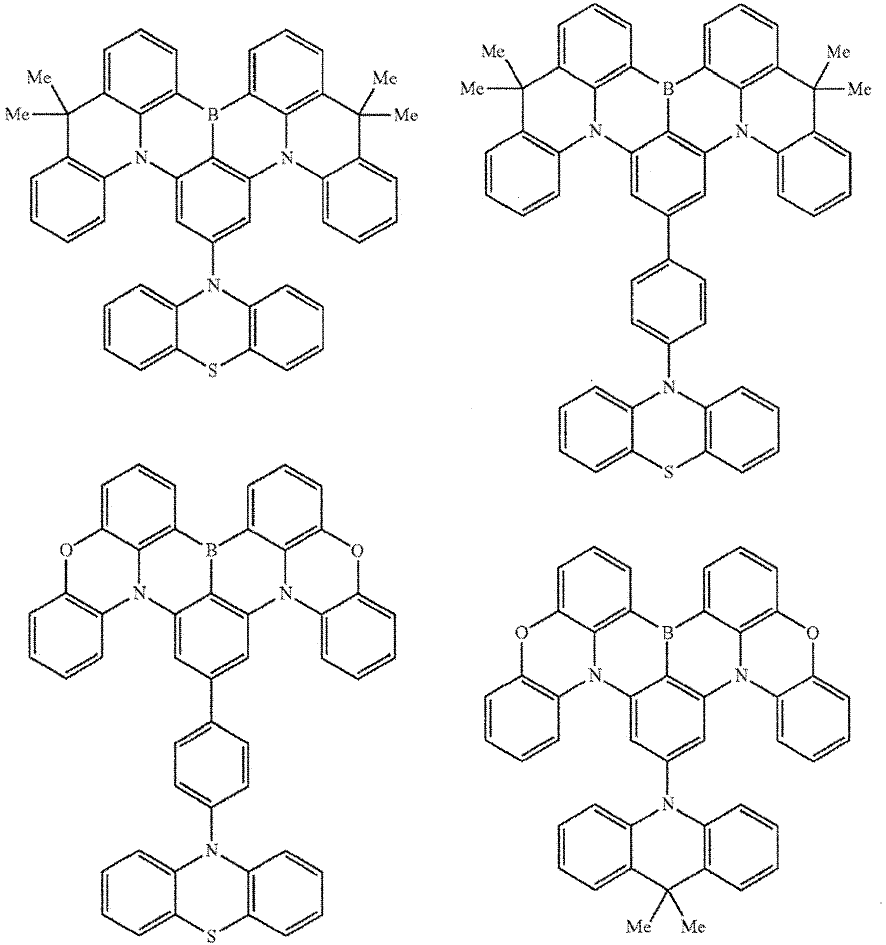

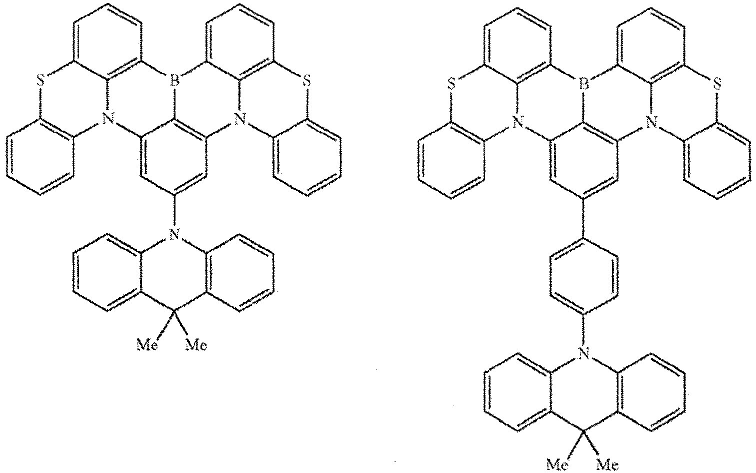

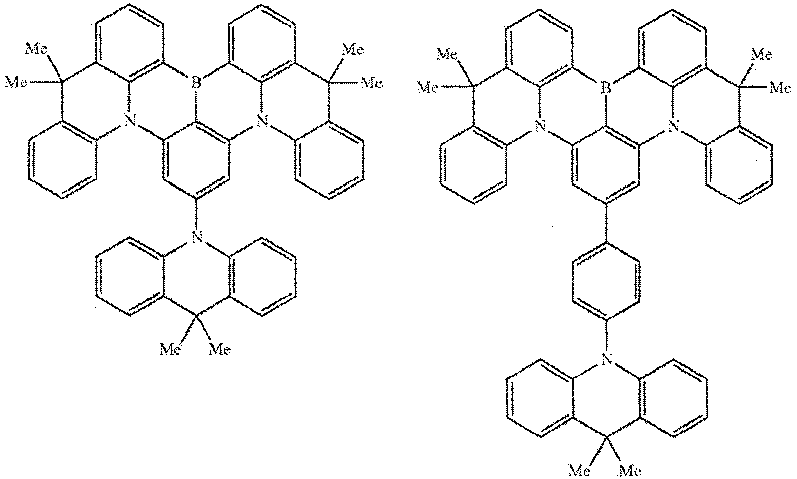

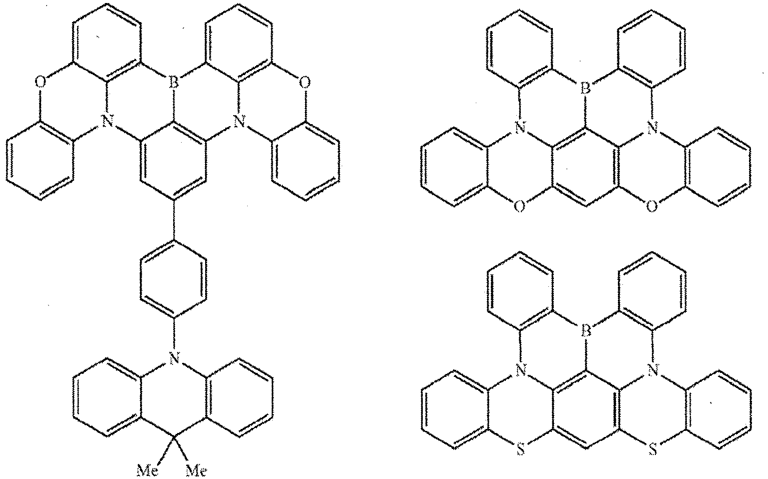

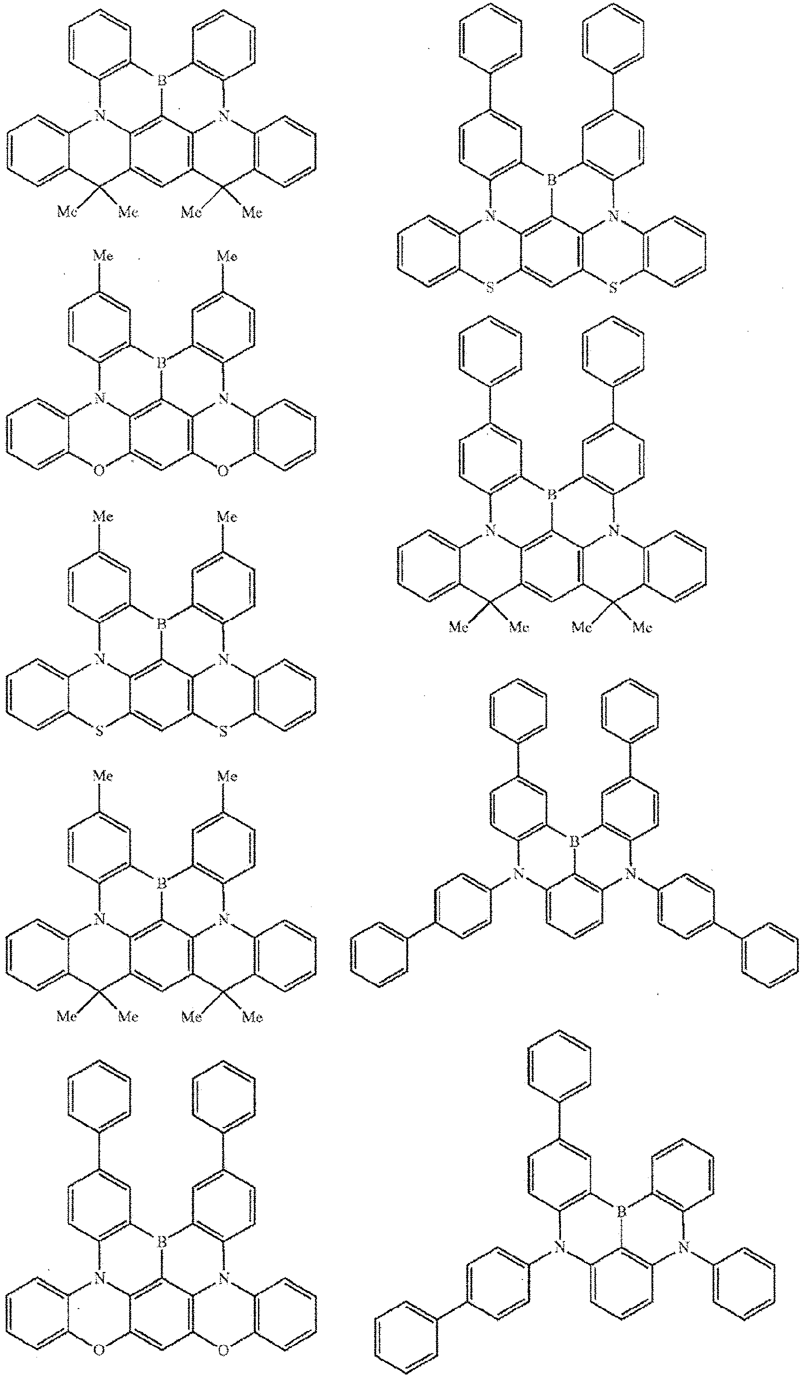

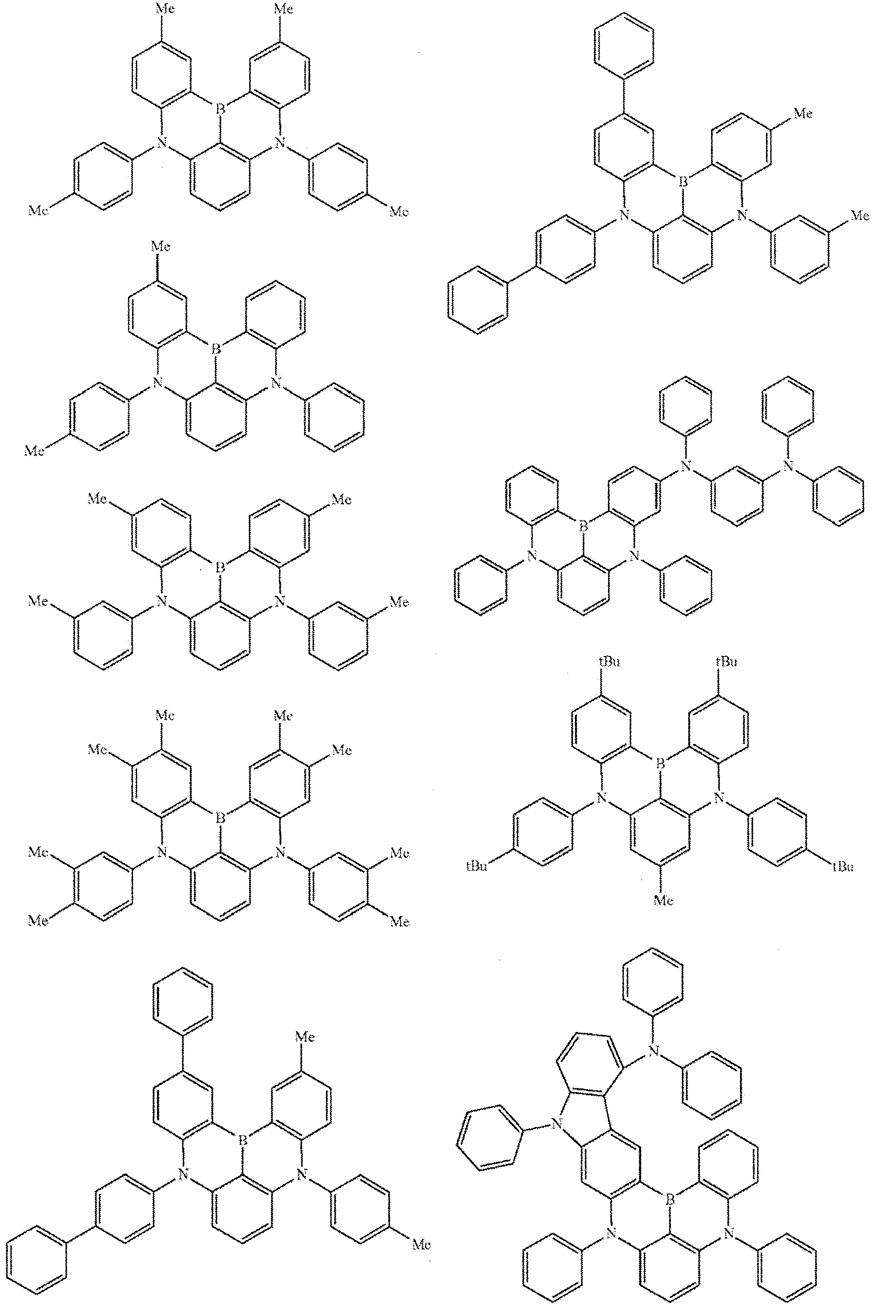

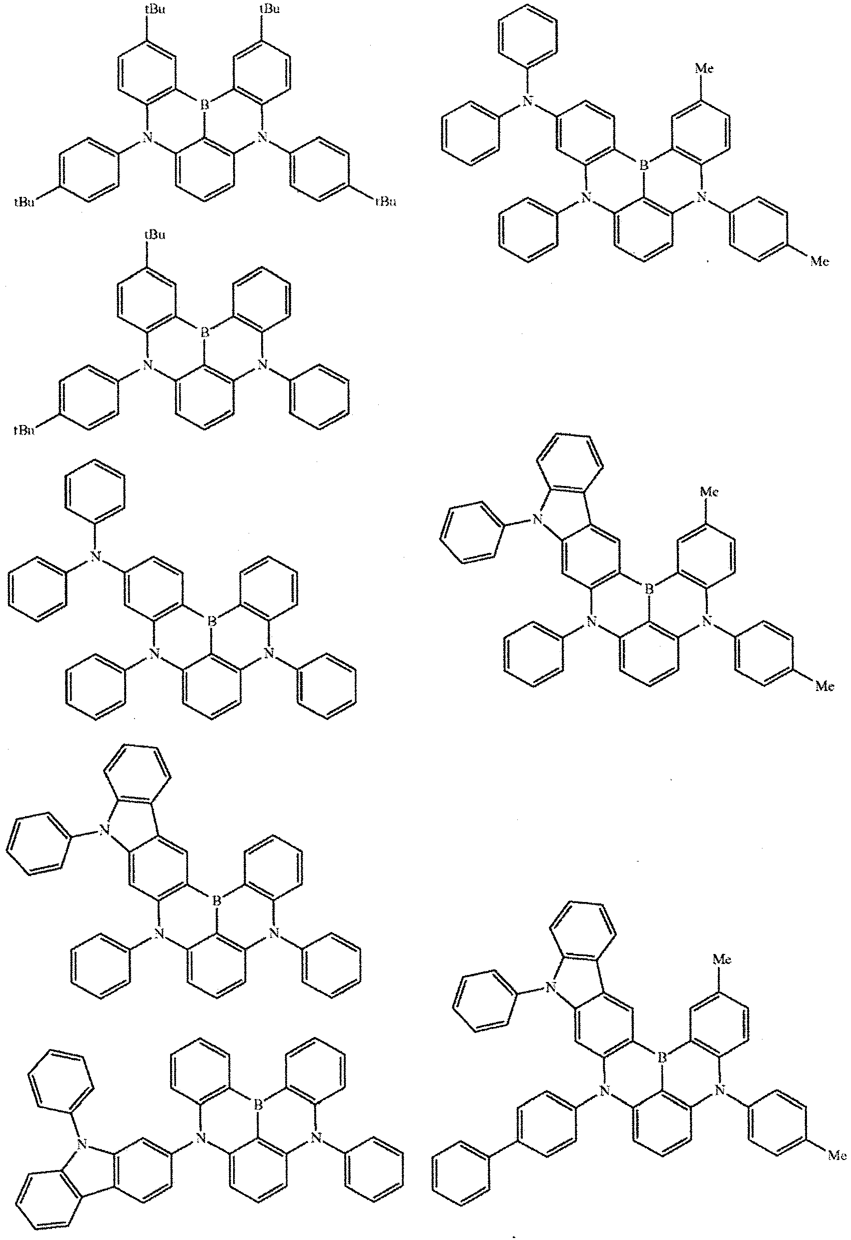

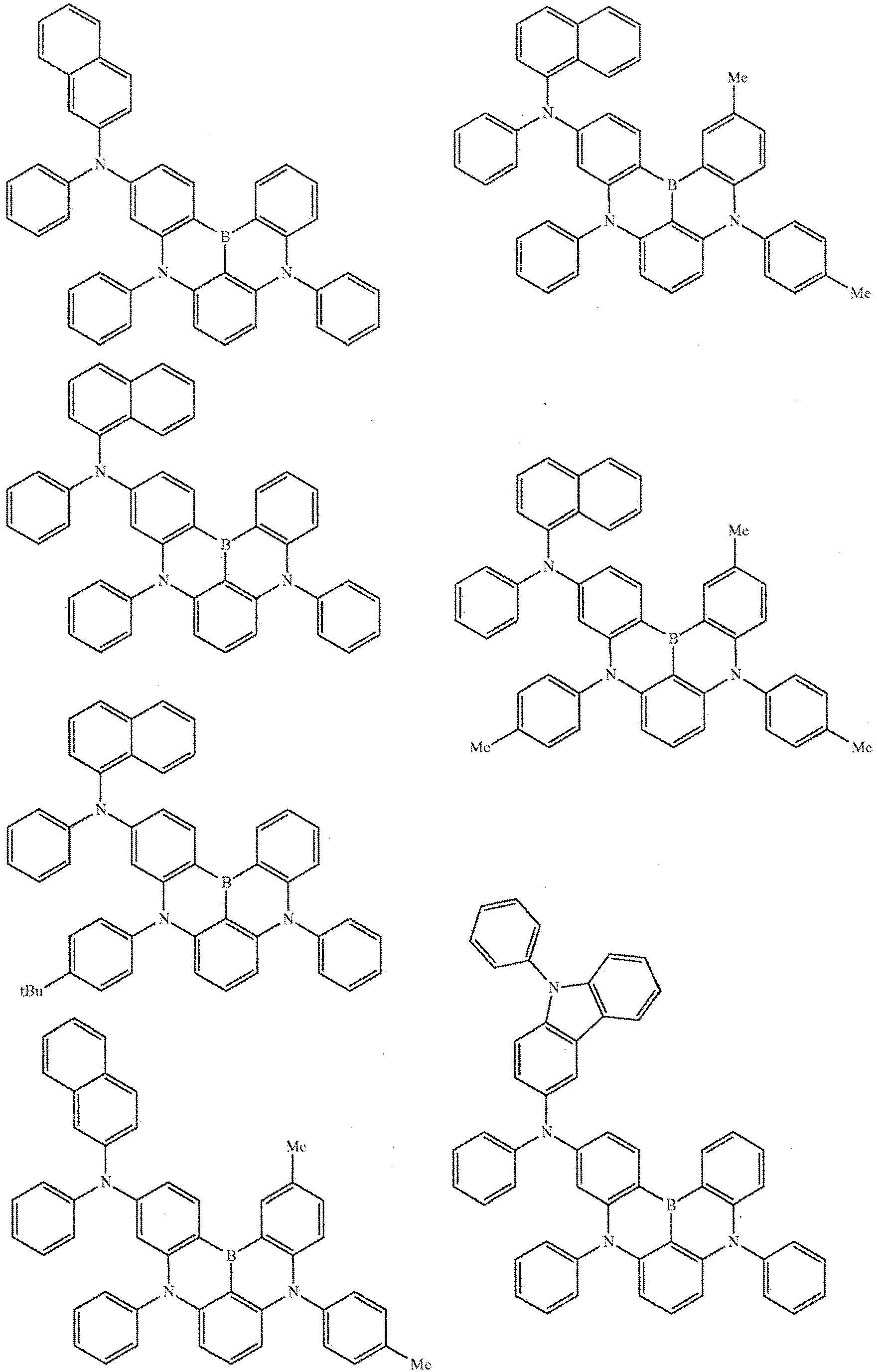

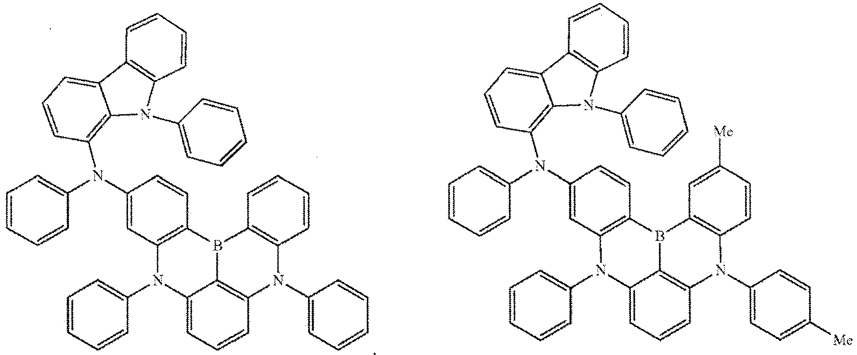

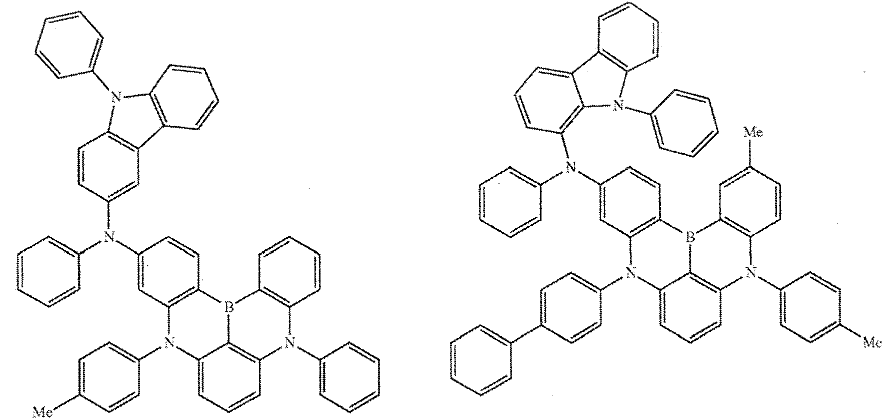

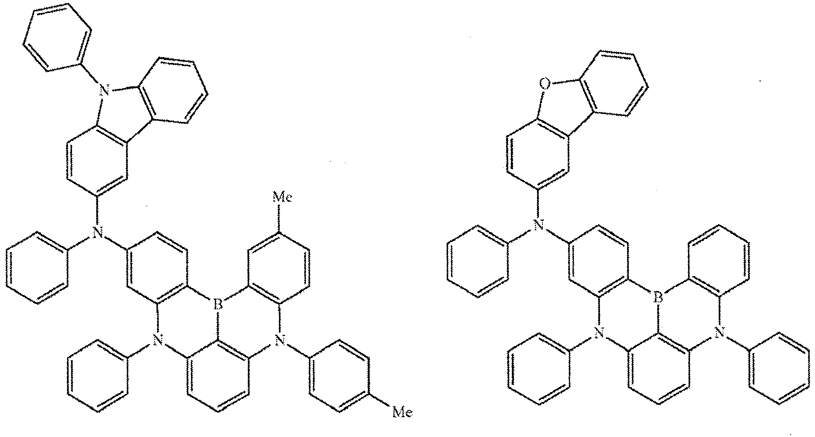

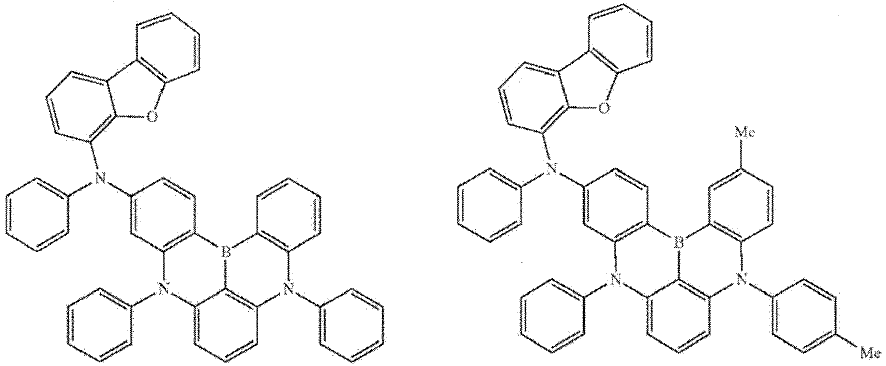

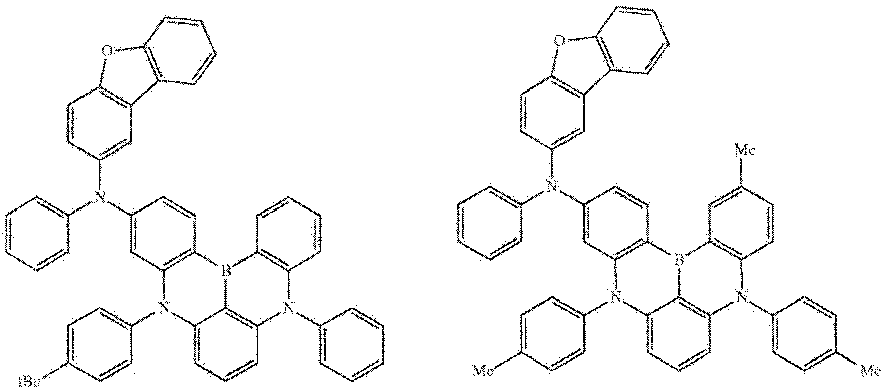

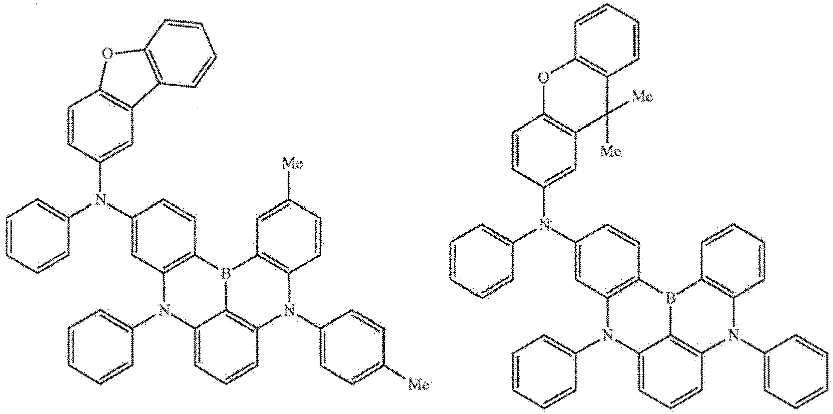

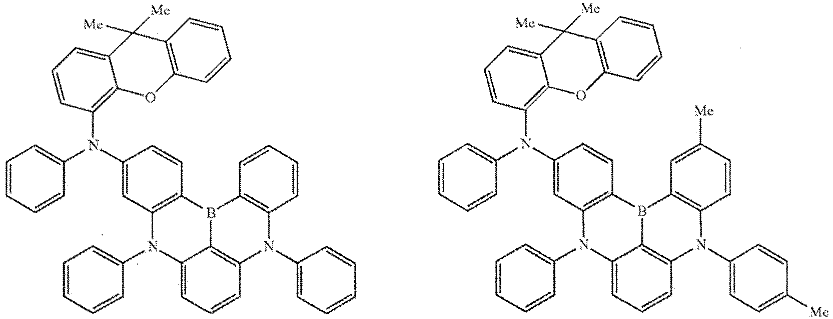

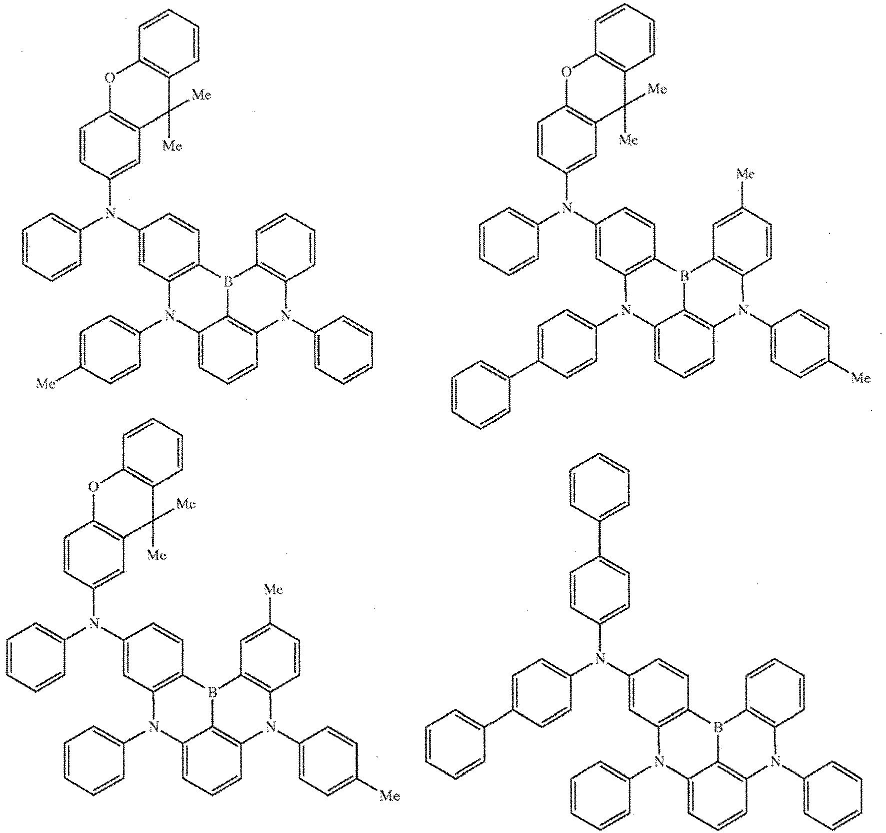

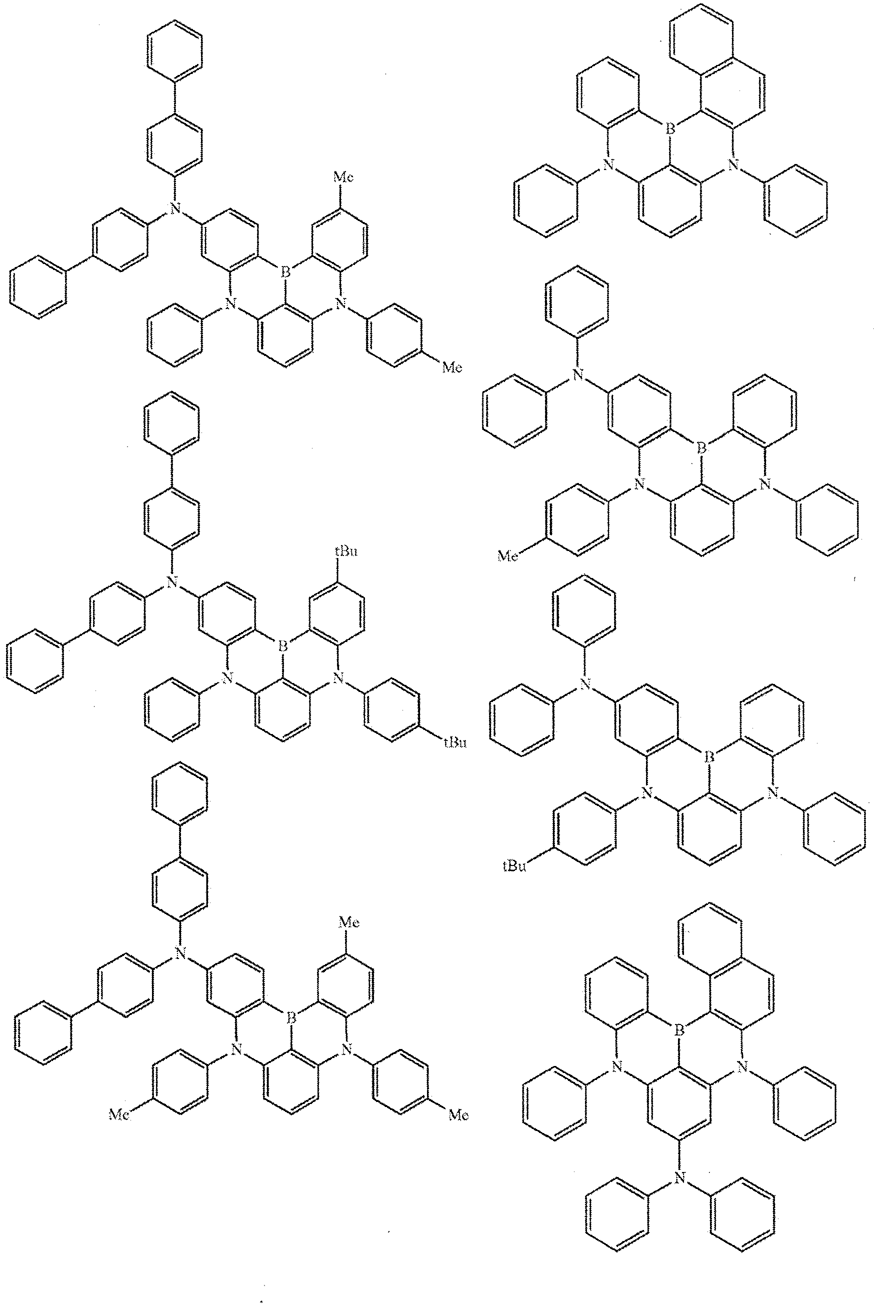

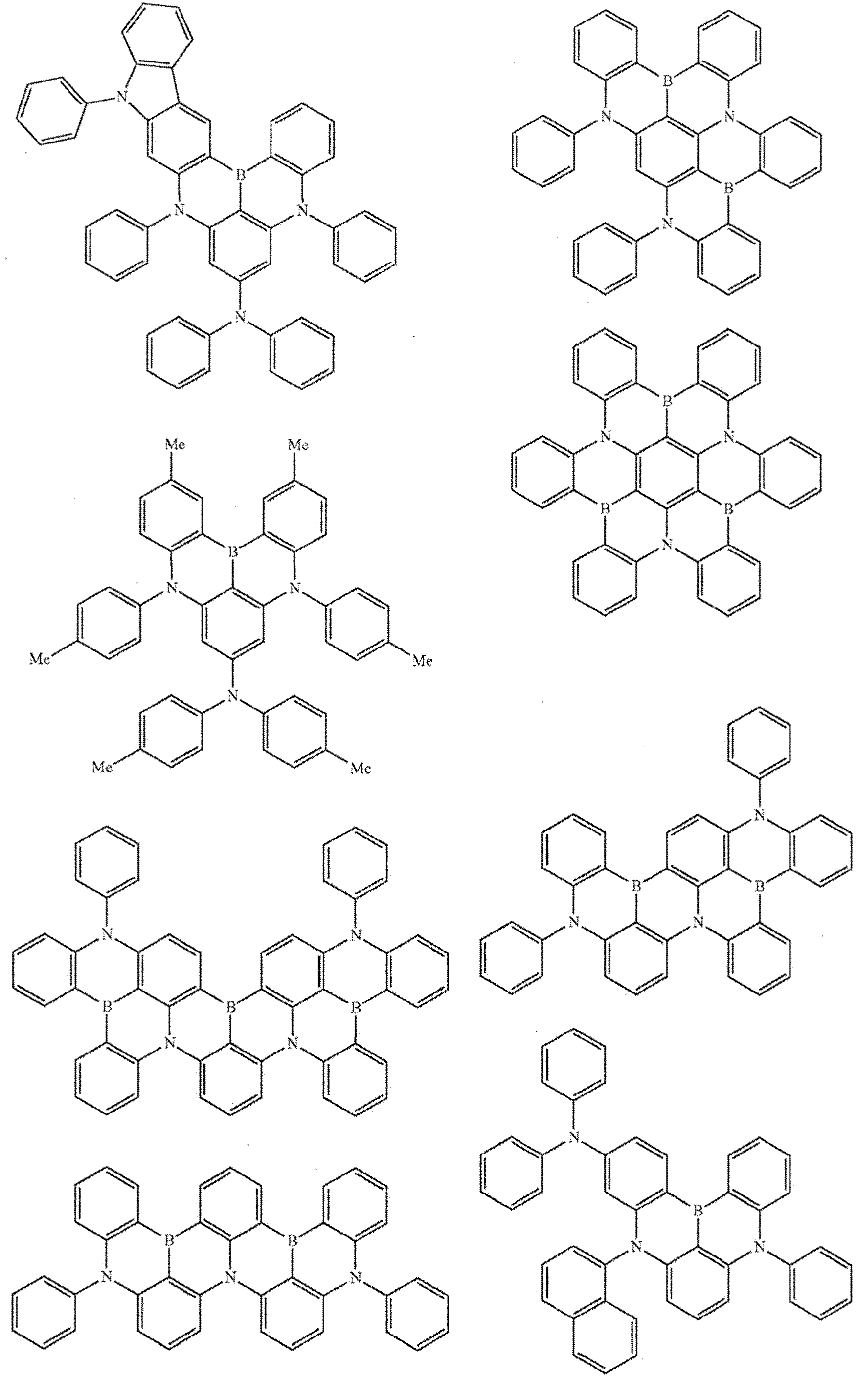

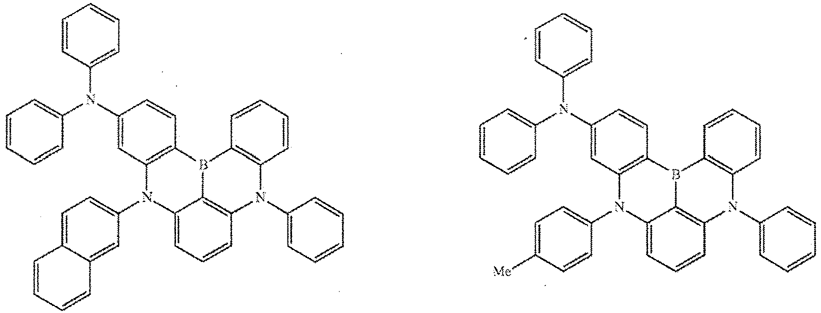

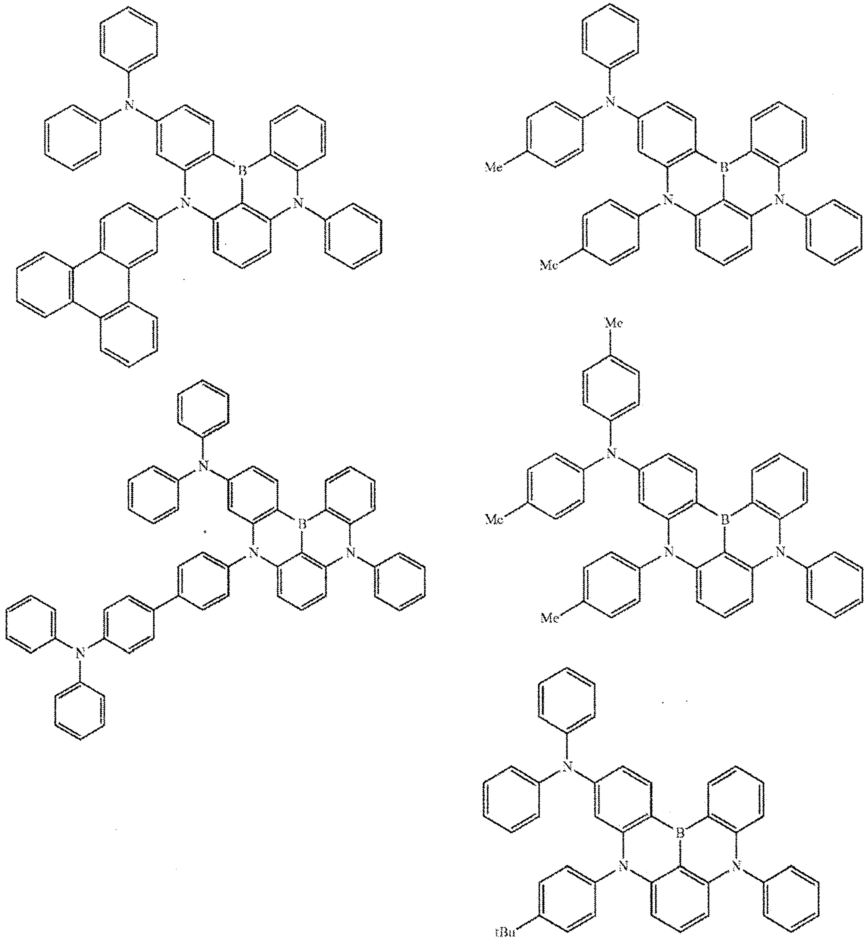

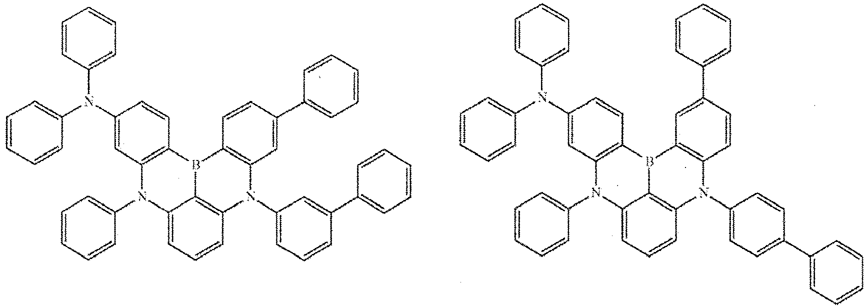

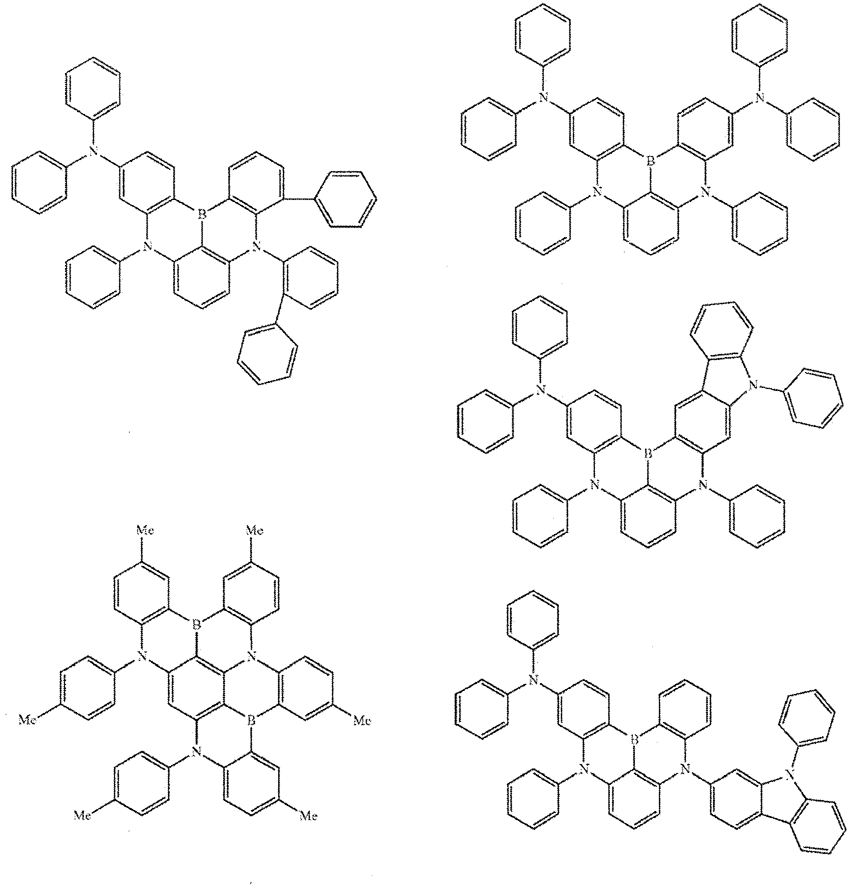

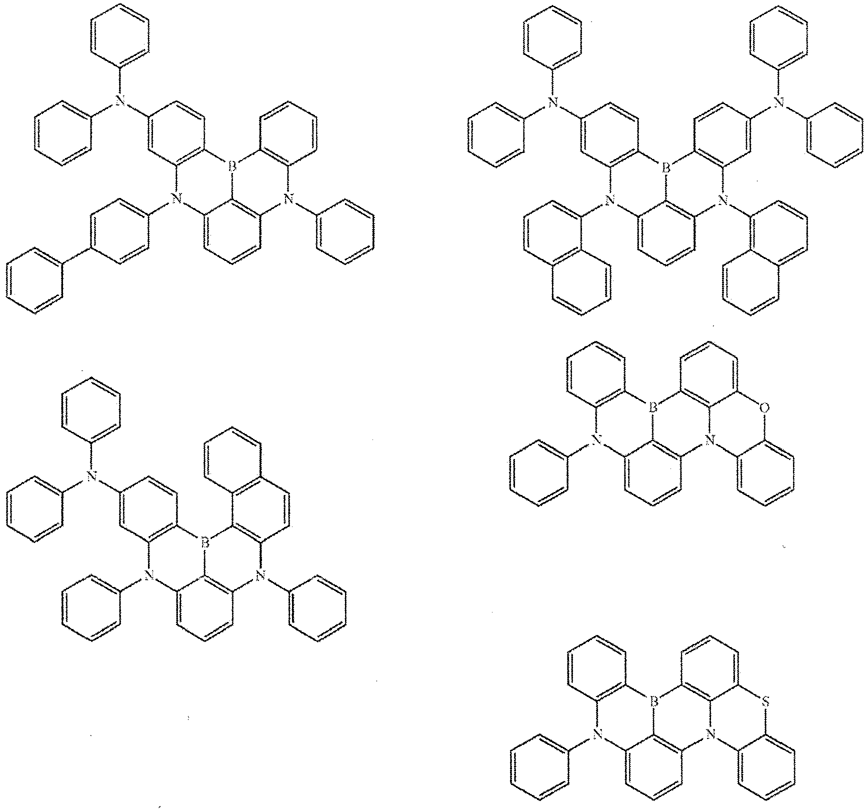

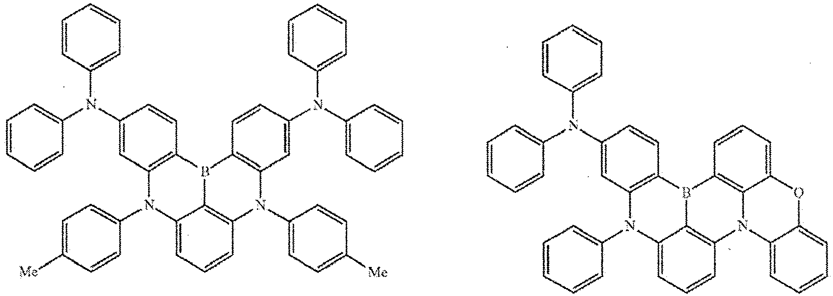

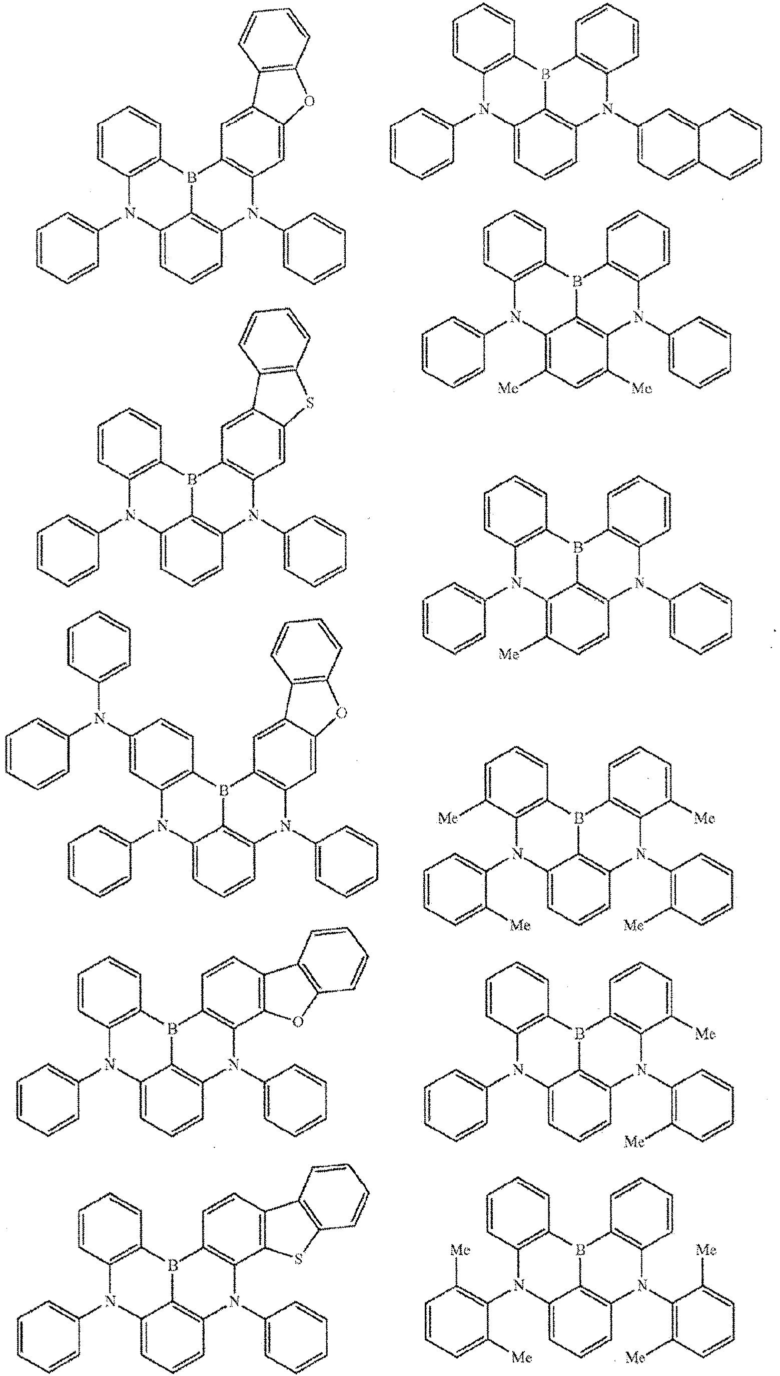

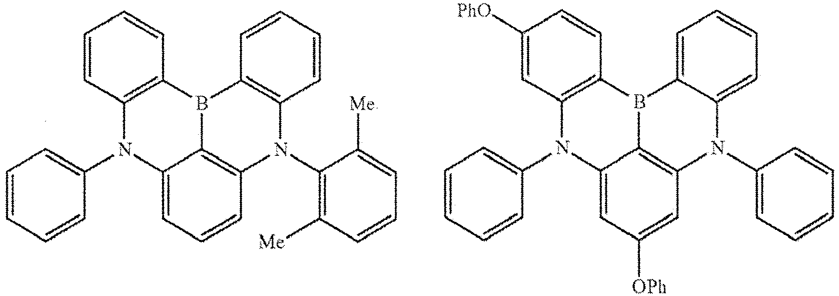

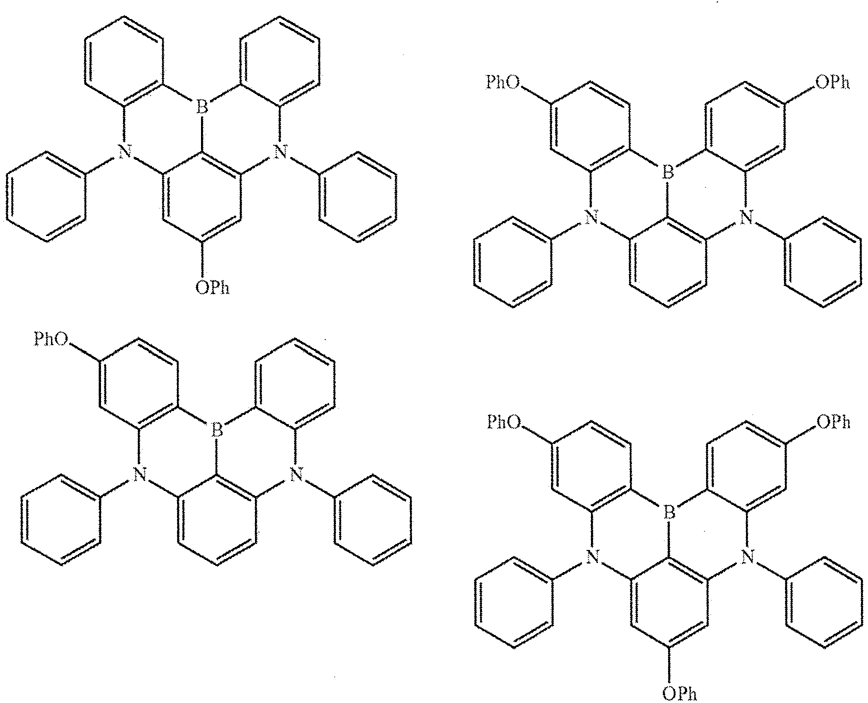

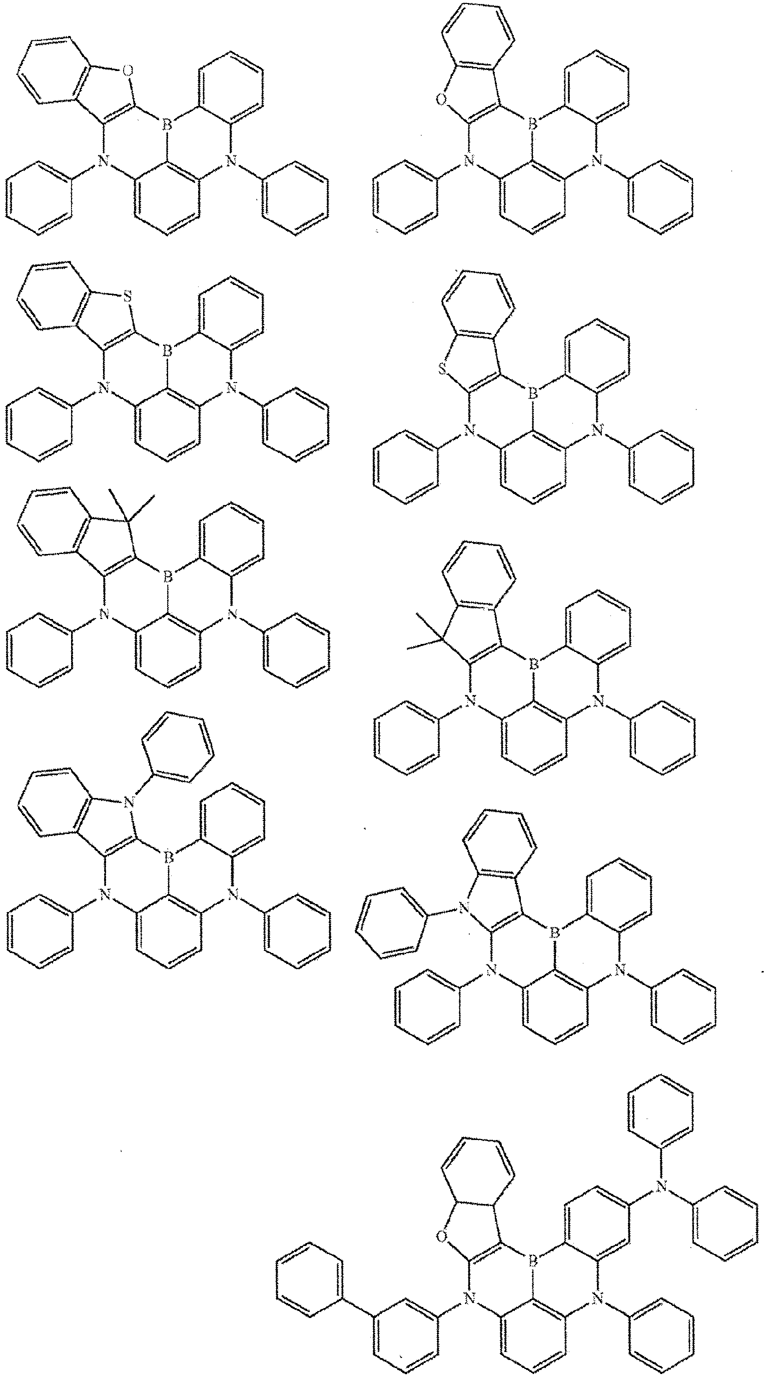

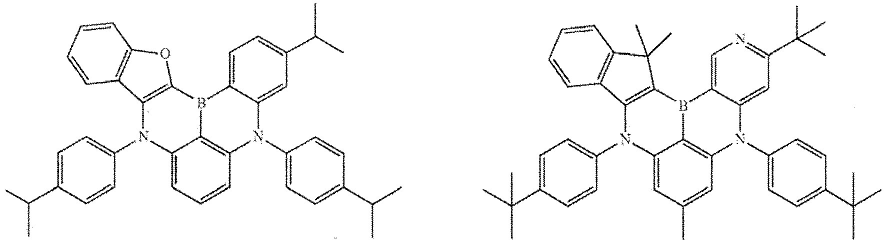

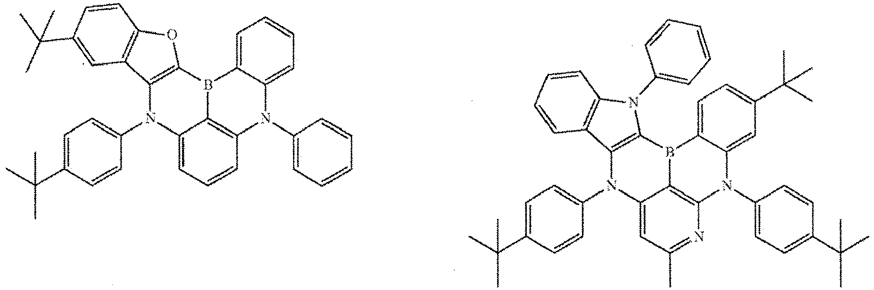

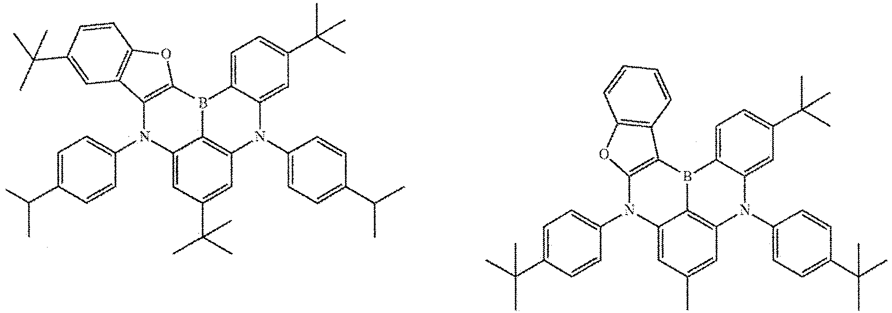

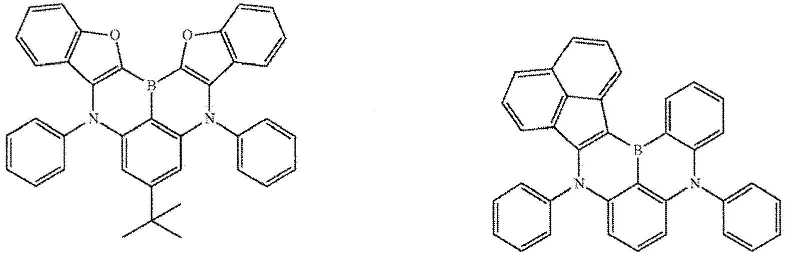

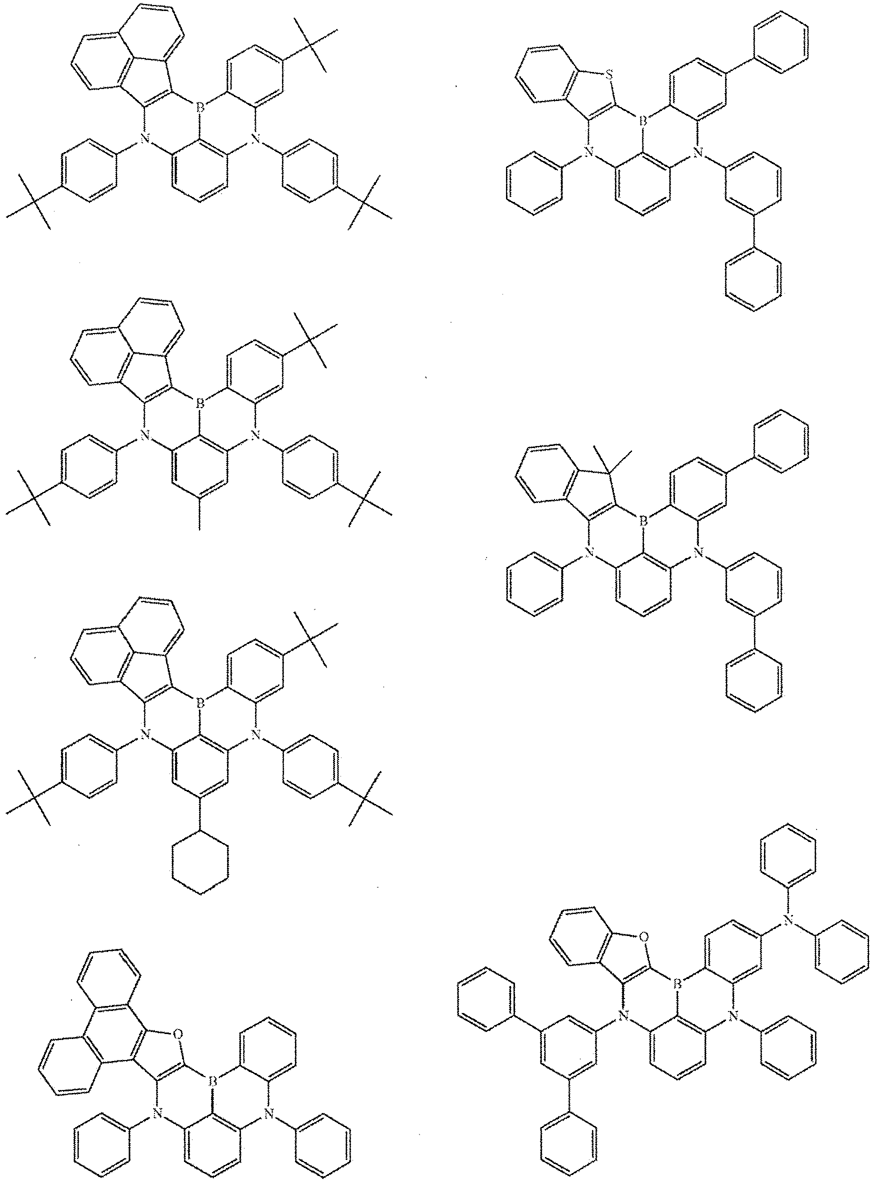

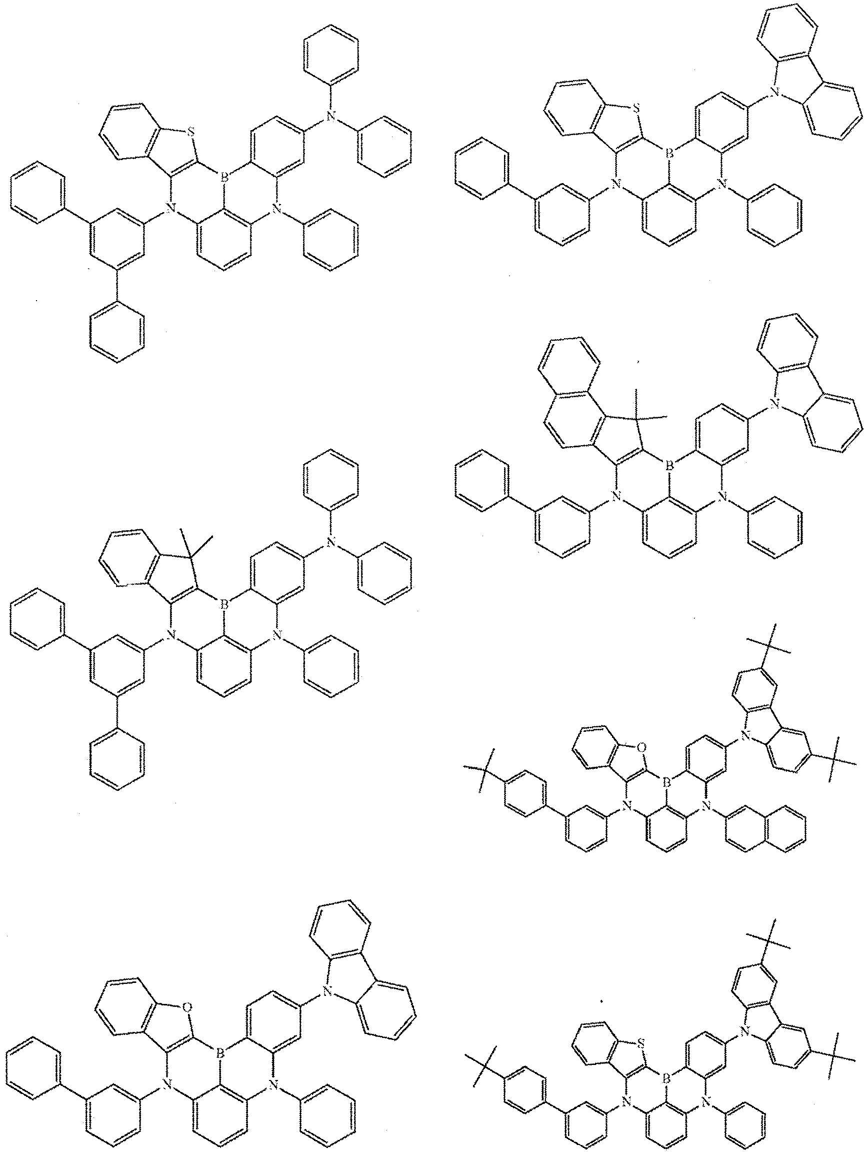

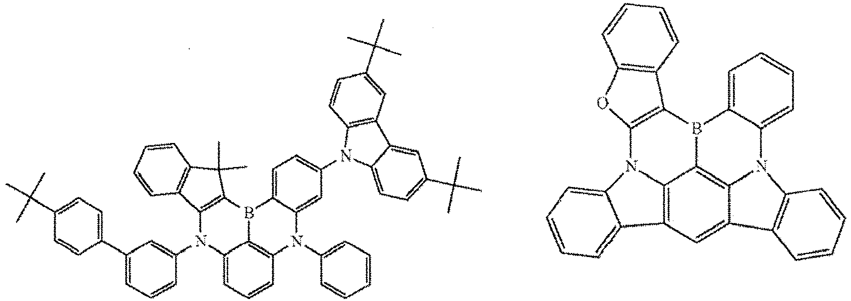

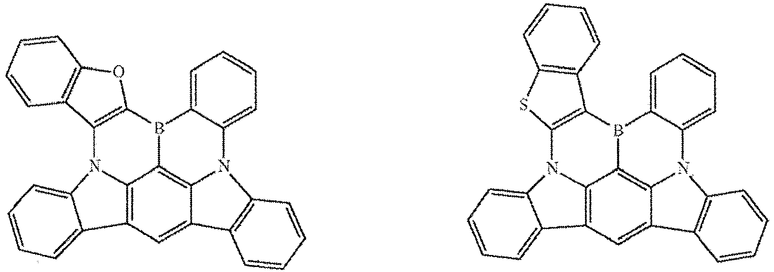

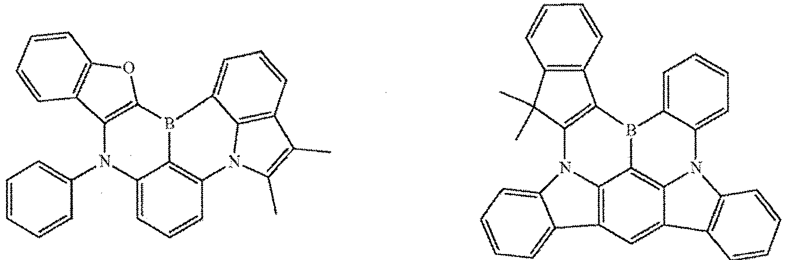

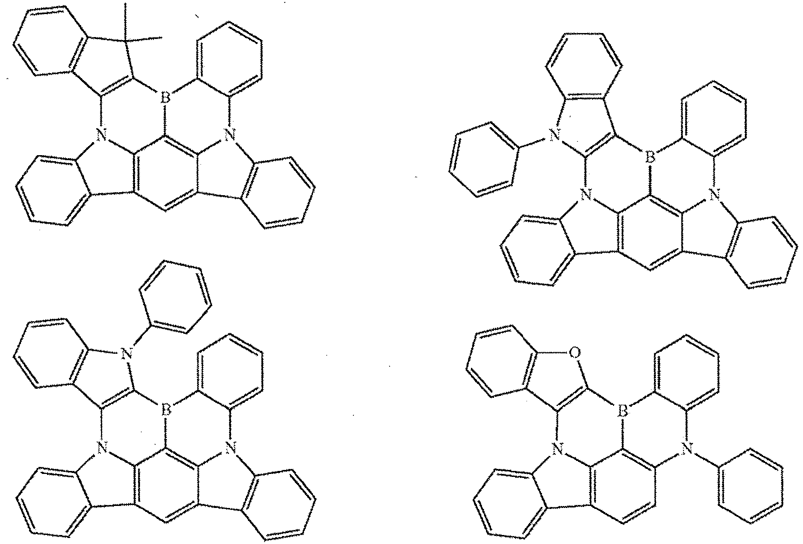

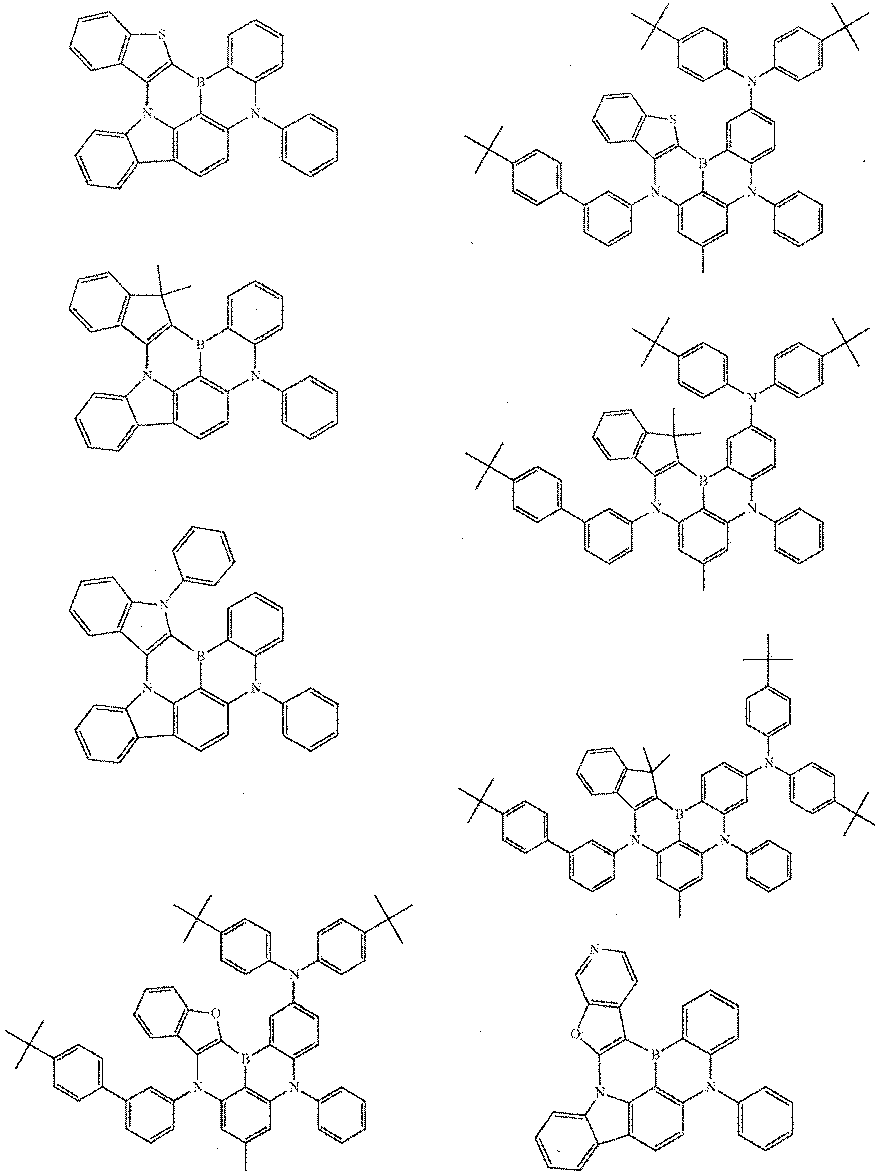

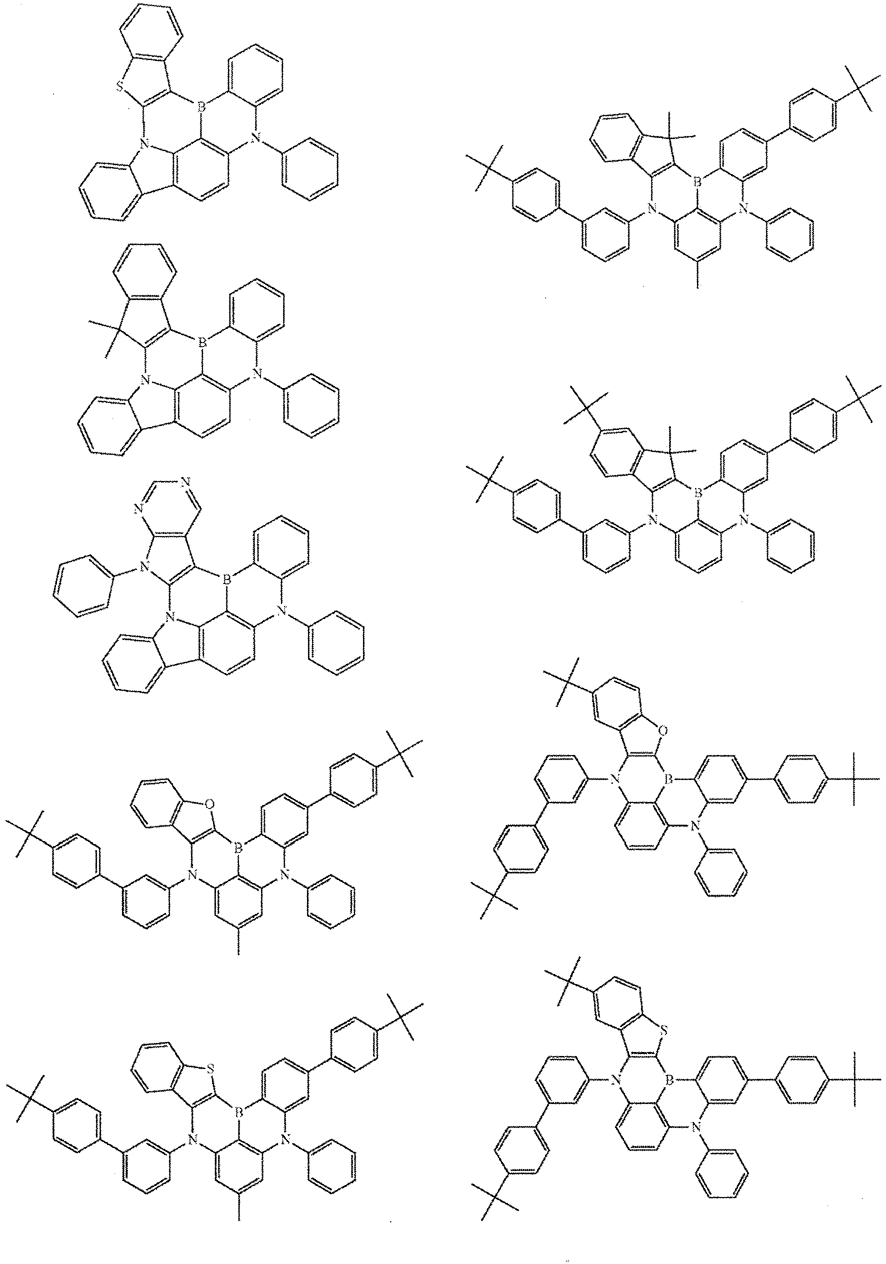

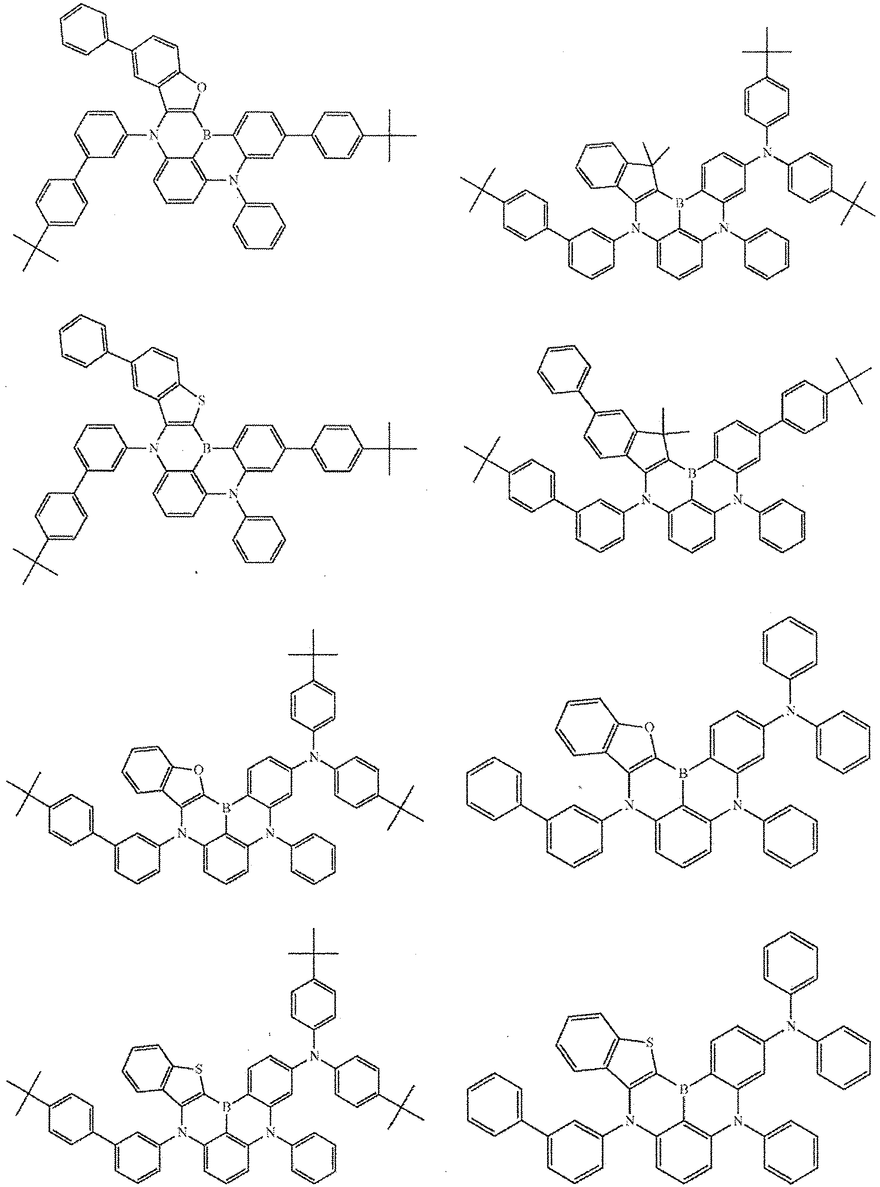

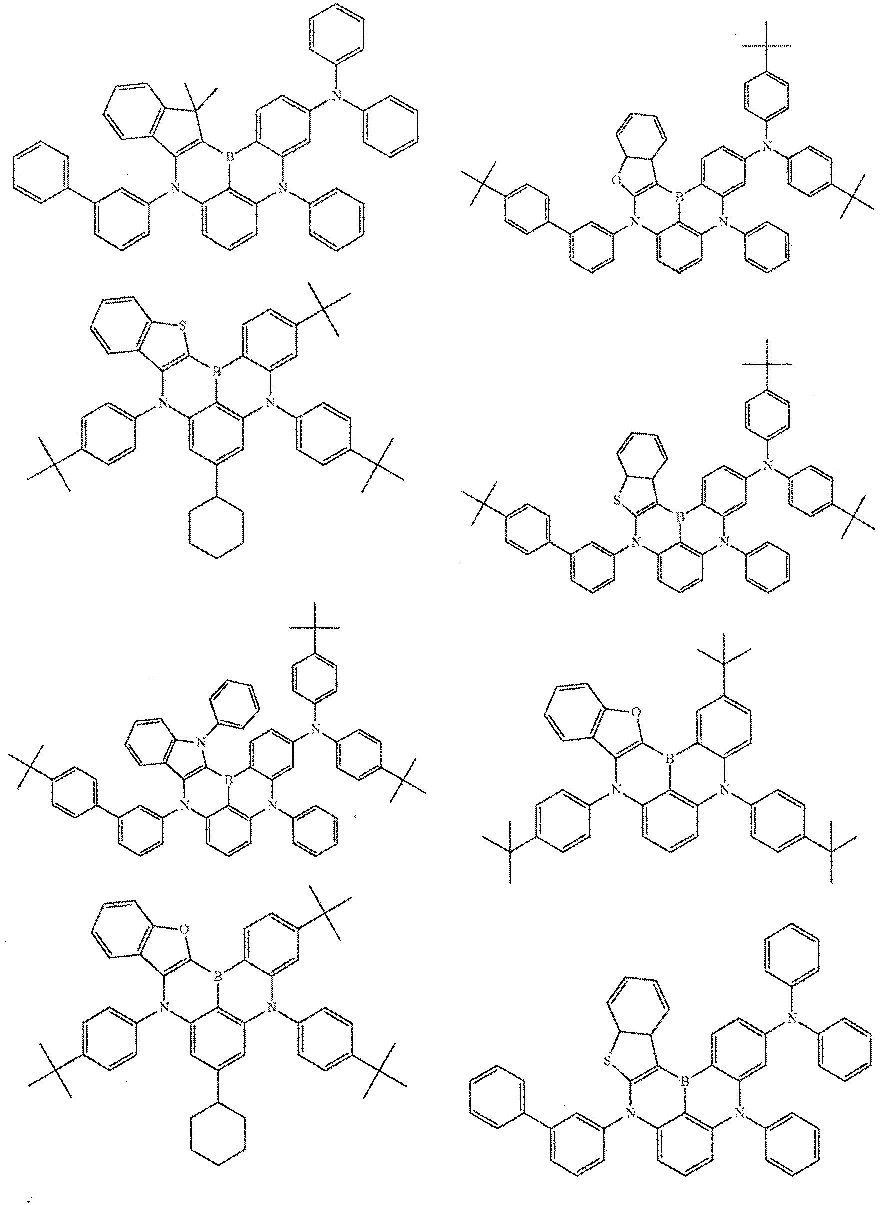

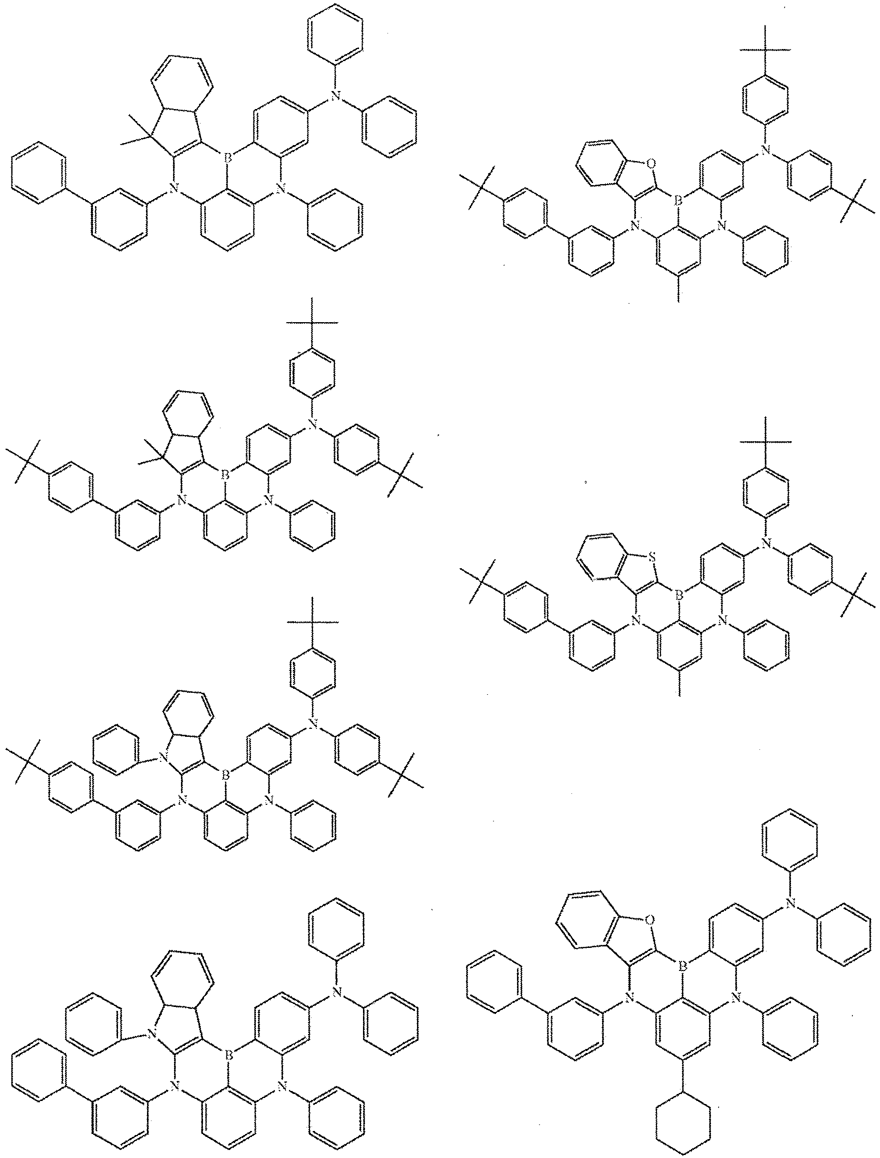

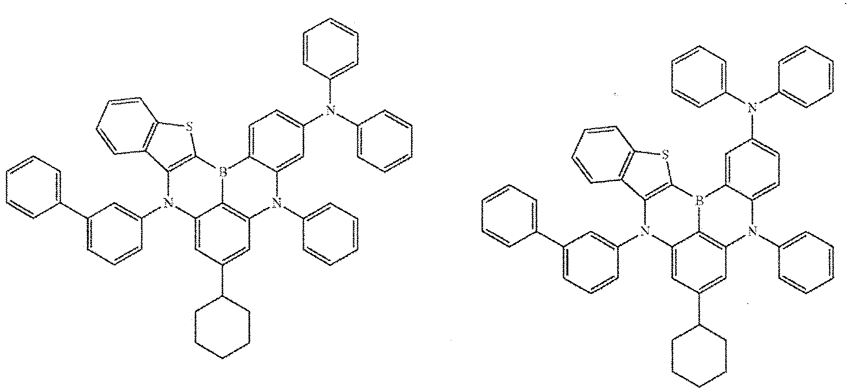

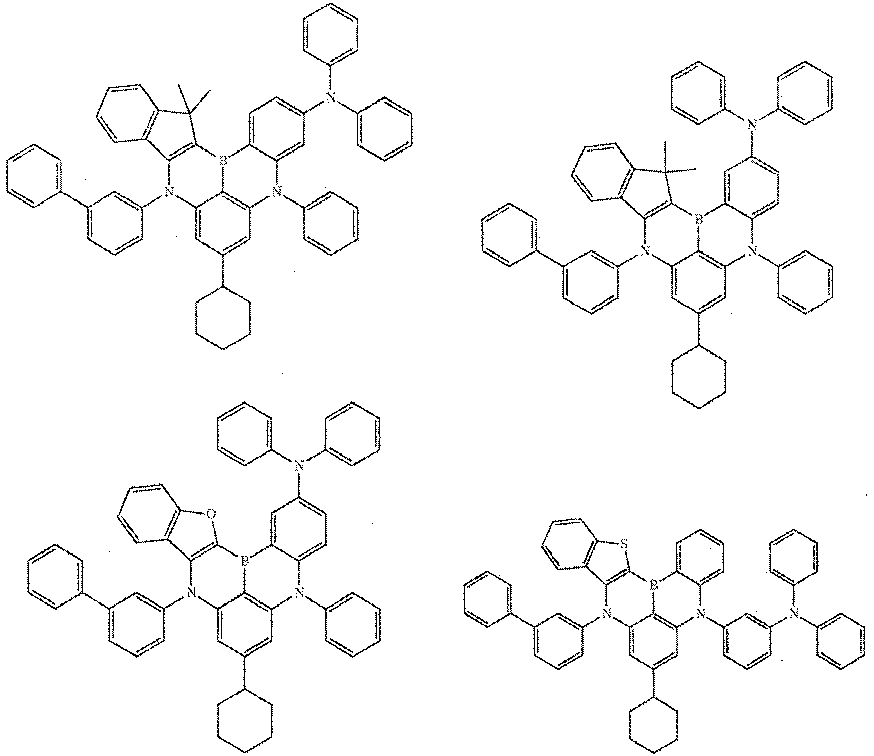

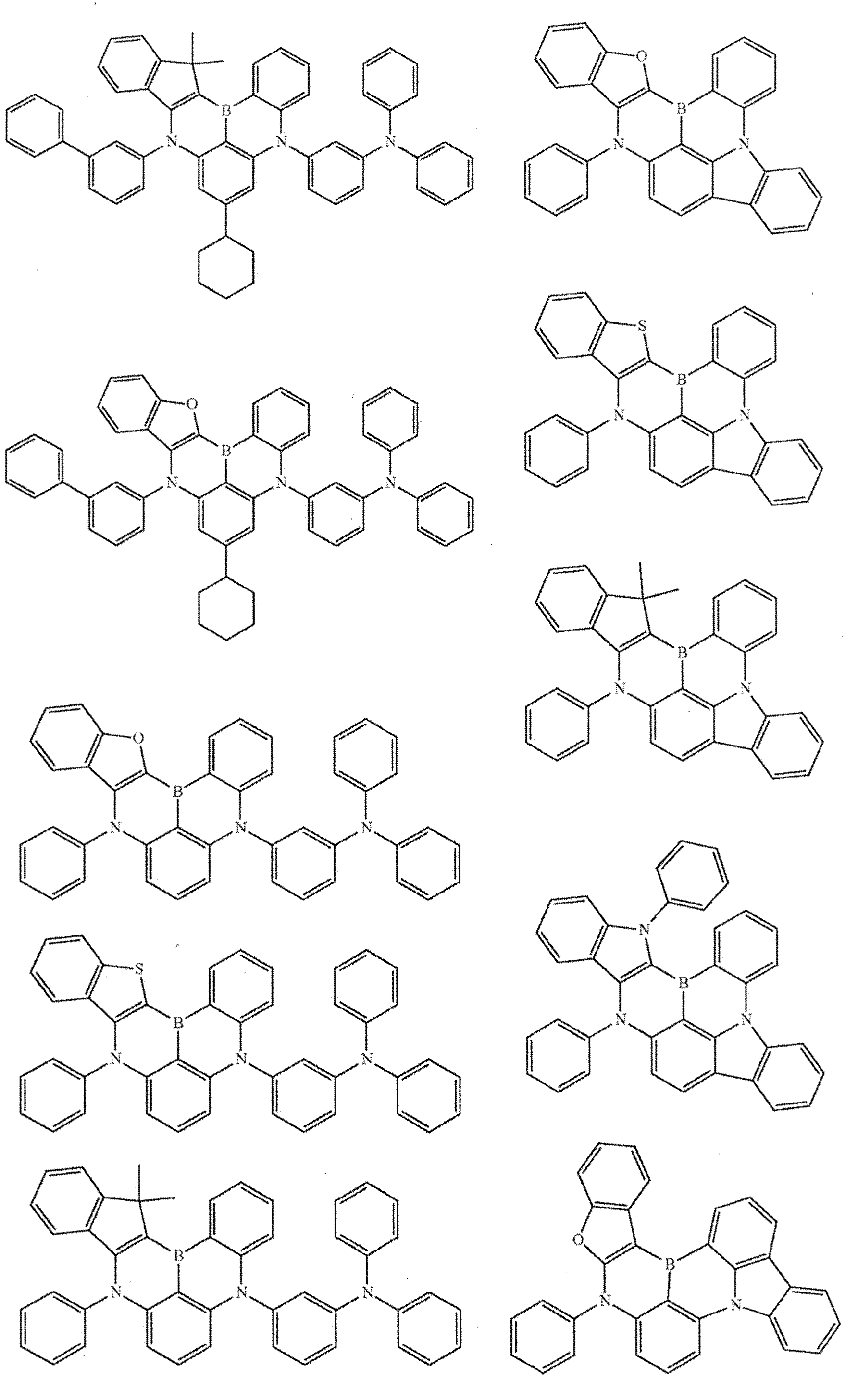

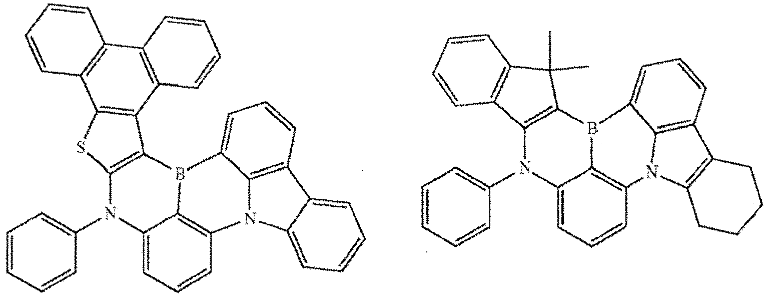

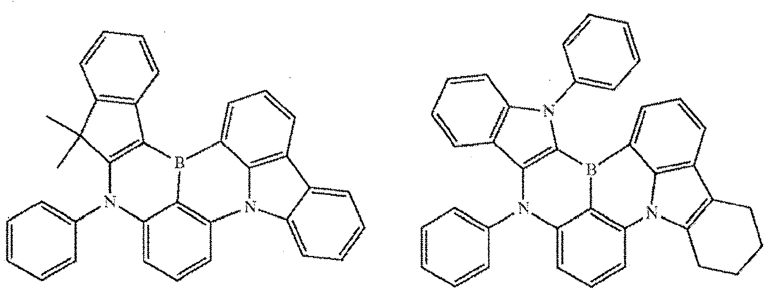

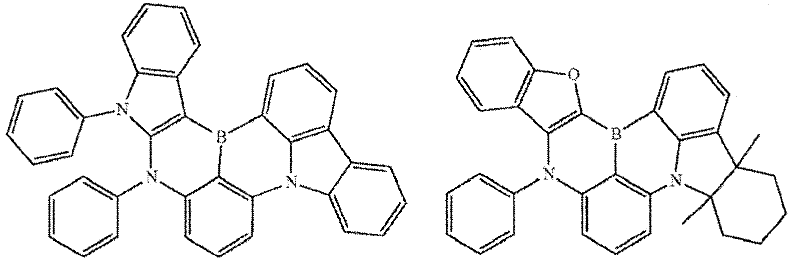

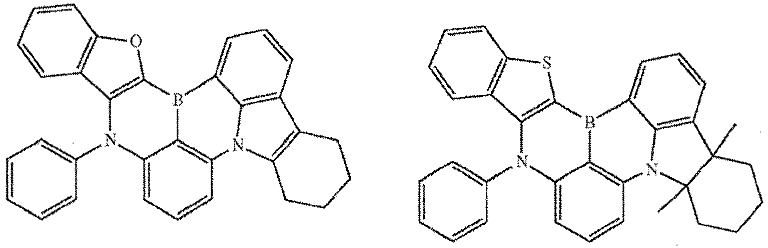

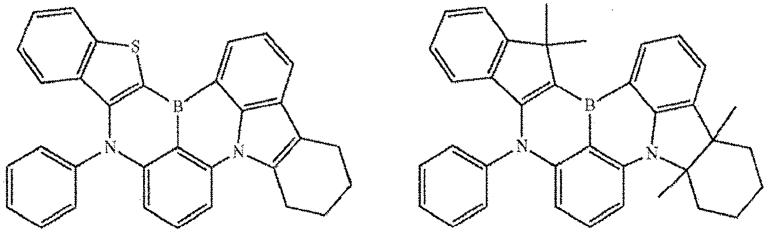

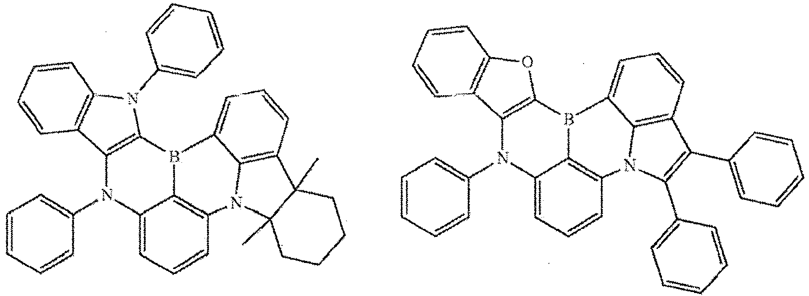

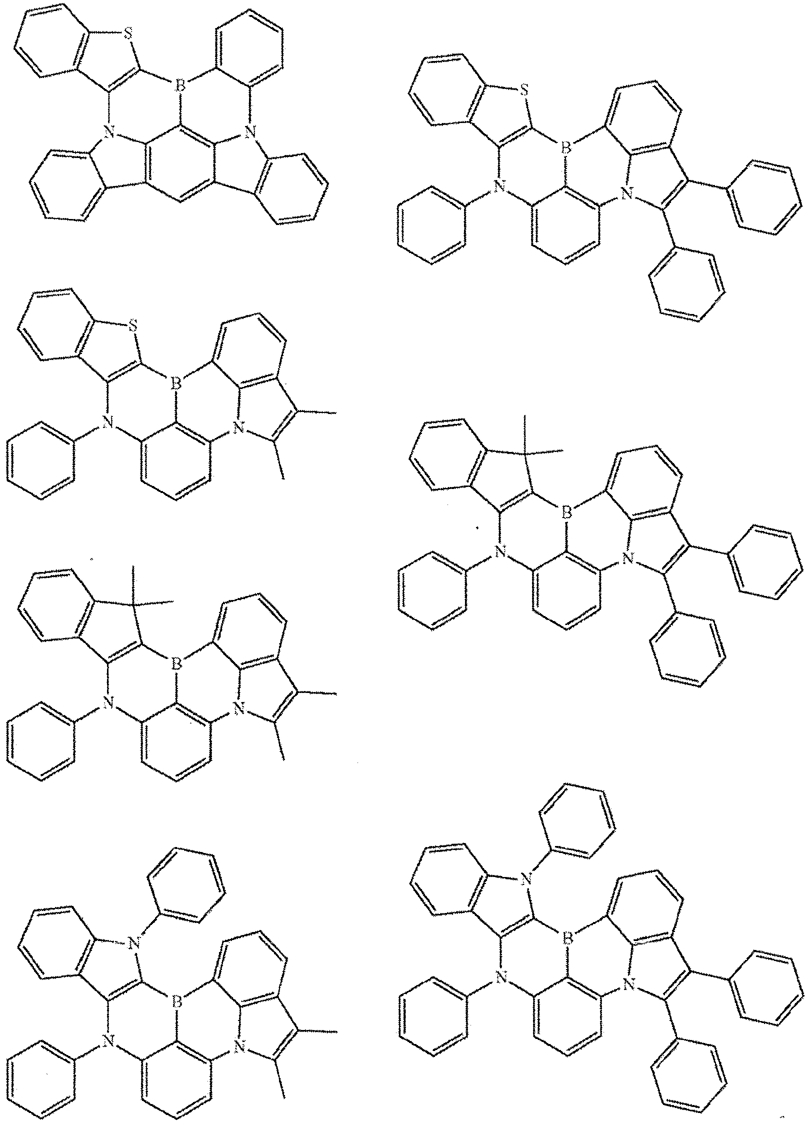

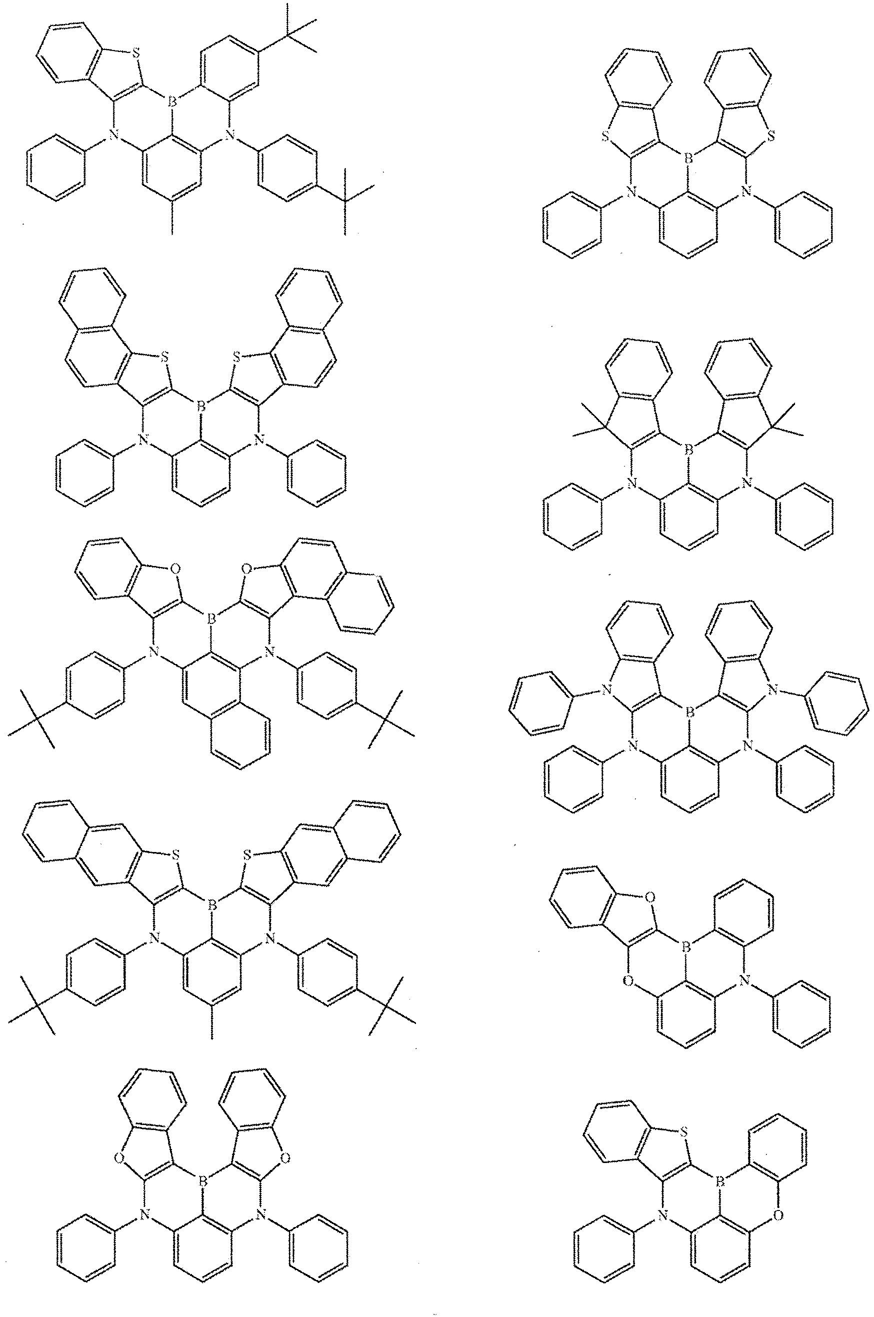

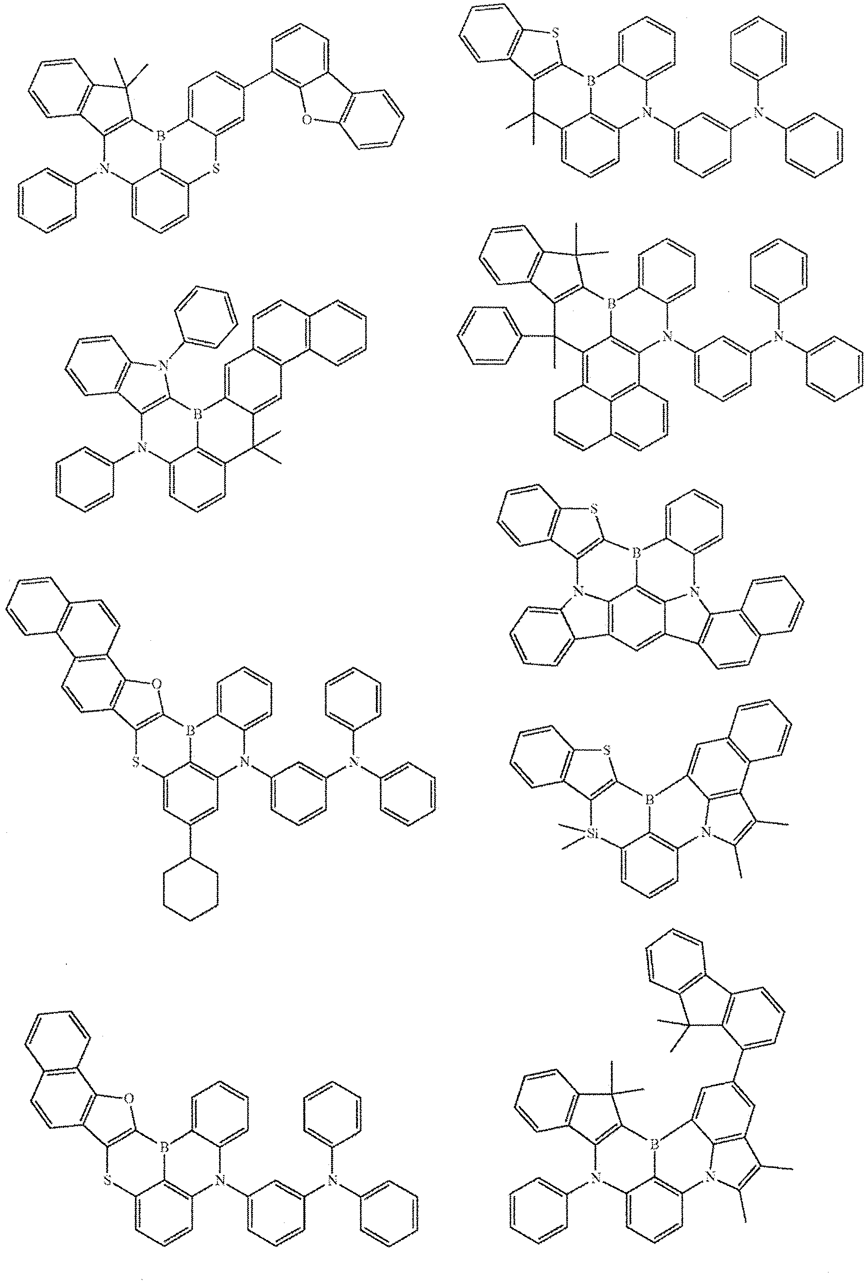

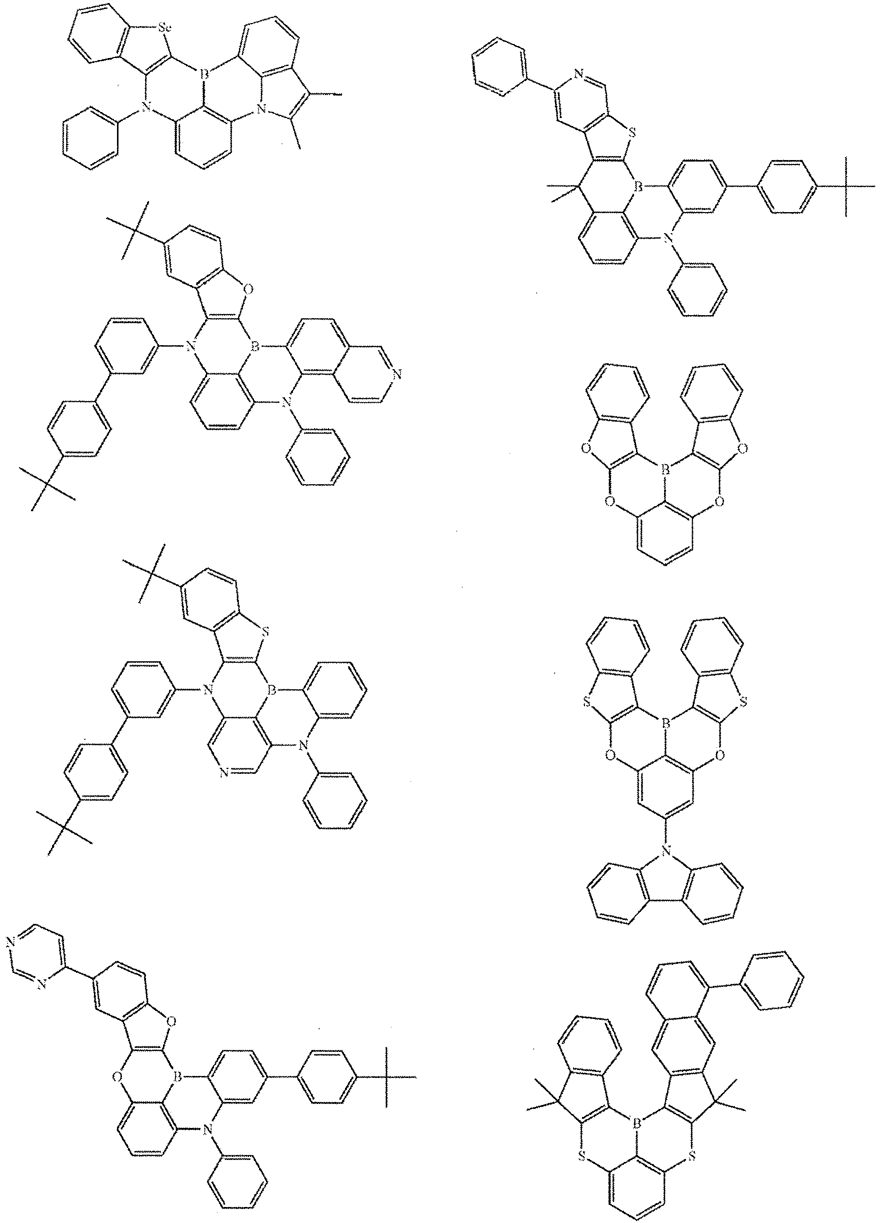

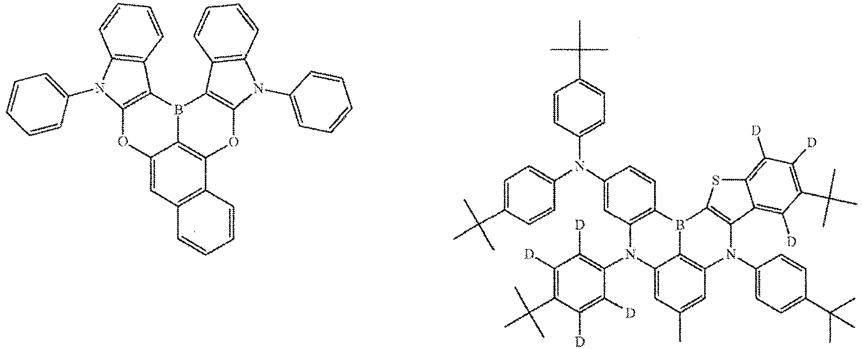

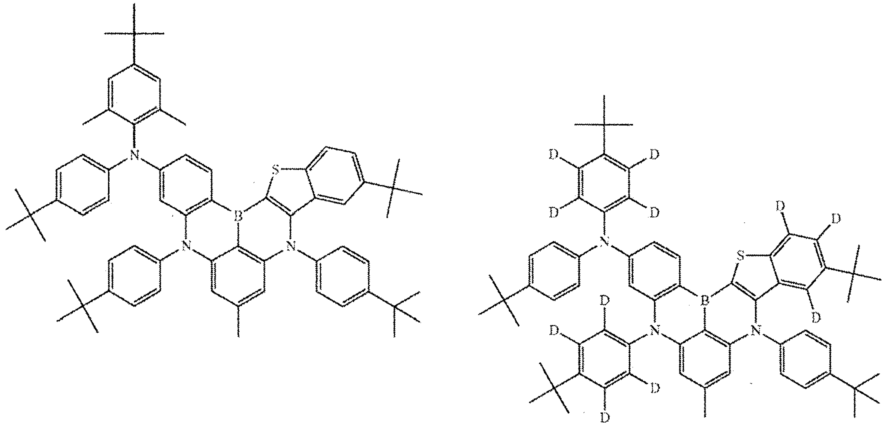

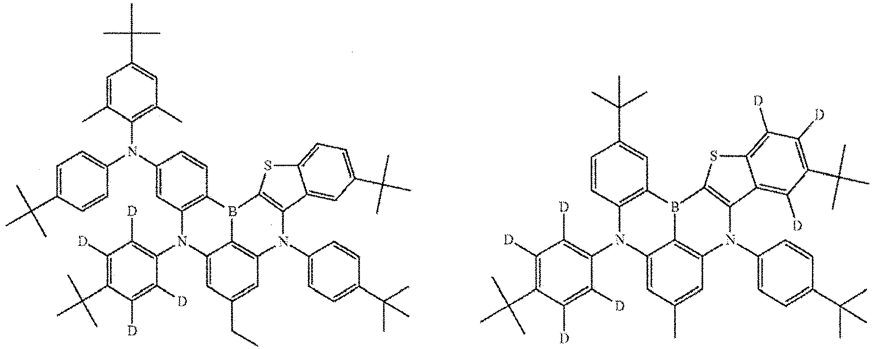

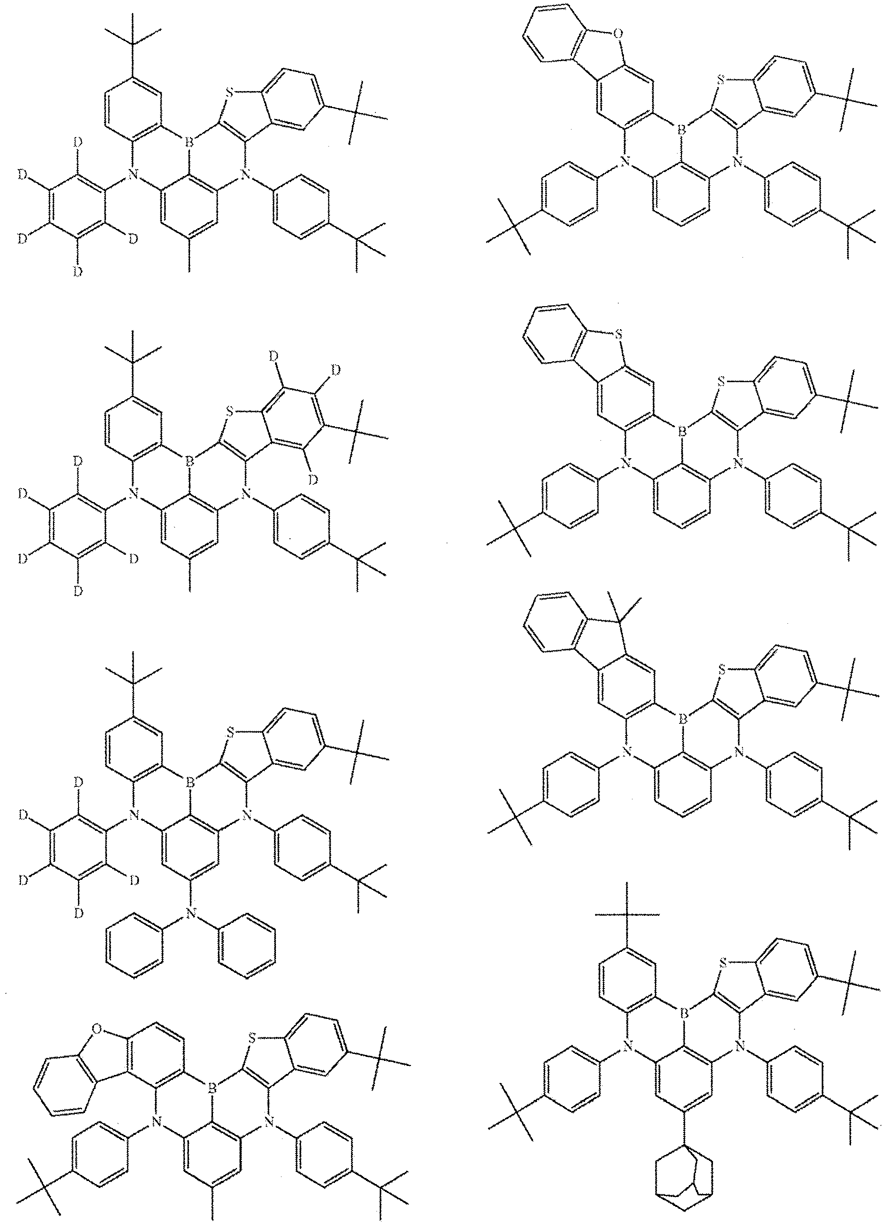

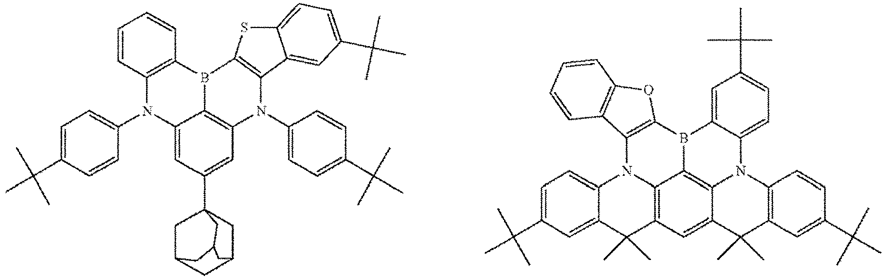

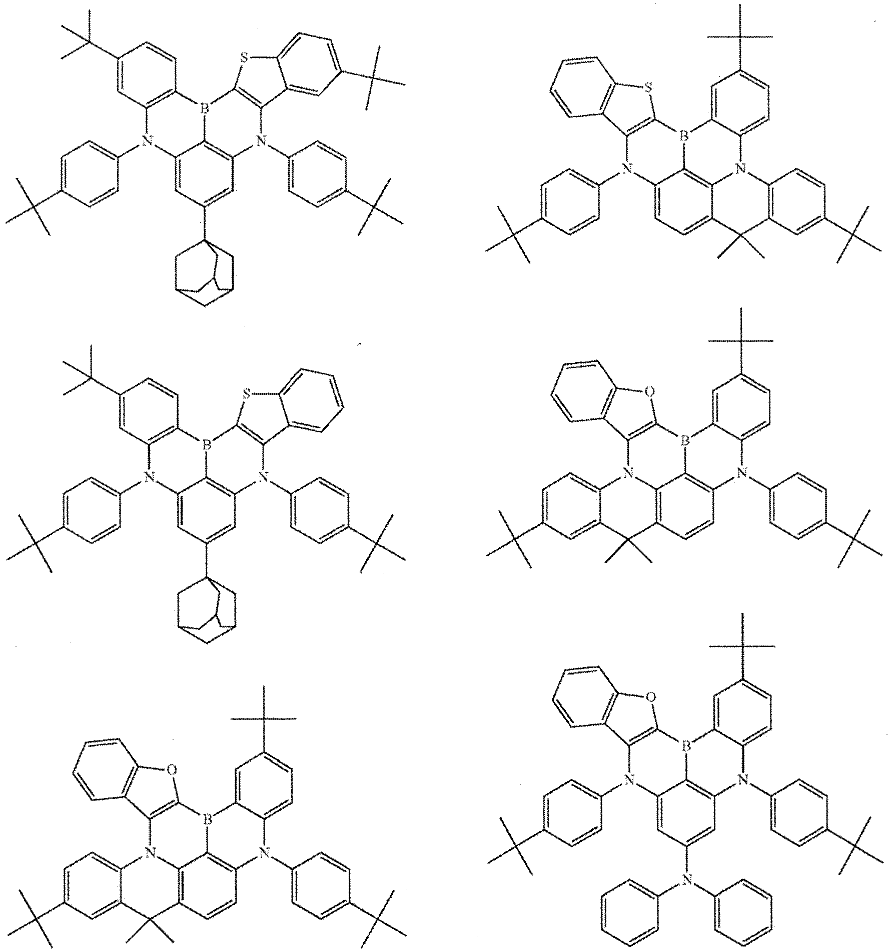

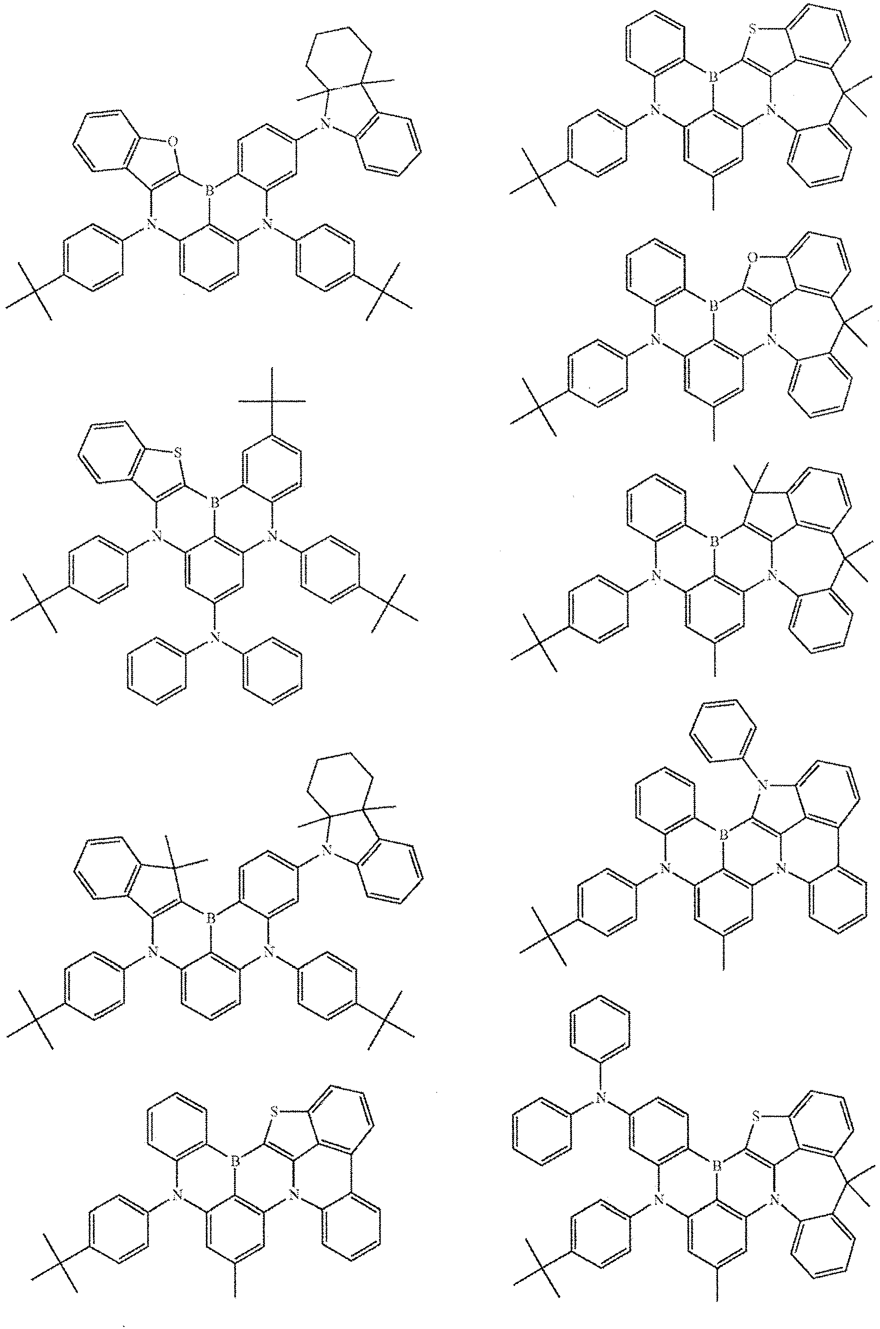

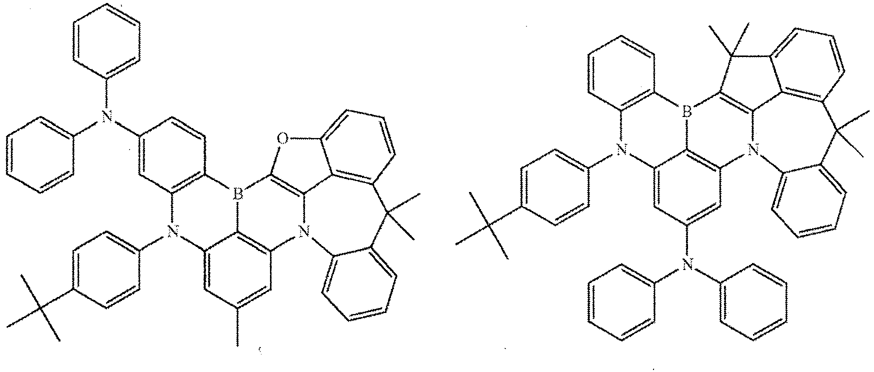

In the organic EL device according to an aspect of the present invention, at least one layer of the one or more emitting layers includes a compound represented by the formula (1A) or (1B), and a compound represented by the formula (41); except that, conventionally-known materials and device configurations can be applied, as long as the effect of the present invention is not impaired.

Members which can be used in the organic EL device according to an aspect of the present invention, materials for forming each layer, other than the above-mentioned compounds, and the like, will be described below.

As the representative device configuration of the organic EL device of the present invention, the following structures may be given:
(1) an anode / an emitting layer / a cathode,
(2) an anode / a hole-injecting layer / an emitting layer / a cathode,
(3) an anode / an emitting layer / an electron-injecting-transporting layer / a cathode,
(4) an anode / a hole-injecting layer / an emitting layer / an electron-injecting-transporting layer / a cathode,
(5) an anode / an organic semiconductor layer / an emitting layer / a cathode,
(6) an anode / an organic semiconductor layer / an electron-barrier layer / an emitting layer / a cathode,
(7) an anode / an organic semiconductor layer / an emitting layer / an adhesion improving layer / a cathode,
(8) an anode / a hole-injecting-transporting layer / an emitting layer / an electron-injecting-transporting layer / a cathode,
(9) an anode / an insulating layer / an emitting layer / an insulating layer / a cathode,
(10) an anode / an inorganic semiconductor layer / an insulating layer / an emitting layer / an insulating layer / a cathode,
(11) an anode / an organic semiconductor layer / an insulating layer / an emitting layer / an insulating layer / a cathode,
(12) an anode / an insulating layer / a hole-injecting-transporting layer / an emitting layer / an insulating layer / a cathode, and
(13) an anode / an insulating layer / a hole-injecting-transporting layer / an emitting layer / an electron-injecting-transporting layer / a cathode.

Among the above-described structures, the configuration of (8) is preferably used, but the device configuration of the organic EL device is not limited thereto.

In the present specification, the term "hole-injecting-transporting layer" means "at least one of the hole-injecting layer and the hole-transporting layer", and the term "electron-injecting-transporting layer" means "at least one of the electron-injecting layer and the electron-transporting layer".

Members which can be used in the organic EL device according to an aspect of the present invention, materials for forming each layer, other than the above-mentioned compounds, and the like, will be described below.

### (Substrate)

The substrate is used as a support of an emitting device. As the substrate, glass, quartz, plastic or the like can be used, for example. Further, a flexible substrate may be used. The term "flexible substrate" means a bendable (flexible) substrate, and specific examples thereof include a plastic substrate formed of polycarbonate, polyvinyl chloride or the like.

### (Anode)

For the anode formed on the substrate, metals, alloys, electrically conductive compounds, mixtures thereof, and the like, which have large work function (specifically 4.0 eV or more) are preferably used. Specific examples thereof include indium oxide-tin oxide (ITO: Indium Tin Oxide), indium oxide-tin oxide containing silicon or silicon oxide, indium oxide-zinc oxide, tungsten oxide, indium oxide containing zinc oxide, graphene, and the like. In addition thereto, specific examples thereof include gold (Au), platinum (Pt), a nitride of a metallic material (for example, titanium nitride), or the like.

### (Hole-injecting layer)

The hole-injecting layer is a layer containing a substance having high hole-injecting property. As the substance having high hole-injecting property, molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, an aromatic amine compound, a polymer compound (oligomers, dendrimers, polymers, and the like), or the like can be given.

### (Hole-transporting layer)

The hole-transporting layer is a layer containing a substance having high hole-transporting property. For the hole-transporting layer, an aromatic amine compound, a carbazole derivative, an anthracene derivative, or the like can be used. A polymer compound such as poly(N-vinylcarbazole) (abbreviation: PVK) and poly(4-vinyltriphenylamine) (abbreviation: PVTPA) can also be used. Provided that a substance other than the above-described substances may be used as long as the substance has higher hole-transporting property than electron-transporting property. The layer containing the substance having high hole-transporting property may be not only a single layer, but also layers in which two or more layers formed of the above-described substances are stacked.

### (Guest (dopant) material of emitting layer)

The emitting layer is a layer containing a substance having high luminous property, and various materials can be used in addition to the material (the compound represented by the formula (41)) used in the present invention described above. For example, as the substance having high emitting property, a fluorescent compound which emits fluorescence or a phosphorescent compound which emits phosphorescence can be used. The fluorescent compound is a compound which can emit from a singlet excited state, and the phosphorescent compound is a compound which can emit from a triplet excited state.

As a blue fluorescent emitting material which can be used for the emitting layer, pyrene derivatives, styrylamine derivatives, chrysene derivatives, fluoranthene derivatives, fluorene derivatives, diamine derivatives, triarylamine derivatives, and the like can be used. As a green fluorescent emitting material which can be used for the emitting layer, aromatic amine derivatives and the like can be used. As a red fluorescent emitting material which can be used for the emitting layer, tetracene derivatives, diamine derivatives and the like can be used.

As a blue phosphorescent emitting material which can be used for the emitting layer, metal complexes such as iridium complexes, osmium complexes and platinum complexes are used. As a green phosphorescent emitting material which can be used for the emitting layer, iridium complexes and the like are used. As a red phosphorescent emitting material which can be used for the emitting layer, metal complexes such as iridium complexes, platinum complexes, terbium complexes and europium complexes are used.

In an aspect of the present invention, the emitting layer may include or may not include another substance described above as the dopant material in addition to the compound represented by the formula (41).

### (Host material for emitting layer)

The emitting layer may have a constitution in which the substance having high emitting property (guest material) is dispersed in another substance (host material). As a substance for dispersing the substance having high emitting property, a variety of substances can be used in addition to the material (the compound represented by the formula (1), the compound represented by the formula (1A), or the compound represented by the formula (1B)) used in the present invention described above, and it is preferable to use a substance having a higher lowest unoccupied molecular orbital level (LUMO level) and a lower highest occupied molecular orbital level (HOMO level) than a substance having high emitting property.

As a substance (host material) for dispersing the substance having high emitting property, 1) a metal complex such as an aluminum complex, a beryllium complex, and a zinc complex, 2) a heterocyclic compound such as an oxadiazole derivative, a benzimidazole derivative, and a phenanthroline derivative, 3) a fused aromatic compound such as a carbazole derivative, an anthracene derivative, a phenanthrene derivative, a pyrene derivative, and a chrysene derivative, and 4) an aromatic amine compound such as a triarylamine derivative and a fused polycyclic aromatic amine derivative are used.

A compound having delayed fluorescence (thermally activated delayed fluorescence) can also be used as the host material. It is also preferable that the emitting layer includes the material used in the present invention described above and the host compound having delayed fluorescence.

### (Electron-transporting layer)

The electron-transporting layer is a layer containing a substance having high electron-transporting property. For the electron-transporting layer, 1) a metal complex such as an aluminum complex, a beryllium complex, and a zinc complex; 2) a heteroaromatic complex such as an imidazole derivative, a benzimidazole derivative, an azine derivative, carbazole derivative, and a phenanthroline derivative; and 3) a polymer compound can be used.

### (Electron-injecting layer)

The electron-injecting layer is a layer containing a substance having high electron-injecting property. For the electron-injecting layer, lithium (Li), ytterbium (Yb), lithium fluoride (LiF), cesium fluoride (CsF), calcium fluoride (CaF₂), a metal complex compound such as 8-hydroxyquinolinolato-lithium (Liq), an alkali metal such as lithium oxide (LiOₓ), an alkaline earth metal, or a compound thereof can be used.

### (Cathode)

For the cathode, metals, alloys, electrically conductive compounds, mixtures thereof, and the like, which have small work function (specifically 3.8 eV or less) are preferably used. Specific examples of such a cathode material include an element belonging to Group 1 or Group 2 of the Periodic Table of the Elements, i.e., an alkali metal such as lithium (Li) and cesium (Cs), an alkaline earth metal such as magnesium (Mg), calcium (Ca) and strontium (Sr), and an alloy containing these (e.g., MgAg and AlLi); a rare earth metal such as europium (Eu) and ytterbium (Yb), and an alloy containing these.

The cathode is usually formed by a vacuum deposition method or a sputtering method. Further, when silver paste or the like is used, it is possible to use the coating method, the inkjet method or the like.

When the electron-injecting layer is provided, the cathode can be formed using various conductive materials such as aluminum, silver, ITO, graphene, indium oxide-tin oxide containing silicon or silicon oxide, regardless of the work function value.

### (Electron-blocking layer, hole-blocking layer, exciton-blocking layer)

An electron-blocking layer, a hole-blocking layer, an exciton (triplet)-blocking layer, and the like may be provided adjacent to the emitting layer.

The electron-blocking layer is a layer which has a function of preventing leakage of electrons from the emitting layer to the hole-transporting layer. The hole-blocking layer is a layer which has a function of preventing leakage of holes from the emitting layer to the electron-transporting layer. The exciton-blocking layer is a layer which has a function of preventing diffusion of excitons generated in the emitting layer into the adjacent layers to confine the excitons within the emitting layer.

In the organic EL device according to an aspect of the present invention, the thickness of each layer is not particularly limited, but is normally preferable several nm to 1 µm generally in order to suppress defects such as pinholes, to suppress applied voltages to be low, and to improve luminous efficiency.

In the organic EL device according to an aspect of the present invention, the method for forming each layer is not particularly limited. A conventionally-known method for forming each layer such as a vacuum deposition process and a spin coating process can be used. Each layer such as the emitting layer can be formed by a known method such as a vacuum deposition process, a molecular beam deposition process (MBE process), or an application process such as a dipping process, a spin coating process, a casting process, a bar coating process and a roll coating process, using a solution prepared by dissolving the material in a solvent.

In the organic EL device according to an aspect of the present invention, the emitting layer can be formed, for example, by vaporizing and simultaneously depositing the compound represented by the formula (1A) or the compound represented by the formula (1B), and the compound represented by the formula (41) each in different deposition sources (crucibles).

Further, the emitting layer may be formed, for example, by mixing the compound represented by the formula (1A) or the compound represented by the formula (1B), and the compound represented by the formula (41) in advance, and then depositing them in the same deposition source. The above method has an advantage in that a fabrication apparatus and a fabrication step can be simplified.

### [Electronic apparatus]

An electronic apparatus according to an aspect of the present invention is characterized by including the organic EL device according to an aspect of the present invention.

Specific examples of the electronic apparatus include display components such as an organic EL panel module; display devices for a television, a cellular phone and a personal computer; and emitting devices such as a light and a vehicular lamp; and the like.

### Examples

Hereinafter, Examples according to the present invention will be described. The present invention is not limited to these Examples.

### <Compound>

Compounds represented by the formula (1), (1A), or (1B) used in the fabrication of the organic EL devices of Examples 1 to 21 are shown below.

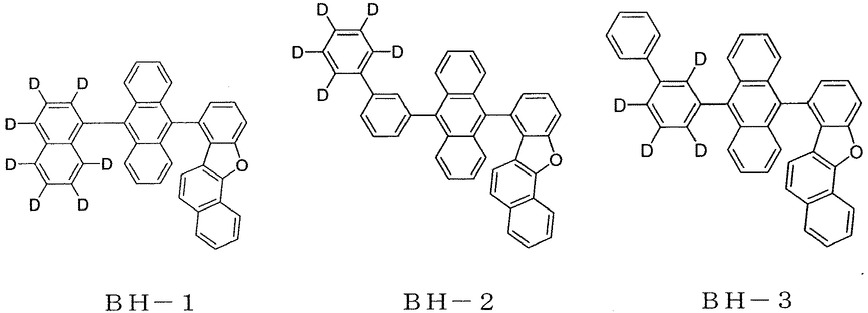

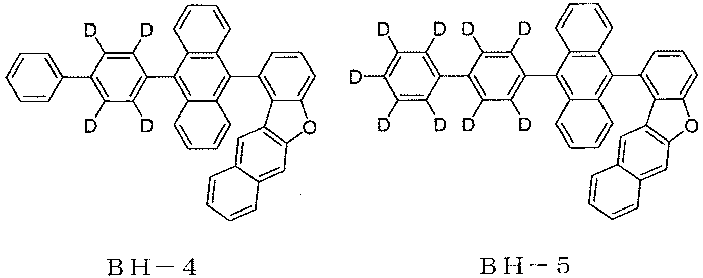

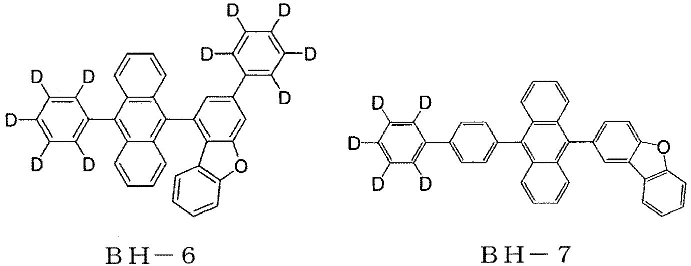

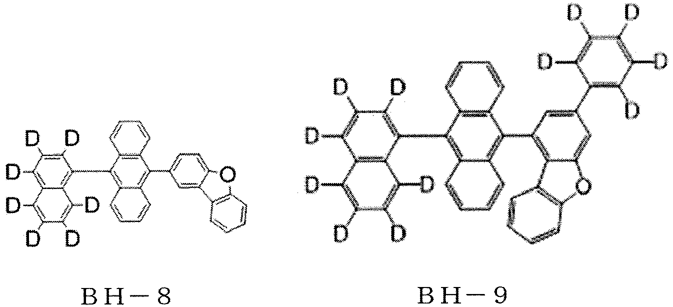

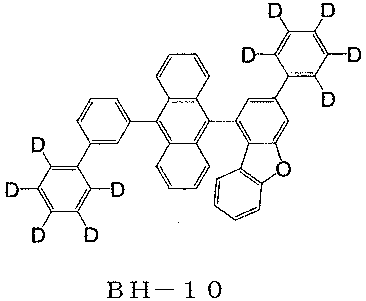

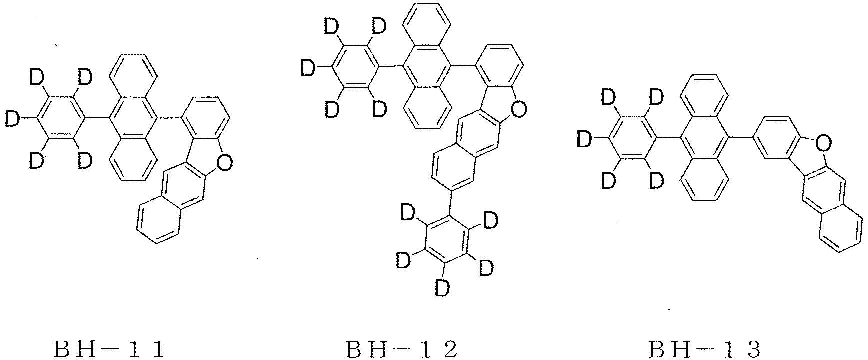

Compounds represented by the formula (41) used in the fabrication of the organic EL devices of Examples 1 to 21 and Comparative Examples 1 to 18 are shown below.

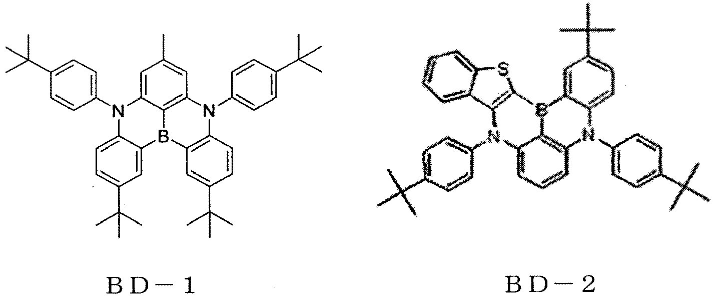

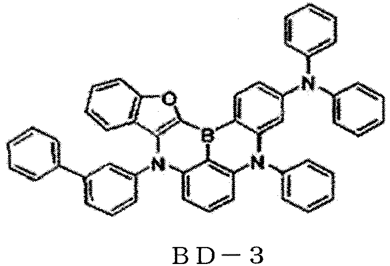

Comparative compounds used in the fabrication of the organic EL devices of Comparative Examples 1 to 18 are shown below.

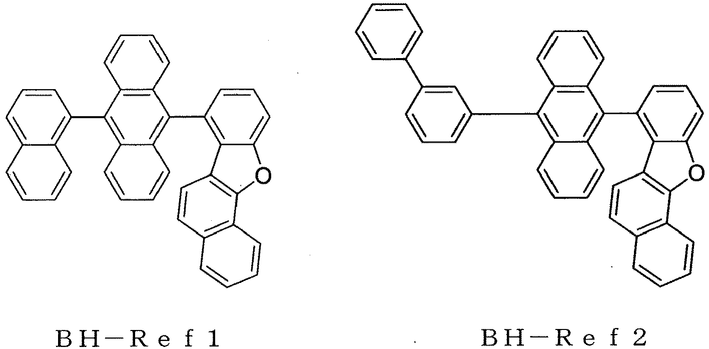

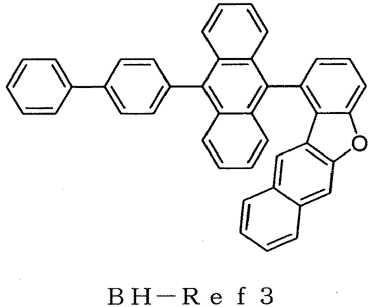

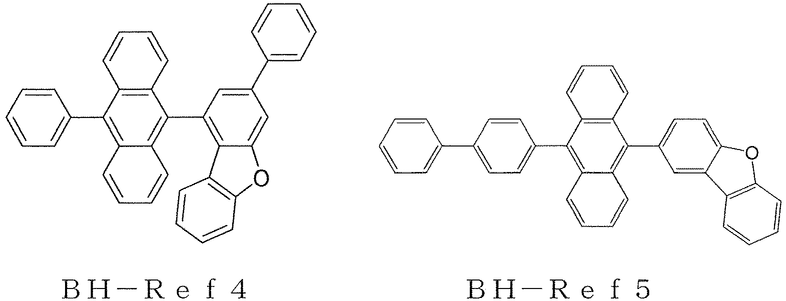

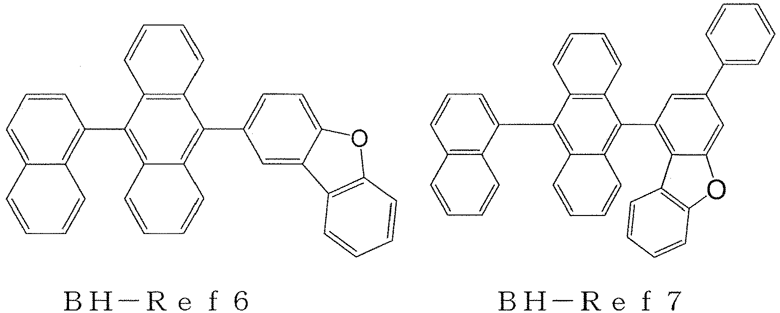

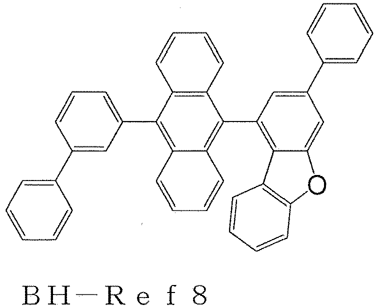

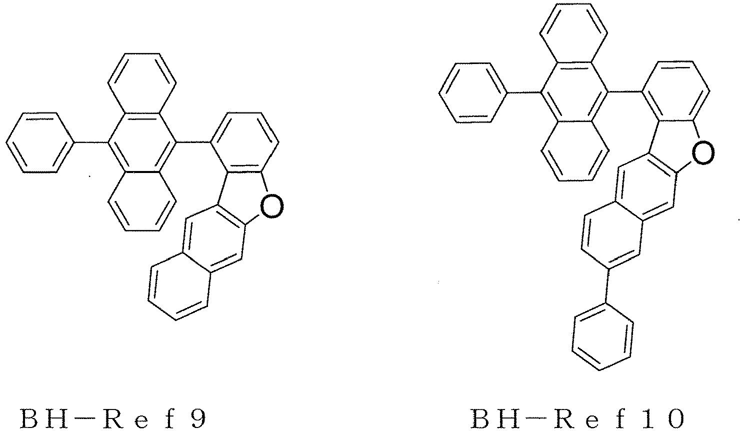

Other compound structures used in the fabrication of the organic EL devices of Examples 1 to 21 and Comparative Examples 1 to 18 are shown below.

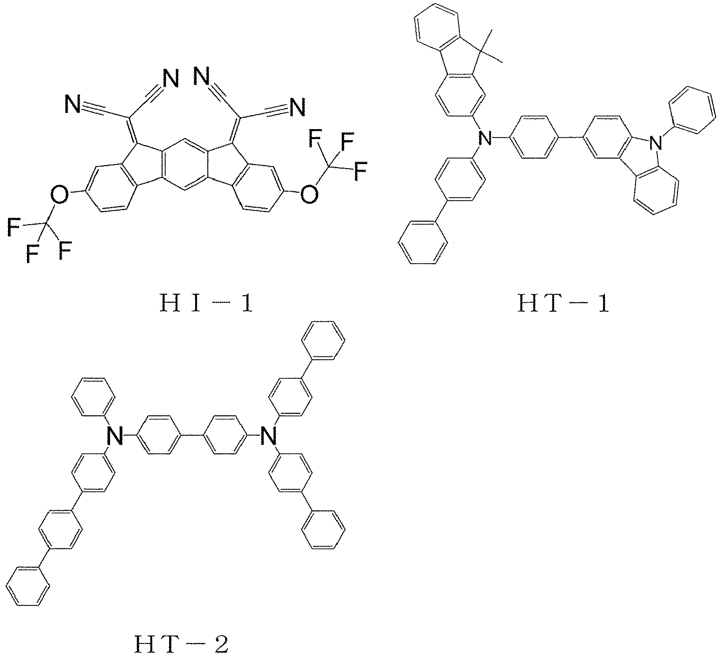

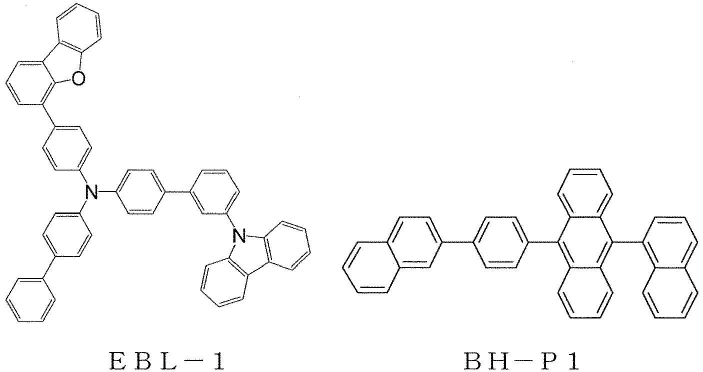

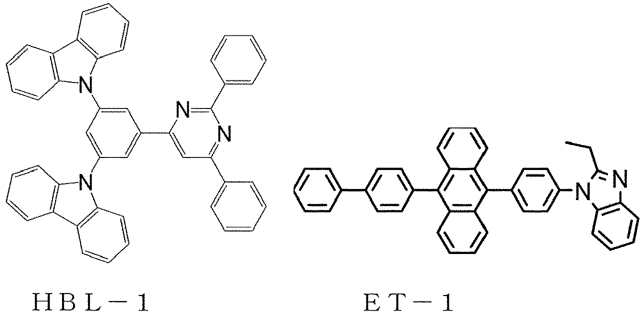

### Example 1

### <Fabrication of organic EL device>

An organic EL device was fabricated as follows.

A 25 mm × 75 mm × 1.1 mm-thick glass substrate with an ITO transparent electrode (anode) (manufactured by GEOMATEC Co., Ltd.) was subjected to ultrasonic cleaning in isopropyl alcohol for 5 minutes, and then subjected to UV-ozone cleaning for 30 minutes. The ITO has the film thickness of 130 nm.

The glass substrate with the transparent electrode after being cleaned was mounted onto a substrate holder in a vacuum vapor deposition apparatus. First, a compound HI-1 was deposited on the surface on the side where the transparent electrode was formed so as to cover the transparent electrode to form a compound HI-1 film having the thickness of 5 nm. The HI-1 film functions as a hole-injecting layer.

Subsequent to the formation of the HI-1 film, a compound HT-1 was deposited thereon to form an HT-1 film having the thickness of 80 nm on the HI-1 film. The HT-1 film functions as a first hole-transporting layer.

Following the formation of the HT-1 film, a compound EBL-1 was deposited thereon to form an EBL-1 film having the thickness of 10 nm on the HT-1 film. The EBL-1 film functions as a second hole-transporting layer.

BH-1 (host material) and BD-1 (dopant material) were co-deposited on the EBL-1 film to be 2% in a proportion (weight ratio) of the compound BD-1 to form an emitting layer having the thickness of 25 nm.

A compound HBL-1 was deposited on the emitting layer to form an electron-transporting layer having the thickness of 10 nm. A compound ET-1 being an electron-injecting material was deposited on the electron-transporting layer to form an electron-injecting layer having the thickness of 15 nm. LiF was deposited on the electron-injecting layer to form a LiF film having the thickness of 1 nm. Metal Al was deposited on the LiF film to form a metal cathode having the thickness of 80 nm.

The device configuration of the organic EL device of Example 1 is schematically shown as follows.
ITO(130)/HI-1(5)/HT-1(80)/EBL-1(10)/BH-1:BD-1(25:2%)/HBL-1(10)/ET-1(15)/LiF(1)/AI(80)

The numerical values in parentheses indicate the film thickness (unit: nm). The numerical values represented by percent in parentheses indicate a proportion (% by mass) of the latter compound in the layer.

### <Evaluation of organic EL device>

### • Driving voltage

The initial property of the organic EL device was measured by driving it using DC (direct current) constant current of 10 mA/cm² at room temperature. The results are shown in Table 1.

### • External quantum efficiency

A voltage was applied to the organic EL device so that the current density became 10 mA/cm², and the EL emission spectrum was measured by using Spectroradiometer CS-2000 (manufactured by KONICA MINOLTA, INC.). External quantum efficiency (EQE) (%) was calculated from the obtained spectral emission luminance spectrum. The results are shown in Table 1.

### •Device lifetime

Regarding the obtained organic EL device, a voltage was applied to the obtained organic EL device at room temperature so that the current density became 50 mA/cm², and the time until the luminance became 95% of the initial luminance (LT95 (unit: hours)) was measured. The results are shown in Table 1.

### (Comparative Example 1)

An organic EL device was fabricated and evaluated in the same manner as in Example 1, except that BH-Ref1 was used instead of the compound BH-1 of Example 1 in formation of the emitting layer.

**Table 1**

| | BH | Voltage [V] | EQE [%] | LT95 [h] |
|---|---|---|---|---|
| Example 1 | BH-1 | 3.4 | 8.5 | 55 |
| Comparative Example 1 | BH-Ref1 | 3.4 | 8.5 | 45 |

### (Example 2)

An organic EL device was fabricated and evaluated in the same manner as in Example 1, except that BH-2 was used instead of the compound BH-1 of Example 1 in formation of the emitting layer. The results are shown in Table 2.

### (Example 3)

An organic EL device was fabricated and evaluated in the same manner as in Example 1, except that BH-3 was used instead of the compound BH-1 of Example 1 in formation of the emitting layer. The results are shown in Table 2.

### (Comparative Example 2)

An organic EL device was fabricated and evaluated in the same manner as in Example 2, except that BH-Ref2 was used instead of the compound BH-1 of Example 1 in formation of the emitting layer. The results are shown in Table 2.

**Table 2**

| | BH | Voltage [V] | EQE [%] | LT95 [h] |
|---|---|---|---|---|
| Example 2 | BH-2 | 3.3 | 8.6 | 60 |
| Example 3 | BH-3 | 3.3 | 8.6 | 65 |
| Comparative Example 2 | BH-Ref2 | 3.3 | 8.6 | 50 |

### (Example 4)

An organic EL device was fabricated and evaluated in the same manner as in Example 1, except that BH-4 was used instead of the compound BH-1 of Example 1 in formation of the emitting layer. The results are shown in Table 3.

### (Example 5)

An organic EL device was fabricated and evaluated in the same manner as in Example 1, except that BH-5 was used instead of the compound BH-1 of Example 1 in formation of the emitting layer. The results are shown in Table 3.

### (Comparative Example 3)

An organic EL device was fabricated and evaluated in the same manner as in Example 1, except that BH-Ref3 was used instead of the compound BH-1 of Example 1 in formation of the emitting layer. The results are shown in Table 3.

**Table 3**

| | BH | Voltage [V] | EQE [%] | LT95 [h] |
|---|---|---|---|---|
| Example 4 | BH-4 | 3.2 | 8.1 | 45 |
| Example 5 | BH-5 | 3.2 | 8.1 | 50 |
| Comparative Example 3 | BH-Ref3 | 3.2 | 8.1 | 40 |

### Example 6

### <Fabrication of organic EL device>

An organic EL device was fabricated as follows.

A 25 mm × 75 mm × 1.1 mm-thick glass substrate with an ITO transparent electrode (anode) (manufactured by GEOMATEC Co., Ltd.) was subjected to ultrasonic cleaning in isopropyl alcohol for 5 minutes, and then subjected to UV-ozone cleaning for 30 minutes. The ITO has the film thickness of 130 nm.

The glass substrate with the transparent electrode after being cleaned was mounted onto a substrate holder in a vacuum vapor deposition apparatus. First, a compound HI-1 was deposited on the surface on the side where the transparent electrode was formed so as to cover the transparent electrode to form a compound HI-1 film having the thickness of 5 nm. The HI-1 film functions as a hole-injecting layer.

Subsequent to the formation of the HI-1 film, a compound HT-1 was deposited thereon to form an HT-1 film having the thickness of 80 nm on the HI-1 film. The HT-1 film functions as a first hole-transporting layer.

Following the formation of the HT-1 film, a compound EBL-1 was deposited thereon to form an EBL-1 film having the thickness of 10 nm on the HT-1 film. The EBL-1 film functions as a second hole-transporting layer.

BH-6 (host material) and BD-1 (dopant material) were co-deposited on the EBL-1 film to be 2% in a proportion (weight ratio) of the compound BD-1 to form an emitting layer having the thickness of 25 nm.

A compound HBL-1 was deposited on the emitting layer to form an electron-transporting layer having the thickness of 10 nm. A compound ET-1 being an electron-injecting material was deposited on the electron-transporting layer to form an electron-injecting layer having the thickness of 15 nm. LiF was deposited on the electron-injecting layer to form a LiF film having the thickness of 1 nm. Metal Al was deposited on the LiF film to form a metal cathode having the thickness of 80 nm.

The device configuration of the organic EL device of Example 6 is schematically shown as follows.
ITO(130)/HI-1(5)/HT-1(80)/EBL-1(10)/BH-6:BD-1(25:2%)/HBL-1(10)/ET-1(15)/LiF(1)/AI(80)

The numerical values in parentheses indicate the film thickness (unit: nm).

The numerical values represented by percent in parentheses indicate a proportion (% by mass) of the latter compound in the layer.

### <Evaluation of organic EL device>

Driving voltage, external quantum efficiency, and device lifetime of the organic EL devices were measured in the same manner as in Example 1. The results are shown in Table 4.

### Example 7

An organic EL device was fabricated and evaluated in the same manner as in Example 6, except that BD-2 was used instead of the compound BD-1 of Example 6 in formation of the emitting layer. The results are shown in Table 4.

### Example 8

An organic EL device was fabricated and evaluated in the same manner as in Example 6, except that BD-3 was used instead of the compound BD-1 of Example 6 in formation of the emitting layer. The results are shown in Table 4.

### Comparative Example 4

An organic EL device was fabricated and evaluated in the same manner as in Example 6, except that BH-Ref4 was used instead of the compound BH-6 of Example 6 in formation of the emitting layer. The results are shown in Table 4.

### Comparative Example 5

An organic EL device was fabricated and evaluated in the same manner as in Example 7, except that BH-Ref4 was used instead of the compound BH-6 of Example 7 in formation of the emitting layer. The results are shown in Table 4.

### Comparative Example 6

An organic EL device was fabricated and evaluated in the same manner as in Example 8, except that BH-Ref4 was used instead of the compound BH-6 of Example 8 in formation of the emitting layer. The results are shown in Table 4.

**Table 4**

| | BH | BD | Voltage [V] | EQE [%] | LT95 [h] |
|---|---|---|---|---|---|
| Example 6 | BH-6 | BD-1 | 3.4 | 9.4 | 65 |
| Example 7 | BH-6 | BD-2 | 3.3 | 9.8 | 80 |
| Example 8 | BH-6 | BD-3 | 3.4 | 9.6 | 58 |
| Comparative Example 4 | BH-Ref4 | BD-1 | 3.4 | 9.4 | 45 |
| Comparative Example 5 | BH-Ref4 | BD-2 | 3.3 | 9.7 | 55 |
| Comparative Example 6 | BH-Ref4 | BD-3 | 3.4 | 9.6 | 40 |

### Example 9

An organic EL device was fabricated and evaluated in the same manner as in Example 6, except that BH-7 was used instead of the compound BH-6 of Example 6 in formation of the emitting layer. The results are shown in Table 5.

### Example 10

An organic EL device was fabricated and evaluated in the same manner as in Example 9, except that BD-2 was used instead of the compound BD-1 of Example 9 in formation of the emitting layer. The results are shown in Table 5.

### Example 11

An organic EL device was fabricated and evaluated in the same manner as in Example 9, except that BD-3 was used instead of the compound BD-1 of Example 9 in formation of the emitting layer. The results are shown in Table 5.

### Comparative Example 7

An organic EL device was fabricated and evaluated in the same manner as in Example 9, except that BH-Ref5 was used instead of the compound BH-7 of Example 9 in formation of the emitting layer. The results are shown in Table 5.

### Comparative Example 8

An organic EL device was fabricated and evaluated in the same manner as in Example 10, except that BH-Ref5 was used instead of the compound BH-7 of Example 10 in formation of the emitting layer. The results are shown in Table 5.

### Comparative Example 9

An organic EL device was fabricated and evaluated in the same manner as in Example 11, except that BH-Ref5 was used instead of the compound BH-7 of Example 11 in formation of the emitting layer. The results are shown in Table 5.

**Table 5**

| | BH | BD | Voltage [V] | EQE [%] | LT95 [h] |
|---|---|---|---|---|---|
| Example 9 | BH-7 | BD-1 | 3.6 | 8.7 | 83 |
| Example 10 | BH-7 | BD-2 | 3.5 | 9.0 | 94 |
| Example 11 | BH-7 | BD-3 | 3.6 | 8.8 | 71 |
| Comparative Example 7 | BH-Ref5 | BD-1 | 3.6 | 8.7 | 72 |
| Comparative Example 8 | BH-Ref5 | BD-2 | 3.5 | 8.9 | 79 |
| Comparative Example 9 | BH-Ref5 | BD-3 | 3.6 | 8.8 | 67 |

### Example 12

### <Fabrication of organic EL device>

An organic EL device was fabricated as follows.

A 25 mm × 75 mm × 1.1 mm-thick glass substrate with an ITO transparent electrode (anode) (manufactured by GEOMATEC Co., Ltd.) was subjected to ultrasonic cleaning in isopropyl alcohol for 5 minutes, and then subjected to UV-ozone cleaning for 30 minutes. The ITO has the film thickness of 130 nm.

The glass substrate with the transparent electrode after being cleaned was mounted onto a substrate holder in a vacuum vapor deposition apparatus. First, a compound HI-1 was deposited on the surface on the side where the transparent electrode was formed so as to cover the transparent electrode to form a compound HI-1 film having the thickness of 5 nm. The HI-1 film functions as a hole-injecting layer.

Subsequent to the formation of the HI-1 film, a compound HT-2 was deposited thereon to form an HT-2 film having the thickness of 80 nm on the HI-1 film. The HT-2 film functions as a first hole-transporting layer.

Following the formation of the HT-1 film, a compound EBL-1 was deposited thereon to form an EBL-1 film having the thickness of 10 nm on the HT-1 film. The EBL-1 film functions as a second hole-transporting layer.

BH-8 (host material) and BD-1 (dopant material) were co-deposited on the EBL-1 film to be 2% in a proportion (weight ratio) of the compound BD-1 to form an emitting layer having the thickness of 25 nm.

A compound HBL-1 was deposited on the emitting layer to form an electron-transporting layer having the thickness of 10 nm. A compound ET-1 being an electron-injecting material was deposited on the electron-transporting layer to form an electron-injecting layer having the thickness of 15 nm. LiF was deposited on the electron-injecting layer to form a LiF film having the thickness of 1 nm. Metal Al was deposited on the LiF film to form a metal cathode having the thickness of 80 nm.

The device configuration of the organic EL device of Example 12 is schematically shown as follows.
ITO(130)/HI-1(5)/HT-2(80)/EBL-1(10)/BH-8:BD-1(25:2%)/HBL-1(10)/ET-1(15)/LiF(1)/AI(80)

The numerical values in parentheses indicate the film thickness (unit: nm).

The numerical values represented by percent in parentheses indicate a proportion (% by mass) of the latter compound in the layer.

### <Evaluation of organic EL device>

Driving voltage, external quantum efficiency, and device lifetime of the organic EL devices were measured in the same manner as in Example 1. The results are shown in Table 6.

### Example 13

An organic EL device was fabricated and evaluated in the same manner as in Example 12, except that BD-2 was used instead of the compound BD-1 of Example 12 in formation of the emitting layer. The results are shown in Table 6.

### Example 14

An organic EL device was fabricated and evaluated in the same manner as in Example 12, except that BD-3 was used instead of the compound BD-1 of Example 12 in formation of the emitting layer. The results are shown in Table 6.

### Example 15

An organic EL device was fabricated and evaluated in the same manner as in Example 12, except that BH-9 was used instead of the compound BH-8 of Example 12 in formation of the emitting layer. The results are shown in Table 6.

### Example 16

An organic EL device was fabricated and evaluated in the same manner as in Example 12, except that BH-10 was used instead of the compound BH-8 of Example 12 in formation of the emitting layer. The results are shown in Table 6.

### Comparative Example 10

An organic EL device was fabricated and evaluated in the same manner as in Example 12, except that BH-Ref6 was used instead of the compound BH-8 of Example 12 in formation of the emitting layer. The results are shown in Table 6.

### Comparative Example 11

An organic EL device was fabricated and evaluated in the same manner as in Example 13, except that BH-Ref6 was used instead of the compound BH-8 of Example 13 in formation of the emitting layer. The results are shown in Table 6.

### Comparative Example 12

An organic EL device was fabricated and evaluated in the same manner as in Example 14, except that BH-Ref6 was used instead of the compound BH-8 of Example 14 in formation of the emitting layer. The results are shown in Table 6.

### Comparative Example 13

An organic EL device was fabricated and evaluated in the same manner as in Example 15, except that BH-Ref7 was used instead of the compound BH-9 of Example 15 in formation of the emitting layer. The results are shown in Table 6.

### Comparative Example 14

An organic EL device was fabricated and evaluated in the same manner as in Example 16, except that BH-Ref8 was used instead of the compound BH-10 of Example 16 in formation of the emitting layer. The results are shown in Table 6.

**Table 6**

| | BH | BD | Voltage [V] | EQE [%] | LT95 [h] |
|---|---|---|---|---|---|
| Example 12 | BH-8 | BD-1 | 3.8 | 8.2 | 87 |
| Example 13 | BH-8 | BD-2 | 3.7 | 8.5 | 99 |
| Example 14 | BH-8 | BD-3 | 3.8 | 8.3 | 70 |
| Example 15 | BH-9 | BD-1 | 3.8 | 8.2 | 32 |
| Example 16 | BH-10 | BD-1 | 3.7 | 8.6 | 41 |
| Comparative Example 10 | BH-Ref6 | BD-1 | 3.8 | 8.2 | 82 |
| Comparative Example 11 | BH-Ref6 | BD-2 | 3.7 | 8.5 | 93 |
| Comparative Example 12 | BH-Ref6 | BD-3 | 3.8 | 8.2 | 67 |
| Comparative Example 13 | BH-Ref7 | BD-1 | 3.8 | 8.2 | 30 |
| Comparative Example 14 | BH-Ref8 | BD-1 | 3.7 | 8.6 | 39 |

### Example 17

### <Fabrication of organic EL device>

An organic EL device was fabricated as follows.

A 25 mm × 75 mm × 1.1 mm-thick glass substrate with an ITO transparent electrode (anode) (manufactured by GEOMATEC Co., Ltd.) was subjected to ultrasonic cleaning in isopropyl alcohol for 5 minutes, and then subjected to UV-ozone cleaning for 30 minutes. The ITO has the film thickness of 130 nm.

The glass substrate with the transparent electrode after being cleaned was mounted onto a substrate holder in a vacuum vapor deposition apparatus. First, a compound HI-1 was deposited on the surface on the side where the transparent electrode was formed so as to cover the transparent electrode to form a compound HI-1 film having the thickness of 5 nm. The HI-1 film functions as a hole-injecting layer.

Subsequent to the formation of the HI-1 film, a compound HT-1 was deposited thereon to form an HT-1 film having the thickness of 80 nm on the HI-1 film. The HT-1 film functions as a first hole-transporting layer.

Following the formation of the HT-1 film, a compound EBL-1 was deposited thereon to form an EBL-1 film having the thickness of 10 nm on the HT-1 film. The EBL-1 film functions as a second hole-transporting layer.

BH-11 (host material) and BD-1 (dopant material) were co-deposited on the EBL-1 film to be 4% in a proportion (weight ratio) of the compound BD-1 to form an emitting layer having the thickness of 25 nm.

A compound HBL-1 was deposited on the emitting layer to form an electron-transporting layer having the thickness of 10 nm. A compound ET-1 being an electron-injecting material was deposited on the electron-transporting layer to form an electron-injecting layer having the thickness of 15 nm. LiF was deposited on the electron-injecting layer to form a LiF film having the thickness of 1 nm. Metal Al was deposited on the LiF film to form a metal cathode having the thickness of 80 nm.

The device configuration of the organic EL device of Example 1 is schematically shown as follows.
ITO(130)/HI-1(5)/HT-1(80)/EBL-1(10)/BH-11:BD-1(25:4%)/HBL-1(10)/ET-1(15)/LiF(1)/AI(80)

The numerical values in parentheses indicate the film thickness (unit: nm).

The numerical values represented by percent in parentheses indicate a proportion (% by mass) of the latter compound in the layer.

### <Evaluation of organic EL device>

Driving voltage, external quantum efficiency, and device lifetime of the organic EL devices were measured in the same manner as in Example 1. The results are shown in Table 7.

### Example 18

An organic EL device was fabricated and evaluated in the same manner as in Example 17, except that BD-2 was used instead of the compound BD-1 of Example 17 in formation of the emitting layer. The results are shown in Table 7.

### Example 19

An organic EL device was fabricated and evaluated in the same manner as in Example 17, except that BD-3 was used instead of the compound BD-1 of Example 17 in formation of the emitting layer. The results are shown in Table 7.

### Example 20

An organic EL device was fabricated and evaluated in the same manner as in Example 17, except that BH-12 was used instead of the compound BH-11 of Example 17 in formation of the emitting layer. The results are shown in Table 7.

### Comparative Example 15

An organic EL device was fabricated and evaluated in the same manner as in Example 17, except that BH-Ref9 was used instead of the compound BH-11 of Example 17 in formation of the emitting layer. The results are shown in Table 7.

### Comparative Example 16

An organic EL device was fabricated and evaluated in the same manner as in Example 18, except that BH-Ref9 was used instead of the compound BH-11 of Example 18 in formation of the emitting layer. The results are shown in Table 7.

### Comparative Example 17

An organic EL device was fabricated and evaluated in the same manner as in Example 19, except that BH-Ref9 was used instead of the compound BH-11 of Example 19 in formation of the emitting layer. The results are shown in Table 7.

### Comparative Example 4

An organic EL device was fabricated and evaluated in the same manner as in Example 17, except that BH-Ref10 was used instead of the compound BH-11 of Example 17 in formation of the emitting layer. The results are shown in Table 7.

**Table 7**

| | BH | BD | Voltage [V] | EQE [%] | LT95 [h] |
|---|---|---|---|---|---|
| Example 17 | BH-11 | BD-1 | 3.3 | 8.6 | 155 |
| Example 18 | BH-11 | BD-2 | 3.3 | 8.9 | 170 |
| Example 19 | BH-11 | BD-3 | 3.3 | 8.8 | 150 |
| Example 20 | BH-12 | BD-1 | 3.3 | 8.6 | 160 |
| Comparative Example 15 | BH-Ref9 | BD-1 | 3.3 | 8.6 | 105 |
| Comparative Example 16 | BH-Ref9 | BD-2 | 3.3 | 8.9 | 115 |
| Comparative Example 17 | BH-Ref9 | BD-3 | 3.3 | 8.8 | 100 |
| Comparative Example 18 | BH-Ref10 | BD-1 | 3.3 | 8.6 | 85 |

### Example 21

An organic EL device was fabricated and evaluated in the same manner as in Example 17, except that mixed powder which was produced by weighting a compound BH-P1 and a compound BH-13 to satisfy a molar ratio of 35:65 and then mixing them with pulverizing in a mortar was used instead of the compound BH-11 of Example 17 in formation of the emitting layer. The results are shown in Table 8.

**Table 8**

| | BH | BD | Voltage [V] | EQE [%] | LT95 [h] |
|---|---|---|---|---|---|
| Example 21 | BH-P1:BH-13 (35:65) | BD-1 | 3.6 | 9.0 | 80 |

### <Synthesis of compound>

(Synthesis Example 1) The anthracene derivative (compound BH-1) was synthesized through the synthetic route described below.

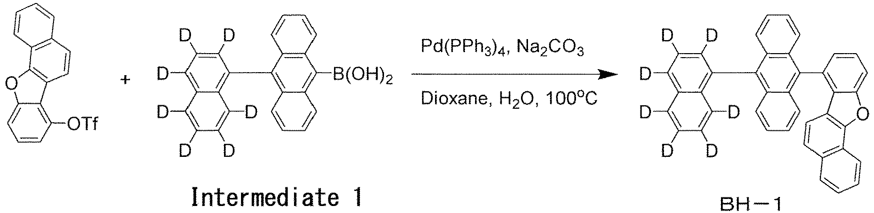

2.06 g of naphtho[2,3-b]benzofuran-1-yl trifluoromethanesulfonate which was synthesized by a known method, 2.00 g of boronic acid (Intermediate 1) which was synthesized by a known method, 0.26 g of tetrakis(triphenylphosphine)palladium (0) (Pd(PPh₃)₄), 1.19 g of sodium carbonate, 42 mL of 1,4-dioxane, and 15 mL of ion-exchanged water were added to a flask under an argon atmosphere, and they were stirred at reflux for 18 hours. It was cooled to room temperature, and then it was filtered to collect precipitated solids. The obtained solids were washed with water and with acetone, and then they were recrystallized using a mixed solvent of toluene and cyclohexane to obtain 2.22 g of pale yellow solid (75% yield). The pale yellow solid was analyzed by using mass spectrum. As a results, it was the compound BH-1, and exhibited m/e = 528 for a molecular weight of 527.67.

(Synthesis Example 2) The anthracene derivative (compound BH-2) was synthesized through the synthetic route described below.

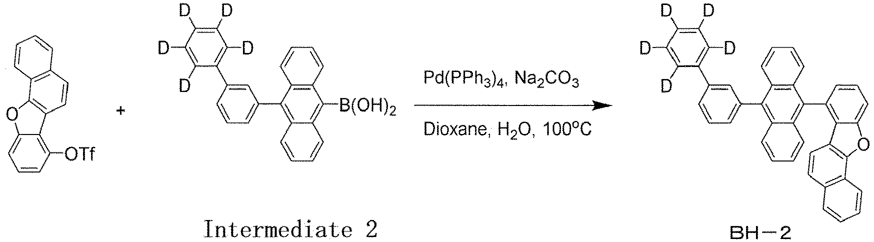

1.93 g of naphtho[1,2-b]benzofuran-7-yl trifluoromethanesulfonate which was synthesized by a known method, 2.00 g of boronic acid (Intermediate 2) which was synthesized by a known method, 0.24 g of tetrakis(triphenylphosphine)palladium (0) (Pd(PPh₃)₄), 1.21 g of sodium carbonate, 40 mL of toluene, and 13 mL of ion-exchanged water were added to a flask under an argon atmosphere, and they were stirred at reflux for 18 hours. It was cooled to room temperature, and then it was filtered to collect precipitated solids. The obtained solids were washed with water and with acetone, and then they were recrystallized using a mixed solvent of toluene and hexane to obtain 2.02 g of pale yellow solid (69% yield). The pale yellow solid was analyzed by using mass spectrum. As a results, it was the compound BH-2, and exhibited m/e = 552 for a molecular weight of 551.70.

(Synthesis Example 3) The anthracene derivative (compound BH-3) was synthesized through the synthetic route described below.

### (1) Synthesis of Intermediate 3

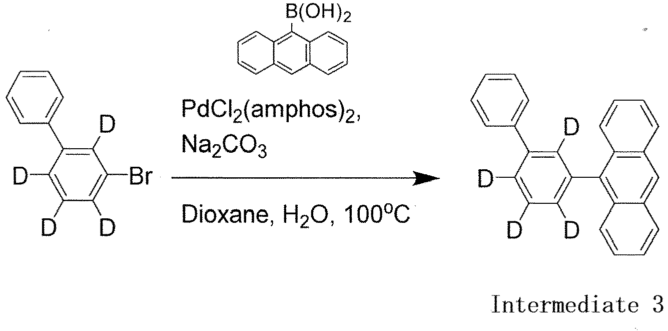

5.01 g of 3-bromo-1,1'-biphenyl-2,4,5,6-d₄ which was synthesized by a known method, 4.80 g of anthracene-9-yl boronic acid, 0.90 g of dichlorobis[di-TERT-butyl(4-dimethyaminophenyl)phosphine]palladium (II), 3.51 g of sodium carbonate, 135 mL of 1,4-dioxane, and 15 mL of ion-exchanged water were added to a flask under an argon atmosphere, and they were heated and stirred at 100°C for 18 hours. It was cooled to room temperature, and then it was filtered to collect precipitated solids. The obtained solids were washed with water and with acetone, and then they were recrystallized using a mixed solvent of toluene and cyclohexane to obtain 5.03 g of pale yellow solid (71% yield). The pale yellow solid was analyzed by using mass spectrum. As a results, it was the Intermediate 3, and exhibited m/e = 334 for a molecular weight of 334.45.

### (2) Synthesis of Intermediate 4

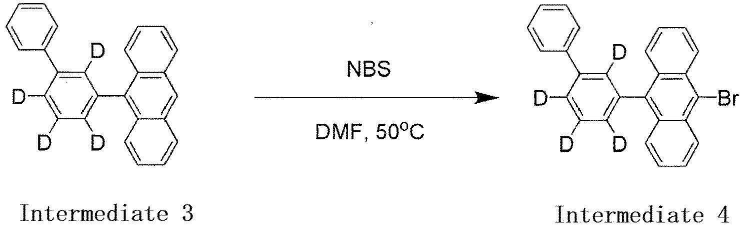

5.40 g of Intermediate 3, 3.10 g of N-bromosuccinimide, and 100 mL of N,N-dimethylformamide were added to a flask under an argon atmosphere, and they were heated and stirred at 50°C for 10 hours. It was cooled to room temperature, and then it was filtered to collect precipitated solids. The obtained solids were washed with water and with methanol, and then they were recrystallized using a mixed solvent of toluene and cyclohexane to obtain 4.38 g of pale yellow solid (66% yield). The pale yellow solid was analyzed by using mass spectrum. As a results, it was the Intermediate 4, and exhibited m/e = 413 for a molecular weight of 413.35.

### (3) Synthesis of Intermediate 5

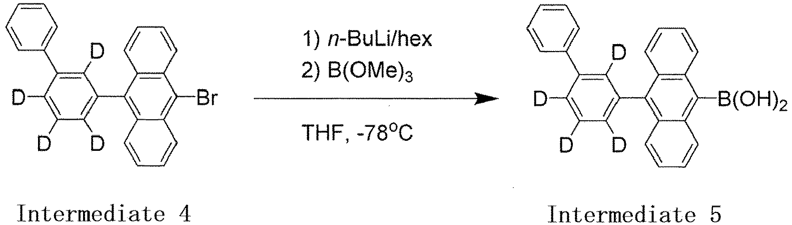

3.00 g of Intermediate 4 and 30 mL of tetrahydrofuran were added to a flask under an argon atmosphere, and they were stirred at -78°C for an hour. 5.80 mL of a hexane solution of n-butyl lithium (1.55 moL/L) was added dropwise thereto, and then it was stirred at -78°C for an hour. 2.51 mL of trimethoxyborane was added to the reaction solution, and then it was stirred for two hours while increasing the temperature thereof with going the flow. After being stirred, 40 mL of 1 N of aqueous hydrochloric acid solution was added thereto, and an organic phase was extracted by using a separating funnel. The organic phase was dried with magnesium sulfate, and it was dried up by using an evaporator. The obtained crude product was recrystallized using a mixed solvent of tetrahydrofuran and hexane to obtain 1.80 g of pale yellow solid (66% yield). The pale yellow solid was analyzed by using mass spectrum. As a results, it was the Intermediate 5, and exhibited m/e = 379 for a molecular weight of 378.27.

### (4) Synthesis of anthracene derivative (compound BH-3)

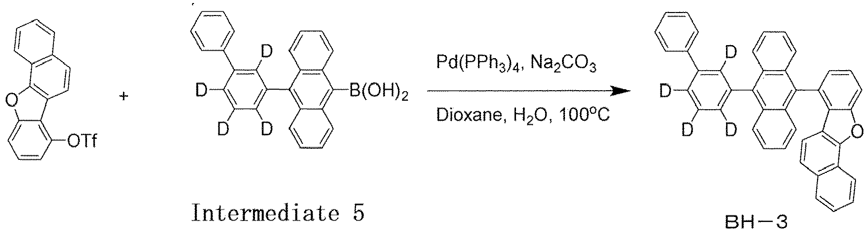

1.94 g of naphtho[1,2-b]benzofuran-7-yl trifluoromethanesulfonate which was synthesized by a known method, 2.00 g of Intermediate 5, 0.24 g of tetrakis(triphenylphosphine)palladium (0) (Pd(PPh₃)₄), 1.21 g of sodium carbonate, 40 mL of toluene, and 13 mL of ion-exchanged water were added to a flask under an argon atmosphere, and they were stirred at reflux for 18 hours. It was cooled to room temperature, and then it was filtered to collect precipitated solids. The obtained solids were washed with water and with acetone, and then they were recrystallized using a mixed solvent of toluene and hexane to obtain 2.22 g of pale yellow solid (76% yield). The pale yellow solid was analyzed by using mass spectrum. As a results, it was the compound BH-3, and exhibited m/e = 551 for a molecular weight of 550.69.

(Synthesis Example 4) The anthracene derivative (compound BH-4) was synthesized through the synthetic route described below.

### (1) Synthesis of Intermediate 6

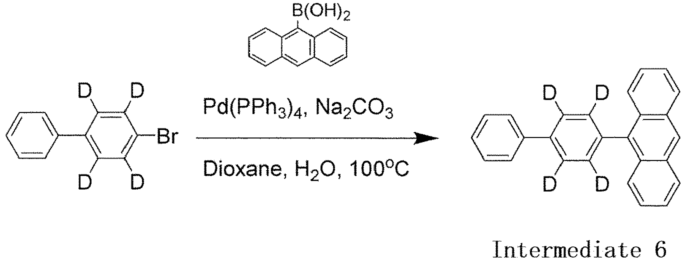

A pale yellow solid was obtained in the same manner as in the Synthesis of Intermediate 3, except that 4-bromo-1,1'-biphenyl-2,3,5,6-d₄ which was synthesized by a known method was used instead of 3-bromo-1,1'-biphenyl-2,4,5,6-d₄. The pale yellow solid was analyzed by using mass spectrum. As a results, it was the Intermediate 6, and exhibited m/e = 334 for a molecular weight of 334.45.

### (2) Synthesis of Intermediate 7

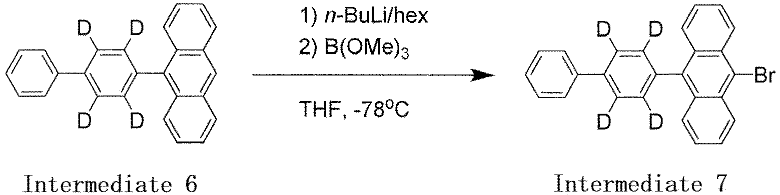

A compound was synthesized to obtain a pale yellow solid in the same manner as in the Synthesis of Intermediate 4, except that the Intermediate 6 was used instead of the Intermediate 3. The pale yellow solid was analyzed by using mass spectrum. As a results, it was the Intermediate 7, and exhibited m/e = 413 for a molecular weight of 413.35.

### (3) Synthesis of Intermediate 9

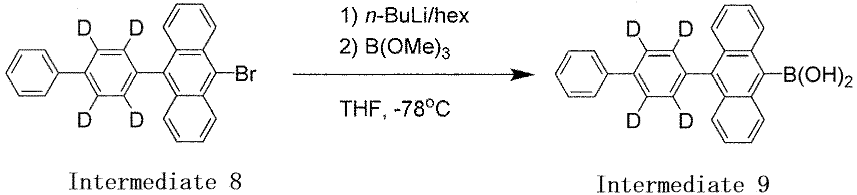

A compound was synthesized to obtain a pale yellow solid in the same manner as in the Synthesis of Intermediate 5, except that the Intermediate 8 was used instead of the Intermediate 4. The pale yellow solid was analyzed by using mass spectrum. As a results, it was the Intermediate 9, and exhibited m/e = 378 for a molecular weight of 378.27.

### (4) Synthesis of anthracene derivative (BH-4)

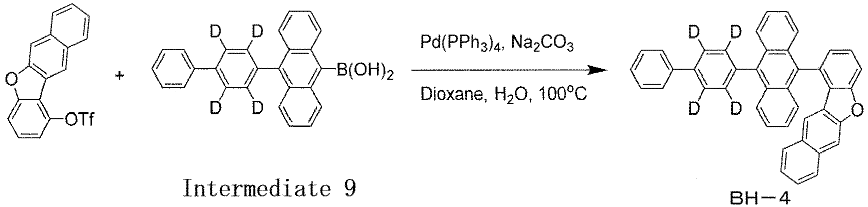

A compound was synthesized to obtain a pale yellow solid in the same manner as in the Synthesis of BH-3, except that the Intermediate 9 was used instead of the Intermediate 5. The pale yellow solid was analyzed by using mass spectrum. As a results, it was the compound BH-4, and exhibited m/e = 552 for a molecular weight of 551.70.

(Synthesis Example 5) The anthracene derivative (compound BH-5) was synthesized through the synthetic route described below.

### (1) Synthesis of Intermediate 10

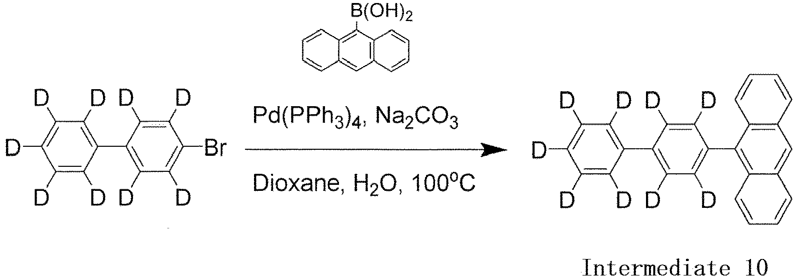

A compound was synthesized to obtain a pale yellow solid in the same manner as in the Synthesis of Intermediate 3, except that 4-bromo-1,1'-biphenyl-d₉ which was synthesized by a known method was used instead of 3-bromo-1,1'-biphenyl-2,4,5,6-d₄. The pale yellow solid was analyzed by using mass spectrum. As a results, it was the Intermediate 10, and exhibited m/e = 339 for a molecular weight of 339.49.

### (2) Synthesis of Intermediate 11

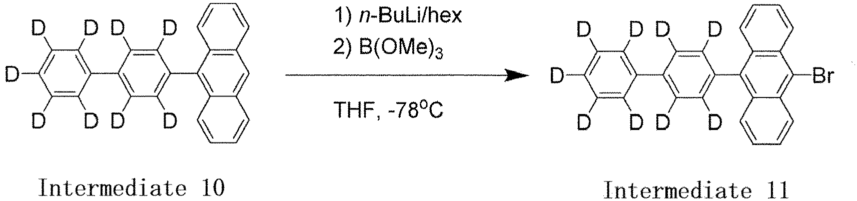

A compound was synthesized to obtain a pale yellow solid in the same manner as in the Synthesis of Intermediate 4, except that the Intermediate 10 was used instead of the Intermediate 3. The pale yellow solid was analyzed by using mass spectrum. As a results, it was the Intermediate 11, and exhibited m/e = 418 for a molecular weight of 418.38.

### (3) Synthesis of Intermediate 12

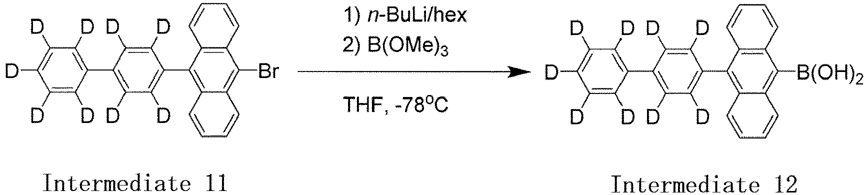

A compound was synthesized to obtain a pale yellow solid in the same manner as in the Synthesis of Intermediate 5, except that the Intermediate 11 was used instead of the Intermediate 4. The pale yellow solid was analyzed by using mass spectrum. As a results, it was the Intermediate 12, and exhibited m/e = 383 for a molecular weight of 383.30.

### (4) Synthesis of anthracene derivative (compound BH-5)

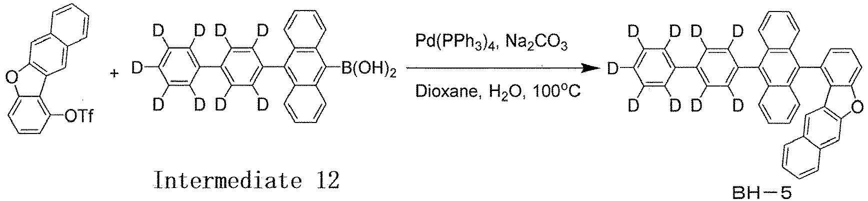

A compound was synthesized to obtain a pale yellow solid in the same manner as in the Synthesis of BH-3, except that the Intermediate 12 was used instead of the Intermediate 5. The pale yellow solid was analyzed by using mass spectrum. As a results, it was the compound BH-5, and exhibited m/e = 556 for a molecular weight of 555.72.

(Synthesis Example 6) The anthracene derivative (compound BH-14) was synthesized through the synthetic route described below.

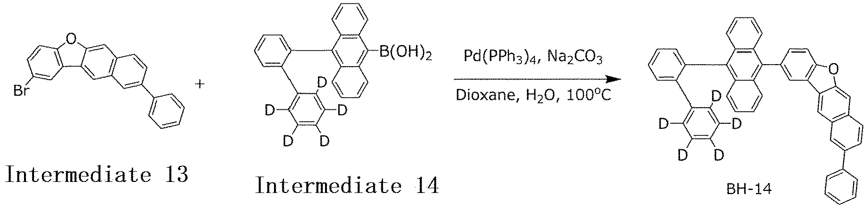

A compound was synthesized to obtain a pale yellow solid in the same manner as in the Synthesis of BH-3, except that the Intermediate 13 and the Intermediate 14 were used instead of naphtho[1,2-b]benzofuran-7-yl trifluoromethanesulfonate and the Intermediate 5. The pale yellow solid was analyzed by using mass spectrum. As a results, it was the compound BH-14, and exhibited m/e = 628 for a molecular weight of 627.80.

### (Synthesis Example 7) Synthesis (A) of BH-15

The compound BH-15 was synthesized through the synthetic route described below.

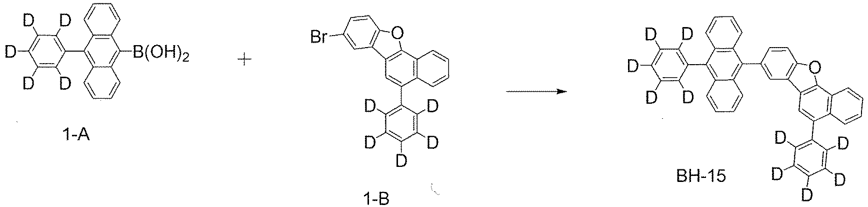

1-A: 0.61 g (2.0 mmol), 1-B: 0.76 g (2.0 mmol), tetrakis(triphenylphosphine)palladium (0): 0.05 g (0.04 mmol), 2 M of aqueous solution of sodium carbonate: 3 ml (6 mmol), 1,4-dioxane: 20 ml were added to a flask under an argon atmosphere, and they were heated and stirred at 85°C for 8 hours. The reaction solution was cooled to room temperature (25°C), and then a solid precipitated by adding 300 ml of water was collected by filtration.

Subsequently, the obtained solid was purified by silica gel column chromatography to obtain 0.5 g of pale yellow solid (45% yield). The pale yellow solid was identified as BH-15 by using LC-MS analysis.

### (Synthesis Example 8) Synthesis (B) of BH-15

The compound BH-15 was synthesized through the synthetic route described below.

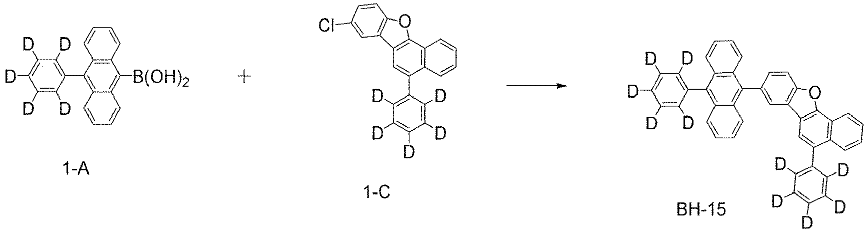

1-A: 0.61 g (2.0 mmol), 1-C: 0.67 g (2.0 mmol), dichlorobisamphospalladium (II): 0.03 g (0.04 mmol), 2 M of aqueous solution of sodium carbonate: 3 ml (6 mmol), 1,4-dioxane: 20 ml were added to a flask under an argon atmosphere, and they were heated and stirred at 85°C for 8 hours. The reaction solution was cooled to room temperature (25°C), and then a solid precipitated by adding 300 ml of water was collected by filtration.

Subsequently, the obtained solid was purified by silica gel column chromatography to obtain 0.25 g of pale yellow solid (22% yield). The pale yellow solid was identified as BH-15 by using LC-MS analysis.

### (Synthesis Example 9) Synthesis (A) of BH-16

The compound BH-16 was synthesized through the synthetic route described below.

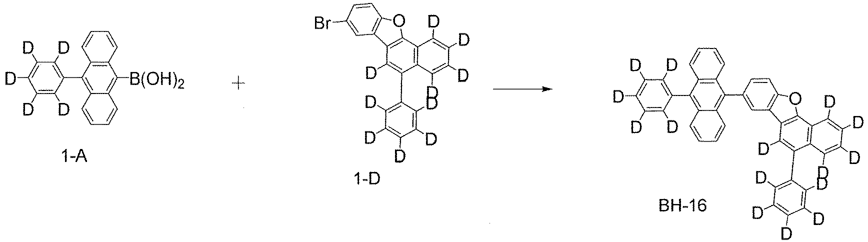

A compound was synthesized to obtain 0.92 g of pale yellow solid (69% yield) in the same manner as in the Synthesis (A) of BH-15, except that 1-D was used instead of 1-B.

The pale yellow solid was identified as BH-16 by using LC-MS analysis.

### (Synthesis Example 10) Synthesis (B) of BH-16

The compound BH-16 was synthesized through the synthetic route described below.

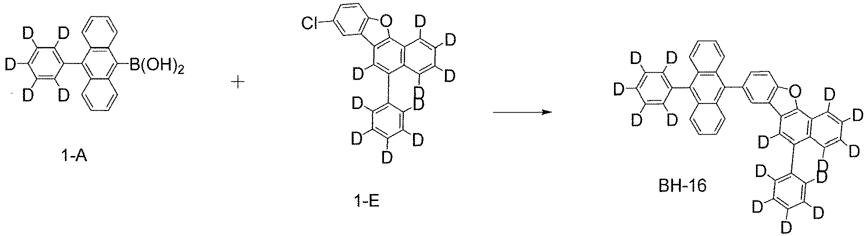

A compound was synthesized to obtain 0.33 g of pale yellow solid (39% yield) in the same manner as in the Synthesis (B) of BH-15, except that 1-E was used instead of 1-C.

The pale yellow solid was identified as BH-16 by using LC-MS analysis.

### (Synthesis Example 11) Synthesis of BH-17

The compound BH-17 was synthesized through the synthetic route described below.

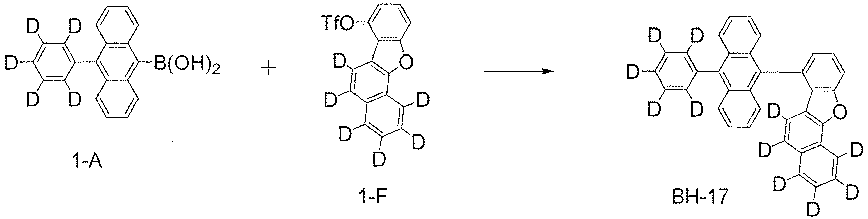

A compound was synthesized to obtain 1.4 g of pale yellow solid (72% yield) in the same manner as in the Synthesis (A) of BH-15, except that 1-F was used instead of 1-B.

The pale yellow solid was identified as BH-17 by using LC-MS analysis.

### (Synthesis Example 12) Synthesis (A) of BH-12

The compound BH-12 was synthesized through the synthetic route described below.

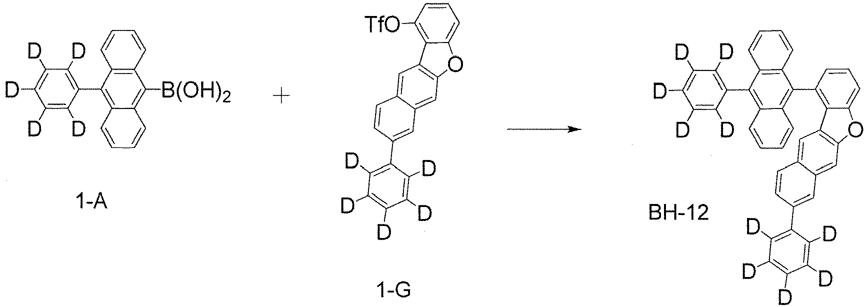

A compound was synthesized to obtain 0.3 g of pale yellow solid (12% yield) in the same manner as in the Synthesis (A) of BH-15, except that 1-G was used instead of 1-B.

The pale yellow solid was identified as BH-12 by using LC-MS analysis.

### (Synthesis Example 13) Synthesis (B) of BH-12

The compound BH-12 was synthesized through the synthetic route described below.

### (1) Synthesis of 4-C

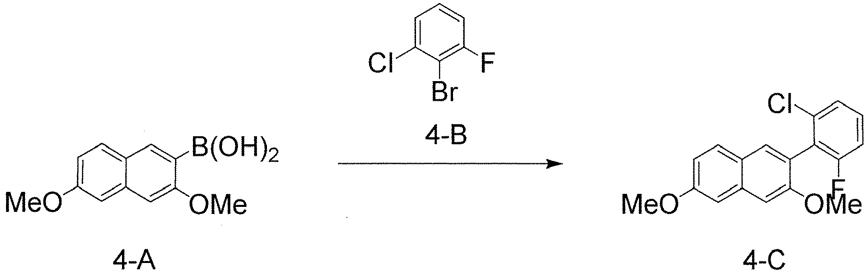

A compound was synthesized to obtain 12.3 g of white solid (77% yield) in the same manner as in the Synthesis (A) of BH-15, except that 4-A and 4-B were used instead of 1-A and 1-B.

### (2) Synthesis of 4-D

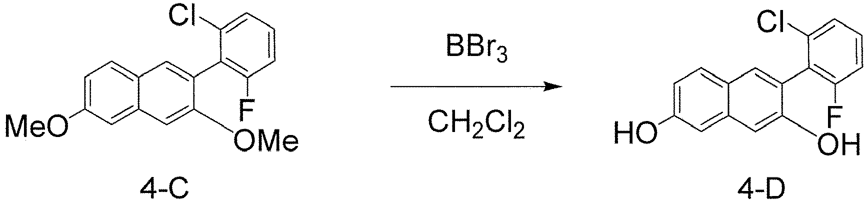

A compound was synthesized to obtain 9.2 g of white solid (93% yield) in the same manner as in the Synthesis of 3-F, except that 4-C was used instead of 3-E.

### (3) Synthesis of 4-E

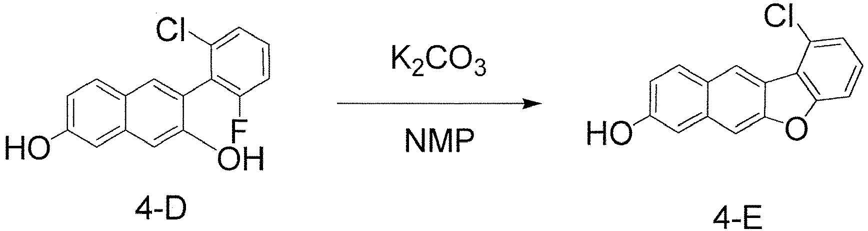

A compound was synthesized to obtain 6.8 g of white solid (72% yield) in the same manner as in the Synthesis of 3-G, except that 4-D was used instead of 3-F.

### (4) Synthesis of 4-F

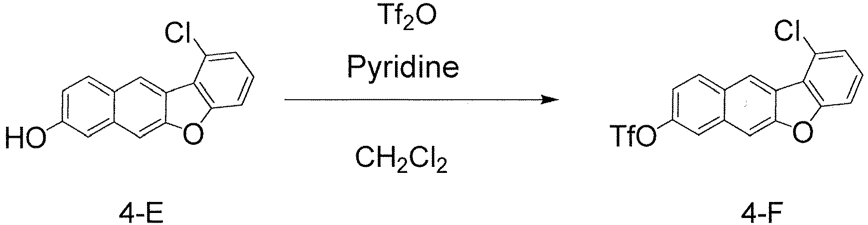

4-E: 4.9 g (18.25 mmol), dichloromethane: 91 ml were added to a flask under an argon atmosphere, and the solution was cooled to 0°C. Subsequently, pyridine: 1.7 ml (21.9 mmol) was added dropwise into the system, and then trifluoromethanesulfonic acid anhydride: 3.59 ml (21.9 mmol) was added dropwise into the system, followed by stirring for an hour as it was.

It was further stirred for 6 hours while being cooled to room temperature (25°C), subsequently, ice water was added to the reaction solution. It was steadily stirred, and then an aqueous phase was removed. A remaining organic phase was concentrated, and the residue was purified by silica gel column chromatography to obtain 6.2 g of white solid (87% yield).

### (5) Synthesis of 4-H

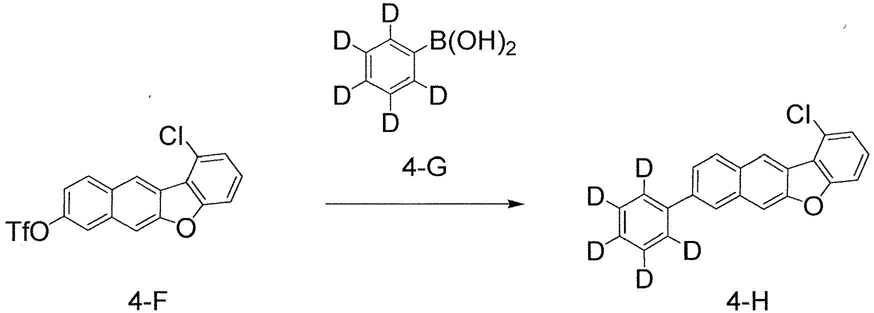

A compound was synthesized to obtain 2.3 g of white solid (68% yield) in the same manner as in the Synthesis (A) of BH-15, except that 4-F and 4-G were used instead of 1-A and 1-B.

### (6) Synthesis of BH-12

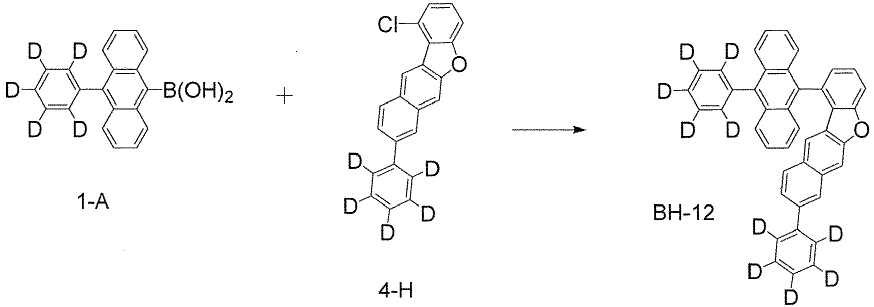

A compound was synthesized to obtain 1.2 g of pale yellow solid (23% yield) in the same manner as in the Synthesis (B) of BH-15, except that 4-H was used instead of 1-C.

The pale yellow solid was identified as BH-12 by using LC-MS analysis.

### (Synthesis Example 14) Synthesis of BH-18

The compound BH-18 was synthesized through the synthetic route described below.

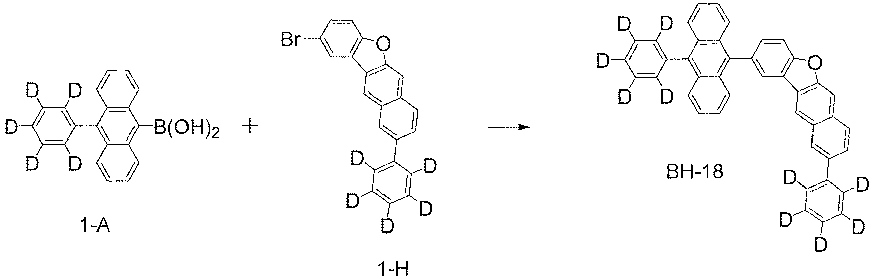

A compound was synthesized to obtain 1.6 g of pale yellow solid (45% yield) in the same manner as in the Synthesis (A) of BH-15, except that 1-H was used instead of 1-B.

The pale yellow solid was identified as BH-18 by using LC-MS analysis.

### (Synthesis Example 15) Synthesis of BH-Ref10

The compound BH-Ref10 was synthesized through the synthetic route described below.

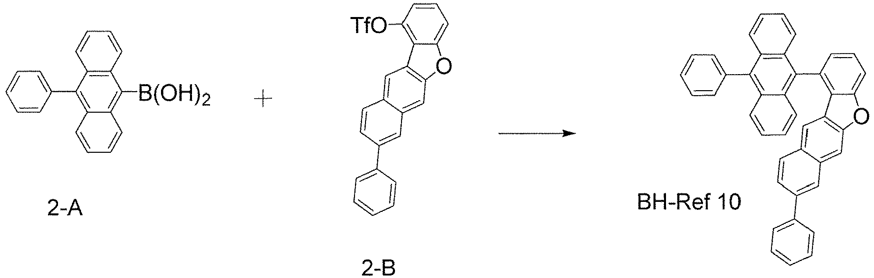

A compound was synthesized to obtain 1.6 g of pale yellow solid (45% yield) in the same manner as in the Synthesis (A) of BH-15, except that 2-A and 2-B were used instead of 1-A and 1-B.

The pale yellow solid was identified as BH-Ref10 by using LC-MS analysis.

### (Synthesis Example 16) Synthesis of BH-19

The compound BH-19 was synthesized through the synthetic route described below.

### (1) Synthesis of 3-B

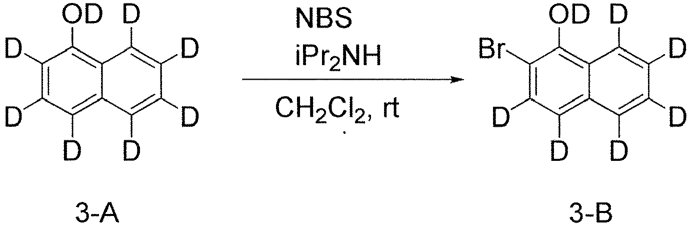

3-A: 10.1 g (70.0 mmol), diisopropylamine: 0.71 g (7.0 mmol), N-bromosuccinimide: 13.7 g (77.0 mmol), dichloromethane: 200 ml were added to a flask under an argon atmosphere, and they were heated and stirred at room temperature for 6 hours. Thereafter, 300 ml of water was added to the reaction solution, and then an organic phase was extracted. Subsequently, the obtained organic phase was purified by silica gel column chromatography to obtain 6.88 g of white solid (43% yield). The white solid was identified as 3-B by using LC-MS analysis.

### (2) Synthesis of 3-C

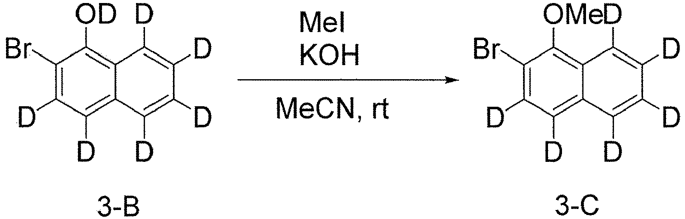

3-B: 1.14 g (4.98 mmol), iodomethane: 0.30 mL (4.80 mmol), potassium carbonate: 0.70 g (5.06 mmol), acetonitrile: 40 ml were added to a flask under an argon atmosphere, and they were heated and stirred at room temperature for 6 hours. Thereafter, 300 ml of water was added thereto, and then an organic phase was extracted. Subsequently, the obtained solid was purified by silica gel column chromatography to obtain 5.06 g of white solid (88% yield). The white solid was identified as 3-C by using LC-MS analysis.

### (3) Synthesis of 3-D

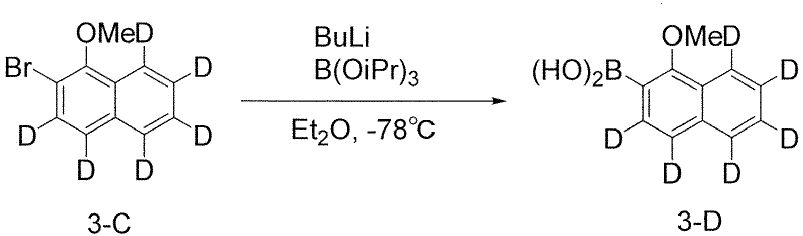

3-C: 7.74 g (31.8 mmol) and diethylether: 130 ml were added to a flask under an argon atmosphere, and they were cooled to -78°C. Subsequently, normalbutyl lithium: 30.8 mL (47.8 mmol, 1.55 M) was added dropwise thereto, and then they were stirred for 30 minutes while maintaining them at -78°C. Thereafter, trimethoxyborane: 7.08 mL (63.7 mmol) was added dropwise thereto, and then it was stirred for 6 hours while slowly increasing the temperature thereof to room temperature. Thereafter, 150 ml of water was added thereto, and then an organic phase was extracted. Subsequently, the obtained solid was purified by silica gel column chromatography to obtain 5.82 g of white solid (62% yield). The white solid was identified as 3-D by using LC-MS analysis.

### (4) Synthesis of 3-E

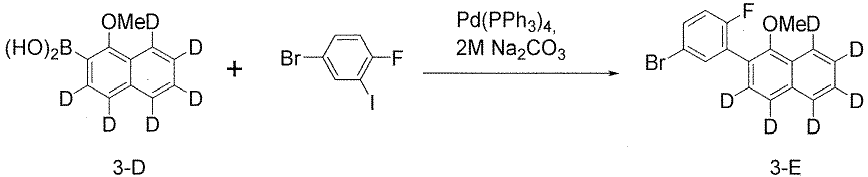

A compound was synthesized to obtain 4.46 g of white solid (71% yield) in the same manner as in the Synthesis (A) of BH-15, except that 3-D and 4-bromo-1-fluoromethane-2-iodobenzene were used instead of 1-A and 1-B. The white solid was identified as 3-E by using LC-MS analysis.

### (5) Synthesis of 3-F

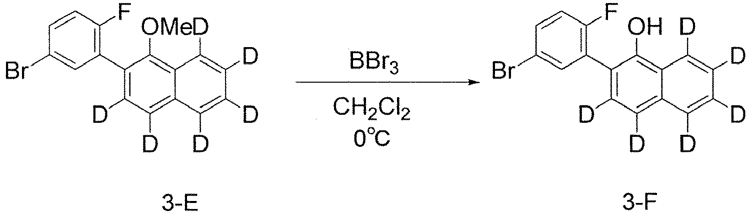

3-E: 4.05 g (12.0 mmol), dichloromethane: 40 ml were added to a flask under an argon atmosphere, and the solution was cooled to 0°C. Subsequently, 1 M of a dichloromethane solution of boron tribromide: 48 ml (48 mmol) was added dropwise into the system, followed by stirring for an hour as it was.

It was further stirred for 6 hours while being cooled to room temperature (25°C), subsequently, ice water was added to the reaction solution. It was steadily stirred, and then an aqueous phase was removed. A remaining organic phase was concentrated, and the residue was purified by silica gel column chromatography to obtain 3.49 g of white solid (90% yield). The white solid was identified as 3-F by using LC-MS analysis.

### (6) Synthesis of 3-G

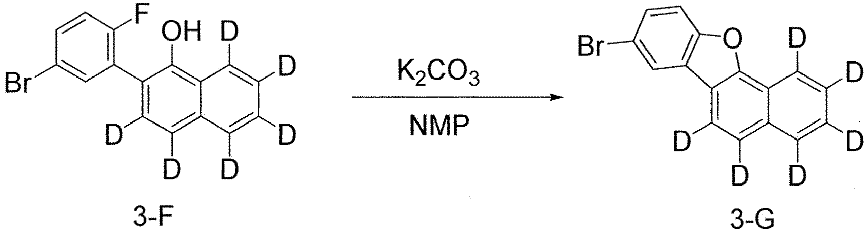

3-F: 4.72 g (14.6 mmol), N-methylpyrrolidone: 80 ml, and potassium carbonate: 2.62 g (19.0 mmol) were added to a flask under an argon atmosphere, and they were stirred at 150°C for 6 hours. Subsequently, it was naturally cooled, 50 ml of water and 150 mL of dichloromethane was added thereto, and then an organic phase was extracted. Subsequently, the obtained solid was purified by silica gel column chromatography to obtain 2.74 g of white solid (62% yield). The white solid was identified as 3-G by using LC-MS analysis.

### (7) Synthesis of BH-19

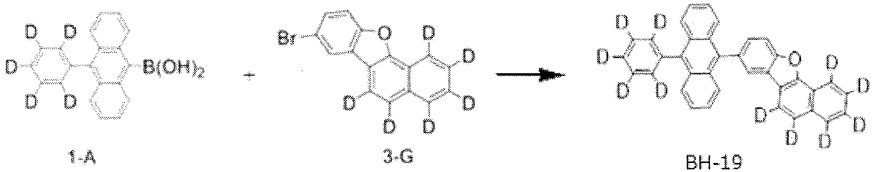

1-A: 0.61 g (2.0 mmol), 3-G: 0.76 g (2.0 mmol), tetrakis(triphenylphosphine)palladium (0): 0.05 g (0.04 mmol), 2 M of aqueous solution of sodium carbonate: 3 ml (6 mmol), 1,4-dioxane: 20 ml were added to a flask under an argon atmosphere, and they were heated and stirred at 85°C for 8 hours. The reaction solution was cooled to room temperature (25°C), and then a solid precipitated by adding 300 ml of water was collected by filtration.

Subsequently, the obtained solid was purified by silica gel column chromatography to obtain 0.5 g of pale yellow solid (45% yield). The pale yellow solid was identified as BH-19 by using LC-MS analysis.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.

The documents described in the specification and the specification of Japanese application(s) on the basis of which the present application claims Paris convention priority are incorporated herein by reference in its entirety.

## Claims

1. A compound represented by the following formula (1): 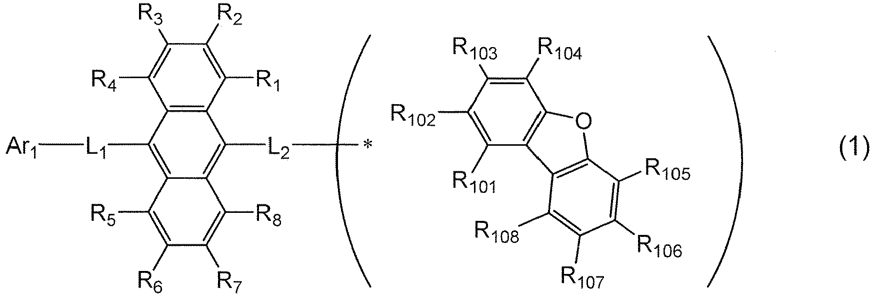 wherein in the formula (1),
R₁ to R₈ are independently a hydrogen atom, or a substituent R;
L₁ and L₂ are independently
a single bond,
a substituted or unsubstituted phenylene group,
a substituted or unsubstituted naphthylene group, or
a divalent group in which these two groups are combined;
Ar1 is
a substituted or unsubstituted phenyl group,
a substituted or unsubstituted p-biphenyl group,
a substituted or unsubstituted m-biphenyl group,
a substituted or unsubstituted o-biphenyl group,
a substituted or unsubstituted 1-naphthyl group,
a substituted or unsubstituted 2-naphthyl group, or
a substituted or unsubstituted phenanthryl group;
the adjacent two of R₁₀₁ to R₁₀₈ form a substituted or unsubstituted benzene ring by bonding with each other, or do not bond with each other;
one of, R₁₀₁ to R₁₀₈ which do not form the substituted or unsubstituted benzene ring, and carbon atoms in the substituted or unsubstituted benzene ring in the case where the adjacent two of R₁₀₁ to R₁₀₈ form a substituted or unsubstituted benzene ring by bonding with each other is bonded with L₂ via a single bond;
R₁₀₁ to R₁₀₈ which do not form the substituted or unsubstituted benzene ring and which are not bonded with L₂ are independently a hydrogen atom, or a substituent R;
the substituent R is selected from the group consisting of
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
-Si(R₉₀₁)(R₉₀₂)(R₉₀₃),
-O-(R₉₀₄),
-S-(R₉₀₅),
-N(R₉₀₆)(R₉₀₇)
(wherein R₉₀₁ to R₉₀₇ are independently
a hydrogen atom,
a substituted or unsubstituted alkyl group having 1 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
a halogen atom, a cyano group, a nitro group,
a substituted or unsubstituted aryl group having 6 to 50 ring carbon atoms, and
a substituted or unsubstituted monovalent heterocyclic group having 5 to 50 ring atoms; provided that the compound represented by the formula (1) satisfies all of the following conditions 1 to 3;
condition 1: R₁ to R₈ which are hydrogen atoms are not a deuterium atom;
condition 2: one or more of, hydrogen atoms possessed by L₁, L₂ and Ar1, hydrogen atoms possessed by the substituent in the case where L₁, L₂ and Ar1 are substituted, hydrogen atoms possessed by R₁ to R₈ which are the substituents R, R₁₀₁ to R₁₀₈ which are hydrogen atoms, hydrogen atoms possessed by R₁₀₁ to R₁₀₈ which are the substituents R, and hydrogen atoms possessed by the substituted or unsubstituted benzene ring in the case where the adjacent two of R₁₀₁ to R₁₀₈ form a substituted or unsubstituted benzene ring by bonding with each other are a deuterium atom; and
condition 3: when L₁ is a single bond, Ar1 is not a phenyl group.

2. The compound according to claim 1, wherein R₁₀₁ is bonded with L₂ via a single bond.

3. The compound according to claim 1 or 2, wherein the compound represented by the formula (1) is a compound represented by the following formula (1-1): 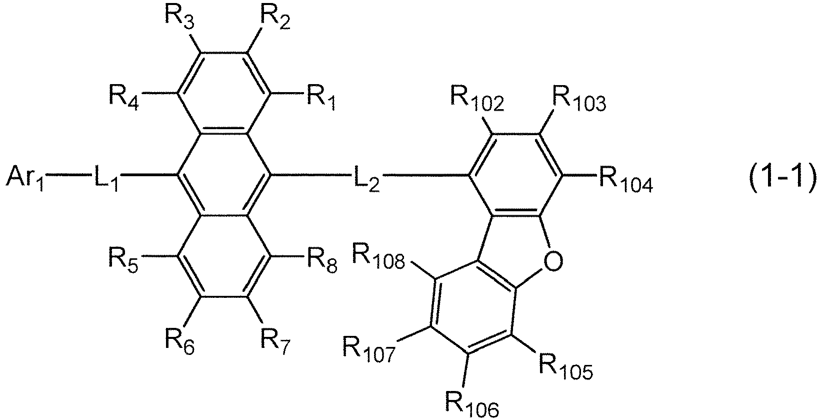 wherein in the formula (1-1), R₁ to R₈, R₁₀₂ to R₁₀₈, L₁, L₂ and Ar1 are the same as defined in the formula (1).

4. The compound according to any one of claims 1 to 3, wherein the adjacent two of R₁₀₅ to R₁₀₈ form a substituted or unsubstituted benzene ring by bonding with each other.

5. The compound according to any one of claims 1 to 4, wherein the compound represented by the formula (1) is a compound represented by any one of the following formulas (1-11) to (1-13): 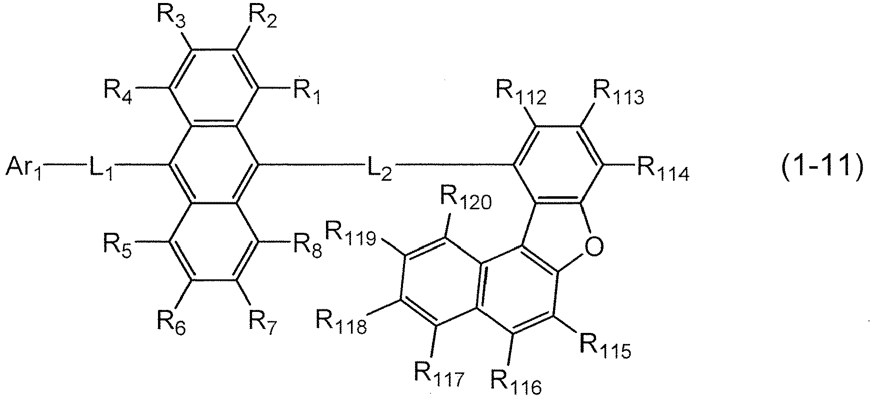 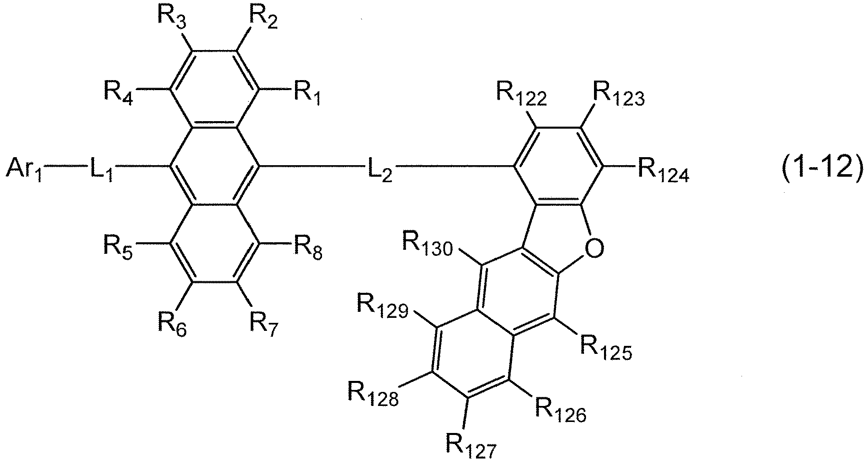 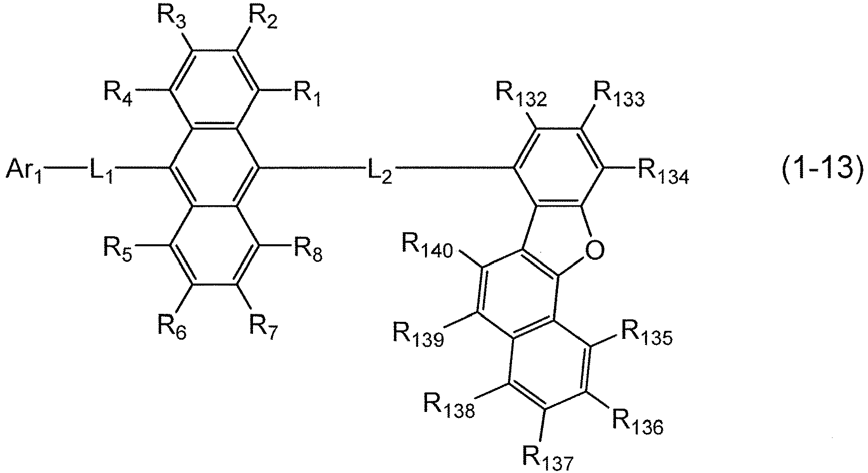 wherein in the formulas (1-11) to (1-13), R₁ to R₈, L₁, L₂ and Ar1 are the same as defined in the formula (1);
in the formula (1-11), R₁₁₂ to R₁₂₀ are independently a hydrogen atom, or a substituent R;
in the formula (1-12), R₁₂₂ to R₁₃₀ are independently a hydrogen atom, or a substituent R;
in the formula (1-13), R₁₃₂ to R₁₄₀ are independently a hydrogen atom, or a substituent R; and the substituent R is the same as defined in the formula (1).

6. The compound according to any one of claims 1 to 5, wherein L₁ is
a substituted or unsubstituted phenylene group, or
a substituted or unsubstituted naphthylene group; and
one or more of hydrogen atoms possessed by L₁ are a deuterium atom.

7. The compound according to claim 5 or 6, wherein the compounds represented by the formulas (1-11) to (1-13) are a compound represented by any one of the following formulas (1-21) to (1-23): 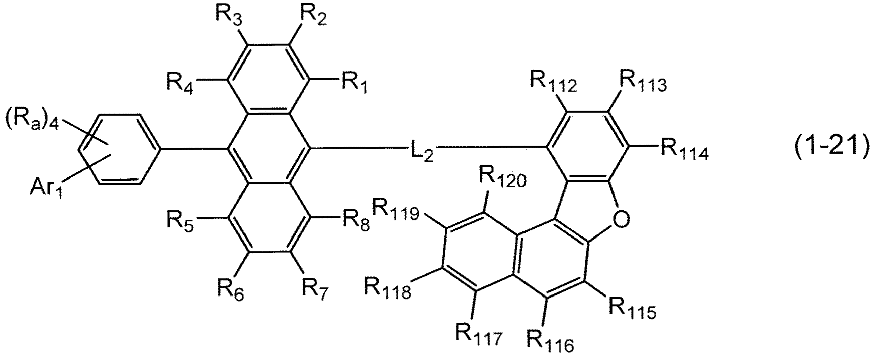 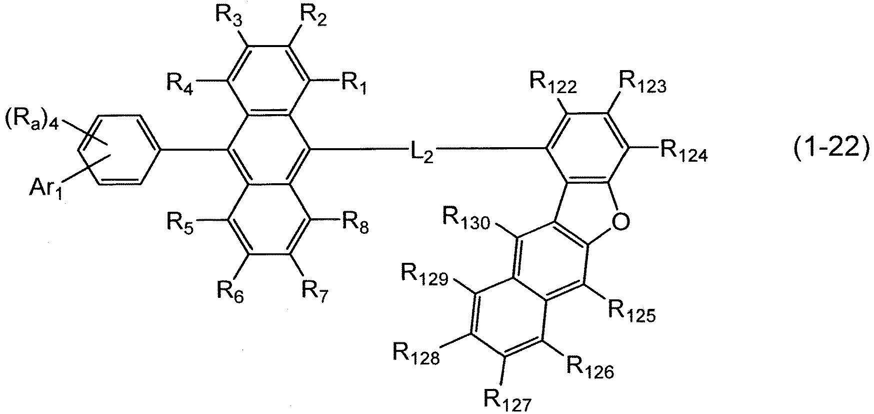 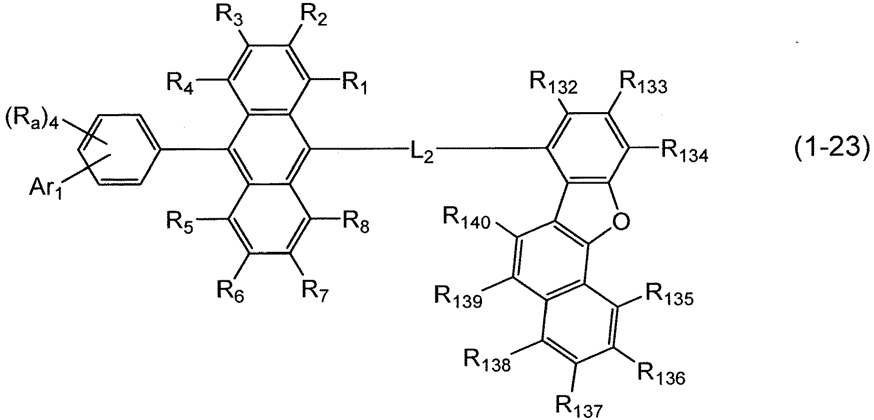 wherein in the formulas (1-21) to (1-23), R₁ to R₈, R₁₁₂ to R₁₂₀, R₁₂₂ to R₁₃₀, R₁₃₂ to R₁₄₀, L₂ and Ar1 are the same as defined in the formulas (1-11) to (1-13);
four Rₐ's are independently a hydrogen atom, or a substituent R;
provided that one or more of the four Rₐ's are a deuterium atom; and
the substituent R is the same as defined in the formula (1).

8. The compound according to any one of claims 1 to 7, wherein hydrogen atoms possessed by Ar1 are not a deuterium atom.

9. The compound according to any one of claims 1 to 8, wherein a substituent in the case of "substituted or unsubstituted" is selected from the group consisting of
an alkyl group having 1 to 50 carbon atoms,
a haloalkyl group having 1 to 50 carbon atoms,
an alkenyl group having 2 to 50 carbon atoms,
an alkynyl group having 2 to 50 carbon atoms,
a cycloalkyl group having 3 to 50 ring carbon atoms,
an alkoxy group having 1 to 50 carbon atoms,
an alkylthio group having 1 to 50 carbon atoms,
an aryloxy group having 6 to 50 ring carbon atoms,
an arylthio group having 6 to 50 ring carbon atoms,
an aralkyl group having 7 to 50 carbon atoms,
-Si(R₄₁)(R₄₂)(R₄₃),
-C(=O)R₄₄, -COOR₄₅,
-S(=O)₂R₄₆,
-P(=O)(R₄₇)(R₄₈),
-Ge(R₄₉)(R₅₀)(R₅₁),
-N(R₅₂)(R₅₃) (wherein, R₄₁ to R₅₃ are independently a hydrogen atom, an alkyl group having 1 to 50 carbon atoms, an aryl group having 6 to 50 ring carbon atoms, or a monovalent heterocyclic group having 5 to 50 ring atoms; when two or more of each of R₄₁ to R₅₃ are present, the two or more of each of R₄₁ to R₅₃ may be the same as or different from each other),
a hydroxy group,
a halogen atom,
a cyano group,
a nitro group,
an aryl group having 6 to 50 ring carbon atoms, and
a monovalent heterocyclic group having 5 to 50 ring atoms.

10. The compound according to any one of claims 1 to 9, wherein a substituent in the case of "substituted or unsubstituted" is selected from the group consisting of
an alkyl group having 1 to 18 carbon atoms,
an aryl group having 6 to 18 ring carbon atoms, and
a monovalent heterocyclic group having 5 to 18 ring atoms.

11. An organic electroluminescence device comprising
a cathode;
an anode; and
one or more organic layers arranged between the cathode and the anode,
wherein at least one layer of the one or more organic layers comprises the compound according to any one of claims 1 to 10.

12. An organic electroluminescence device comprising
a cathode;
an anode; and
one or more emitting layers arranged between the cathode and the anode,
wherein at least one layer of the one or more emitting layers comprises the compound according to any one of claims 1 to 10.

13. The organic electroluminescence device according to claim 11 or 12, wherein a hole-transporting layer is provided between the anode and the emitting layer.

14. The organic electroluminescence device according to any one of claims 11 to 13, wherein an electron-transporting layer is provided between the cathode and the emitting layer.

15. The organic electroluminescence device according to any one of claims 11 to 14, wherein the at least one layer of the one or more emitting layers further comprises one or more fused aromatic compounds selected from the group consisting of a carbazole derivative, an anthracene derivative, a phenanthrene derivative, a pyrene derivative, a naphthacene derivative, a fluoranthene derivative, a triphenylene derivative, a fluorene derivative, and a chrysene derivative.

16. The organic electroluminescence device according to any one of claims 11 to 15, wherein the at least one layer of the one or more emitting layers further comprises a host compound having delayed fluorescence.

17. An organic electroluminescence device comprising
a cathode;
an anode; and
one or more emitting layers arranged between the cathode and the anode,
wherein at least one layer of the one or more emitting layers comprises
a compound represented by the following formula (1A), and
a compound represented by the following formula (41): 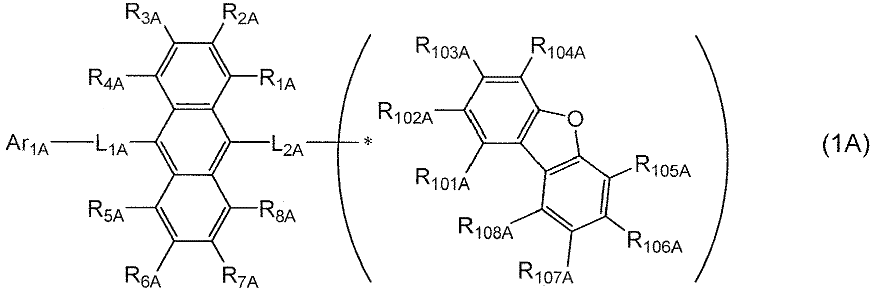 wherein in the formula (1A),
R_{1A} to R_{8A} are independently a hydrogen atom, or a substituent R;
L_{1A} and L_{2A} are independently
a single bond,
a substituted or unsubstituted arylene group having 6 to 18 ring carbon atoms, or
a substituted or unsubstituted divalent heterocyclic group having 5 to 18 ring atoms;
Ar_{1A} is
a substituted or unsubstituted phenyl group,
a substituted or unsubstituted p-biphenyl group,
a substituted or unsubstituted m-biphenyl group,
a substituted or unsubstituted o-biphenyl group,
a substituted or unsubstituted 1-naphthyl group,
a substituted or unsubstituted 2-naphthyl group, or
a substituted or unsubstituted phenanthryl group;
one of R_{101A} to R_{104A} is bonded with L_{2A} via a single bond;
R_{101A} to R_{104A} which are not bonded with L_{2A} are independently
a hydrogen atom,
a substituted or unsubstituted aryl group having 6 to 12 ring carbon atoms, or
a substituted or unsubstituted monovalent heterocyclic group having 5 to 12 ring atoms;
R_{105A} to R_{108A} are hydrogen atoms;
the substituent R is selected from the group consisting of
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
-Si(R₉₀₁)(R₉₀₂)(R₉₀₃),
-O-(R₉₀₄),
-S-(R₉₀₅),
-N(R₉₀₆)(R₉₀₇)
(wherein R₉₀₁ to R₉₀₇ are independently
a hydrogen atom,
a substituted or unsubstituted alkyl group having 1 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
a halogen atom, a cyano group, a nitro group,
a substituted or unsubstituted aryl group having 6 to 50 ring carbon atoms, and
a substituted or unsubstituted monovalent heterocyclic group having 5 to 50 ring atoms;
provided that the compound represented by the formula (1A) satisfies the following conditions A1 and A2;
condition A1: R_{1A} to R_{8A} which are hydrogen atoms are not a deuterium atom;
condition A2: one or more of, hydrogen atoms possessed by L_{1A}, L_{2A} and Ar_{1A}; hydrogen atoms possessed by the substituent in the case where L_{1A}, L_{2A} and Ar_{1A} are substituted; hydrogen atoms possessed by R_{1A} to R_{8A} which are the substituents R; R_{101A} to R_{108A} which are hydrogen atoms; and hydrogen atoms possessed by R_{101A} to R_{104A} which are a substituted or unsubstituted aryl group having 6 to 12 ring carbon atoms, or a substituted or unsubstituted monovalent heterocyclic group having 5 to 12 ring atoms are a deuterium atom: 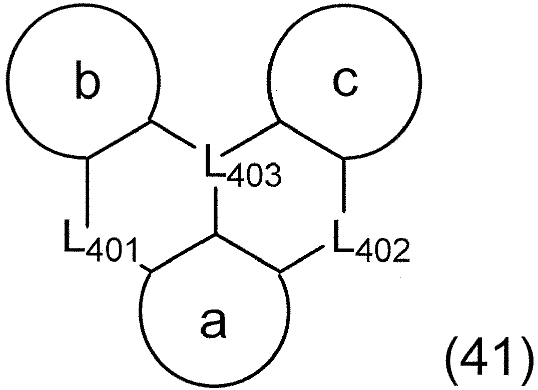 wherein in the formula (41),
a ring a, a ring b and a ring c are independently
a substituted or unsubstituted aromatic hydrocarbon ring having 6 to 50 ring carbon atoms, or
a substituted or unsubstituted heterocycle having 5 to 50 ring atoms;
L₄₀₁ and L₄₀₂ are independently O, S, Se, NR₄₀₁, C(R₄₀₂)(R₄₀₃), or Si(R₄₀₄)(R₄₀₅);
(wherein R₄₀₁ to R₄₀₅ form a substituted or unsubstituted, saturated or unsaturated ring by bonding with the ring a, the ring b or the ring c, or do not form the substituted or unsubstituted, saturated or unsaturated ring;
R₄₀₂ and R₄₀₃ form a substituted or unsubstituted, saturated or unsaturated ring by bonding with each other, or do not form the substituted or unsubstituted, saturated or unsaturated ring;
R₄₀₄ and R₄₀₅ form a substituted or unsubstituted, saturated or unsaturated ring by bonding with each other, or do not form the substituted or unsubstituted, saturated or unsaturated ring;
R₄₀₁ to R₄₀₅ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
when two or more of each of R₄₀₁ to R₄₀₅ are present, the two or more of each of R₄₀₁ to R₄₀₅ may be the same as or different from each other; and
L₄₀₃ is B, P, or P=O.

18. The organic electroluminescence device according to claim 17, wherein the compound represented by the formula (1A) is a compound represented by the following formula (1A-1): 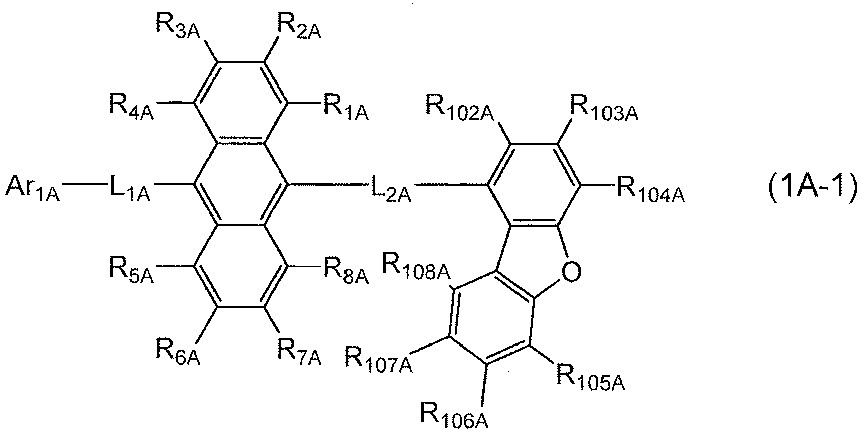 wherein in the formula (1A-1), R_{1A} to R_{8A}, R_{102A} to R_{108A}, L_{1A}, L_{2A} and Ar_{1A} are the same as defined in the formula (1A).

19. The organic electroluminescence device according to claim 17 or 18, wherein the compound represented by the formula (1A) is a compound represented by any one of the following formulas (1A-11) to (1A-14): 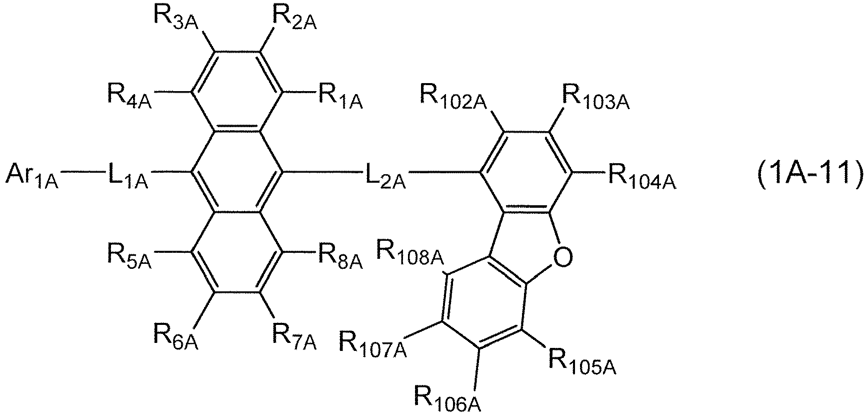 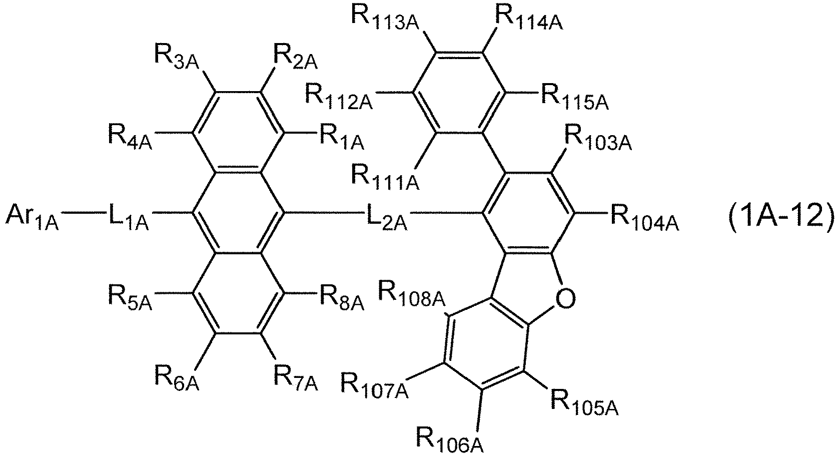 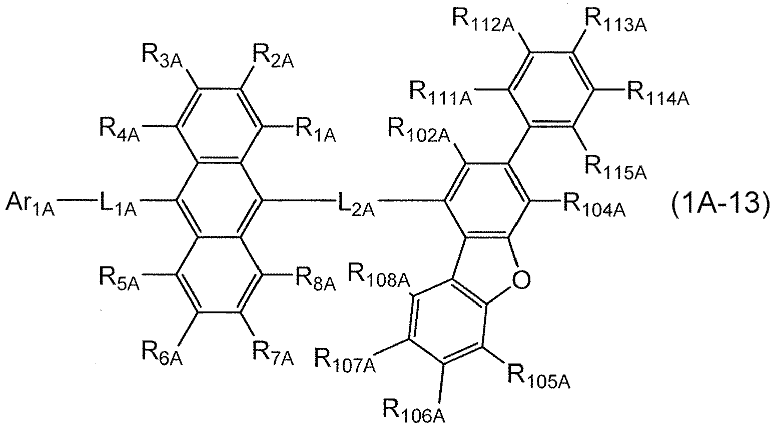 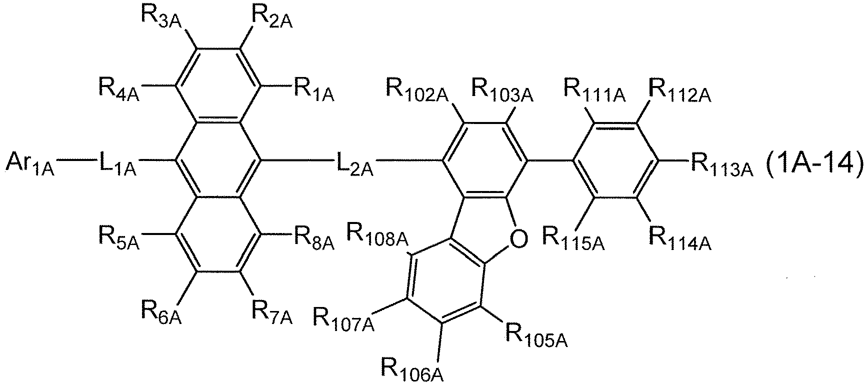 wherein in the formulas (1A-11) to (1A-14), R_{1A} to R_{8A}, R_{105A} to R_{108A}, L_{1A}, L_{2A} and Ar_{1A} are the same as defined in the formula (1A);
in the formula (1A-11), R_{102A} to R_{104A} are a hydrogen atom;
in the formula (1A-12), R_{103A}, R_{104A} and R_{111A} to R_{115A} are a hydrogen atom;
in the formula (1A-13), R_{102A}, R_{104A} and R_{111A} to R_{115A} are a hydrogen atom; and
in the formula (1A-14), R_{102A}, R_{103A} and R_{111A} to R_{115A} are a hydrogen atom.

20. The organic electroluminescence device according to any one of claims 17 to 19, wherein L_{1A} is
a substituted or unsubstituted phenylene group, or
a substituted or unsubstituted naphthylene group; and
one or more of hydrogen atoms possessed by L_{1A} are a deuterium atom.

21. The organic electroluminescence device according to claim 19, wherein the compounds represented by the formulas (1A-11) to (1A-14) are a compound represented by any one of the following formulas (1A-21) to (1A-24): 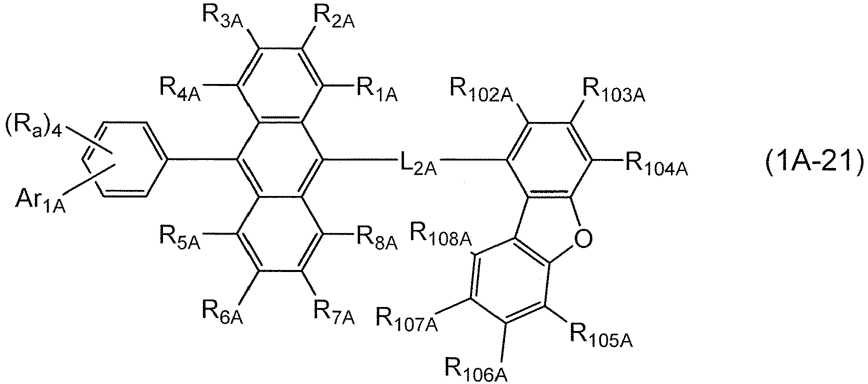 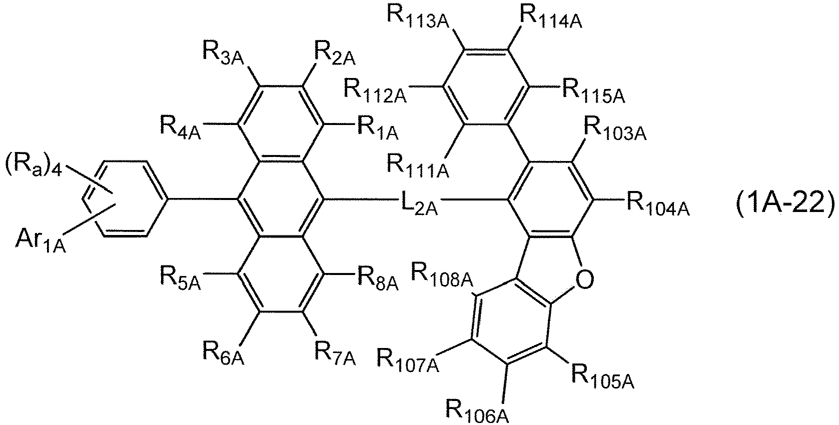 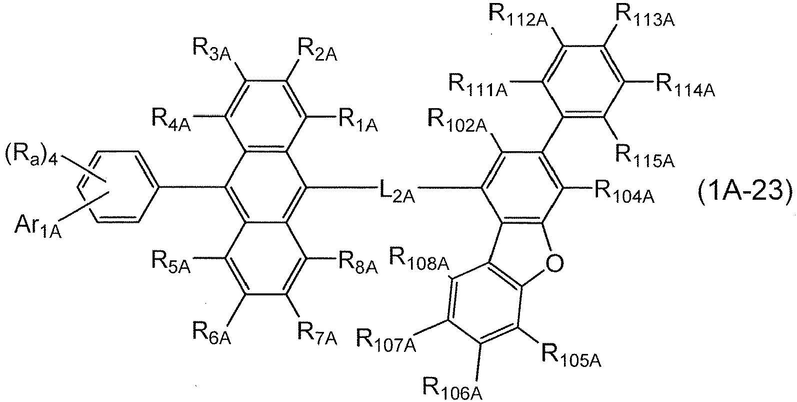 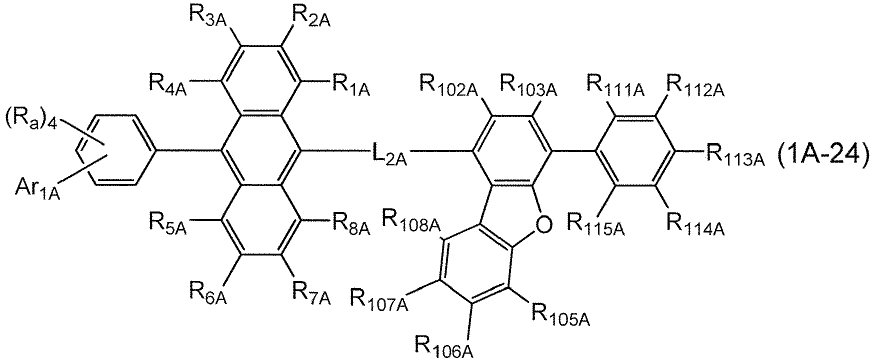 wherein in the formulas (1A-21) to (1A-24), R_{1A} to R_{8A}, R_{102A} to R_{108A}, R_{111A} to R_{115A}, L_{2A} and Ar_{1A} are the same as defined in the formulas (1A-11) to (1A-14);
four Rₐ's are independently a hydrogen atom, or a substituent R;
provided that one or more of the four Rₐ's are a deuterium atom; and
the substituent R is the same as defined in the formula (1A).

22. The organic electroluminescence device according to any one of claims 17 to 21, wherein hydrogen atoms possessed by Ar_{1A} are not a deuterium atom.

23. The organic electroluminescence device according to claim 19, wherein the compound represented by the formula (1A) is a compound represented by the formula (1A-13).

24. The organic electroluminescence device according to any one of claims 17 to 23, wherein the compound represented by the formula (41) is selected from the group consisting of compounds represented by the following formulas (41-1) to (41-6): 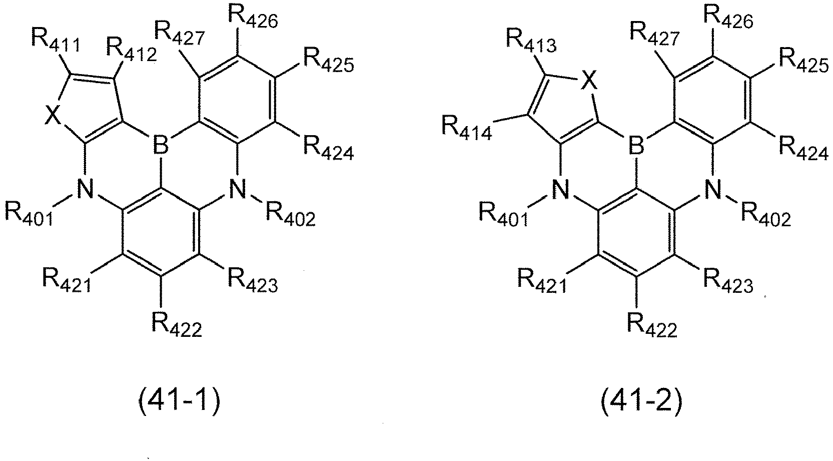 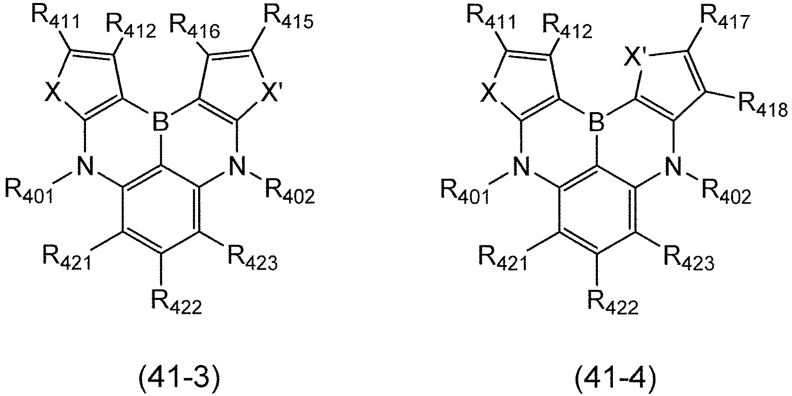 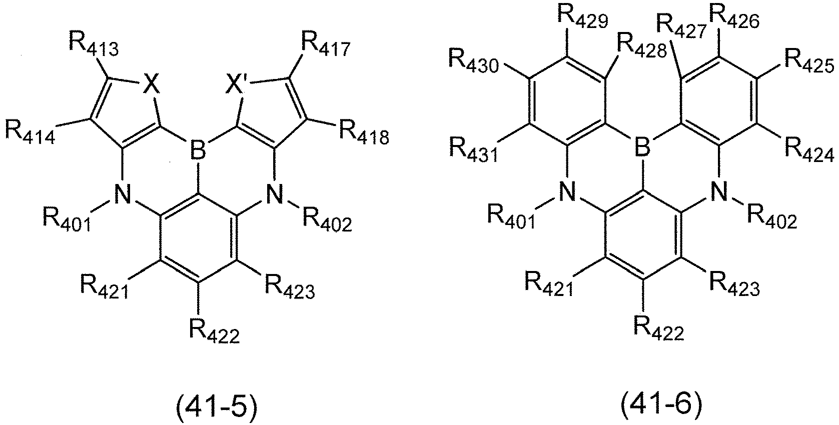 wherein in the formula (41-1),
X is O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₂₇ and R₄₁₂; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, and R₄₂₁ to R₄₂₇ which do not form the substituted or unsubstituted, saturated or unsaturated ring are independently a hydrogen atom or a substituent R;
in the formula (41-2),
X is O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₁₃ and R₄₁₄; and R₄₁₄ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₃, R₄₁₄, and R₄₂₁ to R₄₂₇ which do not form the substituted or unsubstituted, saturated or unsaturated ring are independently a hydrogen atom or a substituent R;
in the formula (41-3),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₁₅ and R₄₁₆; R₄₁₆ and R₄₁₂; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, R₄₁₅, R₄₁₆, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-4),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₁₈; R₄₁₈ and R₄₁₇; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, R₄₁₇, R₄₁₈, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-5),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₁₈; R₄₁₈ and R₄₁₇; R₄₁₃ and R₄₁₄; and R₄₁₄ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, R₄₁₇, R₄₁₈, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-6),
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₂₇ and R₄₂₈; the adjacent two or more of R₄₂₈ to R₄₃₁; and R₄₃₁ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₂₁ to R₄₃₁ which do not form the substituted or unsubstituted, saturated or unsaturated ring are independently a hydrogen atom or a substituent R; and
the substituent R is the same as defined in the formula (1).

25. An organic electroluminescence device comprising
a cathode;
an anode; and
one or more emitting layers arranged between the cathode and the anode,
wherein at least one layer of the one or more emitting layers comprises
a compound represented by the following formula (1B), and
a compound represented by the following formula (41): 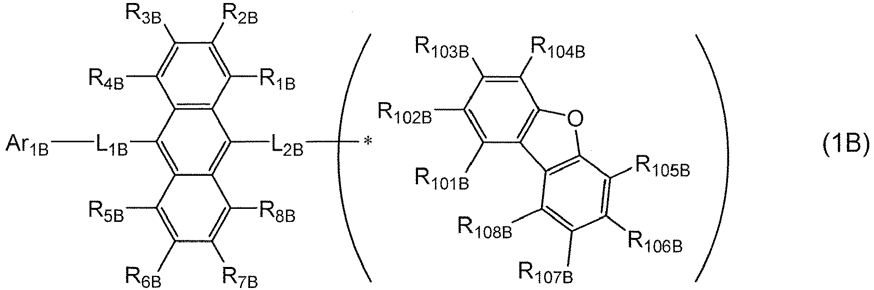 wherein in the formula (1B),
R_{1B} to R_{8B} are independently a hydrogen atom, or a substituent R;
L_{1B} and L_{2B} are independently
a single bond,
a substituted or unsubstituted arylene group having 6 to 18 ring carbon atoms, or
a substituted or unsubstituted divalent heterocyclic group having 5 to 18 ring atoms;
Ar_{1B} is
a substituted or unsubstituted phenyl group,
a substituted or unsubstituted p-biphenyl group,
a substituted or unsubstituted m-biphenyl group,
a substituted or unsubstituted o-biphenyl group,
a substituted or unsubstituted 1-naphthyl group,
a substituted or unsubstituted 2-naphthyl group, or
a substituted or unsubstituted phenanthryl group;
the adjacent two of R_{101B} to R_{108B} form a substituted or unsubstituted benzene ring by bonding with each other;
one of, R_{101B} to R_{108B} which do not form the substituted or unsubstituted benzene ring, and carbon atoms in the substituted or unsubstituted benzene ring is bonded with L_{2B} via a single bond;
R_{101B} to R_{108B} which do not form the substituted or unsubstituted benzene ring and which are not bonded with L_{2B} are independently a hydrogen atom, or a substituent R;
the substituent R is selected from the group consisting of
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
-Si(R₉₀₁)(R₉₀₀)(R₉₀₃),
-O-(R₉₀₄),
-S-(R₉₀₅),
-N(R₉₀₆)(R₉₀₇)
(wherein R₉₀₁ to R₉₀₇ are independently
a hydrogen atom,
a substituted or unsubstituted alkyl group having 1 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms; when two or more of each of R₉₀₁ to R₉₀₇ are present, the two or more of each of R₉₀₁ to R₉₀₇ may be the same as or different from each other),
a halogen atom, a cyano group, a nitro group,
a substituted or unsubstituted aryl group having 6 to 50 ring carbon atoms, and
a substituted or unsubstituted monovalent heterocyclic group having 5 to 50 ring atoms; provided that the compound represented by the formula (1B) satisfies the following conditions B1 and B2;
condition B1: R_{1B} to R_{8B} which are hydrogen atoms are not a deuterium atom;
condition B2: one or more of, hydrogen atoms possessed by L_{1B}, L_{2B} and Ar_{1B}, hydrogen atoms possessed by the substituent in the case where L_{1B}, L_{2B} and Ar_{1B} are substituted, hydrogen atoms possessed by R_{1B} to R_{8B} which are the substituents R, R_{101B} to R_{108B} which are hydrogen atoms, hydrogen atoms possessed by R_{101B} to R_{108B} which are the substituents R, and hydrogen atoms possessed by the substituted or unsubstituted benzene ring are a deuterium atom: 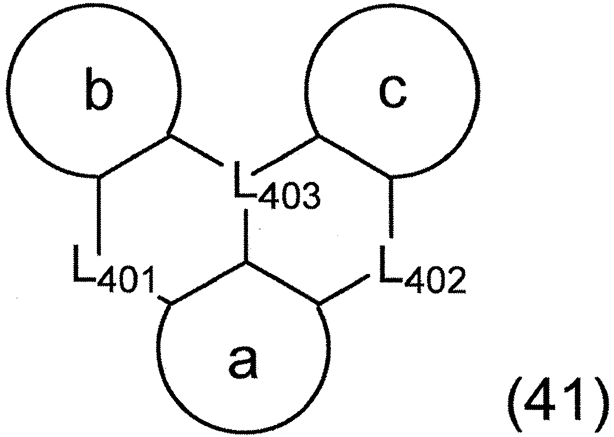 wherein in the formula (41),
a ring a, a ring b and a ring c are independently
a substituted or unsubstituted aromatic hydrocarbon ring having 6 to 50 ring carbon atoms, or
a substituted or unsubstituted heterocycle having 5 to 50 ring atoms;
L₄₀₁ and L₄₀₂ are independently O, S, Se, NR₄₀₁, C(R₄₀₂)(R₄₀₃), or Si(R₄₀₄)(R₄₀₅);
(wherein R₄₀₁ to R₄₀₅ form a substituted or unsubstituted, saturated or unsaturated ring by bonding with the ring a, the ring b or the ring c, or do not form the substituted or unsubstituted, saturated or unsaturated ring;
R₄₀₂ and R₄₀₃ form a substituted or unsubstituted, saturated or unsaturated ring by bonding with each other, or do not form the substituted or unsubstituted, saturated or unsaturated ring;
R₄₀₄ and R₄₀₅ form a substituted or unsubstituted, saturated or unsaturated ring by bonding with each other, or do not form the substituted or unsubstituted, saturated or unsaturated ring;
R₄₀₁ to R₄₀₅ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
when two or more of each of R₄₀₁ to R₄₀₅ are present, the two or more of each of R₄₀₁ to R₄₀₅ may be the same as or different from each other; and
L₄₀₃ is B, P, or P=O.

26. The organic electroluminescence device according to claim 25, wherein in the formula (1B), R_{101B} is bonded with L_{2B} via a single bond.

27. The organic electroluminescence device according to claim 25 or 26, wherein the compound represented by the formula (1B) is a compound represented by the following formula (1B-1): 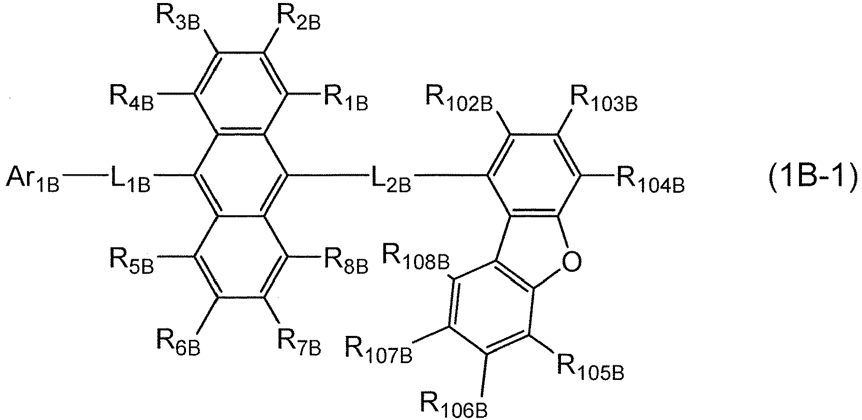 wherein in the formula (1B-1), R_{1B} to R_{8B}, R_{102B} to R_{108B}, L_{1B}, L_{2B} and Ar_{1B} are the same as defined in the formula (1B).

28. The organic electroluminescence device according to any one of claims 25 to 27, wherein in the formula (1B), one or more sets of the adjacent two or more of R_{105B} to R_{108B} form a substituted or unsubstituted benzene ring by bonding with each other.

29. The organic electroluminescence device according to any one of claims 25 to 28, wherein the compound represented by the formula (1) is a compound represented by any one of the following formulas (1B-11) to (1B-13): 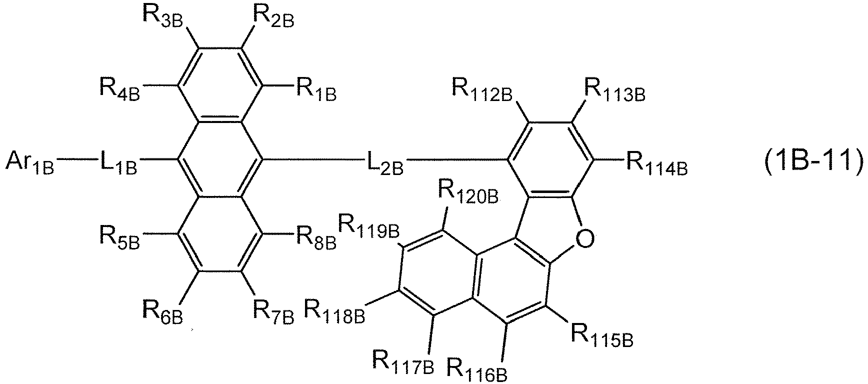 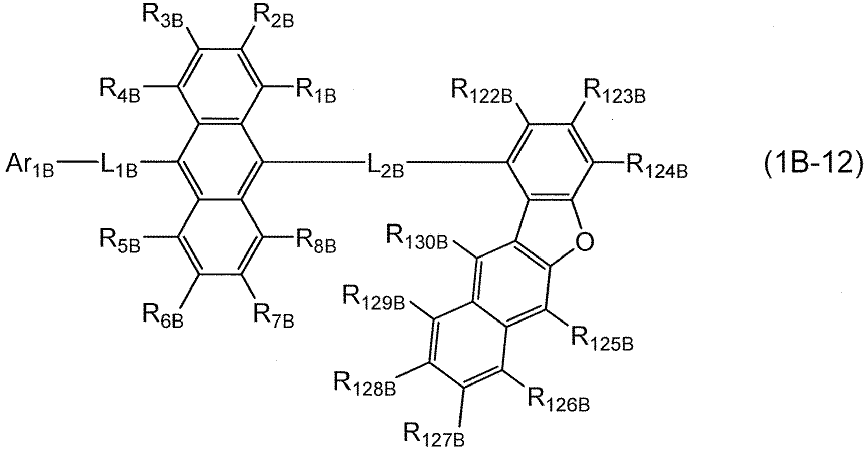 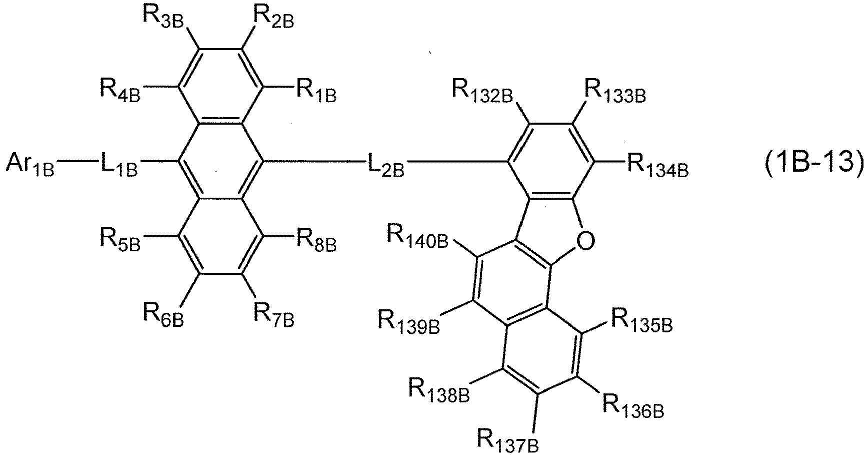 wherein in the formulas (1B-11) to (1B-13), R_{1B} to R_{8B}, L_{1B}, L_{2B} and Ar_{1B} are the same as defined in the formula (1B);
in the formula (1B-11), R_{112B} to R_{120B} are independently a hydrogen atom, or a substituent R;
in the formula (1B-12), R_{122B} to R_{130B} are independently a hydrogen atom, or a substituent R;
in the formula (1B-13), R_{132B} to R_{140B} are independently a hydrogen atom, or a substituent R; and
the substituent R is the same as defined in the formula (1B).

30. The organic electroluminescence device according to any one of claims 25 to 29, wherein L_{1B} is
a substituted or unsubstituted phenylene group, or
a substituted or unsubstituted naphthylene group; and
one or more of hydrogen atoms possessed by L_{1B} are a deuterium atom.

31. The organic electroluminescence device according to claim 29, wherein the compounds represented by the formulas (1B-11) to (1B-13) are a compound represented by any one of the following formulas (1B-21) to (1B-23): 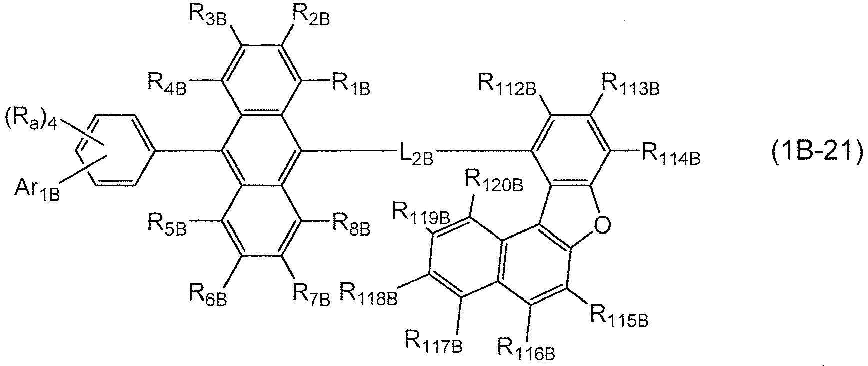 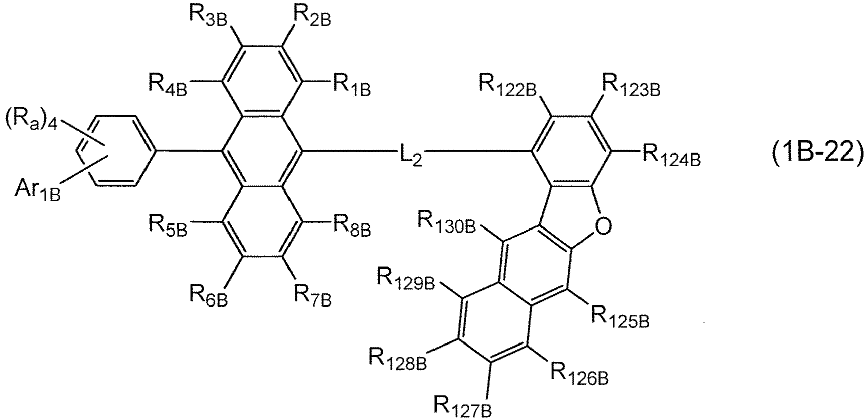 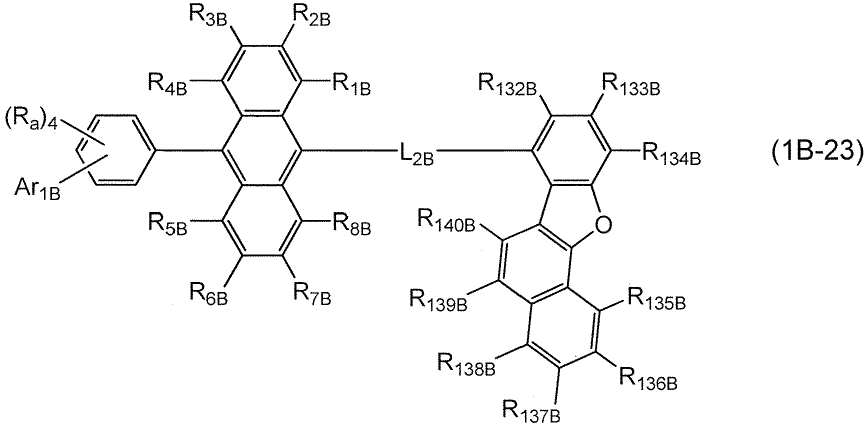 wherein in the formulas (1B-21) to (1B-23), R_{1B} to R_{8B}, R_{112B} to R_{120B}, R_{122B} to R_{130B}, R_{132B} to R_{140B}, L_{2B} and Ar_{1B} are the same as defined in the formulas (1B-11) to (1B-13);
four Rₐ's are independently a hydrogen atom, or a substituent R;
provided that one or more of the four Rₐ's are a deuterium atom; and
the substituent R is the same as defined in the formula (1B).

32. The organic electroluminescence device according to any one of claims 25 to 31, wherein hydrogen atoms possessed by Ar_{1B} are not a deuterium atom.

33. The organic electroluminescence device according to any one of claims 25 to 29, wherein the compound represented by the formula (1B) is a compound represented by the following formula (1B-121): 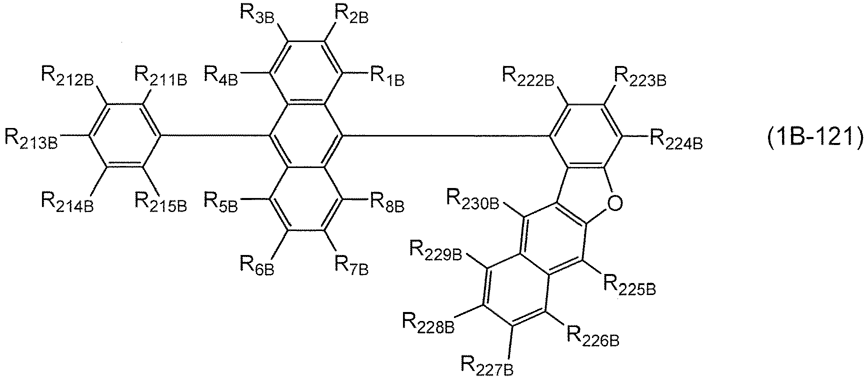 wherein in the formula (1B-121), R_{1B} to R_{8B} are the same as defined in the formula (1B);
R_{211B} to R_{215B} are independently a hydrogen atom, or a substituent R;
R_{222B} to R_{230B} are independently a hydrogen atom, or a substituent R;
the substituent R is the same as defined in the formula (1);
provided that the compound represented by the formula (1B-121) satisfies the following conditions B1-1 and B2-1;
condition B1-1: at least one of R_{211B} to R_{215B} is a deuterium atom; and
condition B2-1: R_{222B} to R_{230B} which are hydrogen atoms are not a deuterium atom.

34. The organic electroluminescence device according to claim 33, wherein R_{211B} to R_{215B} are hydrogen atoms.

35. The organic electroluminescence device according to any one of claims 25 to 34, wherein R_{1B} to R_{8B} are hydrogen atoms.

36. The organic electroluminescence device according to any one of claims 25 to 35, wherein the compound represented by the formula (41) is selected from the group consisting of compounds represented by the following formulas (41-1) to (41-5): 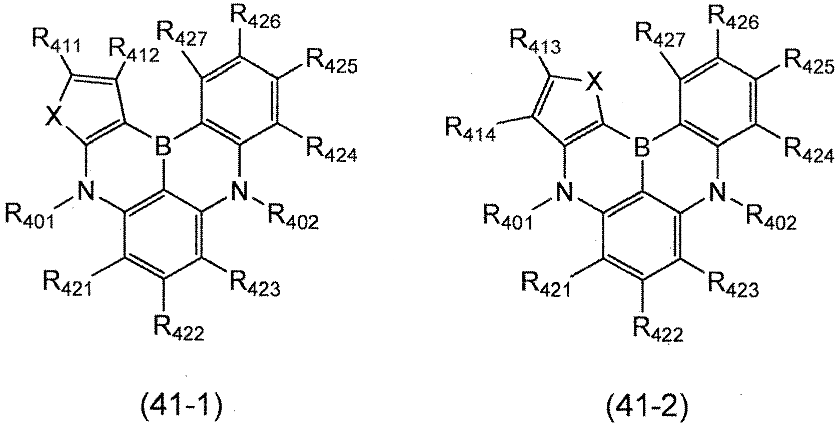 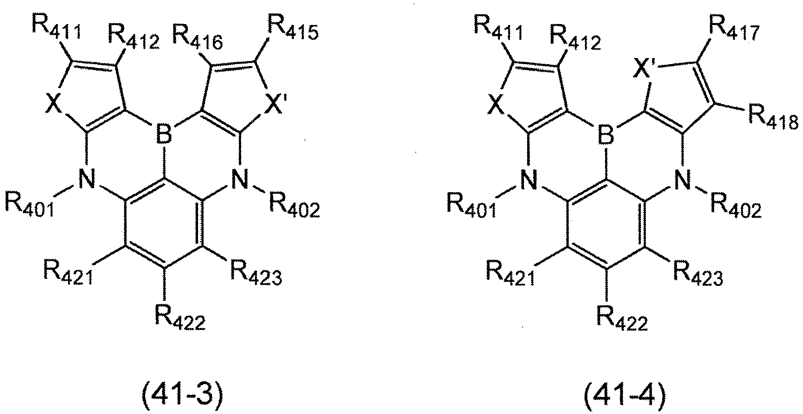 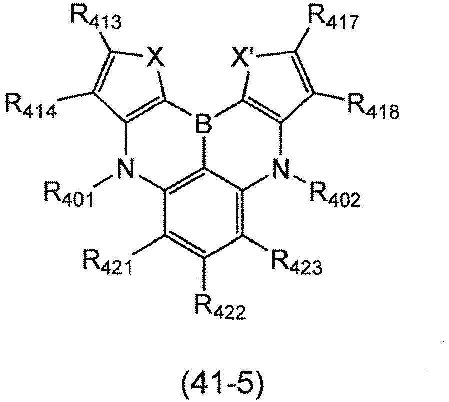 wherein in the formula (41-1),
X is O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₂₇ and R₄₁₂; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, and R₄₂₁ to R₄₂₇ which do not form the substituted or unsubstituted, saturated or unsaturated ring are independently a hydrogen atom or a substituent R;
in the formula (41-2),
X is O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₂₄; the adjacent two or more of R₄₂₄ to R₄₂₇; R₄₁₃ and R₄₁₄; and R₄₁₄ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₃, R₄₁₄, and R₄₂₁ to R₄₂₇ which do not form the substituted or unsubstituted, saturated or unsaturated ring are independently a hydrogen atom or a substituent R;
in the formula (41-3),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₁₅ and R₄₁₆; R₄₁₆ and R₄₁₂; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, R₄₁₅, R₄₁₆, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-4),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₁₈; R₄₁₈ and R₄₁₇; and R₄₁₂ and R₄₁₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₁, R₄₁₂, R₄₁₇, R₄₁₈, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other;
in the formula (41-5),
X and X' are independently O, S, Se, C(R₄₀₃)(R₄₀₄), or NR₄₀₅;
one or more sets of, R₄₀₁ and R₄₂₁; the adjacent two or more of R₄₂₁ to R₄₂₃; R₄₂₃ and R₄₀₂; R₄₀₂ and R₄₁₈; R₄₁₈ and R₄₁₇; R₄₁₃ and R₄₁₄; and R₄₁₄ and R₄₀₁ form a substituted or unsubstituted, saturated or unsaturated ring, or do not form the substituted or unsubstituted, saturated or unsaturated ring;
R₄₀₁ and R₄₀₂ which do not form the substituted or unsubstituted, saturated or unsaturated ring are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
R₄₀₃ to R₄₀₅, and R₄₁₃, R₄₁₄, R₄₁₇, R₄₁₈, and R₄₂₁ to R₄₂₇ which do not form the substituted or unsubstituted, saturated or unsaturated ring are independently a hydrogen atom or a substituent R;
when two or more of each of R₄₀₃ to R₄₀₅ are present, the two or more of each of R₄₀₃ to R₄₀₅ may be the same as or different from each other; and
the substituent R is the same as defined in the formula (1).

37. The organic electroluminescence device according to any one of claims 17 to 36, wherein the compound represented by the formula (41) is a compound represented by the following formula (42-2): 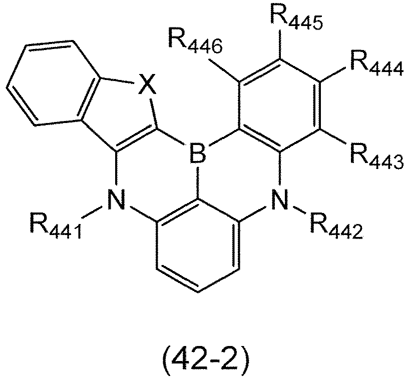 wherein in the formula (42-2),
R₄₄₁ and R₄₄₂ are independently
a substituted or unsubstituted alkyl group having 1 to 50 carbon atoms,
a substituted or unsubstituted alkenyl group having 2 to 50 carbon atoms,
a substituted or unsubstituted alkynyl group having 2 to 50 carbon atoms,
a substituted or unsubstituted cycloalkyl group having 3 to 50 ring carbon atoms,
a substituted or unsubstituted aryl group having 6 to 50 ring carbon atoms, or
a substituted or unsubstituted monovalent heterocyclic group having 5 to 50 ring atoms;
Raas to R₄₄₆ are independently a hydrogen atom or a substituent R;
X is O, or S; and
the substituent R is the same as defined in the formula (1).

38. The organic electroluminescence device according to any one of claims 17 to 37, wherein a substituent in the case of "substituted or unsubstituted" is selected from the group consisting of
an alkyl group having 1 to 50 carbon atoms,
a haloalkyl group having 1 to 50 carbon atoms,
an alkenyl group having 2 to 50 carbon atoms,
an alkynyl group having 2 to 50 carbon atoms,
a cycloalkyl group having 3 to 50 ring carbon atoms,
an alkoxy group having 1 to 50 carbon atoms,
an alkylthio group having 1 to 50 carbon atoms,
an aryloxy group having 6 to 50 ring carbon atoms,
an arylthio group having 6 to 50 ring carbon atoms,
an aralkyl group having 7 to 50 carbon atoms,
-Si(R₄₁)(R₄₂)(R₄₃),
-C(=O)R₄₄, -COOR₄₅,
-S(=O)₂R₄₆,
-P(=O)(R₄₇)(R₄₈),
-Ge(R₄₉)(R₅₀)(R₅₁),
-N(R₅₂)(R₅₃) (wherein, R₄₁ to R₅₃ are independently a hydrogen atom, an alkyl group having 1 to 50 carbon atoms, an aryl group having 6 to 50 ring carbon atoms, or a monovalent heterocyclic group having 5 to 50 ring atoms; when two or more of each of R₄₁ to R₅₃ are present, the two or more of each of R₄₁ to R₅₃ may be the same as or different from each other),
a hydroxy group,
a halogen atom,
a cyano group,
a nitro group,
an aryl group having 6 to 50 ring carbon atoms, and
a monovalent heterocyclic group having 5 to 50 ring atoms.

39. The organic electroluminescence device according to any one of claims 17 to 38, wherein a substituent in the case of "substituted or unsubstituted" is selected from the group consisting of
an alkyl group having 1 to 18 carbon atoms,
an aryl group having 6 to 18 ring carbon atoms, and
a monovalent heterocyclic group having 5 to 18 ring atoms.

40. The organic electroluminescence device according to any one of claims 17 to 39, wherein a hole-transporting layer is provided between the anode and the emitting layer.

41. The organic electroluminescence device according to any one of claims 17 to 40, wherein an electron-transporting layer is provided between the cathode and the emitting layer.

42. The organic electroluminescence device according to any one of claims 17 to 41, wherein the at least one layer of the one or more emitting layers further comprises one or more fused aromatic compounds selected from the group consisting of a carbazole derivative, an anthracene derivative, a phenanthrene derivative, a pyrene derivative, a naphthacene derivative, a fluoranthene derivative, a triphenylene derivative, a fluorene derivative, and a chrysene derivative.

43. The organic electroluminescence device according to any one of claims 17 to 42, wherein the at least one layer of the one or more emitting layers further comprises a host compound having delayed fluorescence.

44. An electronic apparatus comprising the organic electroluminescence device according to any one of claims 17 to 43.
